# EUROPEAN PATENT APPLICATION

(11) **EP 3 957 168 A1**
(43) Date of publication of application: **23.02.2022**
(21) Application number: 20191416.5
(22) Date of filing: 17.08.2020
(51) Int. Cl.: A01H 5/06, A01H 6/02, C07K 14/415

(54) **PLANT RESISTANCE GENE AND MEANS FOR ITS IDENTIFICATION**

(71) Applicant: KWS SAAT SE & Co. KGaA, 37574 Einbeck (DE)
(72) Inventor: Törjék, Ottó, 37574 Einbeck (DE); Borchardt, Dietrich, 37574 Einbeck (DE); Rekoske, Margaret, Shakopee, MN 55379 (US); Schulz, Britta, 37574 Einbeck (DE); Lein, Jens Christoph, 37085 Göttingen (DE)
(74) Representative: Michalski Hüttermann & Partner Patentanwälte mbB

(57) **Abstract**

A more efficient breeding against Cercospora leaf spot disease, or the development of new resistant lines, is enabled via the provision of the Cercospora resistance-mediating gene according to the invention; in particular, a dominant resistance effect in the target plant is evoked by the property of the identified gene alone. The Cercospora resistance-mediating gene, and embodiments of the present invention that are described in the preceding, offer additional applications, e.g., the use of the resistant gene allele in cis-genetic or trans-genetic approaches, with the goal of developing new resistant cultivars.

SEQUENCE LISTING

<110>  KWS SAAT SE & Co. KGaA

<120>  Plant resistance gene and means for its identification

<130>  KWS0270EP4

<160>  228

<170>  PatentIn version 3.5

<210>  1
<211>  3720
<212>  DNA
<213>  Beta vulgaris

<400>  1
   atgaacatga aaatcctcct tttgtttgtc ttccttcatc acctccacta cttcatccat      60
   ggcagaacac ttacagaacg ccaagcttta ctaagtatca aatctgccat tacttatgat     120
   tattataact ctctctcctc atggaaaaac acaacacacc actgcagttg gccatacatc     180
   acttgctcct cctcttcttc ttcttcttct gttatttctc tcaacttcac catgttattt     240
   ctcgaaggaa ttctctcccc tgatataggc ttcctcacca acctgcaaaa cctctctatt     300
   cgatctaacc ttttttctgg cccactcccc cattctctct ctctcctcac ccaactccgc     360
   tatctcgacg tttcccaaaa cagtttcaca ggtccaatcc catcttctct ctctctcctc     420
   acccaactcc gctatctcca cgtttccggc aacagtttca caggtccaat cccatctttt     480
   ctctctctcc tcacccaact ccgctatctc gacgtttccg acaacagttt cacaggtcca     540
   atcccatctt ctctctctct cctcacccaa ctccgctatc tcgacgtttc ctacaacaat     600
   ctaaatggca ctcttccctt atcggtcgtt gagaagatgt cggagctcag ctaccttaac     660
   cttaggtata actctttcta cggtgagatt ccaccggagt ttgggaaact taagaagctt     720
   gaaacattga atcttggtaa caacactctt tctgggagtc ttccatctga gttgggttca     780
   ttaaagagtt tgaaacatat ggacttttct agtaatatgc tatttggtga gatcccacaa     840
   tcttattctc ttcttcgaaa cttaatcgat attgatctta atagaaacaa gttatatggg     900
   agtatacctg attatattgg agattttccg gagttggaat cacttttatt agactcgaat     960
   aacttcacag ggagtatccc acaaaagtta ggtacaaacg ggaagttgca atatctagat    1020
   ataagtaaca acaattttag tggtagtttg ccactaagtc tttgcaaagg agacaaactc    1080
   caagatctgg acgcatccta taatttgttg gttgggtcaa ttcctgagag tttgggaagt    1140
   tgcaagtcac ttgaaggagt gtacatggga aataatttct taaacgggtc gattcctaag    1200
   ggcttgtttg ggagtgatgt ttcacttaat gacaaacttc ttagtggagg tctcgatgag    1260
   aaattcggtg attgcgttaa tcttcgggac attgatctct ctaataataa gctatcaggg    1320
   aagttacctg cgaccatcgg aaactgtatt catcttcggt ccttgacgct ttataataac    1380
   acctgtaccg gacgtatccc tcaagagatt agcaagtgta agcagctaca gaccctcgat 1440
   ctcagccaaa atcagttctc tggtgtgata cccaatgata ttacaggtaa gaaagtatat 1500
   taaacttgtt acttttgaaa atattcgctc tagtttttgt ttcagttggt ccattctcac 1560
   tttgtattat tgaaatatat cccaaaaaag taaatataat tatataaaag aatcttgcta 1620
   aaaataatat gaattatttt tgtatgtgca aaataatgta caaatctaac taatttgttg 1680
   tggataataa tattaattgt gtgaaatagt aaatgtgtgg agatatataa ctttatttat 1740
   catattcact caggttttta ggtatttatt atgagttttg cattggagat atccaacttg 1800
   acaatagtat ttttgtaata taccaatata taaagattac tgtacataac caaaatgtat 1860
   acttttctta tttttataaa cttatatatt cctcttcttt gtatttatca caacattttt 1920
   tatacccttt tgcctcatat taatagcaac acttataatt tatttattta ctttttattt 1980
   cttggtctat aacctcatct acccacatat gacacaccct ataaaggacc cacatgatta 2040
   accaaaatat acaaatatct tcaatgaaat taactttaac actaatatga taaaaatcat 2100
   gtcccgcttt ttatcctcta actaagactc tgcataaagg tatattgcaa ttaatatgag 2160
   atggaagagg tataataatt atatgatcaa attcctggat tgaaaaataa atatgagatt 2220
   aaaagtggta tgtttttggt taaaagaaac tatccataaa gtatgttttt ggttaaaaga 2280
   aactatgcaa cataccaatc aaatgtttat acgcttacaa tttatgtacc acttttttgt 2340
   cattgttttt ctattgtttg ccatacgtac gttactaaat catgttgtct tttcacattt 2400
   taactaacaa taaattacta ttgatacacc aaaaaaatct atgagcattg gagtacgttg 2460
   tttgatagaa gcttcgtgct attatttctt gtcaaagaat ttcatatctc aatatcttct 2520
   aatttaacaa tctaacgaaa tttttttgac ccaggaaaca aatccatttg caatctggaa 2580
   aagatacaaa cacttaaatt atcaaacaat gctttgactg gtgaaatccc tcattgtgtt 2640
   ggaaatatcg agctcatagc attatttctc caatcaaaca aactgaacgg taccataccc 2700
   gcaaacttct caaagttatg tgattcattg atatatctag atcttagtga caatcaactc 2760
   gaaggagttc tacctaagtc cttgtccaaa tgtcaaagtc tagaactcct aaatgtcggg 2820
   aacaataggc taagagataa atttccttca tggttagaca acctcccacg tctccaagtt 2880
   ttcagtgtgc gttttaacgc cttctacggt cctataacta gctcaccaaa agttagtcac 2940
   ccatttccta tgctacaaat tatcgaccta tctaacaata agttttgtgg caagttgcca 3000
   agaagatata tcaaaaactt tgcaaccatg cgcaatatga atgagtctgg tgttgggaat 3060
   ccacagtacc tgggggactc atcaatatat agtattacgt actctatggt attgacattc 3120
   aatgggttac aacaaaaata tgaaaagctt attgtgacga tgtcgacctt tgatatatcc 3180
   agcaacaact ttactggaca gattccatat gttatagggg gattacgctc acttcgtaac 3240
   cttaatctct ctcataatgt cttaaccggg aacattcctc catcaattgc aaaattgtct 3300
   ttgcttcaag atttggacct ttcatcaaac agacttactg gtcgtatccc tcaagaatta 3360
   gttagtttaa catttcttgg gagtttcaat gtttcgaaca atctattgga ggggtctata 3420
   cctcatggtt tcaacttcga cacgtacaca gctaattcat accaggggaa tctcgaatta 3480
   tgtggaaaac cattacctga gtgtggagaa agaagggcaa aaggcaccac taataatcaa 3540
   gatgatccta aaaatgataa tgaacgaatg ttgtcgatgt ccgaaatcgt agttatgggg 3600
   tttggcagtg gtgtactagt tgggttggct tggggatact atatgttttc agtgggaaag 3660
   cccttttggt ttatcaagat ggctagcaaa atggaatcaa tattgattgg ttttttctga 3720

<210> 2
<211> 2652
<212> DNA
<213> Artificial Sequence

<220>
<223> cDNA sequence of the Cercospora resistance-conferring gene

<400> 2
atgaacatga aaatcctcct tttgtttgtc ttccttcatc acctccacta cttcatccat 60

ggcagaacac ttacagaacg ccaagcttta ctaagtatca aatctgccat tacttatgat 120

tattataact ctctctcctc atggaaaaac acaacacacc actgcagttg gccatacatc 180

acttgctcct cctcttcttc ttcttcttct gttatttctc tcaacttcac catgttattt 240

ctcgaaggaa ttctctcccc tgatataggc ttcctcacca acctgcaaaa cctctctatt 300

cgatctaacc ttttttctgg cccactcccc cattctctct ctctcctcac ccaactccgc 360

tatctcgacg tttcccaaaa cagtttcaca ggtccaatcc catcttctct ctctctcctc 420

acccaactcc gctatctcca cgtttccggc aacagtttca caggtccaat cccatctttt 480

ctctctctcc tcacccaact ccgctatctc gacgtttccg acaacagttt cacaggtcca 540

atcccatctt ctctctctct cctcacccaa ctccgctatc tcgacgtttc ctacaacaat 600

ctaaatggca ctcttccctt atcggtcgtt gagaagatgt cggagctcag ctaccttaac 660

cttaggtata actctttcta cggtgagatt ccaccggagt ttgggaaact taagaagctt 720

gaaacattga atcttggtaa caacactctt tctgggagtc ttccatctga gttgggttca 780

ttaaagagtt tgaaacatat ggacttttct agtaatatgc tatttggtga gatcccacaa 840

tcttattctc ttcttcgaaa cttaatcgat attgatctta atagaaacaa gttatatggg 900

agtatacctg attatattgg agattttccg gagttggaat cacttttatt agactcgaat 960

aacttcacag ggagtatccc acaaaagtta ggtacaaacg ggaagttgca atatctagat 1020

ataagtaaca acaattttag tggtagtttg ccactaagtc tttgcaaagg agacaaactc 1080

caagatctgg acgcatccta taatttgttg gttgggtcaa ttcctgagag tttgggaagt 1140

tgcaagtcac ttgaaggagt gtacatggga aataatttct taaacgggtc gattcctaag 1200

ggcttgtttg ggagtgatgt ttcacttaat gacaaacttc ttagtggagg tctcgatgag 1260

aaattcggtg attgcgttaa tcttcgggac attgatctct ctaataataa gctatcaggg 1320

aagttacctg cgaccatcgg aaactgtatt catcttcggt ccttgacgct ttataataac 1380

acctgtaccg gacgtatccc tcaagagatt agcaagtgta agcagctaca gaccctcgat 1440

ctcagccaaa atcagttctc tggtgtgata cccaatgata ttacaggaaa caaatccatt 1500

tgcaatctgg aaaagataca aacacttaaa ttatcaaaca atgctttgac tggtgaaatc 1560

cctcattgtg ttggaaatat cgagctcata gcattatttc tccaatcaaa caaactgaac 1620

ggtaccatac ccgcaaactt ctcaaagtta tgtgattcat tgatatatct agatcttagt 1680

gacaatcaac tcgaaggagt tctacctaag tccttgtcca aatgtcaaag tctagaactc 1740

ctaaatgtcg ggaacaatag gctaagagat aaatttcctt catggttaga caacctccca 1800

cgtctccaag ttttcagtgt gcgttttaac gccttctacg gtcctataac tagctcacca 1860

aaagttagtc acccatttcc tatgctacaa attatcgacc tatctaacaa taagttttgt 1920

ggcaagttgc caagaagata tatcaaaaac tttgcaacca tgcgcaatat gaatgagtct 1980

ggtgttggga atccacagta cctgggggac tcatcaatat atagtattac gtactctatg 2040

gtattgacat tcaatgggtt acaacaaaaa tatgaaaagc ttattgtgac gatgtcgacc 2100

tttgatatat ccagcaacaa ctttactgga cagattccat atgttatagg gggattacgc 2160

tcacttcgta accttaatct ctctcataat gtcttaaccg ggaacattcc tccatcaatt 2220

gcaaaattgt ctttgcttca agatttggac ctttcatcaa acagacttac tggtcgtatc 2280

cctcaagaat tagttagttt aacatttctt gggagtttca atgtttcgaa caatctattg 2340

gaggggtcta tacctcatgg tttcaacttc gacacgtaca cagctaattc ataccagggg 2400

aatctcgaat tatgtggaaa accattacct gagtgtggag aaagaagggc aaaaggcacc 2460

actaataatc aagatgatcc taaaaatgat aatgaacgaa tgttgtcgat gtccgaaatc 2520

gtagttatgg ggtttggcag tggtgtacta gttgggttgg cttggggata ctatatgttt 2580

tcagtgggaa agcccttttg gtttatcaag atggctagca aaatggaatc aatattgatt 2640

ggtttttct ga 2652

<210> 3
<211> 883
<212> PRT
<213> Beta vulgaris

<400> 3

Met Asn Met Lys Ile Leu Leu Leu Phe Val Phe Leu His His Leu His
1               5                   10                  15

Tyr Phe Ile His Gly Arg Thr Leu Thr Glu Arg Gln Ala Leu Leu Ser
            20                  25                  30

Ile Lys Ser Ala Ile Thr Tyr Asp Tyr Tyr Asn Ser Leu Ser Ser Trp
        35                  40                  45

Lys Asn Thr Thr His His Cys Ser Trp Pro Tyr Ile Thr Cys Ser Ser
    50                  55                  60

Ser Ser Ser Ser Ser Ser Val Ile Ser Leu Asn Phe Thr Met Leu Phe
65                  70                  75                  80

Leu Glu Gly Ile Leu Ser Pro Asp Ile Gly Phe Leu Thr Asn Leu Gln
                85                  90                  95

Asn Leu Ser Ile Arg Ser Asn Leu Phe Ser Gly Pro Leu Pro His Ser
            100                 105                 110

Leu Ser Leu Leu Thr Gln Leu Arg Tyr Leu Asp Val Ser Gln Asn Ser
        115                 120                 125

Phe Thr Gly Pro Ile Pro Ser Ser Leu Ser Leu Leu Thr Gln Leu Arg
    130                 135                 140

Tyr Leu His Val Ser Gly Asn Ser Phe Thr Gly Pro Ile Pro Ser Phe
145                 150                 155                 160

Leu Ser Leu Leu Thr Gln Leu Arg Tyr Leu Asp Val Ser Asp Asn Ser
                165                 170                 175

Phe Thr Gly Pro Ile Pro Ser Ser Leu Ser Leu Leu Thr Gln Leu Arg
            180                 185                 190

Tyr Leu Asp Val Ser Tyr Asn Asn Leu Asn Gly Thr Leu Pro Leu Ser
        195                 200                 205

Val Val Glu Lys Met Ser Glu Leu Ser Tyr Leu Asn Leu Arg Tyr Asn
    210                 215                 220

Ser Phe Tyr Gly Glu Ile Pro Pro Glu Phe Gly Lys Leu Lys Lys Leu
225                 230                 235                 240

Glu Thr Leu Asn Leu Gly Asn Asn Thr Leu Ser Gly Ser Leu Pro Ser
                245                 250                 255

Glu Leu Gly Ser Leu Lys Ser Leu Lys His Met Asp Phe Ser Ser Asn

            260                 265                 270

Met Leu Phe Gly Glu Ile Pro Gln Ser Tyr Ser Leu Leu Arg Asn Leu
        275                 280                 285

Ile Asp Ile Asp Leu Asn Arg Asn Lys Leu Tyr Gly Ser Ile Pro Asp
    290                 295                 300

Tyr Ile Gly Asp Phe Pro Glu Leu Glu Ser Leu Leu Leu Asp Ser Asn
305                 310                 315                 320

Asn Phe Thr Gly Ser Ile Pro Gln Lys Leu Gly Thr Asn Gly Lys Leu
                325                 330                 335

Gln Tyr Leu Asp Ile Ser Asn Asn Asn Phe Ser Gly Ser Leu Pro Leu
            340                 345                 350

Ser Leu Cys Lys Gly Asp Lys Leu Gln Asp Leu Asp Ala Ser Tyr Asn
        355                 360                 365

Leu Leu Val Gly Ser Ile Pro Glu Ser Leu Gly Ser Cys Lys Ser Leu
    370                 375                 380

Glu Gly Val Tyr Met Gly Asn Asn Phe Leu Asn Gly Ser Ile Pro Lys
385                 390                 395                 400

Gly Leu Phe Gly Ser Asp Val Ser Leu Asn Asp Lys Leu Leu Ser Gly
                405                 410                 415

Gly Leu Asp Glu Lys Phe Gly Asp Cys Val Asn Leu Arg Asp Ile Asp
            420                 425                 430

Leu Ser Asn Asn Lys Leu Ser Gly Lys Leu Pro Ala Thr Ile Gly Asn
        435                 440                 445

Cys Ile His Leu Arg Ser Leu Thr Leu Tyr Asn Asn Thr Cys Thr Gly
    450                 455                 460

Arg Ile Pro Gln Glu Ile Ser Lys Cys Lys Gln Leu Gln Thr Leu Asp
465                 470                 475                 480

Leu Ser Gln Asn Gln Phe Ser Gly Val Ile Pro Asn Asp Ile Thr Gly
                485                 490                 495

Asn Lys Ser Ile Cys Asn Leu Glu Lys Ile Gln Thr Leu Lys Leu Ser
            500                 505                 510

Asn Asn Ala Leu Thr Gly Glu Ile Pro His Cys Val Gly Asn Ile Glu
        515                 520                 525

Leu Ile Ala Leu Phe Leu Gln Ser Asn Lys Leu Asn Gly Thr Ile Pro
    530                 535                 540

Ala Asn Phe Ser Lys Leu Cys Asp Ser Leu Ile Tyr Leu Asp Leu Ser
545                 550                 555                 560

Asp Asn Gln Leu Glu Gly Val Leu Pro Lys Ser Leu Ser Lys Cys Gln
                565                 570                 575

Ser Leu Glu Leu Leu Asn Val Gly Asn Asn Arg Leu Arg Asp Lys Phe
            580                 585                 590

Pro Ser Trp Leu Asp Asn Leu Pro Arg Leu Gln Val Phe Ser Val Arg
        595                 600                 605

Phe Asn Ala Phe Tyr Gly Pro Ile Thr Ser Ser Pro Lys Val Ser His
    610                 615                 620

Pro Phe Pro Met Leu Gln Ile Ile Asp Leu Ser Asn Asn Lys Phe Cys
625                 630                 635                 640

Gly Lys Leu Pro Arg Arg Tyr Ile Lys Asn Phe Ala Thr Met Arg Asn
                645                 650                 655

Met Asn Glu Ser Gly Val Gly Asn Pro Gln Tyr Leu Gly Asp Ser Ser
            660                 665                 670

Ile Tyr Ser Ile Thr Tyr Ser Met Val Leu Thr Phe Asn Gly Leu Gln
        675                 680                 685

Gln Lys Tyr Glu Lys Leu Ile Val Thr Met Ser Thr Phe Asp Ile Ser
    690                 695                 700

Ser Asn Asn Phe Thr Gly Gln Ile Pro Tyr Val Ile Gly Gly Leu Arg
705                 710                 715                 720

Ser Leu Arg Asn Leu Asn Leu Ser His Asn Val Leu Thr Gly Asn Ile
                725                 730                 735

Pro Pro Ser Ile Ala Lys Leu Ser Leu Leu Gln Asp Leu Asp Leu Ser
            740                 745                 750

Ser Asn Arg Leu Thr Gly Arg Ile Pro Gln Glu Leu Val Ser Leu Thr
        755                 760                 765

Phe Leu Gly Ser Phe Asn Val Ser Asn Asn Leu Leu Glu Gly Ser Ile
    770                 775                 780

Pro His Gly Phe Asn Phe Asp Thr Tyr Thr Ala Asn Ser Tyr Gln Gly
785                 790                 795                 800

Asn Leu Glu Leu Cys Gly Lys Pro Leu Pro Glu Cys Gly Glu Arg Arg
                805                 810                 815

Ala Lys Gly Thr Thr Asn Asn Gln Asp Asp Pro Lys Asn Asp Asn Glu
            820                 825                 830

Arg Met Leu Ser Met Ser Glu Ile Val Val Met Gly Phe Gly Ser Gly
        835                 840                 845

Val Leu Val Gly Leu Ala Trp Gly Tyr Tyr Met Phe Ser Val Gly Lys
    850                 855                 860

Pro Phe Trp Phe Ile Lys Met Ala Ser Lys Met Glu Ser Ile Leu Ile
865                 870                 875                 880

Gly Phe Phe

<210>  4
<211>  4748
<212>  DNA
<213>  Beta vulgaris

<400>  4
ttactatgaa caatacccta atatcattag gttttcccct tctctctcct aagtgccaaa      60

ctgccaaccc cctcccatct ttatttcaat aagagcacca ttaaattatt gtgtaacaaa     120

gaccattatt ttaagatcac taataaggtt gctctaatta ttcctagaat tctagtgaaa     180

aaagaaagat aaaagatgaa catggggtga tgactgatga ctgagagaca acagacaaca     240

cttggttgag ttgatatttg acgcaaagac ttggcgtgtt ggaaggttca ttacacattt     300

tatccaagtc aactttgaag tcttcttagc tagagactaa tagagtgaac gtgttggaag     360

gttcatgttc atgacattat aaaagtaata atagtgaaat ttcacaaagt atttataaac     420

ccaggacaga ctcaagagct ctacttatta tattagtgaa aaacaaacat acacacgaca     480

ataacacaac ataaacaata atgaacatga aaatcctcct tttgtttgtc ttccttcatc     540

acctccacta cttcatcaat ggcagaacac taacagaaca tcaagcttta ctaagtatca     600

aatctgccat tactaatgat acgaatagct atctctcctt atggaaaaac acaacacacc     660

actgcagttg gccatacatc acttgctcct cctcttcttc ttctgtcatt tctctcgata     720

tctcctactt agagctcacc ggaattctct cccctgatat aggcttcctc accaacctcc 780
aaaacctcac tattcaatgg aacgattttt ctggccccct ccccacttct ctctctctcc 840
tcacccaact ccgccatctc gacgtttcct acaacaattt cacaggtcca atcccatctt 900
ctctctctct cctcacccaa ctccgccatc tcgacgtttc cttcaacagt ttcacaggtc 960
caatcccatc ttctctctct ctcctcaccc aactccgcta tctcgacgtt tcccaaaaca 1020
gtttcacagg tccaatccca tcttctctct ctctcctcac ccaactccgc tatctcgacg 1080
tttccgacaa cagtttcaca ggtccaatcc catcttttct ctctctcctc acccaactcc 1140
gctatctaga cgtttcctac aacaatctaa atggcactct tcccttatcg gtcgttgaga 1200
tgtcggaact caggtacctt aaccttaagt ataactcttt ctacggtgag attccaccgg 1260
agtttgggaa acttaagaag cttcaaacat tggatcttgg taacaactat ctttctgggg 1320
gtcttccatt tgagttgggt tcattaaaga gtttgaaata tattgatctt agtataaaca 1380
atttatatgg gagtatacct gattatattg gagattttcc ggagttggaa tcacttttat 1440
tagactcgaa taacttcaca gggagtatcc cacaaaagtt aggtacaaac gggaagttgc 1500
aatatctaga tataagtaac aacaatttta gtgggagttt gccagcaagt ctttgcaaag 1560
gagacaaact ccaacatttg ggagtatccg ataatttgtt ggttgggcca attcctgaga 1620
gtttgggaag ttgcaagtca cttgaagaag tgaacatggg aaataatttc tttaacgggt 1680
cgattcctaa gggcttgttt ggcctcccaa acattattga tgtttcactc aatgacaatc 1740
ttcttagcgg aggtctcgat gagaaatttg gtgattgtgt taatcttttc aacattgatc 1800
tctctaataa taagctatca gggaagttac ctgcgactat tggaaactgt tctaatcttc 1860
agttgttgat gcttaatcag aataacttca ccggaagtat ccctcaagag attagcaagt 1920
gtaagcagct acgggccctc gatctcagcc aaaatcagtt ctctggtgtg atacccaatg 1980
atattacagg taagaaagta tattaaactt gttacttttg aaaatattcg ctctagtttt 2040
ctttcagttg gtccattctc acttttgcat tattgaaata tatccctaaa aaagtaaatg 2100
taattatata aaagaatctt gctcaaaata atatgaatta tttttgtatg tgcaaaataa 2160
tgtacaatct aactaatttg ttgtgaaaaa taatataatt gtgtgaaata gtaaatgtgt 2220
ggagatatat aactttattt atcatattca ctaagggttt taggtatttt actatgactt 2280
ttgcattatg gagatatcca acttgacaat agtatttttg taatatactt cctccgtttc 2340
taaataagtg caacatttac atagtgttta ctattcacag tttaaacttt aattagcttt 2400
ggtgatttac attttaggaa aaacatagtc atgtgggatc ttattagatt cgtctgaatg 2460
tgaatttttt taatatcaac tttttataat ttttacttat tgacaattga agatattaat 2520
ggttaaaata atgcattggc aaacgtgcaa acaagaaatg ttgcacttat ttagaaacgg 2580
aggaagtatc atatatgaag attattgtac ataacacttt tcttattttt ataaactata 2640

tattcttctt ctttgtattt atcacaacac tttttatatc tttgcctcat attaatggca 2700

acacttttaa tttatctatt tactttttat ttcttggtct atagcccatt tacatactta 2760

tgacacacct cataaaggac ccacacgatt aaccaaaata tacaaatatc ttcaatgaaa 2820

ttaacttcaa tactaatatg ataaaaatca tgccccgctg tttatcctca tcctaagact 2880

ctgcataaaa ttattatttc ttgtccatac ttaatcatgt tgtgttttca cattttaact 2940

aataataaat tacaattgat acaccaaaaa actctatgag cattgggtat gttgtttgat 3000

agaagcttca tgctattatt tcttgtcaaa gaatttcata tctcgatatc ttctatacca 3060

tctaacgaac aattattttc tgcaggaaac aaaaccattt gcaattttga agaaattaaa 3120

ttacttgatt tatcaaacaa tattttgacc ggtgaaatcc ctcgttgtct tggaaatact 3180

agtactcaac tcgaaacatt atttcttcaa tcaaacaaac tgaacggtac catacccgca 3240

aacttctcaa agttatgtga ttcattgatg tatctagatc ttagtgacaa tcaactcgaa 3300

ggagttctac ctaagtcatt gtccaaatgt caaaatttga aactcctaaa tgtcgggaac 3360

aacaggctaa gagataaatt tccctcatgg ctagacaacc tcccacatct ccaagttttc 3420

agtgtgcgtt tcaatgcctt ctacggtcct ataactagct catcaaaggt taatcaccca 3480

tttcctatgc tacaaattat cgacctatct aacaatgagt tttgtggcaa gttgccaaga 3540

agatatatca aaaattttgc aaccatgcgc aatatgaatg agtctggtgt tggggatcca 3600

cagtacctgg aggactcata tagtccgtac tctatggtat tgacattcaa tgggttacaa 3660

caaaaatatg aaaagcttat tgtgacgatg tcgacctttg atatatccaa caacaacttt 3720

actggacaga ttccatatgt tataggggga ttacactcac ttcgtaacct taatctctcg 3780

cataatgtct taaccgggaa cattcctcca tcaattgcaa aattgtcttt gcttcaggat 3840

ttggaccttt catcaaacag acttattggt cgtatccctc aagaattagt tagtttaaca 3900

tttcttggga gcttcaatgt ttcgaacaat ctattggagg ggcctatacc tattggtaac 3960

aacttcaata cattctcgaa taattcatac caggggaatg tcggattgtg tggaaaacca 4020

ttacctgagt gtggagaaag aagggcaaaa agcaccacta ataatcaaga tgttcctaaa 4080

aatgataatg aacgaatgtt gtcgatgtcc gaaatcgtag ttatggggtt tggcagtggt 4140

gtactagttg ggttggcttg gggatactat atgttttcag tgggaaagcc cttttggttt 4200

atcaagatgg ctagcaaaat ggaatcaata ttgattggtt ttttctgacc aacaatttgt 4260

tagccgatga agagcatcaa aaccaaaaaa acaaaaaaat tgagtaatat gcatgagtgt 4320

gaccttgttt tccaaagttt agcattacta ttagtgtctc aattcataat aataaaaaaa 4380

ttagcttgtt caagatttgt attttattca aagatttttt atgtctcttg tgcttctttt 4440

atcttatata tattttttgt atggtttgtt tttgtttaat attagtccct ccgctcaaaa 4500

tgatctttca cgcttgagat tggcattaag gtcaagagat gttgctaagc tttagaataa 4560

aaaaattcca aatgcataga gggaaagaaa gcgagacaaa atgttggaga aggcagagta 4620

aatgatgtga tggaggataa atagtagaag tgtgataccg aaagtttgaa aataataagg 4680

aattttattt cttgctggca cttcgttcta gtacaggttt ttggcccttc aaaatgctta 4740

taatgtag 4748

<210> 5
<211> 2460
<212> DNA
<213> Artificial Sequence

<220>
<223> cDNA sequence of the sensitive variant of the Cercospora
      resistance-mediating gene

<400> 5
atgaacatga aaatcctcct tttgtttgtc ttccttcatc acctccacta cttcatcaat 60

ggcagaacac taacagaaca tcaagcttta ctaagtatca aatctgccat tactaatgat 120

acgaatagct atctctcctt atggaaaaac acaacacacc actgcagttg gccatacatc 180

acttgctcct cctcttcttc ttctgtcatt tctctcgata tctcctactt agagctcacc 240

ggaattctct cccctgatat aggcttcctc accaacctcc aaaacctcac tattcaatgg 300

aacgattttt ctggccccct ccccacttct ctctctctcc tcacccaact ccgccatctc 360

gacgtttcct acaacaattt cacaggtcca atcccatctt ctctctctct cctcacccaa 420

ctccgccatc tcgacgtttc cttcaacagt ttcacaggtc caatcccatc ttctctctct 480

ctcctcaccc aactccgcta tctcgacgtt tcccaaaaca gtttcacagg tccaatccca 540

tcttctctct ctctcctcac ccaactccgc tatctcgacg tttccgacaa cagtttcaca 600

ggtccaatcc catcttttct ctctctcctc acccaactcc gctatctaga cgtttcctac 660

aacaatctaa atggcactct tcccttatcg gtcgttgaga tgtcggaact caggtacctt 720

aaccttaagt ataactcttt ctacggtgag attccaccgg agtttgggaa acttaagaag 780

cttcaaacat tggatcttgg taacaactat ctttctgggg gtcttccatt tgagttgggt 840

tcattaaaga gtttgaaata tattgatctt agtataaaca atttatatgg gagtatacct 900

gattatattg gagattttcc ggagttggaa tcacttttat tagactcgaa taacttcaca 960

gggagtatcc cacaaaagtt aggtacaaac gggaagttgc aatatctaga tataagtaac 1020

aacaatttta gtgggagttt gccagcaagt ctttgcaaag gagacaaact ccaacatttg 1080

ggagtatccg ataatttgtt ggttgggcca attcctgaga gtttgggaag ttgcaagtca 1140

cttgaagaag tgaacatggg aaataatttc tttaacgggt cgattcctaa gggcttgttt 1200

ggcctcccaa acattattga tgtttcactc aatgacaatc ttcttagcgg aggtctcgat 1260

gagaaatttg gtgattgtgt taatcttttc aacattgatc tctctaataa taagctatca 1320

gggaagttac ctgcgactat tggaaactgt tctaatcttc agttgttgat gcttaatcag 1380

aataacttca ccggaagtat ccctcaagag attagcaagt gtaagcagct acgggccctc 1440

gatctcagcc aaaatcagtt ctctggtgtg atacccaatg atattacaga tcttagtgac 1500

aatcaactcg aaggagttct acctaagtca ttgtccaaat gtcaaaattt gaaactccta 1560

aatgtcggga acaacaggct aagagataaa tttccctcat ggctagacaa cctcccacat 1620

ctccaagttt tcagtgtgcg tttcaatgcc ttctacggtc ctataactag ctcatcaaag 1680

gttaatcacc catttcctat gctacaaatt atcgacctat ctaacaatga gttttgtggc 1740

aagttgccaa gaagatatat caaaaatttt gcaaccatgc gcaatatgaa tgagtctggt 1800

gttggggatc cacagtacct ggaggactca tatagtccgt actctatggt attgacattc 1860

aatgggttac aacaaaaata tgaaaagctt attgtgacga tgtcgacctt tgatatatcc 1920

aacaacaact ttactggaca gattccatat gttatagggg gattacactc acttcgtaac 1980

cttaatctct cgcataatgt cttaaccggg aacattcctc catcaattgc aaaattgtct 2040

ttgcttcagg atttggacct ttcatcaaac agacttattg gtcgtatccc tcaagaatta 2100

gttagtttaa catttcttgg gagcttcaat gtttcgaaca atctattgga ggggcctata 2160

cctattggta acaacttcaa tacattctcg aataattcat accaggggaa tgtcggattg 2220

tgtggaaaac cattacctga gtgtggagaa agaagggcaa aaagcaccac taataatcaa 2280

gatgttccta aaaatgataa tgaacgaatg ttgtcgatgt ccgaaatcgt agttatgggg 2340

tttggcagtg gtgtactagt tgggttggct tggggatact atatgttttc agtgggaaag 2400

cccttttggt ttatcaagat ggctagcaaa atggaatcaa tattgattgg ttttttctga 2460

<210> 6
<211> 819
<212> PRT
<213> Beta vulgaris

<400> 6

Met Asn Met Lys Ile Leu Leu Leu Phe Val Phe Leu His His Leu His
1               5                   10                  15

Tyr Phe Ile Asn Gly Arg Thr Leu Thr Glu His Gln Ala Leu Leu Ser
            20                  25                  30

Ile Lys Ser Ala Ile Thr Asn Asp Thr Asn Ser Tyr Leu Ser Leu Trp
        35                  40                  45

Lys Asn Thr Thr His His Cys Ser Trp Pro Tyr Ile Thr Cys Ser Ser
    50                  55                  60

Ser Ser Ser Ser Val Ile Ser Leu Asp Ile Ser Tyr Leu Glu Leu Thr
65                  70                  75                  80

Gly Ile Leu Ser Pro Asp Ile Gly Phe Leu Thr Asn Leu Gln Asn Leu
                85                  90                  95

Thr Ile Gln Trp Asn Asp Phe Ser Gly Pro Leu Pro Thr Ser Leu Ser
            100                 105                 110

Leu Leu Thr Gln Leu Arg His Leu Asp Val Ser Tyr Asn Asn Phe Thr
        115                 120                 125

Gly Pro Ile Pro Ser Ser Leu Ser Leu Leu Thr Gln Leu Arg His Leu
    130                 135                 140

Asp Val Ser Phe Asn Ser Phe Thr Gly Pro Ile Pro Ser Ser Leu Ser
145                 150                 155                 160

Leu Leu Thr Gln Leu Arg Tyr Leu Asp Val Ser Gln Asn Ser Phe Thr
                165                 170                 175

Gly Pro Ile Pro Ser Ser Leu Ser Leu Leu Thr Gln Leu Arg Tyr Leu
            180                 185                 190

Asp Val Ser Asp Asn Ser Phe Thr Gly Pro Ile Pro Ser Phe Leu Ser
        195                 200                 205

Leu Leu Thr Gln Leu Arg Tyr Leu Asp Val Ser Tyr Asn Asn Leu Asn
    210                 215                 220

Gly Thr Leu Pro Leu Ser Val Val Glu Met Ser Glu Leu Arg Tyr Leu
225                 230                 235                 240

Asn Leu Lys Tyr Asn Ser Phe Tyr Gly Glu Ile Pro Pro Glu Phe Gly
                245                 250                 255

Lys Leu Lys Lys Leu Gln Thr Leu Asp Leu Gly Asn Asn Tyr Leu Ser
            260                 265                 270

Gly Gly Leu Pro Phe Glu Leu Gly Ser Leu Lys Ser Leu Lys Tyr Ile
        275                 280                 285

Asp Leu Ser Ile Asn Asn Leu Tyr Gly Ser Ile Pro Asp Tyr Ile Gly
    290                 295                 300

Asp Phe Pro Glu Leu Glu Ser Leu Leu Leu Asp Ser Asn Asn Phe Thr
305                 310                 315                 320

Gly Ser Ile Pro Gln Lys Leu Gly Thr Asn Gly Lys Leu Gln Tyr Leu
                325                 330                 335

Asp Ile Ser Asn Asn Asn Phe Ser Gly Ser Leu Pro Ala Ser Leu Cys
            340                 345                 350

Lys Gly Asp Lys Leu Gln His Leu Gly Val Ser Asp Asn Leu Leu Val
        355                 360                 365

Gly Pro Ile Pro Glu Ser Leu Gly Ser Cys Lys Ser Leu Glu Glu Val
    370                 375                 380

Asn Met Gly Asn Asn Phe Phe Asn Gly Ser Ile Pro Lys Gly Leu Phe
385                 390                 395                 400

Gly Leu Pro Asn Ile Ile Asp Val Ser Leu Asn Asp Asn Leu Leu Ser
                405                 410                 415

Gly Gly Leu Asp Glu Lys Phe Gly Asp Cys Val Asn Leu Phe Asn Ile
            420                 425                 430

Asp Leu Ser Asn Asn Lys Leu Ser Gly Lys Leu Pro Ala Thr Ile Gly
        435                 440                 445

Asn Cys Ser Asn Leu Gln Leu Leu Met Leu Asn Gln Asn Asn Phe Thr
    450                 455                 460

Gly Ser Ile Pro Gln Glu Ile Ser Lys Cys Lys Gln Leu Arg Ala Leu
465                 470                 475                 480

Asp Leu Ser Gln Asn Gln Phe Ser Gly Val Ile Pro Asn Asp Ile Thr
                485                 490                 495

Asp Leu Ser Asp Asn Gln Leu Glu Gly Val Leu Pro Lys Ser Leu Ser
            500                 505                 510

Lys Cys Gln Asn Leu Lys Leu Leu Asn Val Gly Asn Asn Arg Leu Arg
        515                 520                 525

Asp Lys Phe Pro Ser Trp Leu Asp Asn Leu Pro His Leu Gln Val Phe
    530                 535                 540

Ser Val Arg Phe Asn Ala Phe Tyr Gly Pro Ile Thr Ser Ser Ser Lys
545                 550                 555                 560

Val Asn His Pro Phe Pro Met Leu Gln Ile Ile Asp Leu Ser Asn Asn

                565                 570                 575

Glu Phe Cys Gly Lys Leu Pro Arg Arg Tyr Ile Lys Asn Phe Ala Thr
            580                 585                 590

Met Arg Asn Met Asn Glu Ser Gly Val Gly Asp Pro Gln Tyr Leu Glu
        595                 600                 605

Asp Ser Tyr Ser Pro Tyr Ser Met Val Leu Thr Phe Asn Gly Leu Gln
    610                 615                 620

Gln Lys Tyr Glu Lys Leu Ile Val Thr Met Ser Thr Phe Asp Ile Ser
625                 630                 635                 640

Asn Asn Asn Phe Thr Gly Gln Ile Pro Tyr Val Ile Gly Gly Leu His
                645                 650                 655

Ser Leu Arg Asn Leu Asn Leu Ser His Asn Val Leu Thr Gly Asn Ile
            660                 665                 670

Pro Pro Ser Ile Ala Lys Leu Ser Leu Leu Gln Asp Leu Asp Leu Ser
        675                 680                 685

Ser Asn Arg Leu Ile Gly Arg Ile Pro Gln Glu Leu Val Ser Leu Thr
    690                 695                 700

Phe Leu Gly Ser Phe Asn Val Ser Asn Asn Leu Leu Glu Gly Pro Ile
705                 710                 715                 720

Pro Ile Gly Asn Asn Phe Asn Thr Phe Ser Asn Asn Ser Tyr Gln Gly
                725                 730                 735

Asn Val Gly Leu Cys Gly Lys Pro Leu Pro Glu Cys Gly Glu Arg Arg
            740                 745                 750

Ala Lys Ser Thr Thr Asn Asn Gln Asp Val Pro Lys Asn Asp Asn Glu
        755                 760                 765

Arg Met Leu Ser Met Ser Glu Ile Val Val Met Gly Phe Gly Ser Gly
    770                 775                 780

Val Leu Val Gly Leu Ala Trp Gly Tyr Tyr Met Phe Ser Val Gly Lys
785                 790                 795                 800

Pro Phe Trp Phe Ile Lys Met Ala Ser Lys Met Glu Ser Ile Leu Ile
                805                 810                 815

Gly Phe Phe

<210> 7
<211> 1998
<212> DNA
<213> Beta vulgaris

<220>
<221> promoter
<222> (1)..(1998)
<223> native promoter of the Cercospora resistance-conferring gene Beta
      vulgaris subsp. maritima

<400> 7
gagcatagtg agtgcaaaag ccatggaagc tagattaaaa aggccatcat tctaagttag      60

acaattggaa acaacatcga gatacacgta cacataaggg ctgctcttct ctattactcc     120

ctctgttcct aatcatttgc ttttttagcg ggttccaaag gcctatgttt gaccactaat     180

atatttaaat taaaactggt gatatatatt aaaagaaaat tatgatgaat ttaacaaaaa     240

ccatatatgt tatgtccttt tttttcctat attaatgaat ttttacagtc aaagttggtg     300

aactttgacc caaaaaaaga aatggagcaa aaaaaaaaaa aaaaaaaaaa aactagggac     360

aatgagtaac atttttatct atgtcttttt aatatgaata tacgtaacaa attctgcaaa     420

aatagagata gcaactaata acacgcatga aaatgacaag ttatattata cctttttttc     480

tcaatatatg aatatacgta acaaattaac tccagtagtt tttagtaaaa ctattagatt     540

attgtgtaac atatactctg gaaatagtac taagatccat tacaatcttt attgagaaat     600

ttcctcatgt accccctgag gtttggcgta atttccaaat acccctcata tttgaggaat     660

ttctcaaata ccctgatgtt tttgtttaga ctcaaaatac ctttactatg gacagtaccc     720

taatgtcatt aagttttccc cttctctctc cccaattttc tctctcctcc cattccccca     780

cccactaccc actgcccact gccaagtagg ggtgtaagtg gattggactg gattggactt     840

tgccaaattc aaatccagtc caaagttttt tggactcgag aaattgagtc caagtccgat     900

ccaaatattt tttgagtcca gtccaatcta gtccgataat tttttcttga gtccgaatcc     960

agtccagtcc agtccgatta ttatatcttt tttcccgatt taggttcaat gattcacaac    1020

attttttgag atgcttgagc atttgacatc tgattcaatt atcaatatcc acaaataaga    1080

ttgaaagctt aaattaaagt aaaatactat gaataaaaag ttgaattaga tgcttacctt    1140

gatctaagtt gagaggaagc atagagactg agaattaatc tgagggacaa atagagaatg    1200

cgagagtcga gacagtgagg tagaaagaaa atgaagagta agaggaagtg agtattaagg    1260

actgaggagt aaagtaagat agaattagtt ggctactagc ctactaatgc agtattgcta    1320

gtataattta cttatttaac aaatggagct aagtgcaata gtttagcgcc aattgacata    1380

tttagagaga gaaggctgaa aaatccaata tttttaaaat agtatcatta tttttaatat 1440

atacattata tataaaaata tttttggact ggactggaca tattggactc caaagggatg 1500

agtccaaatc cagacaaaaa atatttggac ttgaaaattt aagtccgagt ccagtccgaa 1560

aaattttcag tccaatccag tccgacaaat ttggactgga ctggattgga ctctgaactt 1620

ttcgtagtcc gcttacaccc ctactgccaa gtgccaaact gccaaccccc ttttggttga 1680

gttgatattt gacgcaaaga cttggcgtgt tggaaggttc attacacatt ttatccaagt 1740

caactttgaa gtcttcttag ctagagacta gagtgaacgt gttggaaggt tcattacaca 1800

ttttatccaa tcaaactttg aagtcttctt agctagagac tagagtgaac gtgttggaag 1860

gttcatgttc atgacattat aaaagtaata atagtgaaat ttcacaaagt atttataaac 1920

ccaggacaga ctcaagagct ctacttatta ttagtgaaaa acaaacatac acacgacaat 1980

aacacaacat aaacaata 1998

<210> 8
<211> 2000
<212> DNA
<213> Beta vulgaris

<220>
<221> terminator
<222> (1)..(2000)
<223> native terminator of the Cercospora resistance-conferring gene
      from Beta vulgaris subsp. maritima

<400> 8
ccaacaattt gttagccgat gaagagcatc aaaaccaaaa aaaacaaaaa aaattgatta 60

atatgcatga gtgtgacctt gttttccaaa gtttagcatt actattagtg tctcaattca 120

taataataaa aaaattagct tgttcaagat ttgtattttt attcaaagat tttttttgtc 180

tcttgtgctt cttttatctt atatatattt tttgtatggt ttgtttttgt ttaatattag 240

tccctccgct caaaatgatc tttcacgctt gagattggca ttaaggtcaa gagatgttgc 300

taagctttag aataaaaaaa ttccaaatgc atagagggaa agaaagcgag acaaaatgtt 360

ggagaaggca gagtaaatga tgtgatggag gataaatagt agaagtgtga taccgaaagt 420

ttgaaaataa taaggaattt tatttcttgc tggcactttg ttctagtaca ggtttttagc 480

ccttcaaaat gtttataatg tagagtcaaa attaatatcc ttaactagtt tttaagtccg 540

ggttatatcc tagatattaa taatattcat ttattagtaa cattttattt tataaatata 600

atactaagca ttatttggtt tgctggttaa gactttagtg tatatctatt tctttttttt 660

tttattgtat gcgtgtttac ataaactaaa gactataagg gatagtacca cgtggcgcag 720

ttccttgctt aggaacgtct tttaatatat taactagtat ttgggcccgg gcgttgctcc 780

gggttggtat tgtgtttccg aacatgatgt gcagtttttc ccattcccac taaaatatat 840

aaaggaaaac tcaacattta aaagatacaa atataataat atggacactt aaaacatgat 900

taaaagttga ttgagatggt aattgtgtca tgttataata gtaagaggtt gcctaattga 960

ggttgaggtg gtggagtagt ggtatcgctt cccatctgtt atccctgagg tataaggatc 1020

aaacctcata ggactcattt gagtaatttc ccatatcctc ctctcaaatg agtccttttc 1080

atctgacaaa aaaaaagagt ctaattttaa attaaaatta gacgatcttt tataaaatcg 1140

gcactttctg cacataggtc acaatttttt tgtttctatc tctctgcttt ctttaatttc 1200

acagtctcca actctccatc aacatcttac ttattttaga atagatgatg tatggtagta 1260

ttaaatggta aagtactaaa gctcctataa tacacagaag cttacatagt atagattcgt 1320

acatgagaca aggttacaat atactttctc cgttcttttt atattacaat aattactatt 1380

ttaagtagtt tcacatctat tgtaacaatt ccaattttgt tatagaaagc aactttaata 1440

attgacaata ttgcccttac tttatcttat taaaaccatc attaattact cactttctct 1500

tataaaattg cttttatttt ctaaggatga tttctctcct attctagtta attaaagagt 1560

tacttttgtg ctaaactgct catttattcc aaatccttaa aaattgtgtc caaacgtatt 1620

gttgtaatat aaaaagaaca gaggtactat tagtttgaat aaattttgat cagattaggt 1680

cacctttagg gggcgtttgg ttaggggtat tctggaaagg gtaagggaat caacttactt 1740

aattccctta cttgttgttt gtttgctcaa tttaatgatt ccctttaccc accccttact 1800

cccaaagtcc tttactctca ttctccccac cccccaaggt ttcacttacc ctttcttgat 1860

tcatcattga ccatatcttt gaccacccaa ctaccaccac cacttgacca cctaatcacc 1920

taaccaccta attacccaac cactattacc acccaacccc tccacctgcc caccaatcgg 1980

caccataact gcccaaccgt 2000

<210> 9
<211> 911
<212> PRT
<213> Beta vulgaris

<220>
<221> PEPTIDE
<222> (1)..(911)
<223> Consensus sequence of Fig. 1

<220>
<221> VARIANT
<222> (20)..(20)
<223> N or H

<220>
<221> VARIANT
<222> (27)..(27)
<223> H or R

<220>
<221> VARIANT
<222> (39)..(39)
<223> N or Y

<220>
<221> VARIANT
<222> (41)..(41)
<223> T or Y

<220>
<221> VARIANT
<222> (42)..(42)
<223> N or Y

<220>
<221> VARIANT
<222> (42)..(42)
<223> S or N

<220>
<221> VARIANT
<222> (43)..(43)
<223> Y or S

<220>
<221> VARIANT
<222> (47)..(47)
<223> L or S

<220>
<221> VARIANT
<222> (75)..(75)
<223> D or N

<220>
<221> VARIANT
<222> (76)..(76)
<223> I or F

<220>
<221> VARIANT
<222> (77)..(77)
<223> S or T

<220>
<221> VARIANT
<222> (78)..(78)
<223> Y or M

<220>
<221> VARIANT
<222> (80)..(80)
<223> E or F

<220>
<221> VARIANT
<222> (82)..(82)
<223> T or E

<220>
<221> VARIANT
<222> (99)..(99)

<223> T or S

<220>
<221> VARIANT
<222> (101)..(101)
<223> Q or R

<220>
<221> VARIANT
<222> (102)..(102)
<223> W or S

<220>
<221> VARIANT
<222> (104)..(104)
<223> D or L

<220>
<221> VARIANT
<222> (111)..(111)
<223> T or H

<220>
<221> VARIANT
<222> (121)..(121)
<223> H or Y

<220>
<221> VARIANT
<222> (126)..(126)
<223> Y or Q

<220>
<221> VARIANT
<222> (171)..(171)
<223> D or H

<220>
<221> VARIANT
<222> (174)..(174)
<223> Q or G

<220>
<221> VARIANT
<222> (184)..(184)
<223> S or F

<220>
<221> VARIANT
<222> (208)..(208)
<223> F or S

<220>
<221> VARIANT
<222> (246)..(246)
<223> K or R

<220>
<221> VARIANT
<222> (265)..(265)
<223> Q or E

<220>

<221> VARIANT
<222> (268)..(268)
<223> D or N

<220>
<221> VARIANT
<222> (277)..(277)
<223> G or S

<220>
<221> VARIANT
<222> (280)..(280)
<223> F or S

<220>
<221> VARIANT
<222> (290)..(290)
<223> Y or H

<220>
<221> VARIANT
<222> (318)..(318)
<223> S or N

<220>
<221> VARIANT
<222> (319)..(319)
<223> I or R

<220>
<221> VARIANT
<222> (321)..(321)
<223> N or K

<220>
<221> VARIANT
<222> (376)..(376)
<223> A or L

<220>
<221> VARIANT
<222> (386)..(386)
<223> H or D

<220>
<221> VARIANT
<222> (388)..(388)
<223> G or D

<220>
<221> VARIANT
<222> (389)..(389)
<223> V or A

<220>
<221> VARIANT
<222> (391)..(391)
<223> D or Y

<220>
<221> VARIANT
<222> (397)..(397)
<223> P or S

<220>
<221>  VARIANT
<222>  (410)..(410)
<223>  E or G

<220>
<221>  VARIANT
<222>  (412)..(412)
<223>  N or Y

<220>
<221>  VARIANT
<222>  (418)..(418)
<223>  F or L

<220>
<221>  VARIANT
<222>  (429)..(429)
<223>  L or S

<220>
<221>  VARIANT
<222>  (440)..(440)
<223>  N or K

<220>
<221>  VARIANT
<222>  (457)..(457)
<223>  F or R

<220>
<221>  VARIANT
<222>  (458)..(458)
<223>  N or D

<220>
<221>  VARIANT
<222>  (478)..(478)
<223>  S or I

<220>
<221>  VARIANT
<222>  (479)..(479)
<223>  N or H

<220>
<221>  VARIANT
<222>  (481)..(481)
<223>  Q or R

<220>
<221>  VARIANT
<222>  (482)..(482)
<223>  L or S

<220>
<221>  VARIANT
<222>  (484)..(484)
<223>  M or T

<220>
<221>  VARIANT

<222>  (486)..(486)
<223>  N or Y

<220>
<221>  VARIANT
<222>  (487)..(487)
<223>  Q or N

<220>
<221>  VARIANT
<222>  (489)..(489)
<223>  N or T

<220>
<221>  VARIANT
<222>  (490)..(490)
<223>  F or C

<220>
<221>  VARIANT
<222>  (493)..(493)
<223>  S or R

<220>
<221>  VARIANT
<222>  (505)..(505)
<223>  R or Q

<220>
<221>  VARIANT
<222>  (506)..(506)
<223>  A or T

<220>
<221>  VARIANT
<222>  (605)..(605)
<223>  R or Q

<220>
<221>  VARIANT
<222>  (607)..(607)
<223>  K or E

<220>
<221>  VARIANT
<222>  (629)..(629)
<223>  H or R

<220>
<221>  VARIANT
<222>  (648)..(648)
<223>  S or P

<220>
<221>  VARIANT
<222>  (651)..(651)
<223>  N or S

<220>
<221>  VARIANT
<222>  (666)..(666)
<223>  E or K

<220>
<221> VARIANT
<222> (692)..(692)
<223> D or N

<220>
<221> VARIANT
<222> (697)..(697)
<223> E or G

<220>
<221> VARIANT
<222> (705)..(705)
<223> P or T

<220>
<221> VARIANT
<222> (733)..(733)
<223> H or R

<220>
<221> VARIANT
<222> (733)..(733)
<223> N or S

<220>
<221> VARIANT
<222> (748)..(748)
<223> H or R

<220>
<221> VARIANT
<222> (785)..(785)
<223> I or T

<220>
<221> VARIANT
<222> (811)..(811)
<223> P or S

<220>
<221> VARIANT
<222> (814)..(814)
<223> I or H

<220>
<221> VARIANT
<222> (816)..(816)
<223> N or F

<220>
<221> VARIANT
<222> (819)..(819)
<223> N or D

<220>
<221> VARIANT
<222> (821)..(821)
<223> F or Y

<220>
<221> VARIANT
<222> (822)..(822)

<223>  S or T

<220>
<221>  VARIANT
<222>  (823)..(823)
<223>  N or A

<220>
<221>  VARIANT
<222>  (823)..(823)
<223>  N or A

<220>
<221>  VARIANT
<222>  (830)..(830)
<223>  V or L

<220>
<221>  VARIANT
<222>  (831)..(831)
<223>  G or E

<220>
<221>  VARIANT
<222>  (847)..(847)
<223>  S or G

<220>
<221>  VARIANT
<222>  (854)..(854)
<223>  V or D

<400>  9

Met Asn Met Lys Ile Leu Leu Leu Phe Val Phe Leu His His Leu His
1               5                   10                  15

Tyr Phe Ile Xaa Gly Arg Thr Leu Thr Glu Xaa Gln Ala Leu Leu Ser
            20                  25                  30

Ile Lys Ser Ala Ile Thr Xaa Asp Xaa Xaa Xaa Xaa Leu Ser Xaa Trp
        35                  40                  45

Lys Asn Thr Thr His His Cys Ser Trp Pro Tyr Ile Thr Cys Ser Ser
    50                  55                  60

Ser Ser Ser Ser Ser Ser Val Ile Ser Leu Xaa Xaa Xaa Xaa Leu Xaa
65                  70                  75                  80

Leu Xaa Gly Ile Leu Ser Pro Asp Ile Gly Phe Leu Thr Asn Leu Gln
                85                  90                  95

Asn Leu Xaa Ile Xaa Xaa Asn Xaa Phe Ser Gly Pro Leu Pro Xaa Ser
            100                 105                 110

Leu Ser Leu Leu Thr Gln Leu Arg Xaa Leu Asp Val Ser Xaa Asn Asn

        115                 120                 125

Phe Thr Gly Pro Ile Pro Ser Ser Leu Ser Leu Leu Thr Gln Leu Arg
    130                 135                 140

His Leu Asp Val Ser Phe Asn Ser Phe Thr Gly Pro Ile Pro Ser Ser
145                 150                 155                 160

Leu Ser Leu Leu Thr Gln Leu Arg Tyr Leu Xaa Val Ser Xaa Asn Ser
                165                 170                 175

Phe Thr Gly Pro Ile Pro Ser Xaa Leu Ser Leu Leu Thr Gln Leu Arg
            180                 185                 190

Tyr Leu Asp Val Ser Asp Asn Ser Phe Thr Gly Pro Ile Pro Ser Xaa
        195                 200                 205

Leu Ser Leu Leu Thr Gln Leu Arg Tyr Leu Asp Val Ser Tyr Asn Asn
    210                 215                 220

Leu Asn Gly Thr Leu Pro Leu Ser Val Val Glu Lys Met Ser Glu Leu
225                 230                 235                 240

Xaa Tyr Leu Asn Leu Xaa Tyr Asn Ser Phe Tyr Gly Glu Ile Pro Pro
                245                 250                 255

Glu Phe Gly Lys Leu Lys Lys Leu Xaa Thr Leu Xaa Leu Gly Asn Asn
            260                 265                 270

Xaa Leu Ser Gly Xaa Leu Pro Xaa Glu Leu Gly Ser Leu Lys Ser Leu
        275                 280                 285

Lys Xaa Met Asp Phe Ser Ser Asn Met Leu Phe Gly Glu Ile Pro Gln
    290                 295                 300

Ser Tyr Ser Leu Leu Arg Asn Leu Ile Asp Ile Asp Leu Xaa Xaa Asn
305                 310                 315                 320

Xaa Leu Tyr Gly Ser Ile Pro Asp Tyr Ile Gly Asp Phe Pro Glu Leu
                325                 330                 335

Glu Ser Leu Leu Leu Asp Ser Asn Asn Phe Thr Gly Ser Ile Pro Gln
            340                 345                 350

Lys Leu Gly Thr Asn Gly Lys Leu Gln Tyr Leu Asp Ile Ser Asn Asn
        355                 360                 365

Asn Phe Ser Gly Ser Leu Pro Xaa Ser Leu Cys Lys Gly Asp Lys Leu
    370                 375                 380

Gln Xaa Leu Xaa Xaa Ser Xaa Asn Leu Leu Val Gly Xaa Ile Pro Glu
385                 390                 395                 400

Ser Leu Gly Ser Cys Lys Ser Leu Glu Xaa Val Xaa Met Gly Asn Asn
                405                 410                 415

Phe Xaa Asn Gly Ser Ile Pro Lys Gly Leu Phe Gly Xaa Pro Asn Ile
            420                 425                 430

Ile Asp Val Ser Leu Asn Asp Xaa Leu Leu Ser Gly Gly Leu Asp Glu
        435                 440                 445

Lys Phe Gly Asp Cys Val Asn Leu Xaa Xaa Ile Asp Leu Ser Asn Asn
    450                 455                 460

Lys Leu Ser Gly Lys Leu Pro Ala Thr Ile Gly Asn Cys Xaa Xaa Leu
465                 470                 475                 480

Xaa Xaa Leu Xaa Leu Xaa Xaa Asn Xaa Xaa Thr Gly Xaa Ile Pro Gln
                485                 490                 495

Glu Ile Ser Lys Cys Lys Gln Leu Xaa Xaa Leu Asp Leu Ser Gln Asn
            500                 505                 510

Gln Phe Ser Gly Val Ile Pro Asn Asp Ile Thr Gly Asn Lys Ser Ile
        515                 520                 525

Cys Asn Leu Glu Lys Ile Gln Thr Leu Lys Leu Ser Asn Asn Ala Leu
    530                 535                 540

Thr Gly Glu Ile Pro His Cys Val Gly Asn Ile Glu Leu Ile Ala Leu
545                 550                 555                 560

Phe Leu Gln Ser Asn Lys Leu Asn Gly Thr Ile Pro Ala Asn Phe Ser
                565                 570                 575

Lys Leu Cys Asp Ser Leu Ile Tyr Leu Asp Leu Ser Asp Asn Gln Leu
            580                 585                 590

Glu Gly Val Leu Pro Lys Ser Leu Ser Lys Cys Gln Xaa Leu Xaa Leu
        595                 600                 605

Leu Asn Val Gly Asn Asn Arg Leu Arg Asp Lys Phe Pro Ser Trp Leu
    610                 615                 620

Asp Asn Leu Pro Xaa Leu Gln Val Phe Ser Val Arg Phe Asn Ala Phe
625                 630                 635                 640

Tyr Gly Pro Ile Thr Ser Ser Xaa Lys Val Xaa His Pro Phe Pro Met
                645                 650                 655

Leu Gln Ile Ile Asp Leu Ser Asn Asn Xaa Phe Cys Gly Lys Leu Pro
            660                 665                 670

Arg Arg Tyr Ile Lys Asn Phe Ala Thr Met Arg Asn Met Asn Glu Ser
        675                 680                 685

Gly Val Gly Xaa Pro Gln Tyr Leu Xaa Asp Ser Ser Ile Tyr Ser Ile
    690                 695                 700

Xaa Tyr Ser Met Val Leu Thr Phe Asn Gly Leu Gln Gln Lys Tyr Glu
705                 710                 715                 720

Lys Leu Ile Val Thr Met Ser Thr Phe Asp Ile Ser Xaa Asn Asn Phe
                725                 730                 735

Thr Gly Gln Ile Pro Tyr Val Ile Gly Gly Leu Xaa Ser Leu Arg Asn
            740                 745                 750

Leu Asn Leu Ser His Asn Val Leu Thr Gly Asn Ile Pro Pro Ser Ile
        755                 760                 765

Ala Lys Leu Ser Leu Leu Gln Asp Leu Asp Leu Ser Ser Asn Arg Leu
    770                 775                 780

Xaa Gly Arg Ile Pro Gln Glu Leu Val Ser Leu Thr Phe Leu Gly Ser
785                 790                 795                 800

Phe Asn Val Ser Asn Asn Leu Leu Glu Gly Xaa Ile Pro Xaa Gly Xaa
                805                 810                 815

Asn Phe Xaa Thr Xaa Xaa Xaa Asn Ser Tyr Gln Gly Asn Xaa Xaa Leu
            820                 825                 830

Cys Gly Lys Pro Leu Pro Glu Cys Gly Glu Arg Arg Ala Lys Xaa Thr
        835                 840                 845

Thr Asn Asn Gln Asp Xaa Pro Lys Asn Asp Asn Glu Arg Met Leu Ser
    850                 855                 860

Met Ser Glu Ile Val Val Met Gly Phe Gly Ser Gly Val Leu Val Gly
865                 870                 875                 880

Leu Ala Trp Gly Tyr Tyr Met Phe Ser Val Gly Lys Pro Phe Trp Phe
                885                 890                 895

Ile Lys Met Ala Ser Lys Met Glu Ser Ile Leu Ile Gly Phe Phe
            900                 905                 910

<210>  10
<211>  201
<212>  DNA
<213>  Beta vulgaris

<400>  10
agagcagatt ggcatacttr tgaatattct cactggctat taaattctca gaagaaaaat      60

caacaccaag attatgacat gcttgtgcaa agacacaccc rgtcatgaat gcatcatagc     120

cagcttcatg cttagcccca gagttccaat ttgaggayct gcaagaaaac atgggagtaa     180

gatggtttca cataaaacat g                                               201

<210>  11
<211>  201
<212>  DNA
<213>  Beta vulgaris

<400>  11
gggtttcttc gaagtttgat tttgttacat ttttcaaaga gaaattagtt gttgatgttg      60

aataatgatg ataagtagtt agggttcgta gtaaggtgga sgaragagaa aatggcgtca     120

ctctgayrag cttcttcatt ttgttcttct tccttagctc tgttttcagt cactgcgcca     180

tttttttttt aaaaggaaga t                                               201

<210>  12
<211>  134
<212>  DNA
<213>  Beta vulgaris

<400>  12
caagcacaaa atcaaataat gagaatcaca ctatccaaag aaaatttcca tccacattta      60

tccaacacar ttatctctct tttacaccca aattatgtca accaaaaaca staaaacaag     120

tgagtgcagt agct                                                       134

<210>  13
<211>  134
<212>  DNA
<213>  Beta vulgaris

<400>  13
taagtaaaaa gtggtaaaag aattaccaaa arcgcacara ataaattaat tagytggatw      60

taactawtta acctattcct tttttctgtc gctataacta cttttgctta acttattgat     120

ggtttgatcg ttga                                                    134

<210>  14
<211>  150
<212>  DNA
<213>  Beta vulgaris

<400>  14
ttataatgta gagtcaaaat taatatcctt aactagtttt taagtccggg ttatatccta      60

gatattwata atattcattt attagtaaca ttttatttta taaatataat actaagcatt     120

atttggtttg ctggttaaga ctttagtgta                                      150

<210>  15
<211>  201
<212>  DNA
<213>  Beta vulgaris

<400>  15
acatctacac tgggagactg ataaggacgt ttgcagatgt caagtatggg aatcatcatc      60

taacatgggt ggagattgtg tacaatgtta tttcattcat mgtggcaata attaccattg     120

ttgcgtttac tgtatatgcc aagagagcct tcgaagaact taagagggca gaagctaagg     180

aggatcgaga agaagaaacc t                                               201

<210>  16
<211>  28
<212>  DNA
<213>  Artificial Sequence

<220>
<223>  genomic target sequence with 5'-flanking PAM (4 bp)  5'crRNA # 1

<400>  16
tttatttcga tttcgattct tggattat                                         28

<210>  17
<211>  28
<212>  DNA
<213>  Artificial Sequence

<220>
<223>  genomic target sequence with 5'-flanking PAM (4 bp) 5'crRNA # 2

<400>  17
tttcaaccca gtatccttat ccgtcact                                         28

<210>  18
<211>  28
<212>  DNA
<213>  Artificial Sequence

<220>
<223>  genomic target sequence with 5'-flanking PAM (4 bp) 5'crRNA # 3

<400>  18
tttatttaaa catgatacgt atcatatt                                          28


<210>  19
<211>  28
<212>  DNA
<213>  Artificial Sequence

<220>
<223>  genomic target sequence with 5′-flanking PAM (4 bp) 5′crRNA # 4

<400>  19
tttaaacatg atacgtatca tattgagt                                          28


<210>  20
<211>  28
<212>  DNA
<213>  Artificial Sequence

<220>
<223>  genomic target sequence with 5′-flanking PAM (4 bp) 3′crRNA # 1

<400>  20
tttgtgggtg ggtggttttc acgtgtgt                                          28


<210>  21
<211>  28
<212>  DNA
<213>  Artificial Sequence

<220>
<223>  genomic target sequence with 5′-flanking PAM (4 bp) 3′crRNA # 2

<400>  21
tttcccctcc ctttgccgct gcgaagtt                                          28


<210>  22
<211>  28
<212>  DNA
<213>  Artificial Sequence

<220>
<223>  genomic target sequence with 5′-flanking PAM (4 bp) 3′crRNA # 3

<400>  22
tttcttcttc ttgcttccac cataacac                                          28


<210>  23
<211>  28
<212>  DNA
<213>  Artificial Sequence

<220>
<223>  Mutagenesis primer 1

<400>  23
tcagtgcagc cgtcgtctga aaacgaca                                          28

<210>  24
<211>  28
<212>  DNA
<213>  Artificial Sequence

<220>
<223>  Mutagenesis primer 2

<400>  24
tgtcgttttc agacgacggc tgcactga                                          28


<210>  25
<211>  20
<212>  DNA
<213>  Artificial Sequence

<220>
<223>  pSeq_CRBM4_F1

<400>  25
agcgcaacgc aattaatgtg                                                   20


<210>  26
<211>  20
<212>  DNA
<213>  Artificial Sequence

<220>
<223>  pSeq_CRBM4_R1

<400>  26
gatgaagctg aggtagtacc                                                   20


<210>  27
<211>  21
<212>  DNA
<213>  Artificial Sequence

<220>
<223>  pSeq_CRBM4_F2

<400>  27
aggaaggtta gcaagctcga g                                                 21


<210>  28
<211>  20
<212>  DNA
<213>  Artificial Sequence

<220>
<223>  pSeq_CRBM4_R2

<400>  28
tctcgtcgac cttctggatg                                                   20


<210>  29
<211>  21

<212>  DNA
<213>  Artificial Sequence

<220>
<223>  pSeq_CRBM4_F3

<400>  29
atgctgagta cgatgacatc c                                                21


<210>  30
<211>  22
<212>  DNA
<213>  Artificial Sequence

<220>
<223>  pSeq_CRBM4_R3

<400>  30
tagacctgct tctcaacctt ca                                               22


<210>  31
<211>  20
<212>  DNA
<213>  Artificial Sequence

<220>
<223>  pSeq_CRBM4_F4

<400>  31
accactcact cctcgataag                                                  20


<210>  32
<211>  21
<212>  DNA
<213>  Artificial Sequence

<220>
<223>  pSeq_CRBM4_R4

<400>  32
aacgacaatc tgatcgggta c                                                21


<210>  33
<211>  28
<212>  DNA
<213>  Artificial Sequence

<220>
<223>  Oligonucleotide fw for the generation of short 24-bp protospacers
       (5'crRNA # 1)

<400>  33
agattttcga tttcgattct tggattat                                         28


<210>  34
<211>  28
<212>  DNA
<213>  Artificial Sequence

<220>
<223>  Oligonucleotide rev for the generation of short 24-bp
       protospacers (5′crRNA # 1)

<400>  34
ggccataatc caagaatcga aatcgaaa                                          28


<210>  35
<211>  28
<212>  DNA
<213>  Artificial Sequence

<220>
<223>  Oligonucleotide fw for the generation of short 24-bp protospacers
       (5′crRNA # 2)

<400>  35
agataaccca gtatccttat ccgtcact                                          28


<210>  36
<211>  28
<212>  DNA
<213>  Artificial Sequence

<220>
<223>  Oligonucleotide rev for the generation of short 24-bp
       protospacers (5′crRNA # 2)

<400>  36
ggccagtgac ggataaggat actgggtt                                          28


<210>  37
<211>  28
<212>  DNA
<213>  Artificial Sequence

<220>
<223>  Oligonucleotide fw for the generation of short 24-bp protospacers
       (5′crRNA # 3)

<400>  37
agattttaaa catgatacgt atcatatt                                          28


<210>  38
<211>  28
<212>  DNA
<213>  Artificial Sequence

<220>
<223>  Oligonucleotide rev for the generation of short 24-bp
       protospacers (5′crRNA # 3)

<400>  38
ggccaatatg atacgtatca tgtttaaa                                          28


<210>  39
<211>  28

<212>  DNA
<213>  Artificial Sequence

<220>
<223>  Oligonucleotide fw for the generation of short 24-bp protospacers
       (5'crRNA # 4)

<400>  39
agataacatg atacgtatca tattgagt                                          28


<210>  40
<211>  28
<212>  DNA
<213>  Artificial Sequence

<220>
<223>  Oligonucleotide rev for the generation of short 24-bp
       protospacers (5'crRNA # 4)

<400>  40
ggccactcaa tatgatacgt atcatgtt                                          28


<210>  41
<211>  28
<212>  DNA
<213>  Artificial Sequence

<220>
<223>  Oligonucleotide fw for the generation of short 24-bp protospacers
       (3'crRNA # 1)

<400>  41
agattgggtg ggtggttttc acgtgtgt                                          28


<210>  42
<211>  28
<212>  DNA
<213>  Artificial Sequence

<220>
<223>  Oligonucleotide rev for the generation of short 24-bp
       protospacers (3'crRNA # 1)

<400>  42
ggccacacac gtgaaaacca cccaccca                                          28


<210>  43
<211>  28
<212>  DNA
<213>  Artificial Sequence

<220>
<223>  Oligonucleotide fw for the generation of short 24-bp protospacers
       (3'crRNA # 2)

<400>  43
agatccctcc ctttgccgct gcgaagtt                                          28

<210>  44
<211>  28
<212>  DNA
<213>  Artificial Sequence

<220>
<223>  Oligonukleotid rev fuer die Generierung kurzer 24-bp Protospacer
       (3'crRNA#2)

<400>  44
ggccaacttc gcagcggcaa agggaggg                                          28


<210>  45
<211>  28
<212>  DNA
<213>  Artificial Sequence

<220>
<223>  Oligonucleotide fw for the generation of short 24-bp protospacers
       (3'crRNA # 3)

<400>  45
agatttcttc ttgcttccac cataacac                                          28


<210>  46
<211>  28
<212>  DNA
<213>  Artificial Sequence

<220>
<223>  Oligonucleotide rev for the generation of short 24-bp
       protospacers (3'crRNA # 3)

<400>  46
ggccgtgtta tggtggaagc aagaagaa                                          28


<210>  47
<211>  24
<212>  DNA
<213>  Artificial Sequence

<220>
<223>  Primer pCRBM4_F1

<400>  47
cacattttat ccaatcaaac tttg                                              24


<210>  48
<211>  23
<212>  DNA
<213>  Artificial Sequence

<220>
<223>  Primer pCRBM4_R1

<400>  48
ccttcgagaa ataacatggt gaa                                               23

<210>  49
<211>  25
<212>  DNA
<213>  Artificial Sequence

<220>
<223>  Primer pCRBM4_F2

<400>  49
gtacagtgac ggataaggat actgg                                          25


<210>  50
<211>  25
<212>  DNA
<213>  Artificial Sequence

<220>
<223>  Primer pCRBM4_R2

<400>  50
ttagtggtca aacataggcc tttgg                                          25


<210>  51
<211>  23
<212>  DNA
<213>  Artificial Sequence

<220>
<223>  Primer pCRBM4_F3

<400>  51
agtaagaggt tgcctaattg agg                                            23


<210>  52
<211>  23
<212>  DNA
<213>  Artificial Sequence

<220>
<223>  Primer pCRBM4_R3

<400>  52
ttgccgctgc gaagttccct ctc                                            23


<210>  53
<211>  42480
<212>  DNA
<213>  Beta vulgaris

<400>  53
aaatgataca ggggtatatt tgactctatg aatttcagaa atctaatcaa atttgctaag      60

cttccaatga ttctactaag ccctacaaat tacaagaatt agttactttc atctctctgt     120

cggcttcaga accagaagtg tacaatatct tgtcaaacaa actctgctta gaggagctct     180

ttcgatcatc ttttttcgat ttggaagttc ccggtgatag gattgacatt gctgttttct     240

cggtcaattc ttctggatct tggttctgtc catctatctc tggctccatt aatctggtct     300

tccaattaat tccgatagcc tcagcttgct ctgcaaacaa gacctttgag atcggggagc 360

tgcagatatc cttataaact tcataaccag cagcacaggt tttcccacct tccaacaact 420

ttgataaagg atgtaggaga gagatagaat catcactcgt ttctaaccta tccttcaagg 480

caaggaagtt aacagccaag tctgccttac taaactgaac aaatactgca gtttcatcca 540

agttatagat gcaagcaact gagtatatac caaacacttt acagatgcat tgttttatgt 600

cacttgcctt gagttttggg gagaatcccc aaatcaaaac tatgttagga tgcaaaatgt 660

taagaaacct ccttttagct gaactgataa cgggaatttc attcatatca ccagtgctta 720

gattgatcac atctccactg ttccaactaa gatagagcag attggcatac ttgtgaatat 780

tctcactggc tattaaattc tcagaagaaa aatcaacacc aagattatga catgcttgtg 840

caaagacaca cccggtcatg aatgcatcat agccagcttc atgcttagcc ccagagttcc 900

aatttgagga cctgcaagaa aacatgggag taagatggtt tcacataaaa catgtgtaga 960

agtgcagtga acactggcga aaacaatcta attttacgaa ttcattcact cactcagctt 1020

caaattaagt ttccccttta tttagggtgc cccaaaaaga tacactcttc tgtttacctt 1080

ctctctccaa gcgaccaatc ttttctctct tctccaacat cgttttcttt ttctctctct 1140

acccactatc cattttgtcc tcctacattt gataactatt cttaatctcc aagaaaatcc 1200

aatgtgtgaa ataattacgg gacagggagt atacagaagc agcccccttg ccaatatagt 1260

ttacaaatta ccctcagaat taggcttacc tttcccaaag gagcaataaa ttcaaacaaa 1320

tctaaaaggt acaaggcatt aagtgccgaa cctcatgtca tcaacctgga cctccacctt 1380

cacacatgga tgtacaccac cattagagga ttgtccagag gctatctcag ggcacaacag 1440

agaaaatgct gaggccaatg acgtgctggc tttattcaag aatttttgaa ggctcgtgtc 1500

tgcattcaaa agtattttcg tgtcgacaac atgaggaaaa tacttgtgga tctcgagaac 1560

aaactcttca acagttgatg gaagaggacc aaagaattta tggtaaatat gtgccatatc 1620

tgcaaaaaat tataatggat aagatgacaa gaaaagatac taggaaggcc ttcaagtaca 1680

aatattatat catgatgctg gacgaccgat gctcccacaa ttatgtttgt taccaaatgc 1740

ttcgaaggat aattactaaa ttatgtgaat ggtggttacc aagtgtcccg gaccatgcaa 1800

taacttctcc tttcagtgac caacaagaag aagacgtacc taaaaagcaa ttgtgaccta 1860

caattagctt cttttcagca gcgagaaggt caaggacatg ccggaaacct gcagctgctt 1920

ttattttgcg agttgcttgc tggtgagacc catacttcac ctcctcctac aagaacaaac 1980

agaacaatca cacatgcaga aagttcccca cataccaagt tgctgtctgc taaacactga 2040

aactaactta tctctacaaa caatgaagga agttcctcac cagaaggttg atcttatcat 2100

tgtcagattc tacaaaaaca ataagcttct gcaagatggc actgccgtca tgagcacaca 2160

caaaaacaag atccttgaag tgcttccttg taacctgtaa ttgcagatca ttagtatata 2220

ttcaagatgt tataaattta ttgaaaagca gcgtctaaaa caataaaagt catgcttaag 2280

gcatagagcg atagagcata gacacttcag agtttaataa gagcaaatac tccaggagaa 2340

cataaatata tttcatatca caaatcctag taccaactgg caacggctaa ctgccaattt 2400

atgtactgct caaaaaggcc aagcatctaa aagatggctt aaaagtcgga ttttataaga 2460

aagtcgtcac atgattgcta ttacattgac atatcaaagg tcaaatgctg aaatttggtt 2520

cagcttgata tatattaagc atacaaacga tacgttgaca agaaagccta acaagacatg 2580

aagcatcagg cacataacat tcaaaagatt accaattcaa tcaacttcag ctgatgagaa 2640

gtaaatccat tcaatcgaag agcaggacgc atactaaaaa atatggtttc aaattgttgt 2700

ttagattcaa cagcacttgg aagctcggca tttctgttct gtagcaacat atcatgccaa 2760

tcactgagcc gaattttcat ccgttcggag aataaaatat cagctacatt gcccaaaggt 2820

aaatctctaa cttcattaga atatgcccat ttcccgttat atactgaatt caagcgactc 2880

aaagcctcgt cttcctgtcg tctagataaa taagacacgc ctgagattat acaatgtgta 2940

aatttaccac aataatgaca tcatactgac aaaatctcaa acaaatagtt ctaataaagt 3000

catgttatct gaaatttcta tgaataggaa attgaacaaa accttcatgc ttttttccaa 3060

ctaaaattga catcttctac attaccttca tgtatgcatg cattgaagtc aaactggtat 3120

tttgccaaga agtcaatcga agttgtttgg cacaggaatt catatgatgg gccatcagtg 3180

ggaagctctt gacgtggaaa tatataaaaa ttatgcctga caatgaacca ttgtaaaatt 3240

attagatgga gtatctctat ttattgttta cagccaattg agcttttaac aattactata 3300

ggtagtgttt ggaaacttgt atttcatttc aaataatgga attgaaatct ggaatttaaa 3360

gtttgtattt caattcctaa tcactgtttt gtaaaggggg tttgatagaa gagagagaaa 3420

tagaggttta atggaggaga gagaaaaagt gtgggtttac taaaaaaaag agaaataaat 3480

attagaaagt gtgggtttac tcatagagtt gggatatgta tgaggagaga attttcaaat 3540

gccaaggtaa tagcttgaat gacaaattta ataatttcaa attccatgtc atccaaacaa 3600

tagatttcat ccaaatccaa gatttgaaat gaaatcttgc tatccaaaca tatcataaat 3660

taattagtaa tttagacttg ctttctgctg cacttactta tggaaataat tttacttcag 3720

tccttaaata acccgcaatt tacatcaaag gcactaatat aaacacctag ttacgaaatg 3780

gaaatatcag atatacctgt aaaagtaaag aaacaaaaat acaaccctga gcatgaaggt 3840

atccttcaaa agtgcaatat ctgcatactt agaaccggaa ttagaagtgc gaatgcagac 3900

aataaccatc ccaggatcag aaacgtccaa gaaagttgag attcatcatc cattctcttt 3960

agcaaattta tgaactctaa tatataaatc ataccccccc ccccatccaa aagcaattgt 4020

caagctgcct gaacccctca taatttagga tacaacaaag taatcctaaa agacccttta 4080

caatactagt actcgggtat ttccacaatc ttctcatcat tgaatccaaa gcattgcatt 4140

tgaagaaatc aaatcataat ccattactat attagagcaa aatctatgtc attatagtat 4200

tggagagcaa gtatgactat taccccttta cactaggcaa aacacattgt cacaatgcta 4260

acttagtcat taaccaatat caatatggga ctgtggatat tcataaaatc gaagtttttc 4320

gcttgctcat aaactatctt tcattccagc acagtacaag agagaaaaga cagcattttc 4380

atacacttct ttctttagtt caaattcaca cagcagcaaa aaattcactt cttcatagct 4440

ttagctcagc aaacaaagca caaagcatgc aattactctc acacatagca caccaaaaaa 4500

acaaaaacca ctaaaaattc acacaaaaaa aaccaacaaa aattccatcg caatttcaac 4560

aatcaaaaca atcttctaag ttaaaaagag agataaagat gagaagaaaa actaacggat 4620

gagcaacgaa ggaattcttc gaagaatccc atcgaaatgg acaaacacca aattgaacaa 4680

cggcgaattt ctcagcagaa tctttcattt taaggtatcg aacatcgtgc cgatcaaact 4740

cgaacgattc gcgccaaggt gagcttgtaa ttccagtcat ttcgagatca atggcgacaa 4800

aatcggcaga ttttacatgc gtagtgaggt caattagggt ttcttcgaag tttgattttg 4860

ttacattttt caaagagaaa ttagttgttg atgttgaata atgatgataa gtagttaggg 4920

ttcgtagtaa ggtggaggaa agagaaaatg gcgtcactct gacaagcttc ttcattttgt 4980

tcttcttcct tagctctgtt ttcagtcact gcgccatttt tttaaaaaaa aggaagatga 5040

acaaagcaaa tattgaaccc aaattttgta attttggccc actttatatg tacccctccg 5100

tttcaaaata tggagcacgc cgcacacacg acatttaggg tcgaattttg aacattcttc 5160

aagatgatct aatggtataa tctctataat ttatatgtgg catattataa taagagtttt 5220

atgaagtcaa aaagtggatg tcatatattt aatgcatggt aagtttttcc taaatctgta 5280

tactagggta acatacatat gttgacttga agtatatata attcttgtag tataaatatg 5340

gctttggcca taagtagtaa tacacaacaa ctagaaaaat tgaaatcagt ccactgttat 5400

cttgtactct ataattttct gtttcctttt gtttcgcaac aaagacatat ttgtggtgaa 5460

agataatttt cgtaaattga atgacttata ttttgaaata aagagagtat taggtaaggt 5520

tacgtgcttt tcgcttgaat ttgttagacc tcaaatgtat atgtgattag aacggattgg 5580

ctctagtttt tattttatag aagtatatat gcatttttct tagagcacac tcgaaattac 5640

tttcggatag atatattcgg gaaaaaaaga ggttgaaggg aagttcatca ataattatgg 5700

taaaggaaaa aggacatcgt tacaattcta aattctagat aggatgtgat gataatccaa 5760

aagtcatctg aaaaactaaa caagtccaag atgctaatga ttcgagtaga gattgaatga 5820

gtgaccctaa ggattgtcaa ccctcttatt ctaacgtgtg taaaagaatt gacaactcta 5880

agagttactc aaacattttt cgattcgagt ggttaatata ccaatttgaa actattgaca 5940

ggagttattt taatgagtat aatggtcaat ggagcactga attccatctc acatagtcac 6000

atatttcatc tcaagttctg atgatttcaa acattgaaaa aagatgatac aagcaattaa 6060

ttcctaggga aacatattgt ggttttcatg gatacaagag tgagaataaa tcaaaactta 6120

ggctctaaca tttcttttct ctactagtaa ttgctaatta tatcaattca attgtcagtg 6180

taatcagtta atcaccaaat ctcttgtata gtcagtaaac tatacactgt ttagtcctct 6240

ggattttgcc cggtcgaatt atgcagcata accaaacttt gaagtttagt acttcctttg 6300

cacccaagtt agcttcacgg cccctgcctt ctggtggatg gtcaccctat gctttgagca 6360

ttctctgcaa tgcgcacgat attcaatgag aacgtcgcct tgaaaatcta aattgcaact 6420

aaaaattaga ttgaaatgaa acccacaaga gttgtttttc tgagtagttg gtgtagaatt 6480

cacaagtctt gctccattgt ttgaagatat gaagacaata atgtgctatg taaagtgcag 6540

ccgctagcta acagtggaag tggaaacttg atcattttac actcgcacaa gcgaaagctc 6600

ggctgacgtt gcaaactgaa gaaaaacctc tcaaaccaat tcgacttttg ctcaaagttg 6660

caaactaaag aaaaaggctg aatgcaaagc aagttcacca atgaacaata gatcggtgtt 6720

ggcctgaggc cacatcaagt gaagttgcct aattgcggcc ctctcatctg ttcacaggaa 6780

tcattttcca tatagaatca ctccaaaata aaagagcaaa gctgcaccag atgcagaagc 6840

ataactttca agacaactga tgacagataa atagcaaaag aatgcttaag aaatgatcaa 6900

aattgaatgg ctctggaatt acctcatcag ctgattttcc tttctctcta tctctctatc 6960

tctttactcg tctatggagc taccacatca catggcgttt catatgcttt ctgccgtcga 7020

actagacgtg cagcaaaagc tccatccatt gaatgcttca ctgggcatga gcgataaaac 7080

ccatcttcag ttaaaaagtc agatggaaca tatctgctta cagaatctct ttggaagtcc 7140

tatcacccaa caaagaatat attaaaatag agaaggagaa aagaacgtat ctatctgtca 7200

gcatccatat gaggtggaaa ctaggagtac tatataaagc cagtgcagta gctcctaccg 7260

gatgtctaag aaggaaggca gaaaccctat cttcgttttc ttcaagatca atggagcagg 7320

tactgtacac aagcacgcca tctggtttga ccagcctgta gatgttaaac aatcccacag 7380

acaaaaggga ataatatgag tgaaacaagt caacaggggg aaataaccaa taattctagg 7440

actgtcaaac tcaagctctt caaaaacaaa gatagctctt aatctcactt gcaagcagca 7500

tcgaacagct cgtcctgcaa cttctttagc tcttccatat cctctgactt tctattccaa 7560

cgcaaatccg gcctcttcag caaaaatata aagttggaac aaggctctta gatacaagaa 7620

ctgaaaaacc ttcgacatat aatggactct atcaagggca caatgacaaa ttctaaacat 7680

gagcatgtat atcaataaaa tactaagaac cctttcaatg gtactgctag aaggtttatt 7740

gctacacttt ttagtacacc atctataggt tttatagtac catcaaaatg gttcatggtg 7800

ccataagaaa attttatgta tttatggtac tatctaccat atctaatttt ctctgtaaaa 7860

atgtatttgt agatagagac cacgagttcc tcttttagat actgactttt ttttttctac 7920

atgatggcca acagacttct caaacaaaaa gaaaaagaaa atatttagat aatatgagca 7980

acaaaatagc aaccacctac ttttgatagt acacccaggc ccgaacaagg aacatctaaa 8040

agaactttat caaacttcga agtgttgctg tcctgaaaag aatagaaagt aactgcttca 8100

acaaagaaga agaggcagaa agcaaagcta gtacgcattt tgcaatgact tactgaaaag 8160

gagcgaagat cagcatggat gcaagtgatc acattatcaa cacgctgcag cttggctgtt 8220

tcttcaagta tccgtaaccg acctttattt atgtccattg ctgatatcat acctgaaaag 8280

ctacacattt agaatgcaga accagcatca ttggtagtta agttatcact ataccttggc 8340

cattcaagcg agatgccatg aagagtgtct tccctccagg agcagcacag caatcaatga 8400

tgtgatcacc aggctgtgga tccagaacag aaacagctag acctgcagcg aagtatagat 8460

gtaaacttgg gttgggctgt cacatttttt cacatcttat cttcctttct attctttcaa 8520

aactgaggag aaatggttgg gatttctata aacgtgagaa aaatggcatc agattagatg 8580

gttttactgc atgaaaaaaa ttgaatgtgt ttcggcatca cattactaca aggtcaaaag 8640

cactatcttt gaaaatgtag gacataatgg gacagagatg tgctgacctg cactctcatc 8700

ctggactgag cataaacctt cttttagaag tccagtttgt atcacaatct aggatatgag 8760

aaaacaactc aagatgtaat tgctcctaag atatcaatca tttcataata aacataaaag 8820

ttattattac aagacagcac ctgcatccca cttctgatgc agacaaagtc atccaaatgc 8880

aaggaaggct catgcgggac ctgcggacaa agtgttgtga tgcgcataga tatcgaaaga 8940

aggccctgta tagcactaat gagataagat tcagtaacct tcagcatgtt gagcttcaca 9000

acaaggtcat ctcgagttaa tccttttgca atattggccc tagtaccaag aaaaccatat 9060

gtattaacaa gagaaaagtg gcatagggat ctttatgact taggtaagca gttggcaatt 9120

agagagaata aaacccccaa acctcaagct gaaactcgga acactattgt tccacatcat 9180

caatttgata gctccttctt gcccaagata cttggtccac cgtcttacca tccactgaaa 9240

ggaaggtatt aaaagggaga aaagactcgt cagcatagaa aattgtacat cttaaatttt 9300

agaagtatag caccatcttc aggcatcagt caacgtaaat aaataccaca tctacaaata 9360

gaaccatact ttctggacag tcgggatcat gagcacagac aatactgcat gattattgcc 9420

tcgtattctc atgtatacaa gtatatgtaa cattaaatag cagtatttct tgagaaactc 9480

accacgggat gggaataagt tgtagcaagg gcacgtgctt gtgaacgatc atcaccctcc 9540

aatttgggta caggaaggga gtcattatcc tataaagaga aacagctttt gttttcaacc 9600

atatcaagac aaacagttta ttaaactata aacaacaaca atacacatgc acacacctac 9660

tgggaacaag atatatacta ctgataagta ttttctgatt gaagaaaaaa aatctcattt 9720

atttgcaaat atagatttaa tgacaagaaa gctttgaacc ttaaggaaaa ctagctttcg 9780

gaggatccca ttcaccatgt ttcctgcgcc tggtctaaga gcatacttgg caagattcac 9840

attctgcaat ataatccaac agtaagaaca cgacatggat ttagactcaa gtctctgaac 9900

ctatagaaca agtaaaatta gatcttatct catttgacaa tttaaaatta gatagtgcaa 9960

tattctgcag ttataagact tcatgtgtgc atactgcaca agtcatctta aaggtgttat 10020

taaagcttta attgccattt gacatcccct tgctcaactt tagcatgttt ttaggctaca 10080

acaatacgca ctgtctacat ggacatacaa attacaagcg tatggaaaag caataagcgc 10140

aaggaagtct tcagccagaa actctctatg agtccaacaa tatgcaacta aatatccaag 10200

taccgtgaat gagtaagaac taacctcgtc aacaacagca tatggtggca tttccagttt 10260

cacaatctca tagcatccaa tcctgaggat ctaaaattaa agataaatca atacacaaca 10320

tatgatatgg gtcggagcgt atataacaag tatagcaact acatttgaac agataacagc 10380

ctttgagaca ataaggaact ccgacattcc agtatatgcc agatttcata tctttagctc 10440

taaattgcca cgcaaaatgt tattgggcaa tatacctgta gcaggagagg ttccatgttc 10500

ctaaaggagc tttcatcatg gcatgaagaa acaataagat aatccagata ttttctccaa 10560

cgaattgaac caccaacaat gtcagtgacc tacaaagaca agttgtcaac ttaaaacttt 10620

tgaagcgtca tttcacttct gtagaccaat acaaaagcta ctactgcttt acatcataaa 10680

acctttagtc cttaggttca tctgattggc aaaaaaggtc cagatgcaag aaaagcaagt 10740

agctgtaatg ctgtattata tcagcattat tcagaacaga ataataaata tctacagatt 10800

ttgggtggaa gcttgatgat agagtatctc cacaaagaga actcgcttga gtcccaactc 10860

ccaaatctac ttttttggag tcacattatc agtcattttt tctggactct tataggaata 10920

gtgtgctatg taatgattta tggagcaggg gcatttcatg aatagcttta taagttagta 10980

tgggtgtctt ggggaataag ttaaagggtt agttagaggg aagaagtaca acatatatat 11040

agagcttttg taagaagggt ggttatgttg aaaatagatg agaaattggg tgagctcata 11100

gtagttcaat ttggactttg ggagagaatt aagcctcttg aaagcttgaa tatcatttac 11160

atttgttgtt tttactctta ttaatcaacc aaagttcatt ttcttccttt aatttctcca 11220

ttttagcact atgatttgtc caagctaagt gatttcttag catagtgcac agtgtagtat 11280

atcggagaac tcatttgagt cctgaaaggt cccacaagtt acatttttcc tactactact 11340

tgcaccaaaa caataagcat cattaagaca ttgtcactgg tccttcttag gttcttttgg 11400

aggggattcc tcagatgggg gaggcaccca tgaaggaaca tgttaccaag caatgggaca 11460

atgcaaaatg caccaataca gtagcttcac ttcattgatt gcatctatgt cacggaaaac 11520

tgaagaaaga agcaacacct caactttatc caggacagat atccactaac ctaggatgca 11580

agcttgagac tatttagcaa ttgcctctgg gatattaaat cagattacga ctatatttct 11640

acagttattg cttaagaaaa aggtacgatt tgaagcttgg gaagaaagag aacaagagta 11700

aaagaccaat ctgagatctc tttcatccag gtctctggtg cgaaatccaa gagtcctctc 11760

aacatattcc atctcattgt tccctgaacc ctttcctctc tcatttagaa gatcagcgaa 11820

ggcaccacca aactctatcc gcatcaatct cacagcagcc actggattta cacatgaaag 11880

caaaccagga gaaccataaa aatcacaaca aacttcctga tagcctactc actagcatca 11940

accattgtgt tcagcctaaa atgagcggct gttttcaatt gaacagcaac ttacatggac 12000

cactgcataa aagtgatttc ttaatccaga caaacaaaaa tgtttacttc aaccaactga 12060

atttgcatca gctcattagt gatttgacaa gttctaattt atgtatcaac aaacaagacc 12120

atatagctag gaaacaagag gcttaggcta agcttaatgc gtgaacaatg ttagatttca 12180

acctatcagc actgtggata actgcaaact gcgacttaaa taaggaagat aaaggaactg 12240

aatatgcaat ttcaaggtgc tcagcatttg aatcaacagt tacttcagat aattcagaac 12300

ataaaagatt tgaacattct aaggctacct catgattgca agcaatgtta cctgattcgc 12360

taaccctcac aagccacaag ccaaagaagc aatttggtaa atggttcatg gtacaactgt 12420

tcgcttttgg actaatctaa caatactagg tggtaaatta tgttcccata tctattacca 12480

taatgtacag caaattaggc agcactaatt ccaaatgacc caacaaaaaa agaggaagaa 12540

aatccaaaaa ttcaagccaa catatgcact aaaattacaa gcacaaaatc aaataatgag 12600

aatcacacta tccaaagaaa atttccatcc acatttatcc aacacaatta tctctctttt 12660

acacccaaat tatgtcaacc aaaaacacta aaacaagtga gtgcagtagc ttcacatcaa 12720

agaatatcaa tcacaaacac cacataataa aatttcaact cctgcccaaa caaaaaaaat 12780

ataaagaaaa aaaaacagca aaatttcaaa gataaaatag aaaaaaaaaa atcaaaatac 12840

agggggaaaa aaagtaaatt taccagctct atgaggcgaa acctgcaaat tcagcttctg 12900

ggttttctct gaaatatcaa gcacaataac cagcaattaa aaaaaattat aaataaaatt 12960

aaaaagaaaa gattgataat taaaatcaaa agagagcaat ttaaagcaca atcctttttt 13020

taccattttt tctgggagga agagcatcct tcgttttggg tttagacgaa aaaaatgaga 13080

gttgttgtat ttgtgcgcat gagtgatcat tgctggaaat gaaagtggga aagtggtaaa 13140

tgagtgcttt gtgaaattgg gttttgagga aaagtagaaa gaagaagaag ggtcgatgtc 13200

agagaagaga gagagtggat ggaaagtagt gatgattgcc tccattgttg ccggtgaagt 13260

gagctttctg caaatatttc actggactag ttttttttag cagataacgc taaaacagag 13320

aaagatgttc ggttaatttt aatttttgga catttaaatg actattcaat atgtttcaac 13380

cttttttttt taaaacaaag gaacaatact agtattagat tacgttaatg tttagtacat 13440

ccaatactta tgtgtgtttg acctaactta aaatcgtaag ttgtttaaaa tgtcggtgtc 13500

ttgtttttaa gagatatcat acttactatc tttggttttt actcttccat tgttaacaga 13560

aactgtattt atttgggtaa ggggtttgag tgaattcctg taagtatgag aaagttttga 13620

gtgaagcaag agaaagagag aagaaaggaa cttcgagtga agattgagag aaacaacagt 13680

tagtgggaac tgttgttggg aacttgagtt taggagctca ggttgtaccc cgagagaatt 13740

aataggtttg taacagagtc ggtggcctat tatagtggaa agtttgagtc aaaatccatt 13800

gtggccgatg tcgtttcttc ttattgggcc taggaagttt ttcctcgcta aaatttcctg 13860

tgttcccatt gtgtgttcct tagctagctt tcaattccgc aaaaagttac gtttattctc 13920

tcactataat tcacccccct cttatagtgc tcatattata caacaattga tatcaaagca 13980

ggaactctaa aaatacagaa atcatgttga gttcaagatc ttggaaaata tgaatactac 14040

agaaaaactg gaagaaaggt actctactca gagaccaccg atgttcaatg gcaaattcta 14100

cacaaactgg aagaactgaa tgaagatctt catcaaagcc gacaaatatc aggtttgtag 14160

aatcatagag gcaggcgatt ttgaagtcac taccactaat gacacatatg aggtaattcc 14220

taaattcata actcatttcg ataaagtata tttcgaaaag ttggaaatta acgttcttgc 14280

tattaaactg cttcattgtg gtcttagacc tcatgaacac aatcatgtca tgggatgcaa 14340

aatcgcaaaa caaatttggg atcttcttga agtcactcat gaaggtacgg gtaaagttaa 14400

gagatcaaaa atcgatcttt taatgaatca atatgaactt tttcaaatga aatataagga 14460

gtccactcaa gagatgttta cacgctttac taatactatt aatgagctaa cctctcttgg 14520

aaaagaaatt acatatgatg aacaggtaag aaaggtccca aggatcgttg gatggctaag 14580

gttacgcctt acaaaaaact aaggacttta cgaagttcaa tccggaacaa cttactggct 14640

cccttatgac tcacgagcta cacttggaca ctgagaatgg tgacttgtcc aaacagaagt 14700

cgattgcctt gaaagccatt tttgtcatac cgtcaattaa ttaagtaaaa agtggtaaaa 14760

gaattaccaa aaacgcacaa aataaattaa ttagttggat ataactaatt aacctattcc 14820

ttttttctgt cgctataact acttttgctt aacttattga tggtttgatc gttgaatcca 14880

agttttctcc acccacaaag atattataga ctttacttta aaaggtacga taaataatgt 14940

ttaatcaggt atgcatcaac cttgaaatta ttaatttatt aagatcaaat tatgcatatt 15000

tatattaaac gtacaggact tgtgcacaat ccatggatga tattgtagat tttgttgtaa 15060

aggagttagg gacaaatgat gttgaattaa gaatgatgag gaacaacatt gaggtaccta 15120

atggcataca agattatgtg gtaacaaagg tgaagaagtt ggttgtacca ggcaatacag 15180

cagcggcaag ccatatatag gatgagctac cataccctta tgttgtgaac tattgtcacc 15240

accaacaaga cattggtcat tacgacatca ctttagttga ggaatgataa acctcttttt 15300

gctagatatt tgcaaacatc tagcagataa agaggaataa aacactattt atatttcatg 15360

aacactattt gttagttgca tgaacactat ttttagttac acgaacacta gttttagtag 15420

catcatgaac actatttttt agcatcggaa ttttcacgac tactttttgg tttgactgac 15480

actctgcaat tttcgagata actttttggt gatatgggtc ccatgaaata gaagatttat 15540

atttcatgaa cactatttgt tagttgcatg aacaatattt ttagttacac gaacactagt 15600

tttagtagca tgaacactat tttttagcat cggaatcttt gcgactactt tttggtttga 15660

ctgacacttt gcaattttcg agataacttt tttgtttgac tgacaactat ttcctatata 15720

tattgacagt tttacccctg ttagatgttt gcaaacatct agcaaaaaga ggtttatcat 15780

tcctccactt tagttagccc aacctccagt aacgccatcc agaccactgt cgtttgtcac 15840

tacgacactt acgcttggca accctatgtc ctagcccttc gatacctcga tatccgtccg 15900

ggcaatgtcc ccagtttgtc acttctctgc cattaatgac atattttgga gtatcaaacc 15960

caactccaag tatatatcgc aacatggctc agtaaagaga gtcatataat catgacgtag 16020

tttctatatg ccatcctacg tagtatcttg taacatgaat aacagcctgg tttgcaggtt 16080

gatggtacat ggtataaatt ggtattactc cctccggtct ttattagttt aatcctttct 16140

tttgtacaga gttataggag aaataatatt gtgggtcata gaaggaaaga gaaattatta 16200

ttttatgtta aagttgaatg tatgtgtgat gaaaagttag tagtcccatt tcaaaataga 16260

aaaaaaaaag gtaaactaat aagggacatc ccaaaaagga atacgggtaa actaataaat 16320

atccatgcag gttgttggta catggtacat gaagccgtcc aaaaccttca aaagcagtaa 16380

gtcctgctgc tatgccatat tcaaatattc aactccaaaa aaaaaaaaaa aaaaaatcaa 16440

aaatccgctt ttcagcgaaa atataggaaa taatccaaga atcgaaatcg aaataaagtc 16500

atgatgcaag tttggagagc tgaagttaca ctatatcgga gtacttactc aaatgttgat 16560

tagtactccg tgcgtttgaa gtaaagtcac atatggagta gttccaagct aggttgtaca 16620

gtgacggata aggatactgg gttgaaaagg tgaacgtcga gatttatacg tgtatttatt 16680

taaacaggat acgtatcata ttgggttctc atacgcgtac cagctgtgac ttagaaaaat 16740

taaccacgct atataggttc caagccctca tgattacctt ttcatagtgt aaatttcatg 16800

tagttgaatg gtgggaatcc aatcacaaaa acactgcagg taatggaaat gttccaactt 16860

tttccaagca ttttaaaata agacatgtga ttactaatta gggcgtgttc ggcaacagta 16920

attgtggtga tagtttttag ctgtgagagt agttgttagc tgtgctatta gcttttagtg 16980

gttggtgtgt agctgttagc tgttagatgt ccaagtagcg gtgtaaaata ttgatgttcg 17040

gtaaaagaag ctgtcaaagt agctgtctaa gaataactag ttaaaaattc aaataaaact 17100

ttaacatata atttatacac cactaaaagc tacccaaaag ctacaaattg tagcttttga 17160

caaacactac taaaacacta cttgtaccac taaaagctac ttacaccact atcttgccaa 17220

acactcttat tttttctaat tagtgttttg acctagtcaa gacactaaaa gctacttaaa 17280

aagcttgtgc cgaacatgcc aattctgaac caaggaacaa actataacaa aaaagtgcta 17340
tgtgaaactt ttgtaggcaa cagaagtaag gcatttttgg aatgtactaa caaatccgta 17400
ttaagacttg tacatgaaaa ttaccgtggt aacatttacc cacacttcct cattcacgta 17460
ctccgattca ttcttataag ggcataaccg cataaggcac atcaagatcc atgtatctaa 17520
tagtttaatt tgcctctgtg tttctgtatt aacaatgagc atagtgagtg caaaagccat 17580
ggaagctaga ttaaaaaggc catcattcta agttagacaa ttggaaacaa catcgagata 17640
cacgtacaca taagggctgc tcttctctat tactccctct gttcctaatc atttgctttt 17700
ttagcgggtt ccaaaggcct atgtttgacc actaatatat ttaaattaaa actggtgata 17760
tatattaaaa gaaaattatg atgaatttaa caaaaaccat atatgttatg tccttttttt 17820
tcctatatta atgaattttt acagtcaaag ttggtgaact ttgacccaaa aaaagaaatg 17880
gagcaaaaaa aaaaaaaaaa aaaaaaaact agggacaatg agtaacattt ttatctatgt 17940
ctttttaata tgaatatacg taacaaattc tgcaaaaata gagatagcaa ctaataacac 18000
gcatgaaaat gacaagttat attatacctt tttttctcaa tatatgaata tacgtaacaa 18060
attaactcca gtagttttta gtaaaactat tagattattg tgtaacatat actctggaaa 18120
tagtactaag atccattaca atctttattg agaaatttcc tcatgtaccc cctgaggttt 18180
ggcgtaattt ccaaataccc ctcatatttg aggaatttct caaataccct gatgtttttg 18240
tttagactca aaataccttt actatggaca gtaccctaat gtcattaagt tttccccttc 18300
tctctcccca attttctctc tcctcccatt cccccaccca ctacccactg cccactgcca 18360
agtaggggtg taagtggatt ggactggatt ggactttgcc aaattcaaat ccagtccaaa 18420
gttttttgga ctcgagaaat tgagtccaag tccgatccaa atattttttg agtccagtcc 18480
aatctagtcc gataattttt tcttgagtcc gaatccagtc cagtccagtc cgattattat 18540
atcttttttc ccgatttagg ttcaatgatt cacaacattt tttgagatgc ttgagcattt 18600
gacatctgat tcaattatca atatccacaa ataagattga aagcttaaat taaagtaaaa 18660
tactatgaat aaaaagttga attagatgct taccttgatc taagttgaga ggaagcatag 18720
agactgagaa ttaatctgag ggacaaatag agaatgcgag agtcgagaca gtgaggtaga 18780
aagaaaatga agagtaagag gaagtgagta ttaaggactg aggagtaaag taagatagaa 18840
ttagttggct actagcctac taatgcagta ttgctagtat aatttactta tttaacaaat 18900
ggagctaagt gcaatagttt agcgccaatt gacatattta gagagagaag gctgaaaaat 18960
ccaatatttt taaaatagta tcattatttt taatatatac attatatata aaaatatttt 19020
tggactggac tggacatatt ggactccaaa gggatgagtc caaatccaga caaaaaatat 19080
ttggacttga aaatttaagt ccgagtccag tccgaaaaat tttcagtcca atccagtccg 19140
acaaatttgg actggactgg attggactct gaacttttcg tagtccgctt acacccctac 19200

tgccaagtgc caaactgcca accccctttt ggttgagttg atatttgacg caaagacttg 19260

gcgtgttgga aggttcatta cacattttat ccaagtcaac tttgaagtct tcttagctag 19320

agactagagt gaacgtgttg gaaggttcat tacacatttt atccaatcaa actttgaagt 19380

cttcttagct agagactaga gtgaacgtgt tggaaggttc atgttcatga cattataaaa 19440

gtaataatag tgaaatttca caaagtattt ataaacccag gacagactca agagctctac 19500

ttattattag tgaaaaacaa acatacacac gacaataaca caacataaac aataatgaac 19560

atgaaaatcc tccttttgtt tgtcttcctt catcacctcc actacttcat ccatggcaga 19620

acacttacag aacgccaagc tttactaagt atcaaatctg ccattactta tgattattat 19680

aactctctct cctcatggaa aaacacaaca caccactgca gttggccata catcacttgc 19740

tcctcctctt cttcttcttc ttctgttatt tctctcaact tcaccatgtt atttctcgaa 19800

ggaattctct cccctgatat aggcttcctc accaacctgc aaaacctctc tattcgatct 19860

aacctttttt ctggcccact cccccattct ctctctctcc tcacccaact ccgctatctc 19920

gacgtttccc aaaacagttt cacaggtcca atcccatctt ctctctctct cctcacccaa 19980

ctccgctatc tccacgtttc cggcaacagt ttcacaggtc caatcccatc ttttctctct 20040

ctcctcaccc aactccgcta tctcgacgtt tccgacaaca gtttcacagg tccaatccca 20100

tcttctctct ctctcctcac ccaactccgc tatctcgacg tttcctacaa caatctaaat 20160

ggcactcttc ccttatcggt cgttgagaag atgtcggagc tcagctacct taaccttagg 20220

tataactctt tctacggtga gattccaccg gagtttggga aacttaagaa gcttgaaaca 20280

ttgaatcttg gtaacaacac tctttctggg agtcttccat ctgagttggg ttcattaaag 20340

agtttgaaac atatggactt ttctagtaat atgctatttg gtgagatccc acaatcttat 20400

tctcttcttc gaaacttaat cgatattgat cttaatagaa acaagttata tgggagtata 20460

cctgattata ttggagattt tccggagttg gaatcacttt tattagactc gaataacttc 20520

acagggagta tcccacaaaa gttaggtaca aacgggaagt tgcaatatct agatataagt 20580

aacaacaatt ttagtggtag tttgccacta agtctttgca aaggagacaa actccaagat 20640

ctggacgcat cctataattt gttggttggg tcaattcctg agagtttggg aagttgcaag 20700

tcacttgaag gagtgtacat gggaaataat ttcttaaacg ggtcgattcc taagggcttg 20760

tttgggagtg atgtttcact taatgacaaa cttcttagtg gaggtctcga tgagaaattc 20820

ggtgattgcg ttaatcttcg ggacattgat ctctctaata ataagctatc agggaagtta 20880

cctgcgacca tcggaaactg tattcatctt cggtccttga cgctttataa taacacctgt 20940

accggacgta tccctcaaga gattagcaag tgtaagcagc tacagaccct cgatctcagc 21000

caaaatcagt tctctggtgt gatacccaat gatattacag gtaagaaagt atattaaact 21060

tgttactttt gaaaatattc gctctagttt ttgtttcagt tggtccattc tcactttgta 21120

ttattgaaat atatcccaaa aaagtaaata taattatata aaagaatctt gctaaaaata 21180

atatgaatta tttttgtatg tgcaaaataa tgtacaaatc taactaattt gttgtggata 21240

ataatattaa ttgtgtgaaa tagtaaatgt gtggagatat ataactttat ttatcatatt 21300

cactcaggtt tttaggtatt tattatgagt tttgcattgg agatatccaa cttgacaata 21360

gtatttttgt aatataccaa tatataaaga ttactgtaca taaccaaaat gtatactttt 21420

cttattttta taaacttata tattcctctt ctttgtattt atcacaacat tttttatacc 21480

cttttgcctc atattaatag caacacttat aatttattta tttacttttt atttcttggt 21540

ctataacctc atctacccac atatgacaca ccctataaag gacccacatg attaaccaaa 21600

atatacaaat atcttcaatg aaattaactt taacactaat atgataaaaa tcatgtcccg 21660

ctttttatcc tctaactaag actctgcata aaggtatatt gcaattaata tgagatggaa 21720

gaggtataat aattatatga tcaaattcct ggattgaaaa ataaatatga gattaaaagt 21780

ggtatgtttt tggttaaaag aaactatcca taaagtatgt ttttggttaa aagaaactat 21840

gcaacatacc aatcaaatgt ttatacgctt acaatttatg taccactttt ttgtcattgt 21900

ttttctattg tttgccatac gtacgttact aaatcatgtt gtcttttcac attttaacta 21960

acaataaatt actattgata caccaaaaaa atctatgagc attggagtac gttgtttgat 22020

agaagcttcg tgctattatt tcttgtcaaa gaatttcata tctcaatatc ttctaattta 22080

acaatctaac gaaatttttt tgacccagga aacaaatcca tttgcaatct ggaaaagata 22140

caaacactta aattatcaaa caatgctttg actggtgaaa tccctcattg tgttggaaat 22200

atcgagctca tagcattatt tctccaatca aacaaactga acggtaccat acccgcaaac 22260

ttctcaaagt tatgtgattc attgatatat ctagatctta gtgacaatca actcgaagga 22320

gttctaccta agtccttgtc caaatgtcaa agtctagaac tcctaaatgt cgggaacaat 22380

aggctaagag ataaatttcc ttcatggtta gacaacctcc cacgtctcca agttttcagt 22440

gtgcgtttta acgccttcta cggtcctata actagctcac caaaagttag tcacccattt 22500

cctatgctac aaattatcga cctatctaac aataagtttt gtggcaagtt gccaagaaga 22560

tatatcaaaa actttgcaac catgcgcaat atgaatgagt ctggtgttgg gaatccacag 22620

tacctggggg actcatcaat atatagtatt acgtactcta tggtattgac attcaatggg 22680

ttacaacaaa aatatgaaaa gcttattgtg acgatgtcga cctttgatat atccagcaac 22740

aactttactg gacagattcc atatgttata gggggattac gctcacttcg taaccttaat 22800

ctctctcata atgtcttaac cgggaacatt cctccatcaa ttgcaaaatt gtctttgctt 22860

caagatttgg acctttcatc aaacagactt actggtcgta tccctcaaga attagttagt 22920

ttaacatttc ttgggagttt caatgtttcg aacaatctat tggaggggtc tatacctcat 22980

ggtttcaact tcgacacgta cacagctaat tcataccagg ggaatctcga attatgtgga 23040

aaaccattac ctgagtgtgg agaaagaagg gcaaaaggca ccactaataa tcaagatgat 23100

cctaaaaatg ataatgaacg aatgttgtcg atgtccgaaa tcgtagttat ggggtttggc 23160

agtggtgtac tagttgggtt ggcttgggga tactatatgt tttcagtggg aaagcccttt 23220

tggtttatca agatggctag caaaatggaa tcaatattga ttggtttttt ctgaccaaca 23280

atttgttagc cgatgaagag catcaaaacc aaaaaaaaca aaaaaaattg attaatatgc 23340

atgagtgtga ccttgttttc caaagtttag cattactatt agtgtctcaa ttcataataa 23400

taaaaaaatt agcttgttca agatttgtat ttttattcaa agattttttt tgtctcttgt 23460

gcttctttta tcttatatat attttttgta tggtttgttt ttgtttaata ttagtccctc 23520

cgctcaaaat gatctttcac gcttgagatt ggcattaagg tcaagagatg ttgctaagct 23580

ttagaataaa aaaattccaa atgcatagag ggaaagaaag cgagacaaaa tgttggagaa 23640

ggcagagtaa atgatgtgat ggaggataaa tagtagaagt gtgataccga aagtttgaaa 23700

ataataagga attttatttc ttgctggcac tttgttctag tacaggtttt tagcccttca 23760

aaatgtttat aatgtagagt caaaattaat atccttaact agtttttaag tccgggttat 23820

atcctagata ttaataatat tcatttatta gtaacatttt attttataaa tataatacta 23880

agcattattt ggtttgctgg ttaagacttt agtgtatatc tatttctttt tttttttatt 23940

gtatgcgtgt ttacataaac taaagactat aagggatagt accacgtggc gcagttcctt 24000

gcttaggaac gtcttttaat atattaacta gtatttgggc ccgggcgttg ctccgggttg 24060

gtattgtgtt tccgaacatg atgtgcagtt tttcccattc ccactaaaat atataaagga 24120

aaactcaaca tttaaaagat acaaatataa taatatggac acttaaaaca tgattaaaag 24180

ttgattgaga tggtaattgt gtcatgttat aatagtaaga ggttgcctaa ttgaggttga 24240

ggtggtggag tagtggtatc gcttcccatc tgttatccct gaggtataag gatcaaacct 24300

cataggactc atttgagtaa tttcccatat cctcctctca aatgagtcct tttcatctga 24360

caaaaaaaaa gagtctaatt ttaaattaaa attagacgat cttttataaa atcggcactt 24420

tctgcacata ggtcacaatt tttttgtttc tatctctctg ctttctttaa tttcacagtc 24480

tccaactctc catcaacatc ttacttattt tagaatagat gatgtatggt agtattaaat 24540

ggtaaagtac taaagctcct ataatacaca gaagcttaca tagtatagat tcgtacatga 24600

gacaaggtta caatatactt tctccgttct ttttatatta caataattac tattttaagt 24660

agtttcacat ctattgtaac aattccaatt ttgttataga aagcaacttt aataattgac 24720

aatattgccc ttactttatc ttattaaaac catcattaat tactcacttt ctcttataaa 24780

attgctttta ttttctaagg atgatttctc tcctattcta gttaattaaa gagttacttt 24840

tgtgctaaac tgctcattta ttccaaatcc ttaaaaattg tgtccaaacg tattgttgta 24900
atataaaaag aacagaggta ctattagttt gaataaattt tgatcagatt aggtcacctt 24960
tagggggcgt ttggttaggg gtattctgga aagggtaagg gaatcaactt acttaattcc 25020
cttacttgtt gtttgtttgc tcaatttaat gattcccttt acccacccct tactcccaaa 25080
gtcctttact ctcattctcc ccacccccca aggtttcact taccctttct tgattcatca 25140
ttgaccatat ctttgaccac ccaactacca ccaccacttg accacctaat cacctaacca 25200
cctaattacc caaccactat taccacccaa cccctccacc tgcccaccaa tcggcaccat 25260
aactgcccaa ccgtcgccca atcaagccac ccaaccggca ccataaccgc ccaaccaagc 25320
cacccaaccg gcaccagaaa ttgtaccaag ctacccacac acgtgaaaac cacccaccca 25380
caagccctag aaaaaatgga agaatcgaga gaaagggagg ggagagaaaa gatgcagcga 25440
ctagaagggg aggggaggga tgtgacggca aggggagagg gaacttcgca gcggcaaagg 25500
gaggggaaac gtcgcgtcgg caaagggcta aggtggaatt gacggggttg cagcaacaag 25560
gggagggcat ggagacgtcg taaccgcaag gggaggggca gcggcagtgg aactggggtg 25620
gagaggggta gtggcggcac tagggtgtgg gagaggtggc gggggatatc aagagagggg 25680
ggatatggtg gtgttatggt ggaagcaaga agaagaaaga ggaaagacaa tgtactaacc 25740
aaacaacaca ttaaatctaa gggttttggt ttcctttccc catctacccc tttcttgatt 25800
ccattccctt taccccttta caaccaaact cccccttagt ttttactact tataaccttc 25860
aattttggct gttttttgtg acatttttta cttctccgag cctggtcata ttttctcccg 25920
aaacatttcg aggaaagtcg aagtgacttg tgaagttgtg cgggtgcttg gcaccatttg 25980
tgttgcctcg aaaagcatct gaatacccca tttattcctt tctcctgaaa cccaaaatta 26040
cctcgcaata aacgaaaaga tatccatata tttgttccaa gccacatgac tcctttccaa 26100
cgacctccca tgtgaccatg tccttagaag gcatcccgtg gcgttcgaag ctcggacccc 26160
cggaaagtcc gaaagtgtgt attataactt tcaattttgg ctgtttttgg gatatttttt 26220
acttcttcgg gccttgtcat attttctctc gaaacattca taggattgtc aatgtgactt 26280
gtaagttgta acgttgcacg ggtgcttggc acaatttgca ttgcctcgaa aagcctctga 26340
acaccccatt tgttcatttc tcgtgaaatc caaaattgcc tcgaaaaaaa cgtaaaggca 26400
tccacatatt cgttccaagc cacataactc atttccaatg acctcccata gagtccgtag 26460
ctcggacccc aggaaagtcc aaaaacgtgt actataacct tcaattttgg ctgtttttgg 26520
gacatgtttg gacttcaccg gcctggtcat attatcttcc gaagcattcc tacaaaatcc 26580
gacgagacta gtaacgttgt tacgcgggtg cttgacacca tatgtgttgc cttagaaagc 26640
ctttaaacac cccatttgtt catttttcgt gaaacccaaa attgtcccga aatgaacata 26700
aatgcatcca tgtattcgtt gcaagccaca tgatttcttt ccaatgacct cccatatcct 26760

taggaggcat gcatcatgtg gcgttcggcg agcgggtctc gggaaagtcc gaaagcctgt 26820

gttataacct tcaattttgg ctatttttgg gacatttttg gcctttttca agcgtgttca 26880

tattttctcc cgaagcattc ctaggttagg cgatgtgact tgtaaagcgt gggtacttgg 26940

caccattttc tttgcctcga aaagtctttg agcaccacat ttgttcattt ctcgtgaaat 27000

tcaaaattgc ctcgaaatga acgtaaagac attcacatat tcattccaag ccacacatga 27060

ctcctttcca atgacctccc aagcccctag gagtcgtccc gtggcgttcg gatccggagc 27120

tcgggccccc gagaatgtcc gaaaccgtgt attatgacct tcaatttttg ctgtttttgg 27180

aacatttttt gacttctctg ggctggtcat attttctccc gaaacatttg taggactacc 27240

gacgtgactt gtaatgttgc gtgggtgctt ggcacaattt gcattgcctc gaaaaacctt 27300

taaacaccgc atttgttcat ttctcgtgac acccaaaact gcctcgaaat gaacgtaaag 27360

gcatccatat attcgtttca tgccacatga ctcctttcca ctgacctccc atgtccctag 27420

aaagcacccc atatccgaaa gcttgtatta taaccttcaa ttttggctgt ttttgggaca 27480

cttggacttt ttcggttcgt tcatattttc tctcgaaatg ttcctagaaa aggtgacgtg 27540

agttgtaacg ttgcgcgggt acatggaacc atttgccttg cctcgaaaaa cctctgaaca 27600

ccgcatttgt tcatttctcg tgaaactcat aattacctca aaatgaacgt aaatgcatcc 27660

atatattttt tccaagccac ttgactctta tccaatgaca ttctatgtcc ttagaaggca 27720

ctgcttgtcg tccataattc gggccaggga aatgtatgaa agtgtgtatt ataaccttca 27780

attttggctg tttttgagac aattttttac ttctccggga ctggtcatat tttctcccga 27840

aaaaatactt cgagtgccga cgtgacttgt aacgtcgcgc ggatgcttga caccatttgt 27900

gttacctcga aaagcctttg aacaccacat ttgttcattt ctcgtgaaac ccaaaattgc 27960

ctcgaaatga acgtaaaggc atccacatat ttgttccaag ccacatgact catttccaat 28020

tctctcccat gtccctagga ggcatcccgt ggcgttcgga gctcggaccc tgggaaagtc 28080

cgaaagcgtg tattataacc ttcaattttg gctgtttttg ggtcattttt tgacgtctct 28140

tggcttggtc atattttgtg ccgaaacatt cccaggattg ccgacttgac ttgtaacatt 28200

gctcgagtgc ttggcacaat ttgcattgcc tcaaaaagac tctaaacacc ccatttgttc 28260

atttctcggg aaacccaaaa ttacctcgaa atgaacgtaa aggcatccac atattcgttc 28320

catgccacat gactcttttc caatgacctc ccatgtccct aggaggcatc ccatggcatt 28380

cggagctcga acactgggaa agtccgaaag cgtgtattgt aaccttcaat tttggttgtt 28440

tgtgggacat ttttgggctt ctccgggcct ggccatattt tctcccgaaa cgttccttgg 28500

aaagccgaag tgagttgtaa cattgcacgg gtgtttggca ccattagtgt tgcctcgaaa 28560

agcctttaac caacccattt gttcatttct cgtgaaacct aaaactgcct cgaaatgaac 28620

gtaaatgcat ccacatattc gttccaagcc acatgactcc tttccaatga ccttccaggc 28680

ccctaggagt catcttgtgg cgtttggagc tcagtccccg gtaaagtctg aaagcgtgta 28740

ttataacctt caattttggt tgtttttaag acattatttg acttctccgg gactgggcat 28800

attatctccc gaaacattac taggagtgcc gacgtgactt gtaacgccgc gtgggtgctt 28860

ggcgcaattg tgttgcctcg aaaagccatt gaacaccccc atttgttcat ttctcgagaa 28920

acccaaaatt gcctcgaaat gaatgtaaag gcatcgacat attcattcca agccacatgg 28980

ctcatttcca atgacctccc atatccctag gtgtacaccc catttgtctg atgttataat 29040

agcaagaggt cacgggttca aatcttgtta caagctaatt ttacttttgt taattgacat 29100

gacttatgta cacattggac aattatagtg gagtaacaaa ggtgacatgt gacgcgtata 29160

cattatcaca cacgtctttt aatatatttg tatagatcta gatttaagag taattttttt 29220

aatgcgcaat acttggccaa tttcttctgt atcaaatcat aggtctttgg ttggttcata 29280

agagtaaaga ccaaaataat aatctgaact gcaaaaattt tctccaagag ttaaaagttt 29340

gtataagtta gattaaaaaa attaatgaca tatgatgtag ttggacatta aatatgtaag 29400

tttagaagta attgtgttaa cataaaaaaa gattcgatta taacataaaa actaaagaaa 29460

cacaaaggcg ccgtacaaca atcaatatta cccaagtccc ctcattaata ttaagggatg 29520

acctagctcg tacatattta attatctttg aaaattcgtt gttcagactt gctagttgct 29580

attctatatt tgtatattca ttaatcaatt tttcaatatg tgagcattta cattttaaac 29640

tagagcaaat attgtctctt ttactatttt gttgttgtca aattttcaaa aataaattgc 29700

tcaaatactt ttcctagtga cataaaaaat agagcaaata atcaaacagt agcagaccca 29760

ggaactttta cataatgtag acggcataat gtgttaattt ttgcttcttt tttctaatat 29820

catccaataa cacaattctg cttctattag tttgtagttt cagatgatga tacccaaaca 29880

ataagaccaa gcaacaaatt gataagattt tgcttctctt tcttccactt ggtgtaactg 29940

taacagcttt gaagtttaac ttcagtaatc agttgcatat ttggcatatg atcaaaacaa 30000

tcaaattatt atgtatggaa aagcaaaaaa cttccaggtt tccatctgaa caaggaggcc 30060

aagagggtgg aagcaagcaa ggatatatga tcataaaatc ctatgaatat gatgtacaaa 30120

ccttttctac tgcaattagg taacctaaat gataccacct aggaacagca acaacttatt 30180

tacagcacta aacctaaatc aggttaaagt taatcagacc accatgtatc tgggtggtct 30240

ctcgagggaa agcgtctcca tctgtatccg ggtaacagag gtttcttctt ctcgatcctc 30300

cttggcttct gccctcttaa gttcttcgaa ggctctcttg gcatatacag taaacgcaac 30360

aatggtaatt attgccacta tgaatgaaat aacattgtac acaatctcca cccatgttag 30420

atgatgattc ccatacttga catctgcgaa cgtccttatc agtctcccac tgcaaatgaa 30480

tgctatcagc gtcaatattc gagataccaa ctcatttaac tattgaattg ccaaaaacag 30540

atatctttga ccatatattt gttactaaaa ataacgattg ataatgtgaa actatcactg 30600

atagatttaa aagaactttt ataaaagtat agtttctcta atgtataact gcagaaaata 30660

gaatggggta gacaaatgaa gtaattgttt tgaagaatgc aaaaggtcaa ttcagtaata 30720

cttttatacg tgattggggg aagcattaaa aatcccttct aagataaaga tgacctcatt 30780

ggcaatggaa tcgacatcca cagacccttg cattagaaca gagtggaagt ttctgtgaac 30840

ttacgtgtag atgtaaagaa aagcttctgg caccatccct gcaattgatc cccatagata 30900

aggccaaaac gtcatacttg tcaccacaac tgcgtagttg aagatagtat agggaaatgg 30960

tgaaacccta aagagtgcca ccacgcggaa ctgatgaaac cagctacctt cggcagcaag 31020

cctaagcata gcagccttat ccggccatct ttgcaaccat tgctaacaag gtacaaaaac 31080

ataaacattg tggacttaat tagacaagaa agttaaatta aaatcaacat tagataatca 31140

ataaatcaaa tgtaagcagg gaacatattt cttacatgga ttctatcccg gaagagcaat 31200

ccaagtaaat agggaagaat cattccaata gtagttccaa ccatgattat cacaaaacca 31260

agaccataac caaagatcat gcctgcaagc cacatggatg ggccagaagg aatcagaaat 31320

acagggaaga ttgctaggga agtaacaagg accacagcaa gaaccggacg gccaaaggca 31380

gtggcttccc attgcatcat tggaacaaga acctgcagag aaagtaccaa aaactttgag 31440

gcaaaaattt cctgcttgta tattgcaaaa agtagtacag cgaaggcatt ccgtgcagaa 31500

tggcttatag attggaaata cggagaacaa tgcaactata agcacaggcc catctcttga 31560

cttttgggac aataacatgg acccccagat tgatttataa gttctcacac catagctaga 31620

ttttgttgga actttcataa atcatagtga cataagtata gcataatatt catgccttcg 31680

acagaagttt tcgcatatgg taaggctact attgaaaaaa ttcccttgtg tttgaagtac 31740

gcataaaaat atctagtggc agtcaaccaa ataaaacatt ctaggagtcc ctcaaaaaat 31800

taaagagtca tcagttcaga agactttaat atcaatactt tctattatcc gggtttggca 31860

tgcagtaaat ttcatgagaa aaggaaaaat cagctatttg attatataag gaactaattc 31920

ggatgtatca ctaagctttc catcgactgg aacatcggga gctagtctcc aatactcgtc 31980

aaggatctaa cataaacatc ttctccgcaa tcaaaaagcc aaggtcacat acatctaggc 32040

ctctgtctca ttctgatggc atggtatgat gcaagttaga caacactatt atttggcaga 32100

tgacacttag gggtctaata tttaagctca ttcaagataa tcaagtaatc aagttcaatc 32160

tcaaggtttc agttgcgcta aaaaatgtaa tacttggctc attcagaatt agtttgttga 32220

agctggttgg tatttgcttc atttgttaat ggaaccaggc tcataaacaa gctttcatta 32280

ggctaaactt atttaacaaa atcaaaagct taatactata atttttgata ggatttcttt 32340

tgggcagtta tacatgagta atgaacaagc tctacacaat cttttttaat gaacaagctt 32400

taatcgagct agggtacgtt ctattcaact tattggacct gaacttattg gaacttatct 32460

gaactgaact tattgaacct gaactgaact tattggaact tattaaacct gattggacct 32520

gattcaactt attggacctg attgaacctg attggaactt attggacctg attgaacctg 32580

attgacctta ttggacctta ttggaactta ttgaccctga ttgaaactta ttagacctta 32640

ttggacctga ttgaaactta ttagacctta ttgaacctga ttgaaactta tttgacctta 32700

ttagacaaaa acattattat tattattgtt attattatta ttattattat tattattatt 32760

attattatta ttattattat tattattatt gttaacctga ttgataacat ttatatcttt 32820

catagttatt agtaacgaaa acatgttatc tctagttatt caaagacgaa ttgcaaaata 32880

ttgtaataat aataataata atatattatt attattatta ttgttaacct taattatttg 32940

accatgatta taatattatt caatagcaat atgaataatc aaataataga caataataca 33000

agtataatac tatacattgt ggtactttaa taaaaaaatt ctaataataa cataatcagc 33060

taatagtaat atgaataata aaataataga cataatacaa ataaataata aaataataga 33120

cataatacag ataaataata aaataattta cactaataca agtataatac tatataatca 33180

ttgtggtact ttaattaaaa ttctaataat aacataatcc gctaatagtg atatgaaatt 33240

atgaataaca aaatagtgga caataataca aatgtttatt aaacattgac tatttggacc 33300

ttattggacc ttattagacc tgattggaac ttattggacc ttattagacc tgattggaac 33360

ttattgcacc tgattggaac ttattacacc tgattggaac ttattgcacc tgattggaac 33420

ttattgcacc tgattggaac ttattgcact tattagacct tattgcaact tatctgaact 33480

tatctgaact tattggacct gaaacttaat tttttaagtt gaacagaacg cacccctagt 33540

atccacgaac atagttagtt gttcatcgac aagggtgtta attccttgac tataaaaaaa 33600

atatctgcta atatgtcctc cataccatgt cttgatctga ttcccaaaat cacgtgtttt 33660

cgtgtctggt gaccacgttg ctagacatgg aagacaggtc taattgttca gtttcaagtc 33720

aggttgatta aacatatgtt agcaatatac aatcattatt agtcaaacta attcaactcg 33780

ggtttggttt gattcaggtt atgtcgagga tcaggtccaa atcgggttaa tccttccagg 33840

tcaaatatat ctaagtctgt tttgccaaag tctacttttt gtatccgtgt ccatgctaaa 33900

tgacaaacaa aaagcagctt ttaccaagct cgaatcagat ttgttcgctt aaagagtcac 33960

ttcgctcatt tacagcaaca attaaaggac aaaacattgt ccattcaact acttacggat 34020

attaacttat tggcaactgc tagcgtaata aggcaatcaa cagcactcgg cctcaataat 34080

gaacctacaa ggagtccaat gaccaataca aattatcact ggcatcatct agcacgacaa 34140

tctcttaact ctaagagtct aagtgccttg acatacaaaa gtattccttt taaaagtacc 34200

cccgtgtgga tattctgcca agcaaatgca atcgatacac ccaattaggg cttttccatt 34260

atgagtcctc agagcctcag attgtaaaac aggtcagtaa aagaggaaaa tagtatttga 34320

ttcttttgct aaacccttgg atataagaat ggtgacttgt attgtcacgc caagcttctt 34380

tcataaaagc tgatcatatt attatatgag agttctgagt ttcaaggtcc gcattcgatc 34440

taactagaca tcacttccaa ttaaagttga gaaacgaaac taggtgtcct ctttgtttcc 34500

caaaggtgaa ctttagatac ttattataag catattttgt tatgaatcgg gctaaggaga 34560

gggctactct tggtattgca taattagtta attacttagt agtagcttga ggaataagga 34620

agcaagtaag ttagaggaaa gagtatgaaa atctgctata aagtgaggag aggagggata 34680

gaaggataat cacaaaatta ttgagttaac tttggtttta gttgcttagg ttgggagtgt 34740

ccagccactc gaatgtcttg ggactgtaaa caccattgtt catgatctaa ttgcatcaat 34800

attacaatta actcatttct cttcttatcc atattcatct tcttacaatc acaactattt 34860

ccagatcatc catccaaatc ttcatccact tgccttagtt tctactccag atttcagtct 34920

attacaaatt gatttctaca atatgtcaat tcatcacaaa ttatcatgtt ttctgaacaa 34980

aagttcactg tttcaggaca aatacagaaa gaactacttt gatgcttaga acagatatat 35040

tgtaaaattg tattcggaat ttgggataca actggagaag atatgaataa ataggcattc 35100

agggagctca gaaaaacaga ccgtgccata tggtgctctg ctgcataaca ggaaataatg 35160

gataaagtat gaataacgtt ataacttctt aaaaacctag atgacaagta ttttggttgc 35220

tttttattat tggtaggcaa ggagaatact caacaacagt ttagccttaa actgcttctt 35280

atttctcctc ttcccctttt tcctgatgat ttggggttgt cactcagttc ttttacctct 35340

catttccagg tactttagag ttatattaca caaaggattg caagagaaga acaggtcgcc 35400

ctggcatgca ctcagaaagt atacgaccct tcacaggaaa tgtggtgctc caagacttat 35460

atctcaggct ctcatgagtc atgtcaagga ccatctttaa tcatttgtat tctaggtttc 35520

tcaggcgatg cggtgtgctg gtgtgtctct ccctcccact tgagtgtgtg tattgtttgt 35580

gcccctaagt ttttatctta acaatcacta ctagtcaatt agtcattacc aaccctaccc 35640

acctctcttg ttactgttgt tcttggagat atttcatata tgtcagctta gaacttatat 35700

tacgtttctt attacatatt ctcttaagct cgcgcacata ctctgtgatc gaagggatcc 35760

atattagtta tcttttagtg gagttgttgt gaaaaaagac tgcatagaaa aattaagata 35820

gctcatagtt gtaaatgtaa ttgaactttt agattgatag ccttgaggct gcttgcattg 35880

aaccaaccaa attcagccag gctagtctat gcctctttgg tgtcacctgg taggttgaat 35940

ttgtgtagct gtagttctac aagagactga tttaaaaatg ttttcgcact gaaacagctt 36000

aaaccacaaa acaggaaagt gcagaacaaa ctccagaaaa tggtgcagaa cataccttct 36060

caaaaaggaa aggaactccc cattttaaca gtacgaggac aactgctaca gcactaatgg 36120

aggagatcaa gattttgatc caccagatga aggattctga tcttgtttca gcctgagaat 36180

gtaaggttga agcttcaggc ctctttgtaa tagcagatgt caccagacta acaaattcac 36240

tgtcgtcttg catagcaggc ccaacatcta tgtcatgctt agttagctcc attgaatttg 36300

gcatctccaa gagatctcaa gagctgccca aaaagacggt acaatattat gagcatacat 36360

gacatgatga caacccataa agaatatcat aacctgtcac attttttatt caaagttcaa 36420

cagccctctt acaacatgat tgagaatgga ggggaagaga gagagagttg gtctcagaca 36480

ttgatcacat aatcatttca attagtttta aaggtgctca tgaaatagaa ctagtgtctt 36540

aagctggaga cttctgtatt tttcatggtt ttagattatc aatcatattc ttagaatctt 36600

tgatctctag aactctttcc tttcctccca atattttttc cactttgtct tttgttaatt 36660

acggcttcgc tgcaggcctg caataaatct tttaaatttt tacagatact atgtagagtt 36720

gtatacataa gctctaatct gaagacgatt ggtttcgatg ctagttaata caaataaata 36780

tattatggat ataatatgca gtaaattggg ccatgggcac cagggacaac ttagacaagt 36840

atagtgcaac taccaggaaa tttaagctgg gtacctctga ttcatcatgc tggttgataa 36900

tattattgct tccacaagtg ttcgctacgg ctcaaccaaa ctaagtcaca actcacaagc 36960

tgcacaaccc aactgacaat tatcgcctat tgtctaagct atacattaca ttaccccaat 37020

gccacaacgt ggctcacgcc taggcatggt aaggaagttc agatgtacgc agccttaccc 37080

ttttaataac aaagaggctg tttccaggtg acccttaaat cttaattgca aacaccatct 37140

gctgcttcac ataaataagc gacttcaaaa ttgtaaatta aagaatttga atgcaaattg 37200

tgtgaaaaac aactccatca agaatccatt aagcacgctt tactattagt atcaataata 37260

ggaaaccctt atatcccttt tgacgaaggc acacatgcaa cactaatgtg tccttataaa 37320

cttcatgaaa gtatatctct acgaaaccct tttagtctta tgtgattctt taagtgtcca 37380

actgatgatt ggttacaagg tatttagccc aaagtagcat ttcagagaga tggtgtagaa 37440

tgagtagctt ataaaccgag gttgaggtgt aatcctaata aattaggaac taataccaca 37500

agagagatgg acatgtagag atacaatata gtacagaata agattatttg aaatcttttt 37560

accagggaaa ctccagaggt gttccataaa acacaatacc atataactgg gagatcaata 37620

ttttagatta aaaaatataa aaatctattt gggttgagta tatagttggt tagtccaata 37680

atatataaat ttataaggtg gaggtcttcg gtatatgaca ttccaaattt gagtatcaaa 37740

tgatatatat ggttttccat acttgaatcc cttttcatgt actacctctg tttcaaatta 37800

atagttacac ttacactttt cacgcatgcc aatgcagaac tttgaggaca tatatcttta 37860

gttttgtatt tgtaaaaatt ataaaaagta catattaata aaatacatat taatacgaat 37920

ctaacaagat cccacatgac tatgatttta ttcacgtata aatcacaaac gagggtcaaa 37980

atgcaattgt gaatagtgta aaatgtcaaa gtgtaactat taatttgaaa cggaggtagt 38040

atgtgtttat gcaacacttt tcctttttcc ctttttgcta tttagtaatt tatgtaaaat 38100

acttccattg acccaaaagt tgggtgatta tagtttacat ctatcattat tatttatcat 38160

tactatagat tattcaccat tgtaatcaac tttataaaag tatacacagg taactcagga 38220

gtcaggggtg ctgggccaaa cacttttata gtttaaggtg aaaaatctcg agaatcttct 38280

cctgccacgc aaaatgagtg ttcttccact ttaaagatgt tataacactt atcttaacct 38340

actattcgta aataacactt atcttaacct actattcgtc aagacatact tgcttcatct 38400

cactaagaac gtcttagttt tcatttgaaa ttcgtaccag aaagattcac ttcaaatcta 38460

tttattttta gataaattgt tattaaaaac gacgaagaaa cgtcagagga caacaaatcc 38520

tctaaactcc aaattataag tgagtccaac tatgttgacg taaggtaatt agagtatcca 38580

taaaagccct ggccgctttg gcccacaaag cagcttagaa tactacccaa ccccaaatat 38640

aatcaatcag gtgaggaagc tcgcaacaga tgcgagagtt ccactccaat caaaggcacc 38700

agaacatagc catcgacatc ttctcttctt tacccccctt gaaaccaaca gatcttaagg 38760

aagtccacta gtgaacaagg acataaccac tactcatgtg gaatgccaat cagcctctgt 38820

caaagggaag tccattagtg aacaaggaca tacccactgc tcaaggtagt catgtggaaa 38880

ttggaatccc aatcagcctt tgtcaaaagg aataagccac atcgcaatga agaaaaaggt 38940

gcaaaccaga tttattgcat ctccaacacg acataaatat cgagaatgag gcctttactg 39000

acaaaggaac tctggatttc caatttccac tgagcattgg actcagttga gaagtaattg 39060

gtcttgctag attctgttta cgcacatact cttaatgata aataaatgta acaggccaat 39120

tggtctggaa aaaaacagtt gataaaaggc tagtttgggc cttggggata aatataatct 39180

ggtatgagtt aataaatttc tgtttaaggt aaagagaatg tgttatgtgg gataatttaa 39240

tcaagaaaat cttagtaaga tggaggtagt ctaacttcca ttcctcaaaa tgtgtaattc 39300

cttataaaat cagtcagcct ctagatacat agttagcaaa aatggaaggt atagaagtgg 39360

gggtgaggga agaggaagga aagagaaccg cgatcaatca tattgttcgt gctcaagttt 39420

gagttgtgcc tatagctagt tagagtttgt ctatttcatt gtttttggtc agtgttcata 39480

ttctgagtgt catcgtgttt gggttctaga atgctccttt tcctaatgtc gacatttctc 39540

cactttactc tagaaaaatg atctcattgt agccattcca gcttcaattt taatggatac 39600

taagatccct ttcaggaaca atgttaaggt agatgttagt gttttaacag ccatgtggat 39660

gttagtgtct agaacgagtg gtcaaaacac tactagcctc aaaatattgt gatcagtctg 39720

aaaactctat gttagatggt tgcttttttt ggtaggttcg cttgttttgg ggggttagct 39780

ttgtttattt tcttcacaat ttgcccttaa acttttcaca aaatctacaa ttgaagattc 39840

ttaaatagat aacagacgtg tcagctactt caacagctaa ttgtacgaaa aagttcagct 39900

accttgaaac caaaccacta acagctagta cagtttgttt ctactattac atttatctaa 39960

tataacagct agtatttagt ccaacgatgt ataatatcaa tgaaatggaa ctaatctgta 40020

aattggacct taggcataag agtcgagttg agcaggtaca ctccaatcac caagttattt 40080

aagcttaaaa tgtctaactt ccaatgctgt ttgacgatac tcattgccaa gtgtttgtta 40140

cagatcaacc aagcaaataa agcaacaagt gaacagctgc actagtaccc aactgcgaat 40200

tttcgtcgat tgccaagtgc atgtctggga cacaatacca tcatgtccat acccattacc 40260

ttgcttagcc agctatcgta atccataaca cataaaaacc aacaaagtct tgatagtttc 40320

acaaatcaaa atgttcactt ttcattccaa ccaaaacaag caataaatct cttcatccat 40380

actcacaaga agaacaatct ctcacactac ccacttgatt agtaaaaacc ccaatcaaaa 40440

acaaaatcca acccacataa acaaatcaaa tttagtaact acccataaac tcaaaaacct 40500

caaatcacaa taccaataaa agagatatac aatcaatcaa aaaaaataca acaacagcta 40560

aacaaataac atcataaact aaagttattc attttatttc ctaactagag atcaattaag 40620

cagcataaaa caacatcact aattcaagtt aataatcatc aaattctata ctataaaaca 40680

tacatacctt accaaaacta cccagctgaa aattagggta gagctccaga aatcccggcg 40740

aaaaatccgg tgagaaattc agctaaattt gaaaacttct ttaggttaag tagtgtacac 40800

gatgaattga agatttttac aagcatatga aaatggtggt tgaaattgaa atgggggttt 40860

ttgaaaattg ttgcgacgcg taaaagtgga aaaaaaaaag gagagaatca aagaaatgag 40920

caagtttttg taggtgggtt tactgttgtt gcttttgttt gtgcacatta ctgactattc 40980

ttaattcttc catgcgtgtg ggggtgaagg aattgttttc ctaagttgtt tagccacttc 41040

atagagtcat tggatttgaa taatctaggg aataatgatc atgtgtttag tgtatctata 41100

aattataatt tatgtatgta tattgtatat gtggtgaggc atagaggaca aggtctaaga 41160

ggaatagagg attgtgaggg agtgtttcat gcttttaaga atgatgagtc attgagtgta 41220

ttaagttata agtagtattt gatcgagtag taaagtttgt atcacgtaaa tcagagtgat 41280

aattaggaat tgggatttgc tcaagtggtg agttttccca tctttccgag caaggtttct 41340

agggttcaat tcctacctca agcatttcct tgggatttaa ggggacggct cagaggaatt 41400

cttcttacca atattttaaa aaaaaaaaaa ttaagagtgg taatttagtt cagatcctac 41460

ctttatccgg ttcgaaacga cttcaagaaa aaaaaatccg acatcgttta aaatttttta 41520

cttccgactc atttaatccg cctccaactt tgaaacaagt agtcttattt cttttatgtt 41580

aagaaaattt gccaaaaaaa ccctttttaa agtccagttt tgcgaaaaaa aaaaacctta 41640

taaagcattc tttgtgaaaa caaaccaaaa agtaaattat ttttgcaaaa tgaaacctaa 41700

tctcattttt cggttttgac catggacttt tcgacattga ccacttctat ttatcttctt 41760

cctccataat cacagcctag ccaccactac caacacctgc cgctagcccc cacaacctgc 41820

acccccacaa cctccatcca ccccctcaag cggcaacccc ccttattccc atacgcggca 41880

accctacacc ttatcctcca cccccctccg cccttacctt ttctcctctc ccttcttccc 41940

tccatcaccc ctccccactc tcttctccct ttgcccccca tcgttgcacc acccataatc 42000

cctctctgta accccctctc ctcgcagctc cccctccctc ccagccaagg ttgaaaaatt 42060

acagaggcag tcgcatatgg ggatggggga ctatcgtcta aggggtggag agagggtttg 42120

ggggctgctg gtgggggtgg ggtaggctga atgtggtggg ggctgagggt ggggggtgaa 42180

ggtggggctg caggtcgggc tggcggtatg gagaaagaag ggaaatagaa gtggttaaca 42240

ccggaaagtc catgatcaac accgaaaaat gaaattaggt ttcatcttgc aaaaataatt 42300

tattactttt tgatttgttt tcgcaaagaa tgctttataa ggttttttcg cataacattt 42360

agacttttat catccctctt agatttgaca catattatac gaattatact aaaaagactc 42420

cttatagtaa ttcgactaat gttttattaa aatgaacctt tagaataact cgggtaatat 42480

<210> 54
<211> 201
<212> DNA
<213> Beta vulgaris

<400> 54
agagcagatt ggcatacttr tgaatattct cactggctat taaattctca gaagaaaaat 60

caacaccaag attatgacat gcttgtgcaa agacacaccc agtcatgaat gcatcatagc 120

cagcttcatg cttagcccca gagttccaat ttgaggayct gcaagaaaac atgggagtaa 180

gatggtttca cataaaacat g 201

<210> 55
<211> 201
<212> DNA
<213> Beta vulgaris

<400> 55
agagcagatt ggcatacttr tgaatattct cactggctat taaattctca gaagaaaaat 60

caacaccaag attatgacat gcttgtgcaa agacacaccc ggtcatgaat gcatcatagc 120

cagcttcatg cttagcccca gagttccaat ttgaggayct gcaagaaaac atgggagtaa 180

gatggtttca cataaaacat g 201

<210> 56
<211> 201
<212> DNA
<213> Beta vulgaris

<400> 56
gggtttcttc gaagtttgat tttgttacat ttttcaaaga gaaattagtt gttgatgttg 60

aataatgatg ataagtagtt agggttcgta gtaaggtgga cgaragagaa aatggcgtca 120

ctctgayrag cttcttcatt ttgttcttct tccttagctc tgttttcagt cactgcgcca 180

tttttttttt aaaaggaaga t                                                201

<210>  57
<211>  201
<212>  DNA
<213>  Beta vulgaris

<400>  57
gggtttcttc gaagtttgat tttgttacat ttttcaaaga gaaattagtt gttgatgttg      60

aataatgatg ataagtagtt agggttcgta gtaaggtgga ggaragagaa aatggcgtca     120

ctctgayrag cttcttcatt ttgttcttct tccttagctc tgttttcagt cactgcgcca     180

tttttttttt aaaaggaaga t                                                201

<210>  58
<211>  134
<212>  DNA
<213>  Beta vulgaris

<400>  58
caagcacaaa atcaaataat gagaatcaca ctatccaaag aaaatttcca tccacattta      60

tccaacacag ttatctctct tttacaccca aattatgtca accaaaaaca staaaacaag     120

tgagtgcagt agct                                                        134

<210>  59
<211>  134
<212>  DNA
<213>  Beta vulgaris

<400>  59
caagcacaaa atcaaataat gagaatcaca ctatccaaag aaaatttcca tccacattta      60

tccaacacaa ttatctctct tttacaccca aattatgtca accaaaaaca staaaacaag     120

tgagtgcagt agct                                                        134

<210>  60
<211>  134
<212>  DNA
<213>  Beta vulgaris

<400>  60
taagtaaaaa gtggtaaaag aattaccaaa arcgcacara ataaattaat tagytggatw      60

taactattta acctattcct tttttctgtc gctataacta cttttgctta acttattgat     120

ggtttgatcg ttga                                                        134

<210>  61
<211>  134
<212>  DNA
<213>  Beta vulgaris

<400>  61
taagtaaaaa gtggtaaaag aattaccaaa arcgcacara ataaattaat tagytggatw      60

taactaatta acctattcct tttttctgtc gctataacta cttttgctta acttattgat     120

ggtttgatcg ttga                                                       134


<210>  62
<211>  150
<212>  DNA
<213>  Beta vulgaris

<400>  62
ttataatgta gagtcaaaat taatatcctt aactagtttt taagtccggg ttatatccta      60

gatatttata atattcattt attagtaaca ttttatttta taaatataat actaagcatt     120

atttggtttg ctggttaaga ctttagtgta                                      150


<210>  63
<211>  150
<212>  DNA
<213>  Beta vulgaris

<400>  63
ttataatgta gagtcaaaat taatatcctt aactagtttt taagtccggg ttatatccta      60

gatattaata atattcattt attagtaaca ttttatttta taaatataat actaagcatt     120

atttggtttg ctggttaaga ctttagtgta                                      150


<210>  64
<211>  201
<212>  DNA
<213>  Beta vulgaris

<400>  64
acatctacac tgggagactg ataaggacgt ttgcagatgt caagtatggg aatcatcatc      60

taacatgggt ggagattgtg tacaatgtta tttcattcat cgtggcaata attaccattg     120

ttgcgtttac tgtatatgcc aagagagcct tcgaagaact taagagggca gaagctaagg     180

aggatcgaga agaagaaacc t                                               201


<210>  65
<211>  201
<212>  DNA
<213>  Beta vulgaris

<400>  65
acatctacac tgggagactg ataaggacgt ttgcagatgt caagtatggg aatcatcatc      60

taacatgggt ggagattgtg tacaatgtta tttcattcat agtggcaata attaccattg     120

ttgcgtttac tgtatatgcc aagagagcct tcgaagaact taagagggca gaagctaagg     180

aggatcgaga agaagaaacc t                                               201

<210>  66
<211>  21
<212>  DNA
<213>  Artificial Sequence

<220>
<223>  Primer pCRBM4_S2

<400>  66
gtagttgaat ggtgggaatc c                                                21


<210>  67
<211>  21
<212>  DNA
<213>  Artificial Sequence

<220>
<223>  Primer pCRBM4_S3

<400>  67
caatattgcc cttactttat c                                                21


<210>  68
<211>  20
<212>  DNA
<213>  Artificial Sequence

<220>
<223>  Primer pSeq_LbCpf1_F4

<400>  68
accactcact cctcgataag                                                  20


<210>  69
<211>  22
<212>  DNA
<213>  Artificial Sequence

<220>
<223>  SEQ 70: pSeq_LbCpf1_R3

<400>  69
tagacctgct tctcaacctt ca                                               22


<210>  70
<211>  21
<212>  DNA
<213>  Artificial Sequence

<220>
<223>  Primer pSeq_Ribozyme_F

<400>  70
tgcagcggat ccaaattact g                                                21


<210>  71
<211>  18

<212>  DNA
<213>  Artificial Sequence

<220>
<223>  Primer pSeq_Ribozyme_R

<400>  71
cctggtccca ttcgccat                                                    18


<210>  72
<211>  20
<212>  DNA
<213>  Artificial Sequence

<220>
<223>  Primer pSeq_tDT_F

<400>  72
ttacaagaag ctgtccttcc                                                  20


<210>  73
<211>  20
<212>  DNA
<213>  Artificial Sequence

<220>
<223>  Primer pSeq_tDT_R

<400>  73
gtactgttcc acgatggtgt                                                  20


<210>  74
<211>  19956
<212>  DNA
<213>  Beta vulgaris

<400>  74
aaatgataca ggggtatatt tgactctatg aatttcagaa atctaatcaa atttgctaag      60

cttccaatga ttctactaag ccctacaaat tacaagaatt agttactttc atctctctgt     120

cggcttcaga accagaagtg tacaatatct tgtcaaacaa actctgctta gaggagctct     180

ttcgatcatc ttttttcgat ttggaagttc ccggtgatag gattgacatt gctgttttct      240

cggtcaattc ttctggatct tggttctgtc catctatctc tggctccatt aatctggtct     300

tccaattaat tccgatagcc tcagcttgct ctgcaaacaa gacctttgag atcggggagc     360

tgcagatatc cttataaact tcataaccag cagcacaggt tttcccacct tccaacaact     420

ttgataaagg atgtaggaga gagatagaat catcactcgt ttctaaccta tccttcaagg     480

caaggaagtt aacagccaag tctgccttac taaactgaac aaatactgca gtttcatcca     540

agttatagat gcaagcaact gagtatatac caaacacttt acagatgcat tgttttatgt     600

cacttgcctt gagttttggg gagaatcccc aaatcaaaac tatgttagga tgcaaaatgt     660

taagaaacct ccttttagct gaactgataa cgggaatttc attcatatca ccagtgctta     720

gattgatcac atctccactg ttccaactaa gatagagcag attggcatac ttgtgaatat 780
tctcactggc tattaaattc tcagaagaaa aatcaacacc aagattatga catgcttgtg 840
caaagacaca cccggtcatg aatgcatcat agccagcttc atgcttagcc ccagagttcc 900
aatttgagga cctgcaagaa aacatgggag taagatggtt tcacataaaa catgtgtaga 960
agtgcagtga acactggcga aaacaatcta attttacgaa ttcattcact cactcagctt 1020
caaattaagt ttccccttta tttagggtgc cccaaaaaga tacactcttc tgtttacctt 1080
ctctctccaa gcgaccaatc ttttctctct tctccaacat cgttttcttt ttctctctct 1140
acccactatc cattttgtcc tcctacattt gataactatt cttaatctcc aagaaaatcc 1200
aatgtgtgaa ataattacgg gacagggagt atacagaagc agcccccttg ccaatatagt 1260
ttacaaatta ccctcagaat taggcttacc tttcccaaag gagcaataaa ttcaaacaaa 1320
tctaaaaggt acaaggcatt aagtgccgaa cctcatgtca tcaacctgga cctccacctt 1380
cacacatgga tgtacaccac cattagagga ttgtccagag gctatctcag ggcacaacag 1440
agaaaatgct gaggccaatg acgtgctggc tttattcaag aatttttgaa ggctcgtgtc 1500
tgcattcaaa agtattttcg tgtcgacaac atgaggaaaa tacttgtgga tctcgagaac 1560
aaactcttca acagttgatg gaagaggacc aaagaattta tggtaaatat gtgccatatc 1620
tgcaaaaaat tataatggat aagatgacaa gaaaagatac taggaaggcc ttcaagtaca 1680
aatattatat catgatgctg gacgaccgat gctcccacaa ttatgtttgt taccaaatgc 1740
ttcgaaggat aattactaaa ttatgtgaat ggtggttacc aagtgtcccg gaccatgcaa 1800
taacttctcc tttcagtgac caacaagaag aagacgtacc taaaaagcaa ttgtgaccta 1860
caattagctt cttttcagca gcgagaaggt caaggacatg ccggaaacct gcagctgctt 1920
ttattttgcg agttgcttgc tggtgagacc catacttcac ctcctcctac aagaacaaac 1980
agaacaatca cacatgcaga aagttcccca cataccaagt tgctgtctgc taaacactga 2040
aactaactta tctctacaaa caatgaagga agttcctcac cagaaggttg atcttatcat 2100
tgtcagattc tacaaaaaca ataagcttct gcaagatggc actgccgtca tgagcacaca 2160
caaaaacaag atccttgaag tgcttccttg taacctgtaa ttgcagatca ttagtatata 2220
ttcaagatgt tataaattta ttgaaaagca gcgtctaaaa caataaaagt catgcttaag 2280
gcatagagcg atagagcata gacacttcag agtttaataa gagcaaatac tccaggagaa 2340
cataaatata tttcatatca caaatcctag taccaactgg caacggctaa ctgccaattt 2400
atgtactgct caaaaaggcc aagcatctaa aagatggctt aaaagtcgga ttttataaga 2460
aagtcgtcac atgattgcta ttacattgac atatcaaagg tcaaatgctg aaatttggtt 2520
cagcttgata tatattaagc atacaaacga tacgttgaca agaaagccta acaagacatg 2580

aagcatcagg cacataacat tcaaaagatt accaattcaa tcaacttcag ctgatgagaa 2640

gtaaatccat tcaatcgaag agcaggacgc atactaaaaa atatggtttc aaattgttgt 2700

ttagattcaa cagcacttgg aagctcggca tttctgttct gtagcaacat atcatgccaa 2760

tcactgagcc gaattttcat ccgttcggag aataaaatat cagctacatt gcccaaaggt 2820

aaatctctaa cttcattaga atatgcccat ttcccgttat atactgaatt caagcgactc 2880

aaagcctcgt cttcctgtcg tctagataaa taagacacgc ctgagattat acaatgtgta 2940

aatttaccac aataatgaca tcatactgac aaaatctcaa acaaatagtt ctaataaagt 3000

catgttatct gaaatttcta tgaataggaa attgaacaaa accttcatgc ttttttccaa 3060

ctaaaattga catcttctac attaccttca tgtatgcatg cattgaagtc aaactggtat 3120

tttgccaaga agtcaatcga agttgtttgg cacaggaatt catatgatgg gccatcagtg 3180

ggaagctctt gacgtggaaa tatataaaaa ttatgcctga caatgaacca ttgtaaaatt 3240

attagatgga gtatctctat ttattgttta cagccaattg agcttttaac aattactata 3300

ggtagtgttt ggaaacttgt atttcatttc aaataatgga attgaaatct ggaatttaaa 3360

gtttgtattt caattcctaa tcactgtttt gtaaaggggg tttgatagaa gagagagaaa 3420

tagaggttta atggaggaga gagaaaaagt gtgggtttac taaaaaaaag agaaataaat 3480

attagaaagt gtgggtttac tcatagagtt gggatatgta tgaggagaga attttcaaat 3540

gccaaggtaa tagcttgaat gacaaattta ataatttcaa attccatgtc atccaaacaa 3600

tagatttcat ccaaatccaa gatttgaaat gaaatcttgc tatccaaaca tatcataaat 3660

taattagtaa tttagacttg ctttctgctg cacttactta tggaaataat tttacttcag 3720

tccttaaata acccgcaatt tacatcaaag gcactaatat aaacacctag ttacgaaatg 3780

gaaatatcag atatacctgt aaaagtaaag aaacaaaaat acaaccctga gcatgaaggt 3840

atccttcaaa agtgcaatat ctgcatactt agaaccggaa ttagaagtgc gaatgcagac 3900

aataaccatc ccaggatcag aaacgtccaa gaaagttgag attcatcatc cattctcttt 3960

agcaaattta tgaactctaa tatataaatc ataccccccc ccccatccaa aagcaattgt 4020

caagctgcct gaacccctca taatttagga tacaacaaag taatcctaaa agacccttta 4080

caatactagt actcgggtat ttccacaatc ttctcatcat tgaatccaaa gcattgcatt 4140

tgaagaaatc aaatcataat ccattactat attagagcaa aatctatgtc attatagtat 4200

tggagagcaa gtatgactat taccccttta cactaggcaa aacacattgt cacaatgcta 4260

acttagtcat taaccaatat caatatggga ctgtggatat tcataaaatc gaagtttttc 4320

gcttgctcat aaactatctt tcattccagc acagtacaag agagaaaaga cagcattttc 4380

atacacttct ttctttagtt caaattcaca cagcagcaaa aaattcactt cttcatagct 4440

ttagctcagc aaacaaagca caaagcatgc aattactctc acacatagca caccaaaaaa 4500

acaaaaacca ctaaaaattc acacaaaaaa aaccaacaaa aattccatcg caatttcaac 4560

aatcaaaaca atcttctaag ttaaaaagag agataaagat gagaagaaaa actaacggat 4620

gagcaacgaa ggaattcttc gaagaatccc atcgaaatgg acaaacacca aattgaacaa 4680

cggcgaattt ctcagcagaa tctttcattt taaggtatcg aacatcgtgc cgatcaaact 4740

cgaacgattc gcgccaaggt gagcttgtaa ttccagtcat ttcgagatca atggcgacaa 4800

aatcggcaga ttttacatgc gtagtgaggt caattagggt ttcttcgaag tttgattttg 4860

ttacattttt caaagagaaa ttagttgttg atgttgaata atgatgataa gtagttaggg 4920

ttcgtagtaa ggtggaggaa agagaaaatg gcgtcactct gacaagcttc ttcattttgt 4980

tcttcttcct tagctctgtt ttcagtcact gcgccatttt tttaaaaaaa aggaagatga 5040

acaaagcaaa tattgaaccc aaattttgta attttggccc actttatatg tacccctccg 5100

tttcaaaata tggagcacgc cgcacacacg acatttaggg tcgaattttg aacattcttc 5160

aagatgatct aatggtataa tctctataat ttatatgtgg catattataa taagagtttt 5220

atgaagtcaa aaagtggatg tcatatattt aatgcatggt aagtttttcc taaatctgta 5280

tactagggta acatacatat gttgacttga agtatatata attcttgtag tataaatatg 5340

gctttggcca taagtagtaa tacacaacaa ctagaaaaat tgaaatcagt ccactgttat 5400

cttgtactct ataattttct gtttcctttt gtttcgcaac aaagacatat ttgtggtgaa 5460

agataatttt cgtaaattga atgacttata ttttgaaata aagagagtat taggtaaggt 5520

tacgtgcttt tcgcttgaat ttgttagacc tcaaatgtat atgtgattag aacggattgg 5580

ctctagtttt tattttatag aagtatatat gcatttttct tagagcacac tcgaaattac 5640

tttcggatag atatattcgg gaaaaaaaga ggttgaaggg aagttcatca ataattatgg 5700

taaaggaaaa aggacatcgt tacaattcta aattctagat aggatgtgat gataatccaa 5760

aagtcatctg aaaaactaaa caagtccaag atgctaatga ttcgagtaga gattgaatga 5820

gtgaccctaa ggattgtcaa ccctcttatt ctaacgtgtg taaaagaatt gacaactcta 5880

agagttactc aaacattttt cgattcgagt ggttaatata ccaatttgaa actattgaca 5940

ggagttattt taatgagtat aatggtcaat ggagcactga attccatctc acatagtcac 6000

atatttcatc tcaagttctg atgatttcaa acattgaaaa aagatgatac aagcaattaa 6060

ttcctaggga aacatattgt ggttttcatg gatacaagag tgagaataaa tcaaaactta 6120

ggctctaaca tttcttttct ctactagtaa ttgctaatta tatcaattca attgtcagtg 6180

taatcagtta atcaccaaat ctcttgtata gtcagtaaac tatacactgt ttagtcctct 6240

ggattttgcc cggtcgaatt atgcagcata accaaacttt gaagtttagt acttcctttg 6300

cacccaagtt agcttcacgg cccctgcctt ctggtggatg gtcaccctat gctttgagca 6360

ttctctgcaa tgcgcacgat attcaatgag aacgtcgcct tgaaaatcta aattgcaact 6420

aaaaattaga ttgaaatgaa acccacaaga gttgtttttc tgagtagttg gtgtagaatt 6480

cacaagtctt gctccattgt ttgaagatat gaagacaata atgtgctatg taaagtgcag 6540

ccgctagcta acagtggaag tggaaacttg atcattttac actcgcacaa gcgaaagctc 6600

ggctgacgtt gcaaactgaa gaaaaacctc tcaaaccaat tcgacttttg ctcaaagttg 6660

caaactaaag aaaaaggctg aatgcaaagc aagttcacca atgaacaata gatcggtgtt 6720

ggcctgaggc cacatcaagt gaagttgcct aattgcggcc ctctcatctg ttcacaggaa 6780

tcattttcca tatagaatca ctccaaaata aaagagcaaa gctgcaccag atgcagaagc 6840

ataactttca agacaactga tgacagataa atagcaaaag aatgcttaag aaatgatcaa 6900

aattgaatgg ctctggaatt acctcatcag ctgattttcc tttctctcta tctctctatc 6960

tctttactcg tctatggagc taccacatca catggcgttt catatgcttt ctgccgtcga 7020

actagacgtg cagcaaaagc tccatccatt gaatgcttca ctgggcatga gcgataaaac 7080

ccatcttcag ttaaaaagtc agatggaaca tatctgctta cagaatctct ttggaagtcc 7140

tatcacccaa caaagaatat attaaaatag agaaggagaa aagaacgtat ctatctgtca 7200

gcatccatat gaggtggaaa ctaggagtac tatataaagc cagtgcagta gctcctaccg 7260

gatgtctaag aaggaaggca gaaaccctat cttcgttttc ttcaagatca atggagcagg 7320

tactgtacac aagcacgcca tctggtttga ccagcctgta gatgttaaac aatcccacag 7380

acaaaaggga ataatatgag tgaaacaagt caacaggggg aaataaccaa taattctagg 7440

actgtcaaac tcaagctctt caaaaacaaa gatagctctt aatctcactt gcaagcagca 7500

tcgaacagct cgtcctgcaa cttctttagc tcttccatat cctctgactt tctattccaa 7560

cgcaaatccg gcctcttcag caaaaatata aagttggaac aaggctctta gatacaagaa 7620

ctgaaaaacc ttcgacatat aatggactct atcaaggcca caatgacaaa ttctaaacat 7680

gagcatgtat atcaataaaa tactaagaac cctttcaatg gtactgctag aaggtttatt 7740

gctacacttt ttagtacacc atctataggt tttatagtac catcaaaatg gttcatggtg 7800

ccataagaaa attttatgta tttatggtac tatctaccat atctaatttt ctctgtaaaa 7860

atgtatttgt agatagagac cacgagttcc tcttttagat actgactttt ttttttctac 7920

atgatggcca acagacttct caaacaaaaa gaaaaagaaa atatttagat aatatgagca 7980

acaaaatagc aaccacctac ttttgatagt acacccaggc ccgaacaagg aacatctaaa 8040

agaactttat caaacttcga agtgttgctg tcctgaaaag aatagaaagt aactgcttca 8100

acaaagaaga agaggcagaa agcaaagcta gtacgcattt tgcaatgact tactgaaaag 8160

gagcgaagat cagcatggat gcaagtgatc acattatcaa cacgctgcag cttggctgtt 8220

tcttcaagta tccgtaaccg acctttattt atgtccattg ctgatatcat acctgaaaag 8280

ctacacattt agaatgcaga accagcatca ttggtagtta agttatcact ataccttggc 8340

cattcaagcg agatgccatg aagagtgtct tccctccagg agcagcacag caatcaatga 8400

tgtgatcacc aggctgtgga tccagaacag aaacagctag acctgcagcg aagtatagat 8460

gtaaacttgg gttgggctgt cacatttttt cacatcttat cttcctttct attctttcaa 8520

aactgaggag aaatggttgg gatttctata aacgtgagaa aaatggcatc agattagatg 8580

gttttactgc atgaaaaaaa ttgaatgtgt ttcggcatca cattactaca aggtcaaaag 8640

cactatcttt gaaaatgtag gacataatgg gacagagatg tgctgacctg cactctcatc 8700

ctggactgag cataaacctt cttttagaag tccagtttgt atcacaatct aggatatgag 8760

aaaacaactc aagatgtaat tgctcctaag atatcaatca tttcataata aacataaaag 8820

ttattattac aagacagcac ctgcatccca cttctgatgc agacaaagtc atccaaatgc 8880

aaggaaggct catgcgggac ctgcggacaa agtgttgtga tgcgcataga tatcgaaaga 8940

aggccctgta tagcactaat gagataagat tcagtaacct tcagcatgtt gagcttcaca 9000

acaaggtcat ctcgagttaa tccttttgca atattggccc tagtaccaag aaaaccatat 9060

gtattaacaa gagaaaagtg gcatagggat ctttatgact taggtaagca gttggcaatt 9120

agagagaata aaacccccaa acctcaagct gaaactcgga acactattgt tccacatcat 9180

caatttgata gctccttctt gcccaagata cttggtccac cgtcttacca tccactgaaa 9240

ggaaggtatt aaaaggagaa aaagactcgt cagcatagaa aattgtacat cttaaatttt 9300

agaagtatag caccatcttc aggcatcagt caacgtaaat aaataccaca tctacaaata 9360

gaaccatact ttctggacag tcgggatcat gagcacagac aatactgcat gattattgcc 9420

tcgtattctc atgtatacaa gtatatgtaa cattaaatag cagtatttct tgagaaactc 9480

accacgggat gggaataagt tgtagcaagg gcacgtgctt gtgaacgatc atcaccctcc 9540

aatttgggta caggaaggga gtcattatcc tataaagaga aacagctttt gttttcaacc 9600

atatcaagac aaacagttta ttaaactata aacaacaaca atacacatgc acacacctac 9660

tgggaacaag atatatacta ctgataagta ttttctgatt gaagaaaaaa aatctcattt 9720

atttgcaaat atagatttaa tgacaagaaa gctttgaacc ttaaggaaaa ctagctttcg 9780

gaggatccca ttcaccatgt ttcctgcgcc tggtctaaga gcatacttgg caagattcac 9840

attctgcaat ataatccaac agtaagaaca cgacatggat ttagactcaa gtctctgaac 9900

ctatagaaca agtaaaatta gatcttatct catttgacaa tttaaaatta gatagtgcaa 9960

tattctgcag ttataagact tcatgtgtgc atactgcaca agtcatctta aaggtgttat 10020

taaagcttta attgccattt gacatcccct tgctcaactt tagcatgttt ttaggctaca 10080

acaatacgca ctgtctacat ggacatacaa attacaagcg tatggaaaag caataagcgc 10140

aaggaagtct tcagccagaa actctctatg agtccaacaa tatgcaacta aatatccaag 10200

taccgtgaat gagtaagaac taacctcgtc aacaacagca tatggtggca tttccagttt 10260

cacaatctca tagcatccaa tcctgaggat ctaaaattaa agataaatca atacacaaca 10320

tatgatatgg gtcggagcgt atataacaag tatagcaact acatttgaac agataacagc 10380

ctttgagaca ataaggaact ccgacattcc agtatatgcc agatttcata tctttagctc 10440

taaattgcca cgcaaaatgt tattgggcaa tatacctgta gcaggagagg ttccatgttc 10500

ctaaaggagc tttcatcatg gcatgaagaa acaataagat aatccagata ttttctccaa 10560

cgaattgaac caccaacaat gtcagtgacc tacaaagaca agttgtcaac ttaaaacttt 10620

tgaagcgtca tttcacttct gtagaccaat acaaaagcta ctactgcttt acatcataaa 10680

acctttagtc cttaggttca tctgattggc aaaaaaggtc cagatgcaag aaaagcaagt 10740

agctgtaatg ctgtattata tcagcattat tcagaacaga ataataaata tctacagatt 10800

ttgggtggaa gcttgatgat agagtatctc cacaaagaga actcgcttga gtcccaactc 10860

ccaaatctac ttttttggag tcacattatc agtcattttt tctggactct tataggaata 10920

gtgtgctatg taatgattta tggagcaggg gcatttcatg aatagcttta taagttagta 10980

tgggtgtctt ggggaataag ttaaagggtt agttagaggg aagaagtaca acatatatat 11040

agagcttttg taagaagggt ggttatgttg aaaatagatg agaaattggg tgagctcata 11100

gtagttcaat ttggactttg ggagagaatt aagcctcttg aaagcttgaa tatcatttac 11160

atttgttgtt tttactctta ttaatcaacc aaagttcatt ttcttccttt aatttctcca 11220

ttttagcact atgatttgtc caagctaagt gatttcttag catagtgcac agtgtagtat 11280

atcggagaac tcatttgagt cctgaaaggt cccacaagtt acatttttcc tactactact 11340

tgcaccaaaa caataagcat cattaagaca ttgtcactgg tccttcttag gttcttttgg 11400

aggggattcc tcagatgggg gaggcaccca tgaaggaaca tgttaccaag caatgggaca 11460

atgcaaaatg caccaataca gtagcttcac ttcattgatt gcatctatgt cacggaaaac 11520

tgaagaaaga agcaacacct caactttatc caggacagat atccactaac ctaggatgca 11580

agcttgagac tatttagcaa ttgcctctgg gatattaaat cagattacga ctatatttct 11640

acagttattg cttaagaaaa aggtacgatt tgaagcttgg gaagaaagag aacaagagta 11700

aaagaccaat ctgagatctc tttcatccag gtctctggtg cgaaatccaa gagtcctctc 11760

aacatattcc atctcattgt tccctgaacc ctttcctctc tcatttagaa gatcagcgaa 11820

ggcaccacca aactctatcc gcatcaatct cacagcagcc actggattta cacatgaaag 11880

caaaccagga gaaccataaa aatcacaaca aacttcctga tagcctactc actagcatca 11940

accattgtgt tcagcctaaa atgagcggct gttttcaatt gaacagcaac ttacatggac 12000

cactgcataa aagtgatttc ttaatccaga caaacaaaaa tgtttacttc aaccaactga 12060

atttgcatca gctcattagt gatttgacaa gttctaattt atgtatcaac aaacaagacc 12120

atatagctag gaaacaagag gcttaggcta agcttaatgc gtgaacaatg ttagatttca 12180

acctatcagc actgtggata actgcaaact gcgacttaaa taaggaagat aaaggaactg 12240

aatatgcaat ttcaaggtgc tcagcatttg aatcaacagt tacttcagat aattcagaac 12300

ataaaagatt tgaacattct aaggctacct catgattgca agcaatgtta cctgattcgc 12360

taaccctcac aagccacaag ccaaagaagc aatttggtaa atggttcatg gtacaactgt 12420

tcgcttttgg actaatctaa caatactagg tggtaaatta tgttcccata tctattacca 12480

taatgtacag caaattaggc agcactaatt ccaaatgacc caacaaaaaa agaggaagaa 12540

aatccaaaaa ttcaagccaa catatgcact aaaattacaa gcacaaaatc aaataatgag 12600

aatcacacta tccaaagaaa atttccatcc acatttatcc aacacaatta tctctctttt 12660

acacccaaat tatgtcaacc aaaaacacta aaacaagtga gtgcagtagc ttcacatcaa 12720

agaatatcaa tcacaaacac cacataataa aatttcaact cctgcccaaa caaaaaaaat 12780

ataaagaaaa aaaaacagca aaatttcaaa gataaaatag aaaaaaaaaa atcaaaatac 12840

agggggaaaa aaagtaaatt taccagctct atgaggcgaa acctgcaaat tcagcttctg 12900

ggttttctct gaaatatcaa gcacaataac cagcaattaa aaaaaattat aaataaaatt 12960

aaaaagaaaa gattgataat taaaatcaaa agagagcaat ttaaagcaca atcctttttt 13020

taccattttt tctgggagga agagcatcct tcgttttggg tttagacgaa aaaaatgaga 13080

gttgttgtat ttgtgcgcat gagtgatcat tgctggaaat gaaagtggga aagtggtaaa 13140

tgagtgcttt gtgaaattgg gttttgagga aaagtagaaa gaagaagaag ggtcgatgtc 13200

agagaagaga gagagtggat ggaaagtagt gatgattgcc tccattgttg ccggtgaagt 13260

gagctttctg caaatatttc actggactag ttttttttag cagataacgc taaaacagag 13320

aaagatgttc ggttaatttt aatttttgga catttaaatg actattcaat atgtttcaac 13380

cttttttttt taaaacaaag gaacaatact agtattagat tacgttaatg tttagtacat 13440

ccaatactta tgtgtgtttg acctaactta aaatcgtaag ttgtttaaaa tgtcggtgtc 13500

ttgtttttaa gagatatcat acttactatc tttggttttt actcttccat tgttaacaga 13560

aactgtattt atttgggtaa ggggtttgag tgaattcctg taagtatgag aaagttttga 13620

gtgaagcaag agaaagagag aagaaaggaa cttcgagtga agattgagag aaacaacagt 13680

tagtgggaac tgttgttggg aacttgagtt taggagctca ggttgtaccc cgagagaatt 13740

aataggtttg taacagagtc ggtggcctat tatagtggaa agtttgagtc aaaatccatt 13800

gtggccgatg tcgtttcttc ttattgggcc taggaagttt ttcctcgcta aaatttcctg 13860

tgttcccatt gtgtgttcct tagctagctt tcaattccgc aaaaagttac gtttattctc 13920

tcactataat tcacccccct cttatagtgc tcatattata caacaattga tatcaaagca 13980

ggaactctaa aaatacagaa atcatgttga gttcaagatc ttggaaaata tgaatactac 14040

agaaaaactg gaagaaaggt actctactca gagaccaccg atgttcaatg gcaaattcta 14100

cacaaactgg aagaactgaa tgaagatctt catcaaagcc gacaaatatc aggtttgtag 14160

aatcatagag gcaggcgatt ttgaagtcac taccactaat gacacatatg aggtaattcc 14220

taaattcata actcatttcg ataaagtata tttcgaaaag ttggaaatta acgttcttgc 14280

tattaaactg cttcattgtg gtcttagacc tcatgaacac aatcatgtca tgggatgcaa 14340

aatcgcaaaa caaatttggg atcttcttga agtcactcat gaaggtacgg gtaaagttaa 14400

gagatcaaaa atcgatcttt taatgaatca atatgaactt tttcaaatga aatataagga 14460

gtccactcaa gagatgttta cacgctttac taatactatt aatgagctaa cctctcttgg 14520

aaaagaaatt acatatgatg aacaggtaag aaaggtccca aggatcgttg gatggctaag 14580

gttacgcctt acaaaaaact aaggacttta cgaagttcaa tccggaacaa cttactggct 14640

cccttatgac tcacgagcta cacttggaca ctgagaatgg tgacttgtcc aaacagaagt 14700

cgattgcctt gaaagccatt tttgtcatac cgtcaattaa ttaagtaaaa agtggtaaaa 14760

gaattaccaa aaacgcacaa aataaattaa ttagttggat ataactaatt aacctattcc 14820

ttttttctgt cgctataact acttttgctt aacttattga tggtttgatc gttgaatcca 14880

agttttctcc acccacaaag atattataga ctttacttta aaaggtacga taaataatgt 14940

ttaatcaggt atgcatcaac cttgaaatta ttaatttatt aagatcaaat tatgcatatt 15000

tatattaaac gtacaggact tgtgcacaat ccatggatga tattgtagat tttgttgtaa 15060

aggagttagg gacaaatgat gttgaattaa gaatgatgag gaacaacatt gaggtaccta 15120

atggcataca agattatgtg gtaacaaagg tgaagaagtt ggttgtacca ggcaatacag 15180

cagcggcaag ccatatatag gatgagctac cataccctta tgttgtgaac tattgtcacc 15240

accaacaaga cattggtcat tacgacatca ctttagttga ggaatgataa acctcttttt 15300

gctagatatt tgcaaacatc tagcagataa agaggaataa aacactattt atatttcatg 15360

aacactattt gttagttgca tgaacactat ttttagttac acgaacacta gttttagtag 15420

catcatgaac actatttttt agcatcggaa ttttcacgac tactttttgg tttgactgac 15480

actctgcaat tttcgagata actttttggt gatatgggtc ccatgaaata gaagatttat 15540

atttcatgaa cactatttgt tagttgcatg aacaatattt ttagttacac gaacactagt 15600

tttagtagca tgaacactat tttttagcat cggaatcttt gcgactactt tttggtttga 15660

ctgacacttt gcaattttcg agataacttt tttgtttgac tgacaactat ttcctatata 15720

tattgacagt tttacccctg ttagatgttt gcaaacatct agcaaaaaga ggtttatcat 15780

tcctccactt tagttagccc aacctccagt aacgccatcc agaccactgt cgtttgtcac 15840

tacgacactt acgcttggca accctatgtc ctagcccttc gatacctcga tatccgtccg 15900

ggcaatgtcc ccagtttgtc acttctctgc cattaatgac atattttgga gtatcaaacc 15960

caactccaag tatatatcgc aacatggctc agtaaagaga gtcatataat catgacgtag 16020

tttctatatg ccatcctacg tagtatcttg taacatgaat aacagcctgg tttgcaggtt 16080

gatggtacat ggtataaatt ggtattactc cctccggtct ttattagttt aatcctttct 16140

tttgtacaga gttataggag aaataatatt gtgggtcata gaaggaaaga gaaattatta 16200

ttttatgtta aagttgaatg tatgtgtgat gaaaagttag tagtcccatt tcaaaataga 16260

aaaaaaaaag gtaaactaat aagggacatc ccaaaaagga atacgggtaa actaataaat 16320

atccatgcag gttgttggta catggtacat gaagccgtcc aaaaccttca aaagcagtaa 16380

gtcctgctgc tatgccatat tcaaatattc aactccaaaa aaaaaaaaaa aaaaaatcaa 16440

aaatccgctt ttcagcgaaa atataggaaa taatccaaga atcgaaatcg aaataaagtc 16500

atgatgcaag tttggagagc tgaagttaca ctatatcgga gtacttactc aaatgttgat 16560

tagtactccg tgcgtttgaa gtaaagtcac atatggagta gttccaagct aggttgtaca 16620

gtgacggata aggatactgg gttgaaaagg tgaacgtcga gatttatacg tgtatttatt 16680

taaacaggat acgtatcata ttgggttctc atacgcgtac cagctgtgac ttagaaaaat 16740

taaccacgct atataggttc caagccctca tgattacctt ttcatagtgt aaatttcatg 16800

tagttgaatg gtgggaatcc aatcacaaaa acactgcagg taatggaaat gttccaactt 16860

tttccaagca ttttaaaata agacatgtga ttactaatta gggcgtgttc ggcaacagta 16920

attgtggtga tagtttttag ctgtgagagt agttgttagc tgtgctatta gcttttagtg 16980

gttggtgtgt agctgttagc tgttagatgt ccaagtagcg gtgtaaaata ttgatgttcg 17040

gtaaaagaag ctgtcaaagt agctgtctaa gaataactag ttaaaaattc aaataaaact 17100

ttaacatata atttatacac cactaaaagc tacccaaaag ctacaaattg tagcttttga 17160

caaacactac taaaacacta cttgtaccac taaaagctac ttacaccact atcttgccaa 17220

acactcttat tttttctaat tagtgttttg acctagtcaa gacactaaaa gctacttaaa 17280

aagcttgtgc cgaacatgcc aattctgaac caaggaacaa actataacaa aaaagtgcta 17340

tgtgaaactt ttgtaggcaa cagaagtaag gcatttttgg aatgtactaa caaatccgta 17400

ttaagacttg tacatgaaaa ttaccgtggt aacatttacc cacacttcct cattcacgta 17460

ctccgattca ttcttataag ggcataaccg cataaggcac atcaagatcc atgtatctaa 17520

tagtttaatt tgcctctgtg tttctgtatt aacaatgagc atagtgagtg caaaagccat 17580

ggaagctaga ttaaaaaggc catcattcta agttagacaa ttggaaacaa catcgagata 17640

cacgtacaca taagggctgc tcttctctat tactccctct gttcctaatc atttgctttt 17700

ttagcgggtt ccaaaggcct atgtttgacc actaatatat ttaaattaaa actggtgata 17760

tatattaaaa gaaaattatg atgaatttaa caaaaaccat atatgttatg tccttttttt 17820

tcctatatta atgaattttt acagtcaaag ttggtgaact ttgacccaaa aaaagaaatg 17880

gagcaaaaaa aaaaaaaaaa aaaaaaaact agggacaatg agtaacattt ttatctatgt 17940

ctttttaata tgaatatacg taacaaattc tgcaaaaata gagatagcaa ctaataacac 18000

gcatgaaaat gacaagttat attatacctt tttttctcaa tatatgaata tacgtaacaa 18060

attaactcca gtagttttta gtaaaactat tagattattg tgtaacatat actctggaaa 18120

tagtactaag atccattaca atctttattg agaaatttcc tcatgtaccc cctgaggttt 18180

ggcgtaattt ccaaataccc ctcatatttg aggaatttct caaataccct gatgtttttg 18240

tttagactca aaataccttt actatggaca gtaccctaat gtcattaagt tttccccttc 18300

tctctcccca attttctctc tcctcccatt cccccaccca ctacccactg cccactgcca 18360

agtaggggtg taagtggatt ggactggatt ggactttgcc aaattcaaat ccagtccaaa 18420

gttttttgga ctcgagaaat tgagtccaag tccgatccaa atattttttg agtccagtcc 18480

aatctagtcc gataattttt tcttgagtcc gaatccagtc cagtccagtc cgattattat 18540

atcttttttc ccgatttagg ttcaatgatt cacaacattt tttgagatgc ttgagcattt 18600

gacatctgat tcaattatca atatccacaa ataagattga aagcttaaat taaagtaaaa 18660

tactatgaat aaaaagttga attagatgct taccttgatc taagttgaga ggaagcatag 18720

agactgagaa ttaatctgag ggacaaatag agaatgcgag agtcgagaca gtgaggtaga 18780

aagaaaatga agagtaagag gaagtgagta ttaaggactg aggagtaaag taagatagaa 18840

ttagttggct actagcctac taatgcagta ttgctagtat aatttactta tttaacaaat 18900

ggagctaagt gcaatagttt agcgccaatt gacatattta gagagagaag gctgaaaaat 18960

ccaatatttt taaaatagta tcattatttt taatatatac attatatata aaaatatttt 19020

tggactggac tggacatatt ggactccaaa gggatgagtc caaatccaga caaaaaatat 19080

ttggacttga aaatttaagt ccgagtccag tccgaaaaat tttcagtcca atccagtccg 19140

acaaatttgg actggactgg attggactct gaacttttcg tagtccgctt acacccctac 19200

tgccaagtgc caaactgcca accccctttt ggttgagttg atatttgacg caaagacttg 19260

gcgtgttgga aggttcatta cacattttat ccaagtcaac tttgaagtct tcttagctag 19320

agactagagt gaacgtgttg gaaggttcat tacacatttt atccaatcaa actttgaagt 19380

cttcttagct agagactaga gtgaacgtgt tggaaggttc atgttcatga cattataaaa 19440

gtaataatag tgaaatttca caaagtattt ataaacccag gacagactca agagctctac 19500

ttattattag tgaaaaacaa acatacacac gacaataaca caacataaac aataatgaac 19560

atgaaaatcc tccttttgtt tgtcttcctt catcacctcc actacttcat ccatggcaga 19620

acacttacag aacgccaagc tttactaagt atcaaatctg ccattactta tgattattat 19680

aactctctct cctcatggaa aaacacaaca caccactgca gttggccata catcacttgc 19740

tcctcctctt cttcttcttc ttctgttatt tctctcaact tcaccatgtt atttctcgaa 19800

ggaattctct cccctgatat aggcttcctc accaacctgc aaaacctctc tattcgatct 19860

aacctttttt ctggcccact cccccattct ctctctctcc tcacccaact ccgctatctc 19920

gacgtttccc aaaacagttt cacaggtcca atccca 19956

<210> 75
<211> 19206
<212> DNA
<213> Beta vulgaris

<400> 75
ccaacaattt gttagccgat gaagagcatc aaaaccaaaa aaaacaaaaa aaattgatta 60

atatgcatga gtgtgacctt gttttccaaa gtttagcatt actattagtg tctcaattca 120

taataataaa aaaattagct tgttcaagat ttgtattttt attcaaagat tttttttgtc 180

tcttgtgctt cttttatctt atatatattt tttgtatggt ttgtttttgt ttaatattag 240

tccctccgct caaaatgatc tttcacgctt gagattggca ttaaggtcaa gagatgttgc 300

taagctttag aataaaaaaa ttccaaatgc atagagggaa agaaagcgag acaaaatgtt 360

ggagaaggca gagtaaatga tgtgatggag gataaatagt agaagtgtga taccgaaagt 420

ttgaaaataa taaggaattt tatttcttgc tggcactttg ttctagtaca ggtttttagc 480

ccttcaaaat gtttataatg tagagtcaaa attaatatcc ttaactagtt tttaagtccg 540

ggttatatcc tagatattaa taatattcat ttattagtaa cattttattt tataaatata 600

atactaagca ttatttggtt tgctggttaa gactttagtg tatatctatt tctttttttt 660

tttattgtat gcgtgtttac ataaactaaa gactataagg gatagtacca cgtggcgcag 720

ttccttgctt aggaacgtct tttaatatat taactagtat ttgggcccgg gcgttgctcc 780

gggttggtat tgtgtttccg aacatgatgt gcagtttttc ccattcccac taaaatatat 840

aaaggaaaac tcaacattta aaagatacaa atataataat atggacactt aaaacatgat 900

taaaagttga ttgagatggt aattgtgtca tgttataata gtaagaggtt gcctaattga 960

ggttgaggtg gtggagtagt ggtatcgctt cccatctgtt atccctgagg tataaggatc 1020

aaacctcata ggactcattt gagtaatttc ccatatcctc ctctcaaatg agtccttttc 1080

atctgacaaa aaaaaagagt ctaattttaa attaaaatta gacgatcttt tataaaatcg 1140

gcactttctg cacataggtc acaatttttt tgtttctatc tctctgcttt ctttaatttc 1200

acagtctcca actctccatc aacatcttac ttattttaga atagatgatg tatggtagta 1260

ttaaatggta aagtactaaa gctcctataa tacacagaag cttacatagt atagattcgt 1320

acatgagaca aggttacaat atactttctc cgttcttttt atattacaat aattactatt 1380

ttaagtagtt tcacatctat tgtaacaatt ccaattttgt tatagaaagc aactttaata 1440

attgacaata ttgcccttac tttatcttat taaaaccatc attaattact cactttctct 1500

tataaaattg cttttatttt ctaaggatga tttctctcct attctagtta attaaagagt 1560

tacttttgtg ctaaactgct catttattcc aaatccttaa aaattgtgtc caaacgtatt 1620

gttgtaatat aaaaagaaca gaggtactat tagtttgaat aaattttgat cagattaggt 1680

cacctttagg gggcgtttgg ttaggggtat tctggaaagg gtaagggaat caacttactt 1740

aattccctta cttgttgttt gtttgctcaa tttaatgatt ccctttaccc accccttact 1800

cccaaagtcc tttactctca ttctccccac cccccaaggt ttcacttacc ctttcttgat 1860

tcatcattga ccatatcttt gaccacccaa ctaccaccac cacttgacca cctaatcacc 1920

taaccaccta attacccaac cactattacc acccaacccc tccacctgcc caccaatcgg 1980

caccataact gcccaaccgt cgcccaatca agccacccaa ccggcaccat aaccgcccaa 2040

ccaagccacc caaccggcac cagaaattgt accaagctac ccacacacgt gaaaaccacc 2100

cacccacaag ccctagaaaa aatggaagaa tcgagagaaa gggaggggag agaaaagatg 2160

cagcgactag aaggggaggg ggaggatgtg acggcaaggg gagagggaac ttcgcagcgg 2220

caaagggagg ggaaacgtcg cgtcggcaaa gggctaaggt ggaattgacg gggttgcagc 2280

aacaagggga gggcatggag acgtcgtaac cgcaagggga ggggcagcgg cagtggaact 2340

ggggtggaga ggggtagtgg cggcactagg gtgtgggaga ggtggcgggg gatatcaaga 2400

gaggggggat atggtggtgt tatggtggaa gcaagaagaa gaaagaggaa agacaatgta 2460

ctaaccaaac aacacattaa atctaagggt tttggtttcc tttccccatc tacccctttc 2520

ttgattccat tccctttacc cctttacaac caaactcccc cttagttttt actacttata 2580

accttcaatt ttggctgttt tttgtgacat tttttacttc tccgagcctg gtcatatttt 2640

ctcccgaaac atttcgagga aagtcgaagt gacttgtgaa gttgtgcggg tgcttggcac 2700

catttgtgtt gcctcgaaaa gcatctgaat accccattta ttcctttctc ctgaaaccca 2760

aaattacctc gcaataaacg aaaagatatc catatatttg ttccaagcca catgactcct 2820

ttccaacgac ctcccatgtg accatgtcct tagaaggcat cccgtggcgt tcgaagctcg 2880

gacccccgga aagtccgaaa gtgtgtatta taactttcaa ttttggctgt ttttgggata 2940

ttttttactt cttcgggcct tgtcatattt tctctcgaaa cattcatagg attgtcaatg 3000

tgacttgtaa gttgtaacgt tgcacgggtg cttggcacaa tttgcattgc ctcgaaaagc 3060

ctctgaacac cccatttgtt catttctcgt gaaatccaaa attgcctcga aaaaaacgta 3120

aaggcatcca catattcgtt ccaagccaca taactcattt ccaatgacct cccatagagt 3180

ccgtagctcg gaccccagga aagtccaaaa acgtgtacta taaccttcaa ttttggctgt 3240

ttttgggaca tgtttggact tcaccggcct ggtcatatta tcttccgaag cattcctaca 3300

aaatccgacg agactagtaa cgttgttacg cgggtgcttg acaccatatg tgttgcctta 3360

gaaagccttt aaacacccca tttgttcatt tttcgtgaaa cccaaaattg tcccgaaatg 3420

aacataaatg catccatgta ttcgttgcaa gccacatgat ttctttccaa tgacctccca 3480

tatccttagg aggcatgcat catgtggcgt tcggcgagcg ggtctcggga aagtccgaaa 3540

gcctgtgtta taaccttcaa ttttggctat ttttgggaca tttttggcct ttttcaagcg 3600

tgttcatatt ttctcccgaa gcattcctag gttaggcgat gtgacttgta aagcgtgggt 3660

acttggcacc attttctttg cctcgaaaag tctttgagca ccacatttgt tcatttctcg 3720

tgaaattcaa aattgcctcg aaatgaacgt aaagacattc acatattcat tccaagccac 3780

acatgactcc tttccaatga cctcccaagc ccctaggagt cgtcccgtgg cgttcggatc 3840

cggagctcgg gcccccgaga atgtccgaaa ccgtgtatta tgaccttcaa tttttgctgt 3900

ttttggaaca ttttttgact tctctgggct ggtcatattt tctcccgaaa catttgtagg 3960

actaccgacg tgacttgtaa tgttgcgtgg gtgcttggca caatttgcat tgcctcgaaa 4020

aacctttaaa caccgcattt gttcatttct cgtgacaccc aaaactgcct cgaaatgaac 4080

gtaaaggcat ccatatattc gtttcatgcc acatgactcc tttccactga cctcccatgt 4140

ccctagaaag caccccatat ccgaaagctt gtattataac cttcaatttt ggctgttttt 4200

gggacacttg gactttttcg gttcgttcat attttctctc gaaatgttcc tagaaaaggt 4260

gacgtgagtt gtaacgttgc gcgggtacat ggaaccattt gccttgcctc gaaaaacctc 4320

tgaacaccgc atttgttcat ttctcgtgaa actcataatt acctcaaaat gaacgtaaat 4380

gcatccatat attttttcca agccacttga ctcttatcca atgacattct atgtccttag 4440

aaggcactgc ttgtcgtcca taattcgggc cagggaaatg tatgaaagtg tgtattataa 4500

ccttcaattt tggctgtttt tgagacaatt ttttacttct ccgggactgg tcatattttc 4560

tcccgaaaaa atacttcgag tgccgacgtg acttgtaacg tcgcgcggat gcttgacacc 4620

atttgtgtta cctcgaaaag cctttgaaca ccacatttgt tcatttctcg tgaaacccaa 4680

aattgcctcg aaatgaacgt aaaggcatcc acatatttgt tccaagccac atgactcatt 4740

tccaattctc tcccatgtcc ctaggaggca tcccgtggcg ttcggagctc ggaccctggg 4800

aaagtccgaa agcgtgtatt ataaccttca attttggctg tttttgggtc attttttgac 4860

gtctcttggc ttggtcatat tttgtgccga aacattccca ggattgccga cttgacttgt 4920

aacattgctc gagtgcttgg cacaatttgc attgcctcaa aaagactcta aacaccccat 4980

ttgttcattt ctcgggaaac ccaaaattac ctcgaaatga acgtaaaggc atccacatat 5040

tcgttccatg ccacatgact cttttccaat gacctcccat gtccctagga ggcatcccat 5100

ggcattcgga gctcgaacac tgggaaagtc cgaaagcgtg tattgtaacc ttcaattttg 5160

gttgtttgtg ggacattttt gggcttctcc gggcctggcc atattttctc ccgaaacgtt 5220

ccttggaaag ccgaagtgag ttgtaacatt gcacgggtgt ttggcaccat tagtgttgcc 5280

tcgaaaagcc tttaaccaac ccatttgttc atttctcgtg aaacctaaaa ctgcctcgaa 5340

atgaacgtaa atgcatccac atattcgttc caagccacat gactcctttc caatgacctt 5400

ccaggcccct aggagtcatc ttgtggcgtt tggagctcag tccccggtaa agtctgaaag 5460

cgtgtattat aaccttcaat tttggttgtt tttaagacat tatttgactt ctccgggact 5520

gggcatatta tctcccgaaa cattactagg agtgccgacg tgacttgtaa cgccgcgtgg 5580

gtgcttggcg caattgtgtt gcctcgaaaa gccattgaac acccccattt gttcatttct 5640

cgagaaaccc aaaattgcct cgaaatgaat gtaaaggcat cgacatattc attccaagcc 5700

acatggctca tttccaatga cctcccatat ccctaggtgt acaccccatt tgtctgatgt 5760

tataatagca agaggtcacg ggttcaaatc ttgttacaag ctaattttac ttttgttaat 5820

tgacatgact tatgtacaca ttggacaatt atagtggagt aacaaaggtg acatgtgacg 5880

cgtatacatt atcacacacg tcttttaata tatttgtata gatctagatt taagagtaat 5940

ttttttaatg cgcaatactt ggccaatttc ttctgtatca aatcataggt ctttggttgg 6000

ttcataagag taaagaccaa aataataatc tgaactgcaa aaatttctcc caagagttaa 6060

aagtttgtat aagttagatt aaaaaaatta atgacatatg atgtagttgg acattaaata 6120

tgtaagttta gaagtaattg tgttaacata aaaaaagatt cgattataac ataaaaacta 6180

aagaaacaca aaggcgccgt acaacaatca atattaccca agtcccctca ttaatattaa 6240

gggatgacct agctcgtaca tatttaatta tctttgaaaa ttcgttgttc agacttgcta 6300

gttgctattc tatatttgta tattcattaa tcaatttttc aatatgtgag catttacatt 6360

ttaaactaga gcaaatattg tctcttttac tattttgttg ttgtcaaatt ttcaaaaata 6420

aattgctcaa atacttttcc tagtgacata aaaaatagag caaataatca aacagtagca 6480

gacccaggaa cttttacata atgtagacgg cataatgtgt taatttttgc ttcttttttc 6540

taatatcatc caataacaca attctgcttc tattagtttg tagtttcaga tgatgatacc 6600

caaacaataa gaccaagcaa caaattgata agatttgctt ctctttcttt ccacttggtg 6660

taactgtaac agctttgaag tttaacttca gtaatcagtt gcatatttgg catatgatca 6720

aaacaatcaa attattatgt atggaaaagc aaaaaacttc caggtttcca tctgaacaag 6780

gaggccaaga gggtggaagc aagcaaggat atatgatcat aaaatcctat gaatatgatg 6840

tacaaacctt ttctactgca attaggtaac ctaaatgata ccacctagga acagcaacaa 6900

cttatttaca gcactaaacc taaatcaggt taaagttaat cagaccacca tgtatctggg 6960

tggtctctcg agggaaagcg tctccatctg tatccgggta acagaggttt cttcttctcg 7020

atcctccttg gcttctgccc tcttaagttc ttcgaaggct ctcttggcat atacagtaaa 7080

cgcaacaatg gtaattattg ccactatgaa tgaaataaca ttgtacacaa tctccaccca 7140

tgttagatga tgattcccat acttgacatc tgcgaacgtc cttatcagtc tcccactgca 7200

aatgaatgct atcagcgtca atattcgaga taccaactca tttaactatt gaattgccaa 7260

aaacagatat ctttgaccat atatttgtta ctaaaaataa cgattgataa tgtgaaacta 7320

tcactgatag atttaaaaga acttttataa aagtatagtt tctctaatgt ataactgcag 7380

aaaatagaat ggggtagaca aatgaagtaa ttgttttgaa gaatgcaaaa ggtcaattca 7440

gtaatacttt tatacgtgat tgggggaagc attaaaaatc ccttctaaga taaagatgac 7500

ctcattggca atggaatcga catccacaga cccttgcatt agaacagagt ggaagtttct 7560

gtgaacttac gtgtagatgt aaagaaaagc ttctggcacc atccctgcaa ttgatcccca 7620

tagataaggc caaaacgtca tacttgtcac cacaactgcg tagttgaaga tagtataggg 7680

aaatggtgaa accctaaaga gtgccaccac gcggaactga tgaaaccagc taccttcggc 7740

agcaagccta agcatagcag ccttatccgg ccatctttgc aaccattgct aacaaggtac 7800

aaaaacataa acattgtgga cttaattaga caagaaagtt aaattaaaat caacattaga 7860

taatcaataa atcaaatgta agcagggaac atatttctta catggattct atcccggaag 7920

agcaatccaa gtaaataggg aagaatcatt ccaatagtag ttccaaccat gattatcaca 7980

aaaccaagac cataaccaaa gatcatgcct gcaagccaca tggatgggcc agaaggaatc 8040

agaaatacag ggaagattgc tagggaagta acaaggacca cagcaagaac cggacggcca 8100

aaggcagtgg cttcccattg catcattgga acaagaacct gcagagaaag taccaaaaac 8160

tttgaggcaa aaatttcctg cttgtatatt gcaaaaagta gtacagcgaa ggcattccgt 8220

gcagaatggc ttatagattg gaaatacgga gaacaatgca actataagca caggcccatc 8280

tcttgacttt tgggacaata acatggaccc ccagattgat ttataagttc tcacaccata 8340

gctagatttt gttggaactt tcataaatca tagtgacata agtatagcat aatattcatg 8400

ccttcgacag aagttttcgc atatggtaag gctactattg aaaaaattcc cttgtgtttg 8460

aagtacgcat aaaaatatct agtggcagtc aaccaaataa aacattctag gagtccctca 8520

aaaaattaaa gagtcatcag ttcagaagac tttaatatca atactttcta ttatccgggt 8580

ttggcatgca gtaaatttca tgagaaaagg aaaaatcagc tatttgatta tataaggaac 8640

taattcggat gtatcactaa gctttccatc gactggaaca tcgggagcta gtctccaata 8700

ctcgtcaagg atctaacata aacatcttct ccgcaatcaa aaagccaagg tcacatacat 8760

ctaggcctct gtctcattct gatggcatgg tatgatgcaa gttagacaac actattattt 8820

ggcagatgac acttaggggt ctaatattta agctcattca agataatcaa gtaatcaagt 8880

tcaatctcaa ggtttcagtt gcgctaaaaa atgtaatact tggctcattc agaattagtt 8940

tgttgaagct ggttggtatt tgcttcattt gttaatggaa ccaggctcat aaacaagctt 9000

tcattaggct aaacttattt aacaaaatca aaagcttaat actataattt ttgataggat 9060

ttcttttggg cagttataca tgagtaatga acaagctcta cacaatcttt tttaatgaac 9120

aagctttaat cgagctaggg tacgttctat tcaacttatt ggacctgaac ttattggaac 9180

ttatctgaac tgaacttatt gaacctgaac tgaacttatt ggaacttatt aaacctgatt 9240

ggacctgatt caacttattg gacctgattg aacctgattg gaacttattg gacctgattg 9300

aacctgattg accttattgg accttattgg aacttattga ccctgattga aacttattag 9360

accttattgg acctgattga aacttattag accttattga acctgattga aacttatttg 9420

accttattag acaaaaacat tattattatt attgttatta ttattattat tattattatt 9480

attattatta ttattattat tattattatt attattgtta acctgattga taacatttat 9540

atctttcata gttattagta acgaaaacat gttatctcta gttattcaaa gacgaattgc 9600

aaaatattgt aataataata ataataatat attattatta ttattattgt taaccttaat 9660

tatttgacca tgattataat attattcaat agcaatatga ataatcaaat aatagacaat 9720

aatacaagta taatactata cattgtggta ctttaataaa aaaattctaa taataacata 9780

atcagctaat agtaatatga ataataaaat aatagacata atacaaataa ataataaaat 9840

aatagacata atacagataa ataataaaat aatttacact aatacaagta taatactata 9900

taatcattgt ggtactttaa ttaaaattct aataataaca taatccgcta atagtgatat 9960

gaaattatga ataacaaaat agtggacaat aatacaaatg tttattaaac attgactatt 10020

tggaccttat tggaccttat tagacctgat tggaacttat tggaccttat tagacctgat 10080

tggaacttat tgcacctgat tggaacttat tacacctgat tggaacttat tgcacctgat 10140

tggaacttat tgcacctgat tggaacttat tgcacttatt agaccttatt gcaacttatc 10200

tgaacttatc tgaacttatt ggacctgaaa cttaattttt taagttgaac agaacgcacc 10260

cctagtatcc acgaacatag ttagttgttc atcgacaagg gtgttaattc cttgactata 10320

aaaaaaatat ctgctaatat gtcctccata ccatgtcttg atctgattcc caaaatcacg 10380

tgttttcgtg tctggtgacc acgttgctag acatggaaga caggtctaat tgttcagttt 10440

caagtcaggt tgattaaaca tatgttagca atatacaatc attattagtc aaactaattc 10500

aactcgggtt tggtttgatt caggttatgt cgaggatcag gtccaaatcg ggttaatcct 10560

tccaggtcaa atatatctaa gtctgttttg ccaaagtcta ctttttgtat ccgtgtccat 10620

gctaaatgac aaacaaaaag cagcttttac caagctcgaa tcagatttgt tcgcttaaag 10680

agtcacttcg ctcatttaca gcaacaatta aaggacaaaa cattgtccat tcaactactt 10740

acggatatta acttattggc aactgctagc gtaataaggc aatcaacagc actcggcctc 10800

aataatgaac ctacaaggag tccaatgacc aatacaaatt atcactggca tcatctagca 10860

cgacaatctc ttaactctaa gagtctaagt gccttgacat acaaaagtat tccttttaaa 10920

agtacccccg tgtggatatt ctgccaagca aatgcaatcg atacacccaa ttagggcttt 10980

tccattatga gtcctcagag cctcagattg taaaacaggt cagtaaaaga ggaaaatagt 11040

atttgattct tttgctaaac ccttggatat aagaatggtg acttgtattg tcacgccaag 11100

cttctttcat aaaagctgat catattatta tatgagagtt ctgagtttca aggtccgcat 11160

tcgatctaac tagacatcac ttccaattaa agttgagaaa cgaaactagg tgtcctcttt 11220

gtttcccaaa ggtgaacttt agatacttat tataagcata ttttgttatg aatcgggcta 11280

aggagagggc tactcttggt attgcataat tagttaatta cttagtagta gcttgaggaa 11340

taaggaagca agtaagttag aggaaagagt atgaaaatct gctataaagt gaggagagga 11400

gggatagaag gataatcaca aaattattga gttaactttg gttttagttg cttaggttgg 11460

gagtgtccag ccactcgaat gtcttgggac tgtaaacacc attgttcatg atctaattgc 11520

atcaatatta caattaactc atttctcttc ttatccatat tcatcttctt acaatcacaa 11580

ctatttccag atcatccatc caaatcttca tccacttgcc ttagtttcta ctccagattt 11640

cagtctatta caaattgatt tctacaatat gtcaattcat cacaaattat catgttttct 11700

gaacaaaagt tcactgtttc aggacaaata cagaaagaac tactttgatg cttagaacag 11760

atatattgta aaattgtatt cggaatttgg gatacaactg gagaagatat gaataaatag 11820

gcattcaggg agctcagaaa aacagaccgt gccatatggt gctctgctgc ataacaggaa 11880

ataatggata aagtatgaat aacgttataa cttcttaaaa acctagatga caagtatttt 11940

ggttgctttt tattattggt aggcaaggag aatactcaac aacagtttag ccttaaactg 12000

cttcttattt ctcctcttcc cctttttcct gatgatttgg ggttgtcact cagttctttt 12060

acctctcatt tccaggtact ttagagttat attacacaaa ggattgcaag agaagaacag 12120

gtcgccctgg catgcactca gaaagtatac gacccttcac aggaaatgtg gtgctccaag 12180

acttatatct caggctctca tgagtcatgt caaggaccat ctttaatcat ttgtattcta 12240

ggtttctcag gcgatgcggt gtgctggtgt gtctctccct cccacttgag tgtgtgtatt 12300

gtttgtgccc ctaagttttt atcttaacaa tcactactag tcaattagtc attaccaacc 12360

ctacccacct ctcttgttac tgttgttctt ggagatattt catatatgtc agcttagaac 12420

ttatattacg tttcttatta catattctct taagctcgcg cacatactct gtgatcgaag 12480

ggatccatat tagttatctt ttagtggagt tgttgtgaaa aaagactgca tagaaaaatt 12540

aagatagctc atagttgtaa atgtaattga acttttagat tgatagcctt gaggctgctt 12600

gcattgaacc aaccaaattc agccaggcta gtctatgcct ctttggtgtc acctggtagg 12660

ttgaatttgt gtagctgtag ttctacaaga gactgattta aaaatgtttt cgcactgaaa 12720

cagcttaaac cacaaaacag gaaagtgcag aacaaactcc agaaaatggt gcagaacata 12780

ccttctcaaa aaggaaagga actccccatt ttaacagtac gaggacaact gctacagcac 12840

taatggagga gatcaagatt ttgatccacc agatgaagga ttctgatctt gtttcagcct 12900

gagaatgtaa ggttgaagct tcaggcctct ttgtaatagc agatgtcacc agactaacaa 12960

attcactgtc gtcttgcata gcaggcccaa catctatgtc atgcttagtt agctccattg 13020

aatttggcat ctccaagaga tctcaagagc tgcccaaaaa gacggtacaa tattatgagc 13080

atacatgaca tgatgacaac ccataaagaa tatcataacc tgtcacattt tttattcaaa 13140

gttcaacagc cctcttacaa catgattgag aatggagggg aagagagaga gagttggtct 13200

cagacattga tcacataatc atttcaatta gttttaaagg tgctcatgaa atagaactag 13260

tgtcttaagc tggagacttc tgtatttttc atggttttag attatcaatc atattcttag 13320

aatctttgat ctctagaact ctttcctttc ctcccaatat tttttccact ttgtcttttg 13380

ttaattacgg cttcgctgca ggcctgcaat aaatctttta aatttttaca gatactatgt 13440

agagttgtat acataagctc taatctgaag acgattggtt tcgatgctag ttaatacaaa 13500

taaatatatt atggatataa tatgcagtaa attgggccat gggcaccagg gacaacttag 13560

acaagtatag tgcaactacc aggaaattta agctgggtac ctctgattca tcatgctggt 13620

tgataatatt attgcttcca caagtgttcg ctacggctca accaaactaa gtcacaactc 13680

acaagctgca caacccaact gacaattatc gcctattgtc taagctatac attacattac 13740

cccaatgcca caacgtggct cacgcctagg catggtaagg aagttcagat gtacgcagcc 13800

ttaccctttt aataacaaag aggctgtttc caggtgaccc ttaaatctta attgcaaaca 13860

ccatctgctg cttcacataa ataagcgact tcaaaattgt aaattaaaga atttgaatgc 13920

aaattgtgtg aaaaacaact ccatcaagaa tccattaagc acgctttact attagtatca 13980

ataataggaa acccttatat cccttttgac gaaggcacac atgcaacact aatgtgtcct 14040

tataaacttc atgaaagtat atctctacga aaccctttta gtcttatgtg attctttaag 14100

tgtccaactg atgattggtt acaaggtatt tagcccaaag tagcatttca gagagatggt 14160

gtagaatgag tagcttataa accgaggttg aggtgtaatc ctaataaatt aggaactaat 14220

accacaagag agatggacat gtagagatac aatatagtac agaataagat tatttgaaat 14280

ctttttacca gggaaactcc agaggtgttc cataaaacac aataccatat aactgggaga 14340

tcaatatttt agattaaaaa atataaaaat ctatttgggt tgagtatata gttggttagt 14400

ccaataatat ataaatttat aaggtggagg tcttcggtat atgacattcc aaatttgagt 14460

atcaaatgat atatatggtt ttccatactt gaatcccttt tcatgtacta cctctgtttc 14520

aaattaatag ttacacttac acttttcacg catgccaatg cagaactttg aggacatata 14580

tctttagttt tgtatttgta aaaattataa aaagtacata ttaataaaat acatattaat 14640

acgaatctaa caagatccca catgactatg attttattca cgtataaatc acaaacgagg 14700

gtcaaaatgc aattgtgaat agtgtaaaat gtcaaagtgt aactattaat ttgaaacgga 14760

ggtagtatgt gtttatgcaa cacttttcct tttccctttt ttgctattta gtaatttatg 14820

taaaatactt ccattgaccc aaaagttggg tgattatagt ttacatctat cattattatt 14880

tatcattact atagattatt caccattgta atcaacttta taaaagtata cacaggtaac 14940

tcaggagtca ggggtgctgg gccaaacact tttatagttt aaggtgaaaa atctcgagaa 15000

tcttctcctg ccacgcaaaa tgagtgttct tccactttaa agatgttata acacttatct 15060

taacctacta ttcgtaaata acacttatct taacctacta ttcgtcaaga catacttgct 15120

tcatctcact aagaacgtct tagttttcat ttgaaattcg taccagaaag attcacttca 15180

aatctattta tttttagata aattgttatt aaaaacgacg aagaaacgtc agaggacaac 15240

aaatcctcta aactccaaat tataagtgag tccaactatg ttgacgtaag gtaattagag 15300

tatccataaa agccctggcc gctttggccc acaaagcagc ttagaatact acccaacccc 15360

aaatataatc aatcaggtga ggaagctcgc aacagatgcg agagttccac tccaatcaaa 15420

ggcaccagaa catagccatc gacatcttct cttctttacc ccccttgaaa ccaacagatc 15480

ttaaggaagt ccactagtga acaaggacat aaccactact catgtggaat gccaatcagc 15540

ctctgtcaaa gggaagtcca ttagtgaaca aggacatacc cactgctcaa ggtagtcatg 15600

tggaaattgg aatcccaatc agcctttgtc aaaaggaata agccacatcg caatgaagaa 15660

aaaggtgcaa accagattta ttgcatctcc aacacgacat aaatatcgag aatgaggcct 15720

ttactgacaa aggaactctg gatttccaat ttccactgag cattggactc agttgagaag 15780

taattggtct tgctagattc tgtttacgca catactctta atgataaata aatgtaacag 15840

gccaattggt ctggaaaaaa acagttgata aaaggctagt ttgggccttg gggataaata 15900

taatctggta tgagttaata aatttctgtt taaggtaaag agaatgtgtt atgtgggata 15960

atttaatcaa gaaaatctta gtaagatgga ggtagtctaa cttccattcc tcaaaatgtg 16020

taattcctta taaaatcagt cagcctctag atacatagtt agcaaaaatg gaaggtatag 16080

aagtgggggt gagggaagag gaaggaaaga gaaccgcgat caatcatatt gttcgtgctc 16140

aagtttgagt tgtgcctata gctagttaga gtttgtctat ttcattgttt ttggtcagtg 16200

ttcatattct gagtgtcatc gtgtttgggt tctagaatgc tccttttcct aatgtcgaca 16260

tttctccact ttactctaga aaaatgatct cattgtagcc attccagctt caattttaat 16320

ggatactaag atccctttca ggaacaatgt taaggtagat gttagtgttt taacagccat 16380

gtggatgtta gtgtctagaa cgagtggtca aaacactact agcctcaaaa tattgtgatc 16440

agtctgaaaa ctctatgtta gatggttgct ttttttggta ggttcgcttg ttttgggggg 16500

ttagctttgt ttattttctt cacaatttgc ccttaaactt ttcacaaaat ctacaattga 16560

agattcttaa atagataaca gacgtgtcag ctacttcaac agctaattgt acgaaaaagt 16620

tcagctacct tgaaaccaaa ccactaacag ctagtacagt ttgtttctac tattacattt 16680

atctaatata acagctagta tttagtccaa cgatgtataa tatcaatgaa atggaactaa 16740

tctgtaaatt ggaccttagg cataagagtc gagttgagca ggtacactcc aatcaccaag 16800

ttatttaagc ttaaaatgtc taacttccaa tgctgtttga cgatactcat tgccaagtgt 16860

ttgttacaga tcaaccaagc aaataaagca acaagtgaac agctgcacta gtacccaact 16920

gcgaattttc gtcgattgcc aagtgcatgt ctgggacaca ataccatcat gtccataccc 16980

attaccttgc ttagccagct atcgtaatcc ataacacata aaaaccaaca aagtcttgat 17040

agtttcacaa atcaaaatgt tcacttttca ttccaaccaa aacaagcaat aaatctcttc 17100

atccatactc acaagaagaa caatctctca cactacccac ttgattagta aaaaccccaa 17160

tcaaaaacaa aatccaaccc acataaacaa atcaaattta gtaactaccc ataaactcaa 17220

aaacctcaaa tcacaatacc aataaaagag atatacaatc aatcaaaaaa aatacaacaa 17280

cagctaaaca aataacatca taaactaaag ttattcattt tatttcctaa ctagagatca 17340

attaagcagc ataaaacaac atcactaatt caagttaata atcatcaaat tctatactat 17400

aaaacataca taccttacca aaactaccca gctgaaaatt agggtagagc tccagaaatc 17460

ccggcgaaaa atccggtgag aaattcagct aaatttgaaa acttctttag gttaagtagt 17520

gtacacgatg aattgaagat ttttacaagc atatgaaaat ggtggttgaa attgaaatgg 17580

gggtttttga aaattgttgc gacgcgtaaa agtggaaaaa aaaaaggaga gaatcaaaga 17640

aatgagcaag tttttgtagg tgggtttact gttgttgctt ttgtttgtgc acattactga 17700

ctattcttaa ttcttccatg cgtgtggggg tgaaggaatt gttttcctaa gttgtttagc 17760

cacttcatag agtcattgga tttgaataat ctagggaata atgatcatgt gtttagtgta 17820

tctataaatt ataatttatg tatgtatatt gtatatgtgg tgaggcatag aggacaaggt 17880

ctaagaggaa tagaggattg tgagggagtg tttcatgctt ttaagaatga tgagtcattg 17940

agtgtattaa gttataagta gtatttgatc gagtagtaaa gtttgtatca cgtaaatcag 18000

agtgataatt aggaattggg atttgctcaa gtggtgagtt ttcccatctt tccgagcaag 18060

gtttctaggg ttcaattcct acctcaagca tttccttggg atttaagggg acggctcaga 18120

ggaattcttc ttaccaatat tttaaaaaaa aaaaaattaa gagtggtaat ttagttcaga 18180

tcctaccttt atccggttcg aaacgacttc aagaaaaaaa aatccgacat cgtttaaaat 18240

tttttacttc cgactcattt aatccgcctc caactttgaa acaagtagtc ttatttcttt 18300

tatgttaaga aaatttgcca aaaaaaccct ttttaaagtc cagttttgcg aaaaaaaaaa 18360

accttataaa gcattctttg tgaaaacaaa ccaaaaagta aattattttt gcaaaatgaa 18420

acctaatctc atttttcggt tttgaccatg gacttttcga cattgaccac ttctatttat 18480

cttcttcctc cataatcaca gcctagccac cactaccaac acctgccgct agcccccaca 18540

acctgcaccc ccacaacctc catccacccc ctcaagcggc aacccccctt attcccatac 18600

gcggcaaccc tacaccttat cctccacccc cctccgccct taccttttct cctctccctt 18660

cttccctcca tcacccctcc ccactctctt ctccctttgc cccccatcgt tgcaccaccc 18720

ataatccctc tctgtaaccc cctctcctcg cagctccccc tccctcccag ccaaggttga 18780

aaaattacag aggcagtcgc atatggggat gggggactat cgtctaaggg gtggagagag 18840

ggtttggggg ctgctggtgg gggtggggta ggctgaatgt ggtgggggct gagggtgggg 18900

ggtgaaggtg gggctgcagg tcgggctggc ggtatggaga aagaagggaa atagaagtgg 18960

ttaacaccgg aaagtccatg atcaacaccg aaaaatgaaa ttaggtttca tcttgcaaaa 19020

ataatttatt actttttgat ttgttttcgc aaagaatgct ttataaggtt ttttcgcata 19080

acatttagac ttttatcatc cctcttagat ttgacacata ttatacgaat tatactaaaa 19140

agactcctta tagtaattcg actaatgttt tattaaaatg aacctttaga ataactcggg 19200

taatat 19206

<210> 76
<211> 2000
<212> DNA
<213> Beta vulgaris

<400> 76
tacgtaacaa attctgcaaa aatagagata gcaactaata acacgcatga aaatgacaag 60

ttatattata cctttttttc tcaatatatg aatatacgta acaaattaac tccagtagtt 120

tttagtaaaa ctattagatt attgtgtaac atatactctg gaaatagtac taagatccat 180

tacaatcttt attgagaaat ttcctcatgt accccctgag gtttggcgta atttccaaat 240

acccctcata tttgaggaat ttctcaaata ccctgatgtt tttgtttaga ctcaaaatac 300

ctttactatg gacagtaccc taatgtcatt aagttttccc cttctctctc cccaattttc 360

tctctcctcc cattccccca cccactaccc actgcccact gccaagtagg ggtgtaagtg 420

gattggactg gattggactt tgccaaattc aaatccagtc caaagttttt tggactcgag 480

aaattgagtc caagtccgat ccaaatattt tttgagtcca gtccaatcta gtccgataat 540

tttttcttga gtccgaatcc agtccagtcc agtccgatta ttatatcttt tttcccgatt 600

taggttcaat gattcacaac attttttgag atgcttgagc atttgacatc tgattcaatt 660

atcaatatcc acaaataaga ttgaaagctt aaattaaagt aaaatactat gaataaaaag 720

ttgaattaga tgcttacctt gatctaagtt gagaggaagc atagagactg agaattaatc 780

tgagggacaa atagagaatg cgagagtcga gacagtgagg tagaaagaaa atgaagagta 840

agaggaagtg agtattaagg actgaggagt aaagtaagat agaattagtt ggctactagc 900

ctactaatgc agtattgcta gtataattta cttatttaac aaatggagct aagtgcaata 960

gtttagcgcc aattgacata tttagagaga gaaggctgaa aaatccaata tttttaaaat 1020

agtatcatta tttttaatat atacattata tataaaaata tttttggact ggactggaca 1080

tattggactc caaagggatg agtccaaatc cagacaaaaa atatttggac ttgaaaattt 1140

aagtccgagt ccagtccgaa aaattttcag tccaatccag tccgacaaat ttggactgga 1200

ctggattgga ctctgaactt ttcgtagtcc gcttacaccc ctactgccaa gtgccaaact 1260

gccaaccccc ttttggttga gttgatattt gacgcaaaga cttggcgtgt tggaaggttc 1320

attacacatt ttatccaagt caactttgaa gtcttcttag ctagagacta gagtgaacgt 1380

gttggaaggt tcattacaca ttttatccaa tcaaactttg aagtcttctt agctagagac 1440

tagagtgaac gtgttggaag gttcatgttc atgacattat aaaagtaata atagtgaaat 1500

ttcacaaagt atttataaac ccaggacaga ctcaagagct ctacttatta ttagtgaaaa 1560

acaaacatac acacgacaat aacacaacat aaacaataat gaacatgaaa atcctccttt 1620

tgtttgtctt ccttcatcac ctccactact tcatccatgg cagaacactt acagaacgcc 1680

aagctttact aagtatcaaa tctgccatta cttatgatta ttataactct ctctcctcat 1740

ggaaaaacac aacacaccac tgcagttggc catacatcac ttgctcctcc tcttcttctt 1800

cttcttctgt tatttctctc aacttcacca tgttatttct cgaaggaatt ctctcccctg 1860

atataggctt cctcaccaac ctgcaaaacc tctctattcg atctaacctt ttttctggcc 1920

cactccccca ttctctctct ctcctcaccc aactccgcta tctcgacgtt tcccaaaaca 1980

gtttcacagg tccaatccca 2000

<210> 77
<211> 2000
<212> DNA
<213> Beta vulgaris

<400> 77
ccaacaattt gttagccgat gaagagcatc aaaaccaaaa aaaacaaaaa aaattgatta 60

atatgcatga gtgtgacctt gttttccaaa gtttagcatt actattagtg tctcaattca 120

taataataaa aaaattagct tgttcaagat ttgtattttt attcaaagat tttttttgtc 180

tcttgtgctt cttttatctt atatatattt tttgtatggt ttgtttttgt ttaatattag 240

tccctccgct caaaatgatc tttcacgctt gagattggca ttaaggtcaa gagatgttgc 300

taagctttag aataaaaaaa ttccaaatgc atagagggaa agaaagcgag acaaaatgtt 360

ggagaaggca gagtaaatga tgtgatggag gataaatagt agaagtgtga taccgaaagt 420

ttgaaaataa taaggaattt tatttcttgc tggcactttg ttctagtaca ggtttttagc 480

ccttcaaaat gtttataatg tagagtcaaa attaatatcc ttaactagtt tttaagtccg 540

ggttatatcc tagatattaa taatattcat ttattagtaa cattttattt tataaatata 600

atactaagca ttatttggtt tgctggttaa gactttagtg tatatctatt tctttttttt 660

tttattgtat gcgtgtttac ataaactaaa gactataagg gatagtacca cgtggcgcag 720

ttccttgctt aggaacgtct tttaatatat taactagtat ttgggcccgg gcgttgctcc 780

gggttggtat tgtgtttccg aacatgatgt gcagtttttc ccattcccac taaaatatat 840

aaaggaaaac tcaacattta aaagatacaa atataataat atggacactt aaaacatgat 900

taaaagttga ttgagatggt aattgtgtca tgttataata gtaagaggtt gcctaattga 960

ggttgaggtg gtggagtagt ggtatcgctt cccatctgtt atccctgagg tataaggatc 1020

aaacctcata ggactcattt gagtaatttc ccatatcctc ctctcaaatg agtccttttc 1080

atctgacaaa aaaaaagagt ctaattttaa attaaaatta gacgatcttt tataaaatcg 1140

gcactttctg cacataggtc acaatttttt tgtttctatc tctctgcttt ctttaatttc 1200

acagtctcca actctccatc aacatcttac ttattttaga atagatgatg tatggtagta 1260

ttaaatggta aagtactaaa gctcctataa tacacagaag cttacatagt atagattcgt 1320

acatgagaca aggttacaat atactttctc cgttcttttt atattacaat aattactatt 1380

ttaagtagtt tcacatctat tgtaacaatt ccaattttgt tatagaaagc aactttaata 1440

attgacaata ttgcccttac tttatcttat taaaaccatc attaattact cactttctct 1500

tataaaattg cttttatttt ctaaggatga tttctctcct attctagtta attaaagagt 1560

tacttttgtg ctaaactgct catttattcc aaatccttaa aaattgtgtc caaacgtatt 1620

gttgtaatat aaaaagaaca gaggtactat tagtttgaat aaattttgat cagattaggt 1680

cacctttagg gggcgtttgg ttaggggtat tctggaaagg gtaagggaat caacttactt 1740

aattccctta cttgttgttt gtttgctcaa tttaatgatt ccctttaccc accccttact 1800

cccaaagtcc tttactctca ttctccccac cccccaaggt ttcacttacc ctttcttgat 1860

tcatcattga ccatatcttt gaccacccaa ctaccaccac cacttgacca cctaatcacc 1920

taaccaccta attacccaac cactattacc acccaacccc tccacctgcc caccaatcgg 1980

caccataact gcccaaccgt 2000

<210> 78
<211> 5488
<212> DNA
<213> Artificial Sequence

<220>

<223>  Codon-optimized LBcpf1

<400>  78
aagcttatcg atgtcgacag gccttaaggg ccagatcccc cgggctgcag gaattcgatc      60

tggcacgaca ggtttcccga ctggaaagcg ggcagtgagc gcaacgcaat taatgtgagt     120

tagctcactc attaggcacc ccaggcttta cactttatgc ttccggctcg tatgttgtgt     180

ggaattgtga gcggataaca atttcacaca ggaaacagct atgacatgat tacgaattca     240

aaaattacgg atatgaatat aggcatatcc gtatccgaat tatccgtttg acagctagca     300

acgattgtac aattgcttct ttaaaaaagg aagaaagaaa gaaagaaaag aatcaacatc     360

agcgttaaca aacggccccg ttacggccca aacggtcata tagagtaacg gcgttaagcg     420

ttgaaagact cctatcgaaa tacgtaaccg caaacgtgtc atagtcagat cccctcttcc     480

ttcaccgcct caaacacaaa aataatcttc tacagcctat atatacaacc cccccttcta     540

tctctccttt ctcacaattc atcatctttc tttctctacc cccaatttta agaaatcctc     600

tcttctcctc ttcattttca aggtaaatct ctctctctct ctctctctct gttattcctt     660

gttttaatta ggtatgtatt attgctagtt tgttaatctg cttatcttat gtatgcctta     720

tgtgaatatc tttatcttgt tcatctcatc cgtttagaag ctataaattt gttgatttga     780

ctgtgtatct acacgtggtt atgtttatat ctaatcagat atgaatttct tcatattgtt     840

gcgtttgtgt gtaccaatcc gaaatcgttg atttttttca tttaatcgtg tagctaattg     900

tacgtataca tatggatcta cgtatcaatt gttcatctgt ttgtgtttgt atgtatacag     960

atctgaaaac atcacttctc tcatctgatt gtgttgttac atacatagat atagatctgt    1020

tatatcattt tttttattaa ttgtgtatat atatatgtgc atagatctgg attacatgat    1080

tgtgattatt tacatgattt tgttatttac gtatgtatat atgtagatct ggactttttg    1140

gagttgttga cttgattgta tttgtgtgtg tatatgtgtg ttctgatctt gatatgttat    1200

gtatgtgcag cgaattcggc gcgccatggc tcctaagaag aagaggaagg ttagcaagct    1260

cgagaagttt accaactgct acagcctctc taagaccctc aggttcaagg ctatccctgt    1320

gggaaagacc caagagaata tcgacaacaa gaggctcctc gtcgaggatg agaagagagc    1380

tgaagattac aagggcgtga agaagctcct cgacaggtac tacctcagct tcatcaacga    1440

tgtgctccac agcatcaagc tcaagaacct caacaactac atcagcctct tccgtaagaa    1500

aaccaggacc gagaaagaga acaaagagct tgagaacctc gagatcaacc tccgtaaaga    1560

gatcgccaag gctttcaagg gaaacgaggg atacaagagc ctcttcaaga aggatattat    1620

cgagacaatc ctgcctgagt tcctggacga taaggatgag atcgctctcg tgaacagctt    1680

caacggattc actactgcct tcaccggatt cttcgacaac agggaaaaca tgttcagcga    1740

agaggccaag agcacctcta tcgctttcag atgcatcaac gagaacctca cgcgttacat    1800

cagcaacatg gacatcttcg agaaggtgga cgccatcttc gataagcacg aggtgcaaga 1860

aatcaaagag aagatcctca acagcgacta cgacgtcgag gacttttttg aaggggagtt 1920

cttcaacttc gttctcaccc aagagggcat cgacgtgtac aacgctatta tcggaggatt 1980

cgtgaccgag tctggggaga agattaaggg actcaacgag tacatcaacc tgtacaacca 2040

gaaaacgaag cagaagctcc cgaagttcaa gccgctctac aagcaggttc tctctgatcg 2100

tgagagcctc tcattttacg gtgagggtta cacctctgac gaggaagtgc ttgaggtttt 2160

ccgtaacacc ctcaacaaga acagcgagat cttctcgtcc atcaagaagt tggagaaact 2220

tttcaagaac ttcgacgagt acagcagcgc tgggatcttc gttaagaacg gacctgctat 2280

cagcaccatc agcaaggata ttttcggcga gtggaacgtg atcagggaca agtggaatgc 2340

tgagtacgat gacatccacc tcaagaagaa ggctgtcgtc actgagaagt acgaggatga 2400

caggcgtaag tcgttcaaga agatcggctc tttcagcctc gagcagcttc aagaatacgc 2460

tgatgctgat ctcagcgtgg tcgagaagct caaagagatc atcatccaga aggtcgacga 2520

gatctacaag gtgtacgggt cctctgagaa gttgttcgat gctgatttcg tcctcgagaa 2580

gagtctgaag aagaacgacg ctgtcgtcgc gatcatgaag gatttgctcg acagcgtgaa 2640

gtccttcgag aactatatca aggccttctt cggagagggc aaagagacta ataggacgga 2700

gtctttctac ggggatttcg tgctcgctta cgatatcctc ctcaaggtgg accatatcta 2760

cgacgccatc agaaactacg tgacccagaa gccttacagc aaggacaagt tcaagttgta 2820

ctttcagaac ccgcagttca tgggcggatg ggacaaagac aaagagacag attacagggc 2880

caccatcctc aggtacgggt ctaagtacta cctggccatc atggacaaga aatacgccaa 2940

gtgcctccaa aagatcgaca aggatgacgt gaacgggaac tatgagaaga tcaactacaa 3000

gctccttccg ggaccgaaca agatgcttcc taaggtgttc ttcagcaaga aatggatggc 3060

ctactacaac ccgtctgagg acatccagaa aatctacaag aacgggacct tcaagaaagg 3120

cgacatgttc aacctcaacg actgccacaa gctcatcgat ttcttcaagg acagcatctc 3180

gcgttacccg aagtggtcta acgcttacga ctttaacttc agcgagacag aaaagtacaa 3240

ggatatcgcc gggttctacc gtgaggttga ggaacagggt tacaaggtta gcttcgagag 3300

cgcctccaag aaagaggttg acaagttggt cgaagagggc aagctctaca tgttccagat 3360

ctataacaag gacttctccg acaagagcca cggaactcct aacctccata cgatgtactt 3420

caagctgctt ttcgacgaga acaaccacgg gcagatcaga ctttctggtg gtgctgaact 3480

cttcatgcgt agggcctcac tcaagaaaga agagttggtt gttcacccgg ccaactctcc 3540

aatcgctaac aagaatcctg acaacccgaa aaagaccacc acgctgtctt acgacgtcta 3600

caaggacaaa aggttcagcg aggaccagta cgagcttcat atcccgatcg ctatcaacaa 3660

gtgcccgaag aacatcttca agatcaatac cgaggtgagg gtgctgctca agcacgatga 3720

taacccttac gtgatcggaa tcgatcgtgg tgagagaaac ctcctctaca tcgttgtggt 3780

ggacggaaag ggaaacatcg tcgagcagta cagcctgaac gagattatca acaatttcaa 3840

cggcatcagg atcaagaccg actaccactc actcctcgat aagaaagaaa aagagcgttt 3900

cgaggccagg cagaactgga cttctatcga aaacatcaaa gagttgaagg ccggctacat 3960

ctctcaggtg gtgcataaga tctgcgagct ggtggaaaag tacgatgctg tgatcgctct 4020

tgaggacctc aactctgggt tcaagaacag tagagtgaag gttgagaagc aggtctacca 4080

aaagttcgag aagatgctca tcgacaagct caactacatg gtggacaaaa agagcaaccc 4140

ttgcgctacc ggtggtgctc ttaagggata ccagatcacg aacaagttcg agtccttcaa 4200

gagcatgagc acccagaacg gcttcatctt ctatatccct gcttggctca ccagcaagat 4260

cgatccttct actggtttcg tgaacctgct caagaccaag tacacctcga tcgccgacag 4320

caagaagttc atctcgtctt tcgacaggat catgtacgtg ccggaagagg atcttttcga 4380

gttcgctctc gactataaga acttcagcag gaccgacgcc gactacatta agaagtggaa 4440

gctctactcc tacgggaacc gtatcaggat cttccgaaat ccgaagaaaa acaacgtgtt 4500

cgactgggaa gaagtgtgcc tcacctctgc ctacaaagaa ctgttcaaca agtacggcat 4560

caactaccag cagggtgata tcagggctct tttgtgcgag cagagcgaca aggcattcta 4620

cagctcattc atggccctca tgtctctcat gctccagatg aggaactcta tcaccggaag 4680

gaccgatgtg gacttcctta tctctccggt caagaactct gacgggatct tctacgacag 4740

ccgtaactat gaggctcaag agaacgctat cctgccgaag aatgctgatg caaacggggc 4800

ttacaacatt gcgagaaagg ttctctgggc tatcgggcag tttaagaaag cggaagatga 4860

gaagctcgac aaggtgaaga tcgccatctc caacaaagag tggcttgagt acgctcagac 4920

ctccgttaag cacaagaggc ctgctgctac taagaaagct ggccaggcca aaaagaagaa 4980

gtgaggcgcg ccgagctcca ggcctcccag ctttcgtccg tatcatcggt ttcgacaacg 5040

ttcgtcaagt tcaatgcatc agtttcattg cccacacacc agaatcctac taagtttgag 5100

tattatggca ttggaaaagc tgttttcttc tatcatttgt tctgcttgta atttactgtg 5160

ttctttcagt ttttgttttc ggacatcaaa atgcaaatgg atggataaga gttaataaat 5220

gatatggtcc ttttgttcat tctcaaatta ttattatctg ttgtttttac tttaatgggt 5280

tgaatttaag taagaaagga actaacagtg tgatattaag gtgcaatgtt agacatataa 5340

aacagtcttt cacctctctt tggttatgtc ttgaattggt ttgtttcttc acttatctgt 5400

gtaatcaagt ttactatgag tctatgatca agtaattatg caatcaagtt aagtacagta 5460

taggcttgag ctccctagga tcaagctt 5488

<210> 79

<211>   989
<212>   DNA
<213>   Petroselinum crispum

<400>   79
aattcgaatc caaaaattac ggatatgaat ataggcatat ccgtatccga attatccgtt      60

tgacagctag caacgattgt acaattgctt ctttaaaaaa ggaagaaaga aagaaagaaa     120

agaatcaaca tcagcgttaa caaacggccc cgttacggcc caaacggtca tatagagtaa     180

cggcgttaag cgttgaaaga ctcctatcga aatacgtaac cgcaaacgtg tcatagtcag     240

atcccctctt ccttcaccgc ctcaaacaca aaaataatct tctacagcct atatatacaa     300

cccccccttc tatctctcct ttctcacaat tcatcatctt tctttctcta cccccaattt     360

taagaaatcc tctcttctcc tcttcatttt caaggtaaat ctctctctct ctctctctct     420

ctgttattcc ttgttttaat taggtatgta ttattgctag tttgttaatc tgcttatctt     480

atgtatgcct tatgtgaata tctttatctt gttcatctca tccgtttaga agctataaat     540

ttgttgattt gactgtgtat ctacacgtgg ttatgtttat atctaatcag atatgaattt     600

cttcatattg ttgcgtttgt gtgtaccaat ccgaaatcgt tgattttttt catttaatcg     660

tgtagctaat tgtacgtata catatggatc tacgtatcaa ttgttcatct gtttgtgttt     720

gtatgtatac agatctgaaa acatcacttc tctcatctga ttgtgttgtt acatacatag     780

atatagatct gttatatcat ttttttattt aattgtgtat atatatatgt gcatagatct     840

ggattacatg attgtgatta tttacatgat tttgttattt acgtatgtat atatgtagat     900

ctggactttt tggagttgtt gacttgattg tatttgtgtg tgtatatgtg tgttctgatc     960

ttgatatgtt atgtatgtgc agctgaacc                                       989


<210>   80
<211>   8726
<212>   DNA
<213>   Artificial Sequence

<220>
<223>   Resistance gene expression cassette

<400>   80
   tttatttaaa catgatacgt atcatattga gtactcatac gcgtaccagc tgtgacttag      60
   aaaaattaac cacgctatat aggttccaag ccctcatgat taccttttca tagtgtaaat     120
   ttcatgtagt tgaatggtgg gaatccaatc acaaaaacac tgcaggtaat ggaaatgttc     180
   caactttttc caagcatttt aaaataagac atgtgattac taattagggc gtgttcggca     240
   acagtaactg tggtgatagt ttttagctgt gagaatagtt gttagctgtg ctgttagctt     300
   ttagtggttg gtgtgtaact gttagctgtt agatgtccaa gtagcggtgt aaaatattga     360
   tgttcgataa aagaagctgt caaagtagct gtttaagaat aactagttat aaattcaaat     420
   aaatctttaa tatataattt atacaccact aaaagctacc caaaagctac aatctaccca 480
   aaagctacaa tctacccaaa agctacaaat tgtagctttt gacaaacact actaaaacac 540
   tacttgtacc actaaaagct acttacacca ctatcttgcc aaacgctctt attttttcta 600
   attagtgttt tgacctaatc aagacactaa aagctactta aaaagcttgt gccgaacacg 660
   ccaattctga accaaggaac aaactataac aaaaaagtgc tatgtggaac ttttgtaggc 720
   aacagaagta aggcattttt ggaatgtact aacaaatccg tattaagact tgtacatgaa 780
   aattaccgtg gtaacatttg cccacacttc ctcattcacg tactccgatt cattctgata 840
   aggcacatca agatccatgt atctaatagt ttaatttgcc tctgtgtttc tgtattaaca 900
   atgagcatag tgagtgcaaa agccatggaa gctagattaa aaaggccatc attctaagtt 960
   agacaattgg aaacaacatc gagatacacg tacacataag ggctgctctt ctctattact 1020
   ccctctgttc ctaatcattt gcttttttag cgggttccaa aggcctatgt ttgaccacta 1080
   atatatttaa attaaaactg gtgatatata ttaaaagaaa attatgatga atttaacaaa 1140
   aaccatatat gttatgtcct tttttttcct atattaatga atttttacag tcaaagttgg 1200
   tgaactttga cccaaaaaaa gaaatggagc aaaaaaaaaa aaaaaaaaaa aaaactaggg 1260
   acaatgagta acatttttat ctatgtcttt ttaatatgaa tatacgtaac aaattctgca 1320
   aaaatagaga tagcaactaa taacacgcat gaaaatgaca agttatatta tacctttttt 1380
   tctcaatata tgaatatacg taacaaatta actccagtag tttttagtaa aactattaga 1440
   ttattgtgta acatatactc tggaaatagt actaagatcc attacaatct ttattgagaa 1500
   atttcctcat gtaccccctg aggtttggcg taatttccaa atacccctca tatttgagga 1560
   atttctcaaa taccctgatg tttttgttta gactcaaaat acctttacta tggacagtac 1620
   cctaatgtca ttaagttttc cccttctctc tccccaattt tctctctcct cccattcccc 1680
   cacccactac ccactgccca ctgccaagta ggggtgtaag tggattggac tggattggac 1740
   tttgccaaat tcaaatccag tccaaagttt tttggactcg agaaattgag tccaagtccg 1800
   atccaaatat tttttgagtc cagtccaatc tagtccgata atttttcttt gagtccgaat 1860
   ccagtccagt ccagtccgat tattatatct tttttcccga tttaggttca atgattcaca 1920
   acattttttg agatgcttga gcatttgaca tctgattcaa ttatcaatat ccacaaataa 1980
   gattgaaagc ttaaattaaa gtaaaatact atgaataaaa agttgaatta gatgcttacc 2040
   ttgatctaag ttgagaggaa gcatagagac tgagaattaa tctgagggac aaatagagaa 2100
   tgcgagagtc gagacagtga ggtagaaaga aaatgaagag taagaggaag tgagtattaa 2160
   ggactgagga gtaaagtaag atagaattag ttggctacta gcctactaat gcagtattgc 2220
   tagtataatt tacttattta acaaatggag ctaagtgcaa tagtttagcg ccaattgaca 2280
   tatttagaga gagaaggctg aaaaatccaa tatttttaaa atagtatcat tatttttaat 2340
   atatacatta tatataaaaa tatttttgga ctggactgga catattggac tccaaaggga 2400
   tgagtccaaa tccagacaaa aaatatttgg acttgaaaat ttaagtccga gtccagtccg 2460
   aaaaattttc agtccaatcc agtccgacaa atttggactg gactggattg gactctgaac 2520
   ttttcgtagt ccgcttacac ccctactgcc aagtgccaaa ctgccaaccc ccttttggtt 2580
   gagttgatat ttgacgcaaa gacttggcgt gttggaaggt tcattacaca ttttatccaa 2640
   gtcaactttg aagtcttctt agctagagac tagagtgaac gtgttggaag gttcattaca 2700
   cattttatcc aatcaaactt tgaagtcttc ttagctagag actagagtga acgtgttgga 2760
   aggttcatgt tcatgacatt ataaaagtaa taatagtgaa atttcacaaa gtatttataa 2820
   acccaggaca gactcaagag ctctacttat tattagtgaa aaacaaacat acacacgaca 2880
   ataacacaac ataaacaata atgaacatga aaatcctcct tttgtttgtc ttccttcatc 2940
   acctccacta cttcatccat ggcagaacac ttacagaacg ccaagcttta ctaagtatca 3000
   aatctgccat tacttatgat tattataact ctctctcctc atggaaaaac acaacacacc 3060
   actgcagttg gccatacatc acttgctcct cctcttcttc ttcttcttct gttatttctc 3120
   tcaacttcac catgttattt ctcgaaggaa ttctctcccc tgatataggc ttcctcacca 3180
   acctgcaaaa cctctctatt cgatctaacc ttttttctgg cccactcccc cattctctct 3240
   ctctcctcac ccaactccgc tatctcgacg tttcccaaaa cagtttcaca ggtccaatcc 3300
   catcttctct ctctctcctc acccaactcc gctatctcca cgtttccggc aacagtttca 3360
   caggtccaat cccatctttt ctctctctcc tcacccaact ccgctatctc gacgtttccg 3420
   acaacagttt cacaggtcca atcccatctt ctctctctct cctcacccaa ctccgctatc 3480
   tcgacgtttc ctacaacaat ctaaatggca ctcttccctt atcggtcgtt gagaagatgt 3540
   cggagctcag ctaccttaac cttaggtata actctttcta cggtgagatt ccaccggagt 3600
   ttgggaaact taagaagctt gaaacattga atcttggtaa caacactctt tctgggagtc 3660
   ttccatctga gttgggttca ttaaagagtt tgaaacatat ggacttttct agtaatatgc 3720
   tatttggtga gatcccacaa tcttattctc ttcttcgaaa cttaatcgat attgatctta 3780
   atagaaacaa gttatatggg agtatacctg attatattgg agattttccg gagttggaat 3840
   cacttttatt agactcgaat aacttcacag ggagtatccc acaaaagtta ggtacaaacg 3900
   ggaagttgca atatctagat ataagtaaca acaattttag tggtagtttg ccactaagtc 3960
   tttgcaaagg agacaaactc caagatctgg acgcatccta taatttgttg gttgggtcaa 4020
   ttcctgagag tttgggaagt tgcaagtcac ttgaaggagt gtacatggga aataatttct 4080
   taaacgggtc gattcctaag ggcttgtttg ggagtgatgt ttcacttaat gacaaacttc 4140
   ttagtggagg tctcgatgag aaattcggtg attgcgttaa tcttcgggac attgatctct 4200
   ctaataataa gctatcaggg aagttacctg cgaccatcgg aaactgtatt catcttcggt 4260
   ccttgacgct ttataataac acctgtaccg gacgtatccc tcaagagatt agcaagtgta 4320
   agcagctaca gaccctcgat ctcagccaaa atcagttctc tggtgtgata cccaatgata 4380
   ttacaggtaa gaaagtatat taaacttgtt acttttgaaa atattcgctc tagtttttgt 4440
   ttcagttggt ccattctcac tttgtattat tgaaatatat cccaaaaaag taaatataat 4500
   tatataaaag aatcttgcta aaaataatat gaattatttt tgtatgtgca aaataatgta 4560
   caaatctaac taatttgttg tggataataa tattaattgt gtgaaatagt aaatgtgtgg 4620
   agatatataa ctttatttat catattcact caggttttta ggtatttatt atgagttttg 4680
   cattggagat atccaacttg acaatagtat ttttgtaata taccaatata taaagattac 4740
   tgtacataac caaaatgtat actttttcta tttttataaa cttatatatt cctcttcttt 4800
   gtatttatca caacattttt tatacccttt tgcctcatat taatagcaac acttataatt 4860
   tatttattta ctttttattt cttggtctat aacctcatct acccacatat gacacaccct 4920
   ataaaggacc cacatgatta accaaaatat acaaatatct tcaatgaaat taactttaac 4980
   actaatatga taaaaatcat gtcccgcttt ttatcctcta actaagactc tgcataaagg 5040
   tatattgcaa ttaatatgag atggaagagg tataataatt atatgatcaa attcctggat 5100
   tgaaaaataa atatgagatt aaaagtggta tgtttttggt taaaagaaac tatccataaa 5160
   gtatgttttt ggttaaaaga aactatgcaa cataccaatc aaatgtttat acgcttacaa 5220
   tttatgtacc acttttttgt cattgttttt ctattgtttg ccatacgtac gttactaaat 5280
   catgttgtct tttcacattt taactaacaa taaattacta ttgatacacc aaaaaaatct 5340
   atgagcattg gagtacgttg tttgatagaa gcttcgtgct attatttctt gtcaaagaat 5400
   ttcatatctc aatatcttct aatttaacaa tctaacgaaa tttttttgac ccaggaaaca 5460
   aatccatttg caatctggaa aagatacaaa cacttaaatt atcaaacaat gctttgactg 5520
   gtgaaatccc tcattgtgtt ggaaatatcg agctcatagc attatttctc caatcaaaca 5580
   aactgaacgg taccataccc gcaaacttct caaagttatg tgattcattg atatatctag 5640
   atcttagtga caatcaactc gaaggagttc tacctaagtc cttgtccaaa tgtcaaagtc 5700
   tagaactcct aaatgtcggg aacaataggc taagagataa atttccttca tggttagaca 5760
   acctcccacg tctccaagtt ttcagtgtgc gttttaacgc cttctacggt cctataacta 5820
   gctcaccaaa agttagtcac ccatttccta tgctacaaat tatcgaccta tctaacaata 5880
   agttttgtgg caagttgcca agaagatata tcaaaaactt tgcaaccatg cgcaatatga 5940
   atgagtctgg tgttgggaat ccacagtacc tgggggactc atcaatatat agtattacgt 6000
   actctatggt attgacattc aatgggttac aacaaaaata tgaaaagctt attgtgacga 6060
   tgtcgacctt tgatatatcc agcaacaact ttactggaca gattccatat gttatagggg 6120
   gattacgctc acttcgtaac cttaatctct ctcataatgt cttaaccggg aacattcctc 6180
   catcaattgc aaaattgtct ttgcttcaag atttggacct ttcatcaaac agacttactg 6240
   gtcgtatccc tcaagaatta gttagtttaa catttcttgg gagtttcaat gtttcgaaca 6300
   atctattgga ggggtctata cctcatggtt tcaacttcga cacgtacaca gctaattcat 6360
   accaggggaa tctcgaatta tgtggaaaac cattacctga gtgtggagaa agaagggcaa 6420
   aaggcaccac taataatcaa gatgatccta aaaatgataa tgaacgaatg ttgtcgatgt 6480
   ccgaaatcgt agttatgggg tttggcagtg gtgtactagt tgggttggct tggggatact 6540
   atatgttttc agtgggaaag cccttttggt ttatcaagat ggctagcaaa atggaatcaa 6600
   tattgattgg ttttttctga ccaacaattt gttagccgat gaagagcatc aaaaccaaaa 6660
   aaaacaaaaa aaattgatta atatgcatga gtgtgacctt gttttccaaa gtttagcatt 6720
   actattagtg tctcaattca taataataaa aaaattagct tgttcaagat ttgtattttt 6780
   attcaaagat tttttttgtc tcttgtgctt cttttatctt atatatattt tttgtatggt 6840
   ttgtttttgt ttaatattag tccctccgct caaaatgatc tttcacgctt gagattggca 6900
   ttaaggtcaa gagatgttgc taagctttag aataaaaaaa ttccaaatgc atagagggaa 6960
   agaaagcgag acaaaatgtt ggagaaggca gagtaaatga tgtgatggag gataaatagt 7020
   agaagtgtga taccgaaagt ttgaaaataa taaggaattt tatttcttgc tggcactttg 7080
   ttctagtaca ggtttttagc ccttcaaaat gtttataatg tagagtcaaa attaatatcc 7140
   ttaactagtt tttaagtccg ggttatatcc tagatattaa taatattcat ttattagtaa 7200
   cattttattt tataaatata atactaagca ttatttggtt tgctggttaa gactttagtg 7260
   tatatctatt tctttttttt tttattgtat gcgtgtttac ataaactaaa gactataagg 7320
   gatagtacca cgtggcgcag ttccttgctt aggaacgtct tttaatatat taactagtat 7380
   ttgggcccgg gcgttgctcc gggttggtat tgtgtttccg aacatgatgt gcagtttttc 7440
   ccattcccac taaaatatat aaaggaaaac tcaacattta aaagatacaa atataataat 7500
   atggacactt aaaacatgat taaaagttga ttgagatggt aattgtgtca tgttataata 7560
   gtaagaggtt gcctaattga ggttgaggtg gtggagtagt ggtatcgctt cccatctgtt 7620
   atccctgaga tataaggatc aaacctcata ggactcattt gagtaatttc ccatatcctc 7680
   ctctcaaatg agtccttttc atctgacaaa aaaaaatgtc taattttaaa ttaaaattag 7740
   acgatctttt ataaaatcgg cactttctgc acataggtca caattttttt gtttctatct 7800
   ctctgctttc tttaattcta cagtctccaa ctctccatca acatcttact tattttagaa 7860
   tagatgatgt atggtagtat taaatggtaa agtactaaag ctcctataat acacagaagc 7920
   ttacatagta tagattcgta catgagacaa ggttacaata tactttctcc gttcttttta 7980
   tattacaata attactattt taagtagttt cacatctatt gtaacaattc caattttgtt 8040
   atagaaagca actttaataa ttgacaatat tgcccttact ttatcttatt aaaaccatca 8100
   ttaattactc actttctctt ataaaattgc ttttattttc taaggataat ttctctccta 8160
   ttctagttaa ttaaagagtt acttttgtgc taaactgctc atttgttcca aatccttaaa 8220
   aattgtgtcc aaacgcattg ttgtaatata aaaagaacag aggtactatt agtttgaata 8280
   aattttgatc ggattaggtc acctttaggg ggcgtttggt taggggtatt ctggaaacgg 8340
   taagggaatc aacttactta attcccttac ttgttgtttg tttgctcaat ttaatgattc 8400
   cctttaccca ccccttactc ccaaagtcct ttactctcat tccccccacc ccccaaggtt 8460
   tcacttaccc tttcttgatt catcattgac catatctttg accacccaac taccaccacc 8520
   acttgaccac ctaatcacct aaccacctaa cccaaccact attaccaccc aacccctcca 8580
   cctgcccacc aatcggcacc agaactgccc aaccgtcgcc caatcaagcc acccaaccgg 8640
   caccataacc gcccaaccaa gccacccaac cggcaccaga aattgtacca agctacccac 8700
   acacgtgaaa accacccacc cacaaa 8726

<210> 81
<211> 20
<212> DNA
<213> Artificial Sequence

<220>
<223> Primer

<400> 81
atgttatctt taccacagtt 20

<210> 82
<211> 24
<212> DNA
<213> Artificial Sequence

<220>
<223> Primer

<400> 82
gtccctaaat gaaatacgta aaac 24

<210> 83
<211> 3706
<212> DNA
<213> Artificial Sequence

<220>
<223> PCR Product

<400> 83
atgttatctt taccacagtt tgttgctctg acacaaccgg taaatgcatt ggcctttgtt 60

tttgatggca tcaactttgg agcatctgat tttgcatatt cagccttttc catggtaatt 120

cttttacaag aattttcatt ctttcttaag tataaacact tagcttggga caaacttctg 180

atcctatttc ttaatttttg caggtgatgg tggctgttat gagcattttg tgtttgatgt 240

ttctttcttc tcattacggt tttattggga tctgggtggc tctaactatt tacatgagcc 300

tccgcgcgtt tgctgaaggc gggaaacgac aatctgatcc ccatcaagct tgagctcagg 360

atttagcagc attccagatt gggttcaatc aacaaggtac gagccatatc actttattca 420

aattggtatc gccaaaacca agaaggaact cccatcctca aaggtttgta aggaagaatt 480

ctcagtccaa agcctcaaca aggtcagggt acagagtctc caaaccatta gccaaaagct 540

acaggagatc aatgaagaat cttcaatcaa agtaaactac tgttccagca catgcatcat 600

ggtcagtaag tttcagaaaa agacatccac cgaagactta aagttagtgg gcatctttga 660

aagtaatctt gtcaacatcg agcagctggc ttgtggggac cagacaaaaa aggaatggtg 720

cagaattgtt aggcgcacct accaaaagca tctttgcctt tattgcaaag ataaagcaga 780

ttcctctagt acaagtgggg aacaaaataa cgtggaaaag agctgtcctg acagcccact 840

cactaatgcg tatgacgaac gcagtgacga ccacaaaaga attccctcta tataagaagg 900

cattcattcc catttgaagg atcatcagat actcaaccaa tccttctaga agatctaagc 960

ttatcgataa gcttgatgta attggaggaa gatcaaaatt ttcaatcccc attcttcgat 1020

tgcttcaatt gaagtttctc cgatggcgca agttagcaga atctgcaatg gtgtgcagaa 1080

cccatctctt atctccaatc tctcgaaatc cagtcaacgc aaatctccct tatcggtttc 1140

tctgaagacg cagcagcatc cacgagctta tccgatttcg tcgtcgtggg gattgaagaa 1200

gagtgggatg acgttaattg gctctgagct tcgtcctctt aaggtcatgt cttctgtttc 1260

cacggcgtgc atgcttcacg gtgcaagcag ccgtccagca actgctcgta agtcctctgg 1320

tctttctgga accgtccgta ttccaggtga caagtctatc tcccacaggt ccttcatgtt 1380

tggaggtctc gctagcggtg aaacccgtat caccggtctt ttggaaggtg aagatgttat 1440

caacactggt aaggctatgc aagctatggg tgccagaatc cgtaaggaag gtgatacttg 1500

gatcattgat ggtgttggta acggtggact ccttgctcct gaggctcctc tcgatttcgg 1560

taacgctgca actggttgcc gtttgactat gggtcttgtt ggtgtttacg atttcgatag 1620

cactttcatt ggtgacgctt ctctcactaa gcgtccaatg ggtcgtgtgt tgaacccact 1680

tcgcgaaatg ggtgtgcagg tgaagtctga agacggtgat cgtcttccag ttaccttgcg 1740

tggaccaaag actccaacgc caatcaccta cagggtacct atggcttccg ctcaagtgaa 1800

gtccgctgtt ctgcttgctg gtctcaacac cccaggtatc accactgtta tcgagccaat 1860

catgactcgt gaccacactg aaaagatgct tcaaggtttt ggtgctaacc ttaccgttga 1920

gactgatgct gacggtgtgc gtaccatccg tcttgaaggt cgtggtaagc tcaccggtca 1980

agtgattgat gttccaggtg atccatcctc tactgctttc ccattggttg ctgccttgct 2040

tgttccaggt tccgacgtca ccatccttaa cgttttgatg aacccaaccc gtactggtct 2100

catcttgact ctgcaggaaa tgggtgccga catcgaagtg atcaacccac gtcttgctgg 2160

tggagaagac gtggctgact tgcgtgttcg ttcttctact ttgaagggtg ttactgttcc 2220

agaagaccgt gctccttcta tgatcgacga gtatccaatt ctcgctgttg cagctgcatt 2280

cgctgaaggt gctaccgtta tgaacggttt ggaagaactc cgtgttaagg aaagcgaccg 2340

tctttctgct gtcgcaaacg gtctcaagct caacggtgtt gattgcgatg aaggtgagac 2400

ttctctcgtc gtgcgtggtc gtcctgacgg taagggtctc ggtaacgctt ctggagcagc 2460

tgtcgctacc cacctcgatc accgtatcgc tatgagcttc ctcgttatgg gtctcgtttc 2520

tgaaaaccct gttactgttg atgatgctac tatgatcgct actagcttcc cagagttcat 2580

ggatttgatg gctggtcttg gagctaagat cgaactctcc gacactaagg ctgcttgatg 2640

agctcaagaa ttcgagctcg gtaccggatc ctctagctag agctttcgtt cgtatcatcg 2700

gtttcgacaa cgttcgtcaa gttcaatgca tcagtttcat tgcgcacaca ccagaatcct 2760

actgagtttg agtattatgg cattgggaaa actgtttttc ttgtaccatt tgttgtgctt 2820

gtaatttact gtgtttttta ttcggttttc gctatcgaac tgtgaaatgg aaatggatgg 2880

agaagagtta atgaatgata tggtcctttt gttcattctc aaattaatat tatttgtttt 2940

ttctcttatt tgttgtgtgt tgaatttgaa attataagag atatgcaaac attttgtttt 3000

gagtaaaaat gtgtcaaatc gtggcctcta atgaccgaag ttaatatgag gagtaaaaca 3060

cttgtagttg taccattatg cttattcact aggcaacaaa tatattttca gacctagaaa 3120

agctgcaaat gttactgaat acaagtatgt cctcttgtgt tttagacatt tatgaacttt 3180

cctttatgta attttccaga atccttgtca gattctaatc attgctttat aattatagtt 3240

atactcatgg atttgtagtt gagtatgaaa atatttttta atgcatttta tgacttgcca 3300

attgattgac aacatgcatc aatcgacctg cagccactcg aagcggccgc cactcgagtg 3360

gtggccgcat cgatcgtgaa gtttctcatc taagccccca tttggacgtg aatgtagaca 3420

cgtcgaaata aagatttccg aattagaata atttgtttat tgctttcgcc tataaatacg 3480

acggatcgta atttgtcgtt ttatcaaaat gtactttcat tttataataa cgctgcggac 3540

atctacattt ttgaattgaa aaaaattggt aattactctt tctttttctc catattgacc 3600

atcatactca ttgctgatcc atgtagattt cccggacatg aagccattta caattgaata 3660

tatcctaagt aaaacctcat aggttttacg tatttcattt agggac 3706

<210> 84
<211> 26
<212> DNA
<213> Artificial Sequence

<220>
<223>  Primer

<400>  84
cgctgcggac atctacattt ttgaat                                          26


<210>  85
<211>  28
<212>  DNA
<213>  Artificial Sequence

<220>
<223>  agttaacttt ccacttatcg gggcactg

<400>  85
agttaacttt ccacttatcg gggcactg                                        28


<210>  86
<211>  751
<212>  DNA
<213>  Artificial Sequence

<220>
<223>  PCR Product

<400>  86
cgctgcggac atctacattt ttgaattgaa aaaaaattgg taattactct ttctttttct      60

ccatattgac catcatactc attgctgatc catgtagatt tcccggacat gaagccattt     120

acaattgaat atatcctaag taaaacctca taggttttac gtatttcatt tagggactaa     180

aatggtttag gataattact ttagctaaca taagataata aataaataaa taaataaaaa     240

taaaatggtt gtagataaat aaggaaatca ataatgaata tgagtgtgag tgataggacg     300

ggaatgggaa acttttacac tactttaacg ctattgaacg agtatgagta tgttataaac     360

gtaaaatgtt ttatgtgtta gacaatggcc tcaagtgaaa gtgaccctat taatggagga     420

aatgcaaacc acgagtctga ggtcacgctc gaagaaatga gggcaaggat cgacgcattg     480

cgtagcgacc ctgtttttgg agatgccacg ggagatgcta gtgataaccg aatggattta     540

atgaggttga tgatgatgga gcttttacaa ggaaatcgac aaaggcctag aactgaacaa     600

gaagagtgct caaacatgtt caagaggttt tcggctcata agcccccaac ttatgatgga     660

aagccagacc ccactgagtt tgaagaatgg ctcaacggca tggaaaaatt gttcgatgcc     720

acccagtgcc ccgataagtg gaaagttaac t                                    751


<210>  87
<211>  30
<212>  DNA
<213>  Artificial Sequence

<220>
<223>  Primer

<400>  87
gctctgacac aaccggtaaa tgcattggcc                                        30


<210>  88
<211>  29
<212>  DNA
<213>  Artificial Sequence

<220>
<223>  Primer

<400>  88
gcagattctg ctaacttgcg ccatcggag                                         29


<210>  89
<211>  1042
<212>  DNA
<213>  Artificial Sequence

<220>
<223>  PCR Product

<400>  89
gctctgacac aaccggtaaa tgcattggcc tttgtttttg atggcatcaa ctttggagca      60

tctgattttg catattcagc cttttccatg gtaattcttt tacaagaatt ttcattcttt     120

cttaagtata aacacttagc ttgggacaaa cttctgatcc tatttcttaa tttttgcagg     180

cgatggtggc tgttatgagc attttgtgtt tgatgtttct ctcttctcat tacggtttta     240

ttgggatctg ggtggctcta actatttaca tgagcctccg cgcgtttgct gaaggcggga     300

aacgacaatc tgatccccat caagcttgag ctcaggattt agcagcattc cagattgggt     360

tcaatcaaca aggtacgagc catatcactt tattcaaatt ggtatcgcca aaaccaagaa     420

ggaactccca tcctcaaagg tttgtaagga agaattctca gtccaaagcc tcaacaaggt     480

cagggtacag agtctccaaa ccattagcca aaagctacag gagatcaatg aagaatcttc     540

aatcaaagta aactactgtt ccagcacatg catcatggtc agtaagtttc agaaaaagac     600

atccaccgaa gacttaaagt tagtgggcat ctttgaaagt aatcttgtca acatcgagca     660

gctggcttgt ggggaccaga caaaaaagga atggtgcaga attgttaggc gcacctacca     720

aaagcatctt tgcctttatt gcaaagataa agcagattcc tctagtacaa gtggggaaca     780

aaataacgtg gaaaagagct gtcctgacag cccactcact aatgcgtatg acgaacgcag     840

tgacgaccac aaaagaattc cctctatata agaaggcatt cattcccatt tgaaggatca     900

tcagatactg aaccaatcct tctagaagat ctaagcttat cgataagctt gatgtaattg     960

gaggaagatc aaaattttca atccccattc ttcgattgct tcaattgaag tttctccgat    1020

ggcgcaagtt agcagaatct gc                                              1042

<210>  90
<211>  665
<212>  PRT
<213>  Beta vulgaris

<400>  90

Met Ala Ala Thr Phe Thr Asn Pro Thr Phe Ser Pro Ser Ser Thr Pro
1               5                   10                  15

Leu Thr Lys Thr Leu Lys Ser Gln Ser Ser Ile Ser Ser Thr Leu Pro
            20                  25                  30

Phe Ser Thr Pro Pro Lys Thr Pro Thr Pro Leu Phe His Arg Pro Leu
        35                  40                  45

Gln Ile Ser Ser Ser Gln Ser His Lys Ser Ser Ala Ile Lys Thr Gln
    50                  55                  60

Thr Gln Ala Pro Ser Ser Pro Ala Ile Glu Asp Ser Ser Phe Val Ser
65                  70                  75                  80

Arg Phe Gly Pro Asp Glu Pro Arg Lys Gly Ser Asp Val Leu Val Glu
                85                  90                  95

Ala Leu Glu Arg Glu Gly Val Thr Asn Val Phe Ala Tyr Pro Gly Gly
            100                 105                 110

Ala Ser Met Glu Ile His Gln Ala Leu Thr Arg Ser Lys Thr Ile Arg
        115                 120                 125

Asn Val Leu Pro Arg His Glu Gln Gly Gly Val Phe Ala Ala Glu Gly
    130                 135                 140

Tyr Ala Arg Ala Thr Gly Lys Val Gly Val Cys Ile Ala Thr Ser Gly
145                 150                 155                 160

Pro Gly Ala Thr Asn Leu Val Ser Gly Leu Ala Asp Ala Leu Leu Asp
                165                 170                 175

Ser Val Pro Leu Val Ala Ile Thr Gly Gln Val Pro Arg Arg Met Ile
            180                 185                 190

Gly Thr Asp Ala Phe Gln Glu Thr Pro Ile Val Glu Val Thr Arg Ser
        195                 200                 205

Ile Thr Lys His Asn Tyr Leu Val Leu Asp Val Glu Asp Ile Pro Arg
    210                 215                 220

Ile Val Lys Glu Ala Phe Phe Leu Ala Asn Ser Gly Arg Pro Gly Pro
225                 230                 235                 240

Val Leu Ile Asp Leu Pro Lys Asp Ile Gln Gln Gln Leu Val Val Pro
                245                 250                 255

Asp Trp Asp Arg Pro Phe Lys Leu Gly Gly Tyr Met Ser Arg Leu Pro
            260                 265                 270

Lys Ser Lys Phe Ser Thr Asn Glu Val Gly Leu Leu Glu Gln Ile Val
        275                 280                 285

Arg Leu Met Ser Glu Ser Lys Lys Pro Val Leu Tyr Val Gly Gly Gly
    290                 295                 300

Cys Leu Asn Ser Ser Glu Glu Leu Arg Arg Phe Val Glu Leu Thr Gly
305                 310                 315                 320

Ile Pro Val Ala Ser Thr Leu Met Gly Leu Gly Ser Tyr Pro Cys Asn
                325                 330                 335

Asp Glu Leu Ser Leu His Met Leu Gly Met His Gly Thr Val Tyr Ala
            340                 345                 350

Asn Tyr Ala Val Asp Lys Ala Asp Leu Leu Leu Ala Phe Gly Val Arg
        355                 360                 365

Phe Asp Asp Arg Val Thr Gly Lys Leu Glu Ala Phe Ala Ser Arg Ala
    370                 375                 380

Lys Ile Val His Ile Asp Ile Asp Ser Ala Glu Ile Gly Lys Asn Lys
385                 390                 395                 400

Gln Pro His Val Ser Ile Cys Ala Asp Val Lys Leu Ala Leu Arg Gly
                405                 410                 415

Met Asn Lys Ile Leu Glu Ser Arg Ile Gly Lys Leu Asn Leu Asp Phe
            420                 425                 430

Ser Lys Trp Arg Glu Glu Leu Gly Glu Gln Lys Lys Glu Phe Pro Leu
        435                 440                 445

Ser Phe Lys Thr Phe Gly Asp Ala Ile Pro Pro Gln Tyr Ala Ile Gln
    450                 455                 460

Val Leu Asp Glu Leu Thr Asn Gly Asn Ala Ile Ile Ser Thr Gly Val
465                 470                 475                 480

Gly Gln His Gln Met Trp Ala Ala Gln His Tyr Lys Tyr Arg Asn Pro
                485                 490                 495

Arg Gln Trp Leu Thr Ser Gly Gly Leu Gly Ala Met Gly Phe Gly Leu
            500                 505                 510

Pro Ala Ala Ile Gly Ala Ala Val Ala Arg Pro Asp Ala Val Val Val
        515                 520                 525

Asp Ile Asp Gly Asp Gly Ser Phe Ile Met Asn Val Gln Glu Leu Ala
    530                 535                 540

Thr Ile Arg Val Glu Asn Leu Pro Val Lys Ile Met Leu Leu Asn Asn
545                 550                 555                 560

Gln His Leu Gly Met Val Val Gln Trp Glu Asp Arg Phe Tyr Lys Ala
                565                 570                 575

Asn Arg Ala His Thr Tyr Leu Gly Asn Pro Ser Lys Ser Ala Asp Ile
            580                 585                 590

Phe Pro Asp Met Leu Lys Phe Ala Glu Ala Cys Asp Ile Pro Ser Ala
        595                 600                 605

Arg Val Ser Asn Val Ala Asp Leu Arg Ala Ala Ile Gln Thr Met Leu
    610                 615                 620

Asp Thr Pro Gly Pro Tyr Leu Leu Asp Val Ile Val Pro His Gln Glu
625                 630                 635                 640

His Val Leu Pro Met Ile Pro Ser Gly Ala Gly Phe Lys Asp Thr Ile
                645                 650                 655

Thr Glu Gly Asp Gly Arg Thr Ser Tyr
            660                 665

<210>  91
<211>  1998
<212>  DNA
<213>  Artificial Sequence

<220>
<223>  Acetolactate synthase with mutation

<400>  91
atggcggcta ccttcacaaa cccaacattt tccccttcct caactccatt aaccaaaacc      60

ctaaaatccc aatcttccat ctcttcaacc ctcccctttt ccacccctcc caaaacccca     120

actccactct ttcaccgtcc cctccaaatc tcatcctccc aatcccacaa atcatccgcc     180

attaaaacac aaactcaagc accttcttct ccagctattg aagattcatc tttcgtttct 240

cgatttggcc ctgatgaacc cagaaaaggg tccgatgtcc tcgttgaagc tcttgagcgt 300

gaaggtgtta ccaatgtgtt tgcttaccct ggtggtgcat ctatggaaat ccaccaagct 360

ctcacacgct ctaaaaccat ccgcaatgtc ctccctcgcc atgaacaagg cggggttttc 420

gccgccgagg gatatgctag agctactgga aaggttggtg tctgcattgc gacttctggt 480

cctggtgcta ccaacctcgt atcaggtctt gctgacgctc tccttgattc tgtccctctt 540

gttgccatca ctggccaagt tccacgccgt atgattggca ctgatgcttt tcaggagact 600

ccaattgttg aggtgacaag gtctattact aagcataatt atttagtttt ggatgtagag 660

gatattccta gaattgttaa ggaagccttt tttttagcta attctggtag gcctggacct 720

gttttgattg atcttcctaa agatattcag cagcaattgg ttgttcctga ttgggatagg 780

ccttttaagt tgggtgggta tatgtctagg ctgccaaagt ccaagttttc gacgaatgag 840

gttggacttc ttgagcagat tgtgaggttg atgagtgagt cgaagaagcc tgtcttgtat 900

gtgggaggtg ggtgtttgaa ttctagtgag gagttgagga gatttgttga gttgacaggg 960

attccggtgg ctagtacttt gatggggttg gggtcttacc cttgtaatga tgaactgtct 1020

cttcatatgt tggggatgca cgggactgtt tatgccaatt atgcggtgga taaggcggat 1080

ttgttgcttg ctttcggggt taggtttgat gatcgtgtga ccgggaagct cgaggcgttt 1140

gctagccgtg ctaagattgt gcatattgat attgactctg ctgagattgg gaagaacaag 1200

cagccccatg tgtccatttg tgctgatgtt aaattggcat tgcggggtat gaataagatt 1260

ctggagtcta gaatagggaa gctgaatttg gatttctcca agtggagaga agaattaggt 1320

gagcagaaga aggaattccc actgagtttt aagacatttg gggatgcaat tcctccacaa 1380

tatgccattc aggtgcttga tgagttgacc aatggtaatg ctattataag tactggtgtt 1440

gggcagcacc aaatgtgggc tgcgcagcat tacaagtaca gaaaccctcg ccaatggctg 1500

acctctggtg ggttgggggc tatgggtttt gggctaccag ccgccattgg agctgcagtt 1560

gctcgaccag atgcagtggt tgtcgatatt gatggggatg gcagttttat tatgaatgtt 1620

caagagttgg ctacaattag ggtggaaaat ctcccagtta agataatgct gctaaacaat 1680

caacatttag gtatggttgt ccaattggaa gataggttct ataaagctaa ccgggcacat 1740

acataccttg gaaacccttc caaatctgct gatatcttcc ctgatatgct caaattcgct 1800

gaggcatgtg atattccttc tgcccgtgtt agcaacgtgg ctgatttgag ggccgccatt 1860

caaacaatgt tggatactcc agggccgtac ctgctcgatg tgattgtacc gcatcaagag 1920

catgtgttgc ctatgattcc aagtggtgcc ggtttcaagg ataccattac agagggtgat 1980

ggaagaacct cttattga 1998

<210>  92
<211>  21
<212>  DNA
<213>  Beta vulgaris

<400>  92
cctgagagtt tgggaagttg c                                                21


<210>  93
<211>  23
<212>  DNA
<213>  Beta vulgaris

<400>  93
atgtcccgaa gattaacgca atc                                              23


<210>  94
<211>  3720
<212>  DNA
<213>  Artificial Sequence

<220>
<223>  modified genomic sequence of the gene mediating resistance
       towards cercospora

<400>  94
atgaacatga aaatcttact tttgtttgtc ttccttcatc acctccacta cttcatccat      60

ggcagaacac ttacagaacg ccaagcttta ctaagtatca aatctgccat tacttatgat     120

tattataact ctctctcctc atggaaaaac acaacacacc actgcagttg gccatacatc     180

acttgctcct cctcttcttc ttcttcttct gttatttctc tcaacttcac catgttattt     240

ctcgaaggaa ttctctcccc tgatataggc ttcctcacca acctgcaaaa cctctctatt     300

cgatctaacc ttttttctgg cccactcccc cattctctct ctctcctcac ccaactccgc     360

tatctcgacg tttcccaaaa cagtttcaca ggtccaatcc catcttctct ctctctcctc     420

acccaactcc gctatctcca cgtttccggc aacagtttca caggtccaat cccatctttt     480

ctctctctcc tcacccaact ccgctatctc gacgtttccg acaacagttt cacaggtcca     540

atcccatctt ctctctctct cctcacccaa ctccgctatc tcgacgtttc ctacaacaat     600

ctaaatggca ctcttccctt atcggtcgtt gagaagatgt cggagctcag ctaccttaac     660

cttaggtata actctttcta cggtgagatt ccaccggagt ttgggaaact taagaagctt     720

gaaacattga atcttggtaa caacactctt tctgggagtc ttccatctga gttgggttca     780

ttaaagagtt tgaaacatat ggacttttct agtaatatgc tatttggtga gatcccacaa     840

tcttattctc ttcttcgaaa cttaatcgat attgatctta atagaaacaa gttatatggg     900

agtatacctg attatattgg agattttccg gagttggaat cacttttatt agactcgaat     960

aacttcacag ggagtatccc acaaaagtta ggtacaaacg ggaagttgca atatctagat    1020

ataagtaaca acaattttag tggtagtttg ccactaagtc tttgcaaagg agacaaactc 1080

caagatctgg acgcatccta taatttgttg gttgggtcaa ttcctgagag tttgggaagt 1140

tgcaagtcac ttgaaggagt gtacatggga aataatttct taaacgggtc gattcctaag 1200

ggcttgtttg ggagtgatgt ttcacttaat gacaaacttc ttagtggagg tctcgatgag 1260

aaattcggtg attgcgttaa tcttcgggac attgatctct ctaataataa gctatcaggg 1320

aagttacctg cgaccatcgg aaactgtatt catcttcggt ccttgacgct ttataataac 1380

acctgtaccg gacgtatccc tcaagagatt agcaagtgta agcagctaca gaccctcgat 1440

ctcagccaaa atcagttctc tggtgtgata cccaatgata ttacaggtaa gaaagtatat 1500

taaacttgtt acttttgaaa atattcgctc tagtttttgt ttcagttggt ccattctcac 1560

tttgtattat tgaaatatat cccaaaaaag taaatataat tatataaaag aatcttgcta 1620

aaaataatat gaattatttt tgtatgtgca aaataatgta caaatctaac taatttgttg 1680

tggataataa tattaattgt gtgaaatagt aaatgtgtgg agatatataa ctttatttat 1740

catattcact caggttttta ggtatttatt atgagttttg cattggagat atccaacttg 1800

acaatagtat ttttgtaata taccaatata taaagattac tgtacataac caaaatgtat 1860

acttttctta tttttataaa cttatatatt cctcttcttt gtatttatca caacattttt 1920

tatacccttt tgcctcatat taatagcaac acttataatt tatttattta ctttttattt 1980

cttggtctat aacctcatct acccacatat gacacaccct ataaaggacc cacatgatta 2040

accaaaatat acaaatatct tcaatgaaat taactttaac actaatatga taaaaatcat 2100

gtcccgcttt ttatcctcta actaagactc tgcataaagg tatattgcaa ttaatatgag 2160

atggaagagg tataataatt atatgatcaa attcctggat tgaaaaataa atatgagatt 2220

aaaagtggta tgtttttggt taaaagaaac tatccataaa gtatgttttt ggttaaaaga 2280

aactatgcaa cataccaatc aaatgtttat acgcttacaa tttatgtacc acttttttgt 2340

cattgttttt ctattgtttg ccatacgtac gttactaaat catgttgtct tttcacattt 2400

taactaacaa taaattacta ttgatacacc aaaaaaatct atgagcattg gagtacgttg 2460

tttgatagaa gcttcgtgct attatttctt gtcaaagaat ttcatatctc aatatcttct 2520

aatttaacaa tctaacgaaa tttttttgac ccaggaaaca aatccatttg caatctggaa 2580

aagatacaaa cacttaaatt atcaaacaat gctttgactg gtgaaatccc tcattgtgtt 2640

ggaaatatcg agctcatagc attatttctc caatcaaaca aactgaacgg taccataccc 2700

gcaaacttct caaagttatg tgattcattg atatatctag atcttagtga caatcaactc 2760

gaaggagttc tacctaagtc cttgtccaaa tgtcaaagtc tagaactcct aaatgtcggg 2820

aacaataggc taagagataa atttccttca tggttagaca acctcccacg tctccaagtt 2880

ttcagtgtgc gttttaacgc cttctacggt cctataacta gctcaccaaa agttagtcac 2940

ccatttccta tgctacaaat tatcgaccta tctaacaata agttttgtgg caagttgcca 3000

agaagatata tcaaaaactt tgcaaccatg cgcaatatga atgagtctgg tgttgggaat 3060

ccacagtacc tgggggactc atcaatatat agtattacgt actctatggt attgacattc 3120

aatgggttac aacaaaaata tgaaaagctt attgtgacga tgtcgacctt tgatatatcc 3180

agcaacaact ttactggaca gattccatat gttatagggg gattacgctc acttcgtaac 3240

cttaatctct ctcataatgt cttaaccggg aacattcctc catcaattgc aaaattgtct 3300

ttgcttcaag atttggacct ttcatcaaac agacttactg gtcgtatccc tcaagaatta 3360

gttagtttaa catttcttgg gagtttcaat gtttcgaaca atctattgga ggggtctata 3420

cctcatggtt tcaacttcga cacgtacaca gctaattcat accaggggaa tctcgaatta 3480

tgtggaaaac cattacctga gtgtggagaa agaagggcaa aaggcaccac taataatcaa 3540

gatgatccta aaaatgataa tgaacgaatg ttgtcgatgt ccgaaatcgt agttatgggg 3600

tttggcagtg gtgtactagt tgggttggct tggggatact atatgttttc agtgggaaag 3660

cccttttggt ttatcaagat ggctagcaaa atggaatcaa tattgattgg ttttttctga 3720

<210> 95
<211> 2652
<212> DNA
<213> Artificial Sequence

<220>
<223> modified cDNA sequence of the gene mediating resistance towards
      cercospora

<220>
<221> variation
<222> (55)..(57)

<400> 95
atgaacatga aaatcctcct tttgtttgtc ttccttcatc acctccacta cttcatacat 60

ggcagaacac ttacagaacg ccaagcttta ctaagtatca aatctgccat tacttatgat 120

tattataact ctctctcctc atggaaaaac acaacacacc actgcagttg gccatacatc 180

acttgctcct cctcttcttc ttcttcttct gttatttctc tcaacttcac catgttattt 240

ctcgaaggaa ttctctcccc tgatataggc ttcctcacca acctgcaaaa cctctctatt 300

cgatctaacc ttttttctgg cccactcccc cattctctct ctctcctcac ccaactccgc 360

tatctcgacg tttcccaaaa cagtttcaca ggtccaatcc catcttctct ctctctcctc 420

acccaactcc gctatctcca cgtttccggc aacagtttca caggtccaat cccatctttt 480

ctctctctcc tcacccaact ccgctatctc gacgtttccg acaacagttt cacaggtcca 540

atcccatctt ctctctctct cctcacccaa ctccgctatc tcgacgtttc ctacaacaat 600

ctaaatggca ctcttccctt atcggtcgtt gagaagatgt cggagctcag ctaccttaac 660

cttaggtata actctttcta cggtgagatt ccaccggagt ttgggaaact taagaagctt 720

gaaacattga atcttggtaa caacactctt tctgggagtc ttccatctga gttgggttca 780

ttaaagagtt tgaaacatat ggacttttct agtaatatgc tatttggtga gatcccacaa 840

tcttattctc ttcttcgaaa cttaatcgat attgatctta atagaaacaa gttatatggg 900

agtatacctg attatattgg agattttccg gagttggaat cacttttatt agactcgaat 960

aacttcacag ggagtatccc acaaaagtta ggtacaaacg ggaagttgca atatctagat 1020

ataagtaaca acaattttag tggtagtttg ccactaagtc tttgcaaagg agacaaactc 1080

caagatctgg acgcatccta taatttgttg gttgggtcaa ttcctgagag tttgggaagt 1140

tgcaagtcac ttgaaggagt gtacatggga aataatttct taaacgggtc gattcctaag 1200

ggcttgtttg ggagtgatgt ttcacttaat gacaaacttc ttagtggagg tctcgatgag 1260

aaattcggtg attgcgttaa tcttcgggac attgatctct ctaataataa gctatcaggg 1320

aagttacctg cgaccatcgg aaactgtatt catcttcggt ccttgacgct ttataataac 1380

acctgtaccg gacgtatccc tcaagagatt agcaagtgta agcagctaca gaccctcgat 1440

ctcagccaaa atcagttctc tggtgtgata cccaatgata ttacaggaaa caaatccatt 1500

tgcaatctgg aaaagataca aacacttaaa ttatcaaaca atgctttgac tggtgaaatc 1560

cctcattgtg ttggaaatat cgagctcata gcattatttc tccaatcaaa caaactgaac 1620

ggtaccatac ccgcaaactt ctcaaagtta tgtgattcat tgatatatct agatcttagt 1680

gacaatcaac tcgaaggagt tctacctaag tccttgtcca aatgtcaaag tctagaactc 1740

ctaaatgtcg ggaacaatag gctaagagat aaatttcctt catggttaga caacctccca 1800

cgtctccaag ttttcagtgt gcgttttaac gccttctacg gtcctataac tagctcacca 1860

aaagttagtc acccatttcc tatgctacaa attatcgacc tatctaacaa taagttttgt 1920

ggcaagttgc caagaagata tatcaaaaac tttgcaacca tgcgcaatat gaatgagtct 1980

ggtgttggga atccacagta cctgggggac tcatcaatat atagtattac gtactctatg 2040

gtattgacat tcaatgggtt acaacaaaaa tatgaaaagc ttattgtgac gatgtcgacc 2100

tttgatatat ccagcaacaa ctttactgga cagattccat atgttatagg gggattacgc 2160

tcacttcgta accttaatct ctctcataat gtcttaaccg ggaacattcc tccatcaatt 2220

gcaaaattgt ctttgcttca agatttggac ctttcatcaa acagacttac tggtcgtatc 2280

cctcaagaat tagttagttt aacatttctt gggagtttca atgtttcgaa caatctattg 2340

gaggggtcta tacctcatgg tttcaacttc gacacgtaca cagctaattc ataccagggg 2400

aatctcgaat tatgtggaaa accattacct gagtgtggag aaagaagggc aaaaggcacc 2460

actaataatc aagatgatcc taaaaatgat aatgaacgaa tgttgtcgat gtccgaaatc 2520

gtagttatgg ggtttggcag tggtgtacta gttgggttgg cttggggata ctatatgttt     2580

tcagtgggaa agcccttttg gtttatcaag atggctagca aaatggaatc aatattgatt     2640

ggtttttct ga                                                          2652

<210>  96
<211>  883
<212>  PRT
<213>  Artificial Sequence

<220>
<223>  modified protein sequence of the gene mediating resistance
       towards cercospora

<220>
<221>  VARIANT
<222>  (209)..(209)

<400>  96

Met Asn Met Lys Ile Leu Leu Leu Phe Val Phe Leu His His Leu His
1               5                   10                  15

Tyr Phe Ile His Gly Arg Thr Leu Thr Glu Arg Gln Ala Leu Leu Ser
            20                  25                  30

Ile Lys Ser Ala Ile Thr Tyr Asp Tyr Tyr Asn Ser Leu Ser Ser Trp
        35                  40                  45

Lys Asn Thr Thr His His Cys Ser Trp Pro Tyr Ile Thr Cys Ser Ser
    50                  55                  60

Ser Ser Ser Ser Ser Ser Val Ile Ser Leu Asn Phe Thr Met Leu Phe
65                  70                  75                  80

Leu Glu Gly Ile Leu Ser Pro Asp Ile Gly Phe Leu Thr Asn Leu Gln
                85                  90                  95

Asn Leu Ser Ile Arg Ser Asn Leu Phe Ser Gly Pro Leu Pro His Ser
            100                 105                 110

Leu Ser Leu Leu Thr Gln Leu Arg Tyr Leu Asp Val Ser Gln Asn Ser
        115                 120                 125

Phe Thr Gly Pro Ile Pro Ser Ser Leu Ser Leu Leu Thr Gln Leu Arg
    130                 135                 140

Tyr Leu His Val Ser Gly Asn Ser Phe Thr Gly Pro Ile Pro Ser Phe
145                 150                 155                 160

Leu Ser Leu Leu Thr Gln Leu Arg Tyr Leu Asp Val Ser Asp Asn Ser
                165                 170                 175

Phe Thr Gly Pro Ile Pro Ser Ser Leu Ser Leu Leu Thr Gln Leu Arg
            180                 185                 190

Tyr Leu Asp Val Ser Tyr Asn Asn Leu Asn Gly Thr Leu Pro Leu Ser
        195                 200                 205

Val Leu Glu Lys Met Ser Glu Leu Ser Tyr Leu Asn Leu Arg Tyr Asn
    210                 215                 220

Ser Phe Tyr Gly Glu Ile Pro Pro Glu Phe Gly Lys Leu Lys Lys Leu
225                 230                 235                 240

Glu Thr Leu Asn Leu Gly Asn Asn Thr Leu Ser Gly Ser Leu Pro Ser
                245                 250                 255

Glu Leu Gly Ser Leu Lys Ser Leu Lys His Met Asp Phe Ser Ser Asn
            260                 265                 270

Met Leu Phe Gly Glu Ile Pro Gln Ser Tyr Ser Leu Leu Arg Asn Leu
        275                 280                 285

Ile Asp Ile Asp Leu Asn Arg Asn Lys Leu Tyr Gly Ser Ile Pro Asp
    290                 295                 300

Tyr Ile Gly Asp Phe Pro Glu Leu Glu Ser Leu Leu Leu Asp Ser Asn
305                 310                 315                 320

Asn Phe Thr Gly Ser Ile Pro Gln Lys Leu Gly Thr Asn Gly Lys Leu
                325                 330                 335

Gln Tyr Leu Asp Ile Ser Asn Asn Asn Phe Ser Gly Ser Leu Pro Leu
            340                 345                 350

Ser Leu Cys Lys Gly Asp Lys Leu Gln Asp Leu Asp Ala Ser Tyr Asn
        355                 360                 365

Leu Leu Val Gly Ser Ile Pro Glu Ser Leu Gly Ser Cys Lys Ser Leu
    370                 375                 380

Glu Gly Val Tyr Met Gly Asn Asn Phe Leu Asn Gly Ser Ile Pro Lys
385                 390                 395                 400

Gly Leu Phe Gly Ser Asp Val Ser Leu Asn Asp Lys Leu Leu Ser Gly
                405                 410                 415

Gly Leu Asp Glu Lys Phe Gly Asp Cys Val Asn Leu Arg Asp Ile Asp
            420                 425                 430

Leu Ser Asn Asn Lys Leu Ser Gly Lys Leu Pro Ala Thr Ile Gly Asn
        435                 440                 445

Cys Ile His Leu Arg Ser Leu Thr Leu Tyr Asn Asn Thr Cys Thr Gly
    450                 455                 460

Arg Ile Pro Gln Glu Ile Ser Lys Cys Lys Gln Leu Gln Thr Leu Asp
465                 470                 475                 480

Leu Ser Gln Asn Gln Phe Ser Gly Val Ile Pro Asn Asp Ile Thr Gly
                485                 490                 495

Asn Lys Ser Ile Cys Asn Leu Glu Lys Ile Gln Thr Leu Lys Leu Ser
            500                 505                 510

Asn Asn Ala Leu Thr Gly Glu Ile Pro His Cys Val Gly Asn Ile Glu
        515                 520                 525

Leu Ile Ala Leu Phe Leu Gln Ser Asn Lys Leu Asn Gly Thr Ile Pro
    530                 535                 540

Ala Asn Phe Ser Lys Leu Cys Asp Ser Leu Ile Tyr Leu Asp Leu Ser
545                 550                 555                 560

Asp Asn Gln Leu Glu Gly Val Leu Pro Lys Ser Leu Ser Lys Cys Gln
                565                 570                 575

Ser Leu Glu Leu Leu Asn Val Gly Asn Asn Arg Leu Arg Asp Lys Phe
            580                 585                 590

Pro Ser Trp Leu Asp Asn Leu Pro Arg Leu Gln Val Phe Ser Val Arg
        595                 600                 605

Phe Asn Ala Phe Tyr Gly Pro Ile Thr Ser Ser Pro Lys Val Ser His
    610                 615                 620

Pro Phe Pro Met Leu Gln Ile Ile Asp Leu Ser Asn Asn Lys Phe Cys
625                 630                 635                 640

Gly Lys Leu Pro Arg Arg Tyr Ile Lys Asn Phe Ala Thr Met Arg Asn
                645                 650                 655

Met Asn Glu Ser Gly Val Gly Asn Pro Gln Tyr Leu Gly Asp Ser Ser

660                 665                 670

Ile Tyr Ser Ile Thr Tyr Ser Met Val Leu Thr Phe Asn Gly Leu Gln
        675                 680                 685

Gln Lys Tyr Glu Lys Leu Ile Val Thr Met Ser Thr Phe Asp Ile Ser
    690                 695                 700

Ser Asn Asn Phe Thr Gly Gln Ile Pro Tyr Val Ile Gly Gly Leu Arg
705                 710                 715                 720

Ser Leu Arg Asn Leu Asn Leu Ser His Asn Val Leu Thr Gly Asn Ile
                725                 730                 735

Pro Pro Ser Ile Ala Lys Leu Ser Leu Leu Gln Asp Leu Asp Leu Ser
            740                 745                 750

Ser Asn Arg Leu Thr Gly Arg Ile Pro Gln Glu Leu Val Ser Leu Thr
        755                 760                 765

Phe Leu Gly Ser Phe Asn Val Ser Asn Asn Leu Leu Glu Gly Ser Ile
    770                 775                 780

Pro His Gly Phe Asn Phe Asp Thr Tyr Thr Ala Asn Ser Tyr Gln Gly
785                 790                 795                 800

Asn Leu Glu Leu Cys Gly Lys Pro Leu Pro Glu Cys Gly Glu Arg Arg
                805                 810                 815

Ala Lys Gly Thr Thr Asn Asn Gln Asp Asp Pro Lys Asn Asp Asn Glu
            820                 825                 830

Arg Met Leu Ser Met Ser Glu Ile Val Val Met Gly Phe Gly Ser Gly
        835                 840                 845

Val Leu Val Gly Leu Ala Trp Gly Tyr Tyr Met Phe Ser Val Gly Lys
    850                 855                 860

Pro Phe Trp Phe Ile Lys Met Ala Ser Lys Met Glu Ser Ile Leu Ile
865                 870                 875                 880

Gly Phe Phe

<210>  97
<211>  2652
<212>  DNA
<213>  Artificial Sequence

<220>
<223>  modified cDNA of the gene mediating resistance towards cercospora

<220>
<221>  variation
<222>  (625)..(627)

<400>  97
   atgaacatga aaatcctcct tttgtttgtc ttccttcatc acctccacta cttcatccat      60
   ggcagaacac ttacagaacg ccaagcttta ctaagtatca aatctgccat tacttatgat     120
   tattataact ctctctcctc atggaaaaac acaacacacc actgcagttg gccatacatc     180
   acttgctcct cctcttcttc ttcttcttct gttatttctc tcaacttcac catgttattt     240
   ctcgaaggaa ttctctcccc tgatataggc ttcctcacca acctgcaaaa cctctctatt     300
   cgatctaacc ttttttctgg cccactcccc cattctctct ctctcctcac ccaactccgc     360
   tatctcgacg tttcccaaaa cagtttcaca ggtccaatcc catcttctct ctctctcctc     420
   acccaactcc gctatctcca cgtttccggc aacagtttca caggtccaat cccatctttt     480
   ctctctctcc tcacccaact ccgctatctc gacgtttccg acaacagttt cacaggtcca     540
   atcccatctt ctctctctct cctcacccaa ctccgctatc tcgacgtttc ctacaacaat     600
   ctaaatggca ctcttccctt atcgctcgtt gagaagatgt cggagctcag ctaccttaac     660
   cttaggtata actctttcta cggtgagatt ccaccggagt ttgggaaact taagaagctt     720
   gaaacattga atcttggtaa caacactctt tctgggagtc ttccatctga gttgggttca     780
   ttaaagagtt tgaaacatat ggacttttct agtaatatgc tatttggtga gatcccacaa     840
   tcttattctc ttcttcgaaa cttaatcgat attgatctta atagaaacaa gttatatggg     900
   agtatacctg attatattgg agattttccg gagttggaat cacttttatt agactcgaat     960
   aacttcacag ggagtatccc acaaaagtta ggtacaaacg ggaagttgca atatctagat    1020
   ataagtaaca acaattttag tggtagtttg ccactaagtc tttgcaaagg agacaaactc    1080
   caagatctgg acgcatccta taatttgttg gttgggtcaa ttcctgagag tttgggaagt    1140
   tgcaagtcac ttgaaggagt gtacatggga aataatttct taaacgggtc gattcctaag    1200
   ggcttgtttg ggagtgatgt ttcacttaat gacaaacttc ttagtggagg tctcgatgag    1260
   aaattcggtg attgcgttaa tcttcgggac attgatctct ctaataataa gctatcaggg    1320
   aagttacctg cgaccatcgg aaactgtatt catcttcggt ccttgacgct ttataataac    1380
   acctgtaccg gacgtatccc tcaagagatt agcaagtgta agcagctaca gaccctcgat    1440
   ctcagccaaa atcagttctc tggtgtgata cccaatgata ttacaggaaa caaatccatt    1500
   tgcaatctgg aaaagataca aacacttaaa ttatcaaaca atgctttgac tggtgaaatc    1560
   cctcattgtg ttggaaatat cgagctcata gcattatttc tccaatcaaa caaactgaac    1620
   ggtaccatac ccgcaaactt ctcaaagtta tgtgattcat tgatatatct agatcttagt 1680
   gacaatcaac tcgaaggagt tctacctaag tccttgtcca aatgtcaaag tctagaactc 1740
   ctaaatgtcg ggaacaatag gctaagagat aaatttcctt catggttaga caacctccca 1800
   cgtctccaag ttttcagtgt gcgttttaac gccttctacg gtcctataac tagctcacca 1860
   aaagttagtc acccatttcc tatgctacaa attatcgacc tatctaacaa taagttttgt 1920
   ggcaagttgc caagaagata tatcaaaaac tttgcaacca tgcgcaatat gaatgagtct 1980
   ggtgttggga atccacagta cctgggggac tcatcaatat atagtattac gtactctatg 2040
   gtattgacat tcaatgggtt acaacaaaaa tatgaaaagc ttattgtgac gatgtcgacc 2100
   tttgatatat ccagcaacaa ctttactgga cagattccat atgttatagg gggattacgc 2160
   tcacttcgta accttaatct ctctcataat gtcttaaccg ggaacattcc tccatcaatt 2220
   gcaaaattgt ctttgcttca agatttggac ctttcatcaa acagacttac tggtcgtatc 2280
   cctcaagaat tagttagttt aacatttctt gggagtttca atgtttcgaa caatctattg 2340
   gaggggtcta tacctcatgg tttcaacttc gacacgtaca cagctaattc ataccagggg 2400
   aatctcgaat tatgtggaaa accattacct gagtgtggag aaagaagggc aaaaggcacc 2460
   actaataatc aagatgatcc taaaaatgat aatgaacgaa tgttgtcgat gtccgaaatc 2520
   gtagttatgg ggtttggcag tggtgtacta gttgggttgg cttggggata ctatatgttt 2580
   tcagtgggaa agcccttttg gtttatcaag atggctagca aaatggaatc aatattgatt 2640
   ggttttttct ga 2652

<210> 98
<211> 21
<212> DNA
<213> Beta vulgaris

<400> 98
cgtttccggc aacagtttca c 21

<210> 99
<211> 21
<212> DNA
<213> Beta vulgaris

<400> 99
agagagagag aggagtgggt t 21

<210> 100
<211> 201
<212> DNA
<213> Beta vulgaris

<400> 100
tgagtagtac tgttgaatct tcacatgyga ttaaaatyaa ycatcatcat matcatcatm 60

ggaaagagca cgtgactagt cacgtgccga tggattgtag aagtgattgt gattcttcat 120

cgtcggtgat tgatgacgtg gacggttgcg atgatgataa tattgggtgt tsttcttctg 180

ttttagccgt tggattaacg a 201

<210> 101
<211> 201
<212> DNA
<213> Beta vulgaris

<400> 101
tgagtagtac tgttgaatct tcacatgyga ttaaaatyaa ycatcatcat matcatcatm 60

ggaaagagca cgtgactagt cacgtgccga tggattgtag gagtgattgt gattcttcat 120

cgtcggtgat tgatgacgtg gacggttgcg atgatgataa tattgggtgt tsttcttctg 180

ttttagccgt tggattaacg a 201

<210> 102
<211> 201
<212> DNA
<213> Beta vulgaris

<400> 102
ttgggtgcat tcctgtcatt atmgcagatg atatcgttct tccatttgca gatgccattc 60

catgggaaga aattgggttg tttgtggatg aaaaggatgt rccraatttg gatacaatac 120

ttacttcaat ccctcctgaa gtcatcttga ggaaacaaag gttacttgct aacccttcag 180

ttaaacaagc tatgctgttc c 201

<210> 103
<211> 201
<212> DNA
<213> Beta vulgaris

<400> 103
aaccgatggt tggatctaat ctgaggtgcc aaaatcacaa aaattgatga ttagttcatt 60

aaatagtgat ggtttttttk agcagccaga gtactaagga yaactcaaca ccaaaaatga 120

gtcgaagcca agttctatcc aacattgatc catacaatgt arycccagct ttgtttttct 180

ccaaatatgt cacaagagcg t 201

<210> 104
<211> 201
<212> DNA
<213> Beta vulgaris

<400> 104
aaccgatggt tggatctaat ctgaggtgcc aaaatcacaa aaattgatga ttagttcatt 60

aaatagtgat ggtttttttk agcagccaga gtactaagga taactcaaca ccaaaaatga 120

gtcgaagcca agttctatcc aacattgatc catacaatgt arycccagct ttgtttttct 180

ccaaatatgt cacaagagcg t 201

<210> 105
<211> 201
<212> DNA
<213> Beta vulgaris

<400> 105
aaccgatggt tggatctaat ctgaggtgcc aaaatcacaa aaattgatga ttagttcatt 60

aaatagtgat ggtttttttk agcagccaga gtactaagga yaactcaaca ccaaaaatga 120

gtcgaagcca agttctatcc aacattgatc catacaatgt arycccagct ttgtttttct 180

ccaaatatgt cacaagagcg t 201

<210> 106
<211> 201
<212> DNA
<213> Beta vulgaris

<400> 106
ctgctctttc atctaactct ctcatctctt catcwtctct tcaacaatct ttkatcaatg 60

tcaataatcc ttcccttaca ctctcaccat tttcactbtc ctcttcttgt ttttcctcat 120

ccaactcctc ttttatctct cccttacatt cgtcgaatcg ctctgcwttc cctctggttt 180

tcagaaacag cagcaatsct g 201

<210> 107
<211> 201
<212> DNA
<213> Beta vulgaris

<400> 107
ctgctctttc atctaactct ctcatctctt catcwtctct tcaacaatct ttkatcaatg 60

tcaataatcc ttcccttaca ctctcaccat tttcactbtc ttcttcttgt ttttcctcat 120

ccaactcctc ttttatctct cccttacatt cgtcgaatcg ctctgcwttc cctctggttt 180

tcagaaacag cagcaatsct g 201

<210> 108
<211> 201
<212> DNA
<213> Beta vulgaris

<400> 108
ctgctctttc atctaactct ctcatctctt catcwtctct tcaacaatct ttkatcaatg 60

tcaataatcc ttcccttaca ctctcaccat tttcactbtc ytcttcttgt ttttcctcat 120

ccaactcctc ttttatctct cccttacatt cgtcgaatcg ctctgcwttc cctctggttt 180

tcagaaacag cagcaatsct g 201

<210>  109
<211>  201
<212>  DNA
<213>  Beta vulgaris

<400>  109
ttgcttgtat cttgtgcagt tgtgctaatg atttgtacct tccatavarg tttgatgtcg        60

tggttgatgt gttgcctgaa gtgagatgga ctcctgagaa cggatacaar aacttaaaaa       120

ttgttgttga aatagataay gaaataactg ctgaggtagc ctgcgagcga gagctaaaac       180

gtcgtcataa gacattaggt c                                                 201


<210>  110
<211>  201
<212>  DNA
<213>  Beta vulgaris

<400>  110
ttgcttgtat cttgtgcagt tgtgctaatg atttgtacct tccatavarg tttgatgtcg        60

tggttgatgt gttgcctgaa gtgagatgga ctcctgagaa tggatacaar aacttaaaaa       120

ttgttgttga aatagataay gaaataactg ctgaggtagc ctgcgagcga gagctaaaac       180

gtcgtcataa gacattaggt c                                                 201


<210>  111
<211>  201
<212>  DNA
<213>  Beta vulgaris

<400>  111
ttgcttgtat cttgtgcagt tgtgctaatg atttgtacct tccatavarg tttgatgtcg        60

tggttgatgt gttgcctgaa gtgagatgga ctcctgagaa yggatacaar aacttaaaaa       120

ttgttgttga aatagataay gaaataactg ctgaggtagc ctgcgagcga gagctaaaac       180

gtcgtcataa gacattaggt c                                                 201


<210>  112
<211>  201
<212>  DNA
<213>  Beta vulgaris

<400>  112
accaagagtt gtgctgattg tcataatttg gtgggatgta gcgaatatgg gaygaagttg        60

gtgaaagyga tgaagacagg gacaagatgc ttcttcagat agagcaggaa tgtttagatg       120

tctacaaaag gaaggttgac ctggctgtca attcaagagc ccaacttctc cagaccttgg       180

cggatgctaa tgttgaactt t                                                 201


<210>  113

<211>  201
<212>  DNA
<213>  Beta vulgaris

<400>  113
accaagagtt gtgctgattg tcataatttg gtgggatgta gcgaatatgg gaygaagttg        60

gtgaaagyga tgaagacagg gacaagatgc ttcttcagat tgagcaggaa tgtttagatg       120

tctacaaaag gaaggttgac ctggctgtca attcaagagc ccaacttctc cagaccttgg       180

cggatgctaa tgttgaactt t                                                 201


<210>  114
<211>  201
<212>  DNA
<213>  Beta vulgaris

<400>  114
accaagagtt gtgctgattg tcataatttg gtgggatgta gcgaatatgg gaygaagttg        60

gtgaaagyga tgaagacagg gacaagatgc ttcttcagat wgagcaggaa tgtttagatg       120

tctacaaaag gaaggttgac ctggctgtca attcaagagc ccaacttctc cagaccttgg       180

cggatgctaa tgttgaactt t                                                 201


<210>  115
<211>  201
<212>  DNA
<213>  Beta vulgaris

<400>  115
aatgtcaatg aatcrcatta wtttttctcc aaaaccakct tgagcagaag cagaaatatg        60

cagtatggaa agcagctgat ataaggaagg ctctaaaaga aggaaggaag cctgttccrg       120

gtcctcctgg tggygaacaa gattcatcca ctccrtcaag tgctccaagc aatgaatatg       180

taagatccca acatggcatg g                                                 201


<210>  116
<211>  201
<212>  DNA
<213>  Beta vulgaris

<400>  116
aatgtcaatg aatcrcatta wtttttctcc aaaaccakct tgagcagaag cagaaatatg        60

cagtatggaa agcagctgat ataaggaagg ctctaaaaga gggaaggaag cctgttccrg       120

gtcctcctgg tggygaacaa gattcatcca ctccrtcaag tgctccaagc aatgaatatg       180

taagatccca acatggcatg g                                                 201


<210>  117
<211>  201
<212>  DNA
<213>  Beta vulgaris

<400>  117
aatgtcaatg aatcrcatta wtttttctcc aaaaccakct tgagcagaag cagaaatatg      60

cagtatggaa agcagctgat ataaggaagg ctctaaaaga dggaaggaag cctgttccrg     120

gtcctcctgg tggygaacaa gattcatcca ctccrtcaag tgctccaagc aatgaatatg     180

taagatccca acatggcatg g                                               201


<210>  118
<211>  201
<212>  DNA
<213>  Beta vulgaris

<400>  118
agcagtgaaa taatcttcgy ttggaaagag caagtaatgc tgccataatc taatcatata      60

tccaaccacc tgacgaacca tgaggaagga aaggattggc gggacctggg gcaaagtcca     120

tkggaatgcc aggataaccc tgtctarggg gttgaggatg atggtagaaa ctagcaccaa     180

cggacctagg gcccaggtct g                                               201


<210>  119
<211>  201
<212>  DNA
<213>  Beta vulgaris

<400>  119
agcagtgaaa taatcttcgy ttggaaagag caagtaatgc tgccataatc taatcatata      60

tccaaccacc tgacgaacca tgaggaagga aaggattggc tggacctggg gcaaagtcca     120

tkggaatgcc aggataaccc tgtctarggg gttgaggatg atggtagaaa ctagcaccaa     180

cggacctagg gcccaggtct g                                               201


<210>  120
<211>  201
<212>  DNA
<213>  Beta vulgaris

<400>  120
agcagtgaaa taatcttcgy ttggaaagag caagtaatgc tgccataatc taatcatata      60

tccaaccacc tgacgaacca tgaggaagga aaggattggc kggacctggg gcaaagtcca     120

tkggaatgcc aggataaccc tgtctarggg gttgaggatg atggtagaaa ctagcaccaa     180

cggacctagg gcccaggtct g                                               201


<210>  121
<211>  201
<212>  DNA
<213>  Beta vulgaris

<400>  121
agatttaaca tttttgtgcw ggatttccgc cgctagtaat agcacataat ggattctcag      60

gattgttccc tccttctagt agttatgcct acaatttggc gatgatgact agtgtgccgg 120

atgtkgttct atggtgtgac gtccagttaa cgaaggatgc ggctggcata tgctttccag 180

atataaagct ggataattcc t 201

<210> 122
<211> 201
<212> DNA
<213> Beta vulgaris

<400> 122
agatttaaca tttttgtgcw ggatttccgc cgctagtaat agcacataat ggattctcag 60

gattgttccc tccttctagt agttatgcct acaatttggc aatgatgact agtgtgccgg 120

atgtkgttct atggtgtgac gtccagttaa cgaaggatgc ggctggcata tgctttccag 180

atataaagct ggataattcc t 201

<210> 123
<211> 201
<212> DNA
<213> Beta vulgaris

<400> 123
agatttaaca tttttgtgcw ggatttccgc cgctagtaat agcacataat ggattctcag 60

gattgttccc tccttctagt agttatgcct acaatttggc ratgatgact agtgtgccgg 120

atgtkgttct atggtgtgac gtccagttaa cgaaggatgc ggctggcata tgctttccag 180

atataaagct ggataattcc t 201

<210> 124
<211> 201
<212> DNA
<213> Beta vulgaris

<400> 124
gatgtaaccc tatcaaggag ttattygagg gagaggagaa gacttggacc ctctagaagt 60

atcggctctg actggagccg ygagaatgag aaggaaggat cattgactga agaagcaatt 120

ccttcgaggg ccatggaaat tgataatgat actttaaaca aggatacwtc taaagatact 180

gatactagtt ttcagaattc t 201

<210> 125
<211> 201
<212> DNA
<213> Beta vulgaris

<400> 125
gatgtaaccc tatcaaggag ttattygagg gagaggagaa gacttggacc ctctagaagt 60

atcggctctg actggagccg ygagaatgag aaggaaggat tattgactga agaagcaatt 120

ccttcgaggg ccatggaaat tgataatgat actttaaaca aggatacwtc taaagatact 180

gatactagtt ttcagaattc t 201

<210> 126
<211> 201
<212> DNA
<213> Beta vulgaris

<400> 126
gatgtaaccc tatcaaggag ttattygagg gagaggagaa gacttggacc ctctagaagt 60

atcggctctg actggagccg ygagaatgag aaggaaggat yattgactga agaagcaatt 120

ccttcgaggg ccatggaaat tgataatgat actttaaaca aggatacwtc taaagatact 180

gatactagtt ttcagaattc t 201

<210> 127
<211> 201
<212> DNA
<213> Beta vulgaris

<400> 127
ttcgagggcc atggaaattg ataatgatac tttaaacaag gatacwtcta aagatactga 60

tactagtttt cagaattctc atccagctga gccagctaat gaggtgatga aaccttctga 120

tgatttagac ttaaagatga cttckttggt gcaaaacaag gatgataatg agatagtgga 180

ascttcagat gtggtgaatg c 201

<210> 128
<211> 201
<212> DNA
<213> Beta vulgaris

<400> 128
ttcgagggcc atggaaattg ataatgatac tttaaacaag gatacwtcta aagatactga 60

tactagtttt cagaattctc atccagctga gccagctaat aaggtgatga aaccttctga 120

tgatttagac ttaaagatga cttckttggt gcaaaacaag gatgataatg agatagtgga 180

ascttcagat gtggtgaatg c 201

<210> 129
<211> 403
<212> DNA
<213> Beta vulgaris

<400> 129
ttcgagggcc atggaaattg ataatgatac tttaaacaag gatacwtcta aagatactga 60

tactagtttt cagaattctc atccagctga gccagctaat daggtgatga aaccttctga 120

tgatttagac ttaaagatga cttckttggt gcaaaacaag gatgataatg agatagtgga 180

ascttcagat gtggtgaatg cttcgagggc catggaaatt gataatgata ctttaaacaa 240

ggatacwtct aaagatactg atactagttt tcagaattct catccagctg agccagctaa 300

tagaggtgat gaaaccttct gatgatttag acttaaagat gacttckttg gtgcaaaaca 360

aggatgataa tgagatagtg gaascttcag atgtggtgaa tgc 403

<210> 130
<211> 201
<212> DNA
<213> Beta vulgaris

<400> 130
actgttgatc trcaaaaatc yatggaatct ctgccattta ccgataaaac gttttcttat 60

gagcaagaat ctggtggatt gcctgaactg gggatgtgca gttctattgt taaagataga 120

ggtcaaaagc grcctgttga ggaggatagt atgatggagg gagccaagaa agctaagcaa 180

caatggcttt ctgttgctcc a 201

<210> 131
<211> 201
<212> DNA
<213> Beta vulgaris

<400> 131
actgttgatc trcaaaaatc yatggaatct ctgccattta ccgataaaac gttttcttat 60

gagcaagaat ctggtggatt gcctgaactg gggatgtgca attctattgt taaagataga 120

ggtcaaaagc grcctgttga ggaggatagt atgatggagg gagccaagaa agctaagcaa 180

caatggcttt ctgttgctcc a 201

<210> 132
<211> 201
<212> DNA
<213> Beta vulgaris

<400> 132
actgttgatc trcaaaaatc yatggaatct ctgccattta ccgataaaac gttttcttat 60

gagcaagaat ctggtggatt gcctgaactg gggatgtgca rttctattgt taaagataga 120

ggtcaaaagc grcctgttga ggaggatagt atgatggagg gagccaagaa agctaagcaa 180

caatggcttt ctgttgctcc a 201

<210> 133
<211> 201
<212> DNA
<213> Beta vulgaris

<400> 133
aaagatgtct ttgtaacatt gatctgtaag gaagatttga tgaygattat tcgtcaaatg 60

aaagctgata atcttcatgg aagaaagttt ggattaggag cactagcaag aacaagcttg 120

gaaggatgtt ctctatccta ctctgtagaa tcaatgtata aaagctgata gtctcttgtg 180

ttgtaaacgg gkggataatc t 201

<210> 134
<211> 201
<212> DNA
<213> Beta vulgaris

<400> 134
aaagatgtct ttgtaacatt gatctgtaag gaagatttga tgaygattat tcgtcaaatg 60

aaagctgata atcttcatgg aagaaagttt ggattaggag gactagcaag aacaagcttg 120

gaaggatgtt ctctatccta ctctgtagaa tcaatgtata aaagctgata gtctcttgtg 180

ttgtaaacgg gkggataatc t 201

<210> 135
<211> 201
<212> DNA
<213> Beta vulgaris

<400> 135
aaagatgtct ttgtaacatt gatctgtaag gaagatttga tgaygattat tcgtcaaatg 60

aaagctgata atcttcatgg aagaaagttt ggattaggag sactagcaag aacaagcttg 120

gaaggatgtt ctctatccta ctctgtagaa tcaatgtata aaagctgata gtctcttgtg 180

ttgtaaacgg gkggataatc t 201

<210> 136
<211> 201
<212> DNA
<213> Beta vulgaris

<400> 136
ttgaatcacc gtaccctgay ccaccacgat atagctcccc ataagatgtg tcaaaaccac 60

ctgttgatga ggcaaatgta gatgccggag ctgcatcagt agtatgwgtt cttccataac 120

ctccacctcc caacccataa ctatcaccaa aaccataccc aagattccca ctgctatatc 180

cagaaccrtt acctccrcct t 201

<210> 137
<211> 201
<212> DNA
<213> Beta vulgaris

<400> 137
ttgaatcacc gtaccctgay ccaccacgat atagctcccc ataagatgtg tcaaaaccac 60

ctgttgatga ggcaaatgta gatgccggag ctgcatcagt ggtatgwgtt cttccataac 120

ctccacctcc caacccataa ctatcaccaa aaccataccc aagattccca ctgctatatc 180

cagaaccrtt acctccrcct t 201

<210>  138
<211>  201
<212>  DNA
<213>  Beta vulgaris

<400>  138
ttgaatcacc gtaccctgay ccaccacgat atagctcccc ataagatgtg tcaaaaccac      60

ctgttgatga ggcaaatgta gatgccggag ctgcatcagt rgtatgwgtt cttccataac     120

ctccacctcc caacccataa ctatcaccaa aaccataccc aagattccca ctgctatatc     180

cagaaccrtt acctccrcct t                                               201


<210>  139
<211>  201
<212>  DNA
<213>  Beta vulgaris

<400>  139
caccgtaccc tgayccacca cgatatagct ccccataaga tgtgtcaaaa ccacctgttg      60

atgaggcaaa tgtagatgcc ggagctgcat cagtrgtatg agttcttcca taacctccac     120

ctcccaaccc ataactatca ccaaaaccat acccaagatt cccactgcta tatccagaac     180

crttacctcc rccttgggat g                                               201


<210>  140
<211>  201
<212>  DNA
<213>  Beta vulgaris

<400>  140
caccgtaccc tgayccacca cgatatagct ccccataaga tgtgtcaaaa ccacctgttg      60

atgaggcaaa tgtagatgcc ggagctgcat cagtrgtatg tgttcttcca taacctccac     120

ctcccaaccc ataactatca ccaaaaccat acccaagatt cccactgcta tatccagaac     180

crttacctcc rccttgggat g                                               201


<210>  141
<211>  201
<212>  DNA
<213>  Beta vulgaris

<400>  141
caccgtaccc tgayccacca cgatatagct ccccataaga tgtgtcaaaa ccacctgttg      60

atgaggcaaa tgtagatgcc ggagctgcat cagtrgtatg wgttcttcca taacctccac     120

ctcccaaccc ataactatca ccaaaaccat acccaagatt cccactgcta tatccagaac     180

crttacctcc rccttgggat g                                               201


<210>  142

<211>  201
<212>  DNA
<213>  Beta vulgaris

<400>  142
tgaaggaggg agcaattacc attcctccat atgctcctcc acagaaaatg caagctcatt      60

taatccctcc agatacacct cgctccatct ttgtttactt ccgaggtctc ttytatgatg     120

tcggaaatga ccccgaagga ggttactatg caagrtttgc tctctctctc tctctctctc     180

tttctctctc tcatagaaga t                                               201


<210>  143
<211>  201
<212>  DNA
<213>  Beta vulgaris

<400>  143
tgaaggaggg agcaattacc attcctccat atgctcctcc acagaaaatg caagctcatt      60

taatccctcc agatacacct cgctccatct ttgtttactt tcgaggtctc ttytatgatg     120

tcggaaatga ccccgaagga ggttactatg caagrtttgc tctctctctc tctctctctc     180

tttctctctc tcatagaaga t                                               201


<210>  144
<211>  201
<212>  DNA
<213>  Beta vulgaris

<400>  144
tgaaggaggg agcaattacc attcctccat atgctcctcc acagaaaatg caagctcatt      60

taatccctcc agatacacct cgctccatct ttgtttactt ycgaggtctc ttytatgatg     120

tcggaaatga ccccgaagga ggttactatg caagrtttgc tctctctctc tctctctctc     180

tttctctctc tcatagaaga t                                               201


<210>  145
<211>  201
<212>  DNA
<213>  Beta vulgaris

<400>  145
ttgggtgcat tcctgtcatt atmgcagatg atatcgttct tccatttgca gatgccattc      60

catgggaaga aattgggttg tttgtggatg aaaaggatgt gccraatttg gatacaatac     120

ttacttcaat ccctcctgaa gtcatcttga ggaaacaaag gttacttgct aacccttcag     180

ttaaacaagc tatgctgttc c                                               201


<210>  146
<211>  201
<212>  DNA
<213>  Beta vulgaris

<400>  146
ttgggtgcat tcctgtcatt atmgcagatg atatcgttct tccatttgca gatgccattc      60

catgggaaga aattgggttg tttgtggatg aaaaggatgt tccraatttg gatacaatac     120

ttacttcaat ccctcctgaa gtcatcttga ggaaacaaag gttacttgct aacccttcag     180

ttaaacaagc tatgctgttc c                                               201


<210>  147
<211>  201
<212>  DNA
<213>  Beta vulgaris

<400>  147
ttgggtgcat tcctgtcatt atmgcagatg atatcgttct tccatttgca gatgccattc      60

catgggaaga aattgggttg tttgtggatg aaaaggatgt kccraatttg gatacaatac     120

ttacttcaat ccctcctgaa gtcatcttga ggaaacaaag gttacttgct aacccttcag     180

ttaaacaagc tatgctgttc c                                               201


<210>  148
<211>  201
<212>  DNA
<213>  Beta vulgaris

<400>  148
tgaccacttt tgggatgaaa gagtctcatt ttgttcagat gtaccgaaga cacgcaaatg      60

cttttcatat caatgtatat tctgcacggr aaaggctgga ttacttgttg aaggttggcg     120

tcaaacacaa cgacatcaag aggatactat tacggcaacc tcagrttctt gggtatactg     180

tggacagcaa tatgaagtcc c                                               201


<210>  149
<211>  201
<212>  DNA
<213>  Beta vulgaris

<400>  149
tgaccacttt tgggatgaaa gagtctcatt ttgttcagat gtaccgaaga cacgcaaatg      60

cttttcatat caatgtatat tctgcacggr aaaggctgga gtacttgttg aaggttggcg     120

tcaaacacaa cgacatcaag aggatactat tacggcaacc tcagrttctt gggtatactg     180

tggacagcaa tatgaagtcc c                                               201


<210>  150
<211>  201
<212>  DNA
<213>  Beta vulgaris

<400>  150
tgaccacttt tgggatgaaa gagtctcatt ttgttcagat gtaccgaaga cacgcaaatg      60

cttttcatat caatgtatat tctgcacggr aaaggctgga ktacttgttg aaggttggcg 120

tcaaacacaa cgacatcaag aggatactat tacggcaacc tcagrttctt gggtatactg 180

tggacagcaa tatgaagtcc c 201

<210> 151
<211> 201
<212> DNA
<213> Beta vulgaris

<400> 151
tgaaattwtg catcttctct gtagcttcag accgtgatga agagctcaaa cagtggcacg 60

accagtacwc cgatctcgca aattctgagc tttacttgga gcaacatcct tcagtttgtg 120

accgtggaag ttggaactag aggctgatgg aatgcaattc aggttactct ggtgtatatg 180

ctgtcagatt ttcaacagag t 201

<210> 152
<211> 201
<212> DNA
<213> Beta vulgaris

<400> 152
tgaaattwtg catcttctct gtagcttcag accgtgatga agagctcaaa cagtggcacg 60

accagtacwc cgatctcgca aattctgagc tttacttgga acaacatcct tcagtttgtg 120

accgtggaag ttggaactag aggctgatgg aatgcaattc aggttactct ggtgtatatg 180

ctgtcagatt ttcaacagag t 201

<210> 153
<211> 201
<212> DNA
<213> Beta vulgaris

<400> 153
tgaaattwtg catcttctct gtagcttcag accgtgatga agagctcaaa cagtggcacg 60

accagtacwc cgatctcgca aattctgagc tttacttgga rcaacatcct tcagtttgtg 120

accgtggaag ttggaactag aggctgatgg aatgcaattc aggttactct ggtgtatatg 180

ctgtcagatt ttcaacagag t 201

<210> 154
<211> 201
<212> DNA
<213> Beta vulgaris

<400> 154
tatatcagtt gtgatggaag aatttgaaga cttggatgtt gatcaatcag gaaccttatc 60

tgtttctgat ttagcactag ctcagtcatc ttcacaagca agasttccgr cttctggyca 120

ggagagacct atttgacatt atttctgaat atattctaga ctttgatttg tctatatctc 180

tgtgaatatt caagatcata t 201

<210> 155
<211> 201
<212> DNA
<213> Beta vulgaris

<400> 155
tatatcagtt gtgatggaag aatttgaaga cttggatgtt gatcaatcag gaaccttatc 60

tgtttctgat ttagcactag ctcagtcatc ttcacaagca ggasttccgr cttctggyca 120

ggagagacct atttgacatt atttctgaat atattctaga ctttgatttg tctatatctc 180

tgtgaatatt caagatcata t 201

<210> 156
<211> 201
<212> DNA
<213> Beta vulgaris

<400> 156
tatatcagtt gtgatggaag aatttgaaga cttggatgtt gatcaatcag gaaccttatc 60

tgtttctgat ttagcactag ctcagtcatc ttcacaagca rgasttccgr cttctggyca 120

ggagagacct atttgacatt atttctgaat atattctaga ctttgatttg tctatatctc 180

tgtgaatatt caagatcata t 201

<210> 157
<211> 201
<212> DNA
<213> Beta vulgaris

<400> 157
tggccgtttg ctcacccagg gcttgaacga ggaactccct tcacttttct cttctcctgg 60

tgtggtcaaa atatcaacaa agaaatctgt ctgcatttct cataattttt gctgccatgg 120

caacatctta tcttctctct tcaccttcat ctcttaattt tattcccaaa accctaaatt 180

catcttctct tcttcctctc a 201

<210> 158
<211> 201
<212> DNA
<213> Beta vulgaris

<400> 158
tggccgtttg ctcacccagg gcttgaacga ggaactccct tcacttttct cttctcctgg 60

tgtggtcaaa atatcaacaa agaaatctgt ctgcatttct gataattttt gctgccatgg 120

caacatctta tcttctctct tcaccttcat ctcttaattt tattcccaaa accctaaatt 180

catcttctct tcttcctctc a 201

<210>  159
<211>  201
<212>  DNA
<213>  Beta vulgaris

<400>  159
tggccgtttg ctcacccagg gcttgaacga ggaactccct tcacttttct cttctcctgg      60

tgtggtcaaa atatcaacaa agaaatctgt ctgcatttct sataattttt gctgccatgg     120

caacatctta tcttctctct tcaccttcat ctcttaattt tattcccaaa accctaaatt     180

catcttctct tcttcctctc a                                               201


<210>  160
<211>  201
<212>  DNA
<213>  Beta vulgaris

<400>  160
ttttttgtca atccaagaaa aatgatgaaa ttgttgatgc cccagataga ttaatctcag      60

caatttgcta tttttacccc ttttttgatg gtgttcaata tggaaaatat gtcattaccc     120

aatttgcycc tcttcaactc attatycaac ctttagttcc agctataagg gtgtttaaaa     180

gcttcccttt taatgggatt t                                               201


<210>  161
<211>  201
<212>  DNA
<213>  Beta vulgaris

<400>  161
ttttttgtca atccaagaaa aatgatgaaa ttgttgatgc cccagataga ttaatctcag      60

caatttgcta tttttacccc ttttttgatg gtgttcaata cggaaaatat gtcattaccc     120

aatttgcycc tcttcaactc attatycaac ctttagttcc agctataagg gtgtttaaaa     180

gcttcccttt taatgggatt t                                               201


<210>  162
<211>  201
<212>  DNA
<213>  Beta vulgaris

<400>  162
ttttttgtca atccaagaaa aatgatgaaa ttgttgatgc cccagataga ttaatctcag      60

caatttgcta tttttacccc ttttttgatg gtgttcaata yggaaaatat gtcattaccc     120

aatttgcycc tcttcaactc attatycaac ctttagttcc agctataagg gtgtttaaaa     180

gcttcccttt taatgggatt t                                               201


<210>  163

<211>  201
<212>  DNA
<213>  Beta vulgaris

<400>  163
cagtgacagc acaactcata ckcrtcaaga ataaggatga aagatacctc atgcttccat      60

tgagacattg aagctctaag tgctccaagg aagacttygg aatccatagg atcctccata      120

ttcacagtga cacctccatg gttgtcgttg actgcaggaa gcarctggmc tctatcttct      180

gcacccatat tatctgtcaa t                                                201


<210>  164
<211>  201
<212>  DNA
<213>  Beta vulgaris

<400>  164
cagtgacagc acaactcata ckcrtcaaga ataaggatga aagatacctc atgcttccat      60

tgagacattg aagctctaag tgctccaagg aagacttygg gatccatagg atcctccata      120

ttcacagtga cacctccatg gttgtcgttg actgcaggaa gcarctggmc tctatcttct      180

gcacccatat tatctgtcaa t                                                201


<210>  165
<211>  201
<212>  DNA
<213>  Beta vulgaris

<400>  165
cagtgacagc acaactcata ckcrtcaaga ataaggatga aagatacctc atgcttccat      60

tgagacattg aagctctaag tgctccaagg aagacttygg ratccatagg atcctccata      120

ttcacagtga cacctccatg gttgtcgttg actgcaggaa gcarctggmc tctatcttct      180

gcacccatat tatctgtcaa t                                                201


<210>  166
<211>  201
<212>  DNA
<213>  Beta vulgaris

<400>  166
acaggctatc ttatcttcac ycacagattc taaactacca ttctcaactg catgatctag      60

agccatacag ctcaacttct gcacattgat atcaaaaggt ggttccctgc cggcactatt      120

tytcatactc agatctgtca ttttcacaca attgtcaaca gcatcatcta aatcttcctc      180

gctgctttca gratcctcca g                                                201


<210>  167
<211>  201
<212>  DNA
<213>  Beta vulgaris

<400>  167
acaggctatc ttatcttcac ycacagattc taaactacca ttctcaactg catgatctag      60

agccatacag ctcaacttct gcacattgat atcaaaaggt tgttccctgc cggcactatt     120

tytcatactc agatctgtca ttttcacaca attgtcaaca gcatcatcta aatcttcctc     180

gctgctttca gratcctcca g                                               201


<210>  168
<211>  201
<212>  DNA
<213>  Beta vulgaris

<400>  168
acaggctatc ttatcttcac ycacagattc taaactacca ttctcaactg catgatctag      60

agccatacag ctcaacttct gcacattgat atcaaaaggt kgttccctgc cggcactatt     120

tytcatactc agatctgtca ttttcacaca attgtcaaca gcatcatcta aatcttcctc     180

gctgctttca gratcctcca g                                               201


<210>  169
<211>  201
<212>  DNA
<213>  Beta vulgaris

<400>  169
aaatcaaaac tatgttagga tgcaaaaygt taagaaacct ccttttagct gaactgataa      60

cgggaatttc attcatatca ccagtgctta gattgatcac gtctccactg ttccaactaa     120

gatagagcag attggcatac ttrtgaatat tctcactggc tattaaattc tcagaagaaa     180

aatcaacacc aagattatga c                                               201


<210>  170
<211>  201
<212>  DNA
<213>  Beta vulgaris

<400>  170
aaatcaaaac tatgttagga tgcaaaaygt taagaaacct ccttttagct gaactgataa      60

cgggaatttc attcatatca ccagtgctta gattgatcac atctccactg ttccaactaa     120

gatagagcag attggcatac ttrtgaatat tctcactggc tattaaattc tcagaagaaa     180

aatcaacacc aagattatga c                                               201


<210>  171
<211>  201
<212>  DNA
<213>  Beta vulgaris

<400>  171
aaatcaaaac tatgttagga tgcaaaaygt taagaaacct ccttttagct gaactgataa      60

cgggaatttc attcatatca ccagtgctta gattgatcac rtctccactg ttccaactaa 120

gatagagcag attggcatac ttrtgaatat tctcactggc tattaaattc tcagaagaaa 180

aatcaacacc aagattatga c 201

<210> 172
<211> 201
<212> DNA
<213> Beta vulgaris

<400> 172
ttttagctga actgataacg ggaatttcat tcatatcacc agtgcttaga ttgatcacrt 60

ctccactgtt ccaactaaga tagagcagat tggcatactt atgaatattc tcactggcta 120

ttaaattctc agaagaaaaa tcaacaccaa gattatgaca tgcttgtgca aagacacacc 180

crgtcatgaa tgcatcatag c 201

<210> 173
<211> 201
<212> DNA
<213> Beta vulgaris

<400> 173
ttttagctga actgataacg ggaatttcat tcatatcacc agtgcttaga ttgatcacrt 60

ctccactgtt ccaactaaga tagagcagat tggcatactt gtgaatattc tcactggcta 120

ttaaattctc agaagaaaaa tcaacaccaa gattatgaca tgcttgtgca aagacacacc 180

crgtcatgaa tgcatcatag c 201

<210> 174
<211> 201
<212> DNA
<213> Beta vulgaris

<400> 174
ttttagctga actgataacg ggaatttcat tcatatcacc agtgcttaga ttgatcacrt 60

ctccactgtt ccaactaaga tagagcagat tggcatactt rtgaatattc tcactggcta 120

ttaaattctc agaagaaaaa tcaacaccaa gattatgaca tgcttgtgca aagacacacc 180

crgtcatgaa tgcatcatag c 201

<210> 175
<211> 162
<212> DNA
<213> Beta vulgaris

<400> 175
tcaaaccaat tcgacttttg ctcraagttg caaactaaag aaaaaggctg aatgcaaagc 60

aagttcayca atgaacaata gaccggtgtt ggcctgaggc cacatcaagt gaagttgcct 120

aattgcggcc ctctcatctg ttcacaggaa tcattttcca ta 162

<210> 176
<211> 162
<212> DNA
<213> Beta vulgaris

<400> 176
tcaaaccaat tcgacttttg ctcraagttg caaactaaag aaaaaggctg aatgcaaagc 60

aagttcayca atgaacaata gatcggtgtt ggcctgaggc cacatcaagt gaagttgcct 120

aattgcggcc ctctcatctg ttcacaggaa tcattttcca ta 162

<210> 177
<211> 162
<212> DNA
<213> Beta vulgaris

<400> 177
tcaaaccaat tcgacttttg ctcraagttg caaactaaag aaaaaggctg aatgcaaagc 60

aagttcayca atgaacaata gaycggtgtt ggcctgaggc cacatcaagt gaagttgcct 120

aattgcggcc ctctcatctg ttcacaggaa tcattttcca ta 162

<210> 178
<211> 128
<212> DNA
<213> Beta vulgaris

<400> 178
attaaagctt taattgccat ttgacatccc cttgctcaac tttagcatgt ttttaggcta 60

caacaaatac gcactgtcta catggacata caaattacaa gcgtatggaa aagcaataag 120

cgcaagga 128

<210> 179
<211> 127
<212> DNA
<213> Beta vulgaris

<400> 179
attaaagctt taattgccat ttgacatccc cttgctcaac tttagcatgt ttttaggcta 60

caacaatacg cactgtctac atggacatac aaattacaag cgtatggaaa agcaataagc 120

gcaagga 127

<210> 180
<211> 128
<212> DNA
<213> Beta vulgaris

<220>
<221> misc_recomb

<222>  (65)..(65)
<223>  deletion

<400>  180
attaaagctt taattgccat ttgacatccc cttgctcaac tttagcatgt ttttaggcta      60

caacaaatac gcactgtcta catggacata caaattacaa gcgtatggaa aagcaataag     120

cgcaagga                                                              128


<210>  181
<211>  201
<212>  DNA
<213>  Beta vulgaris

<400>  181
gggagtatta tccaatatcc atgcaggttg ttggtacatg ttaattgtta catgaagccr      60

tccaaaacct tcaaaagcag taagtcttgc tgctatgyca aattcaaata ttcaactcca     120

aaaaaaaaaa aaaaaaaatt caaaaatccg cttttcagcg aawawatagg aaataatcca     180

agaatcgaaa tcgaaataaa g                                               201


<210>  182
<211>  201
<212>  DNA
<213>  Beta vulgaris

<400>  182
gggagtatta tccaatatcc atgcaggttg ttggtacatg ttaattgtta catgaagccr      60

tccaaaacct tcaaaagcag taagtcttgc tgctatgyca tattcaaata ttcaactcca     120

aaaaaaaaaa aaaaaaaatt caaaaatccg cttttcagcg aawawatagg aaataatcca     180

agaatcgaaa tcgaaataaa g                                               201


<210>  183
<211>  201
<212>  DNA
<213>  Beta vulgaris

<400>  183
gggagtatta tccaatatcc atgcaggttg ttggtacatg ttaattgtta catgaagccr      60

tccaaaacct tcaaaagcag taagtcttgc tgctatgyca wattcaaata ttcaactcca     120

aaaaaaaaaa aaaaaaaatt caaaaatccg cttttcagcg aawawatagg aaataatcca     180

agaatcgaaa tcgaaataaa g                                               201


<210>  184
<211>  134
<212>  DNA
<213>  Beta vulgaris

<400>  184
gctagatttt gttggaactt tcataaatca tastgacata agtatagcat aatattcatg      60

scktccgaca gaagttttcg catatggtaa ggctactatt gaaaaaattc ccttgtgttt     120

gaagtacgca taaa                                                      134

<210>  185
<211>  133
<212>  DNA
<213>  Beta vulgaris

<400>  185
gctagatttt gttggaactt tcataaatca tastgacata agtatagcat aatattcatg      60

scktcgacag aagttttcgc atatggtaag gctactattg aaaaaattcc cttgtgtttg     120

aagtacgcat aaa                                                       133

<210>  186
<211>  134
<212>  DNA
<213>  Beta vulgaris

<220>
<221>  misc_recomb
<222>  (65)..(65)
<223>  deletion

<400>  186
gctagatttt gttggaactt tcataaatca tastgacata agtatagcat aatattcatg      60

scktccgaca gaagttttcg catatggtaa ggctactatt gaaaaaattc ccttgtgttt     120

gaagtacgca taaa                                                      134

<210>  187
<211>  144
<212>  DNA
<213>  Beta vulgaris

<400>  187
aatataaaaa tctatttggg ttgagtatat agttggttag tccaataata tataaattta      60

taaggttgag gtcttcggta tatgacattc caaatttgag tatcaamtga tatatatggk     120

tttccatact tgaattactt gaat                                           144

<210>  188
<211>  135
<212>  DNA
<213>  Beta vulgaris

<400>  188
aatataaaaa tctatttggg ttgagtatat agttggttag tccaataata tataaattta      60

taaggtggag gtcttcggta tatgacattc caaatttgag tatcaamtga tatatatggk     120

tttccatact tgaat                                                     135

<210>  189
<211>  135
<212>  DNA
<213>  Beta vulgaris

<400>  189
aatataaaaa tctatttggg ttgagtatat agttggttag tccaataata tataaattta      60

taaggtkgag gtcttcggta tatgacattc caaatttgag tatcaamtga tatatatggk      120

tttccatact tgaat                                                       135


<210>  190
<211>  201
<212>  DNA
<213>  Beta vulgaris

<400>  190
cttatattat taaaaagttc aggaaaatga caaatttaat aacaaaaaga acatgcaggg      60

atttgtctaa aattaccaca cggccaacaa atttgaagta cgaaagatgt tcagtytggt      120

agacrgaatt agggttaggc tggaaggtcg cattgttccc tactgtggtg aatagtagag      180

ctcccttatc aaataagacc c                                                201


<210>  191
<211>  201
<212>  DNA
<213>  Beta vulgaris

<400>  191
cttatattat taaaaagttc aggaaaatga caaatttaat aacaaaaaga acatgcaggg      60

atttgtctaa aattaccaca cggccaacaa atttgaagta tgaaagatgt tcagtytggt      120

agacrgaatt agggttaggc tggaaggtcg cattgttccc tactgtggtg aatagtagag      180

ctcccttatc aaataagacc c                                                201


<210>  192
<211>  201
<212>  DNA
<213>  Beta vulgaris

<400>  192
cttatattat taaaaagttc aggaaaatga caaatttaat aacaaaaaga acatgcaggg      60

atttgtctaa aattaccaca cggccaacaa atttgaagta ygaaagatgt tcagtytggt      120

agacrgaatt agggttaggc tggaaggtcg cattgttccc tactgtggtg aatagtagag      180

ctcccttatc aaataagacc c                                                201


<210>  193
<211>  201
<212>  DNA
<213>  Beta vulgaris

<400>  193
agkggagagc atggaagcaa aaggtaytgc agttagttgt accatgtaca cgatgatgat      60

ggatggattt tgcaaaaagg ggatgctcac tgaagctgta gatttgctag ctgcaatatc      120

aaaggttkgc gcttktgctg acgtgatcac atattctgtg ctaatcaatg gattctgcag      180

gttggggaag atgcataatg c                                                201


<210>  194
<211>  201
<212>  DNA
<213>  Beta vulgaris

<400>  194
agkggagagc atggaagcaa aaggtaytgc agttagttgt accatgtaca cgatgatgat      60

ggatggattt tgcaaaaagg ggatgctcac tgaagctgta aatttgctag ctgcaatatc      120

aaaggttkgc gcttktgctg acgtgatcac atattctgtg ctaatcaatg gattctgcag      180

gttggggaag atgcataatg c                                                201


<210>  195
<211>  201
<212>  DNA
<213>  Beta vulgaris

<400>  195
agkggagagc atggaagcaa aaggtaytgc agttagttgt accatgtaca cgatgatgat      60

ggatggattt tgcaaaaagg ggatgctcac tgaagctgta ratttgctag ctgcaatatc      120

aaaggttkgc gcttktgctg acgtgatcac atattctgtg ctaatcaatg gattctgcag      180

gttggggaag atgcataatg c                                                201


<210>  196
<211>  201
<212>  DNA
<213>  Beta vulgaris

<400>  196
agtaaataga atttgttcaa ctatcttaag gtctctggtc ttgtactcaa aatgtatcat      60

atcctcaagt gtagagggtt gagatagtgt ggactgtgga ctgttcactc cggatgaagg      120

gagaaaaaca aaaggagagg ygagacgtga gagtatttct agagttgaca agaactgaaa      180

gagtgacatc tggtcatcta c                                                201


<210>  197
<211>  201
<212>  DNA
<213>  Beta vulgaris

<400>  197
agtaaataga atttgttcaa ctatcttaag gtctctggtc ttgtactcaa aatgtatcat      60

atcctcaagt gtagagggtt gagatagtgt ggactgtgga ttgttcactc cggatgaagg 120

gagaaaaaca aaaggagagg ygagacgtga gagtatttct agagttgaca agaactgaaa 180

gagtgacatc tggtcatcta c 201

<210> 198
<211> 201
<212> DNA
<213> Beta vulgaris

<400> 198
agtaaataga atttgttcaa ctatcttaag gtctctggtc ttgtactcaa aatgtatcat 60

atcctcaagt gtagagggtt gagatagtgt ggactgtgga ytgttcactc cggatgaagg 120

gagaaaaaca aaaggagagg ygagacgtga gagtatttct agagttgaca agaactgaaa 180

gagtgacatc tggtcatcta c 201

<210> 199
<211> 201
<212> DNA
<213> Beta vulgaris

<400> 199
aagargarga gaagaaagat gaggarggta aggtagagga tgttgatgag gaaaaagaga 60

aagaagagaa gaagaaaaag aagattaarg aggtctccaa cgaatggtct ttggttaaca 120

agcaaaagcc catctggatg aggaagccag aagagatcac caaagaggag tacgctgcat 180

tttacaagag tcttaccaat g 201

<210> 200
<211> 201
<212> DNA
<213> Beta vulgaris

<400> 200
aagargarga gaagaaagat gaggarggta aggtagagga tgttgatgag gaaaaagaga 60

aagaagagaa gaagaaaaag aagattaarg aggtctccaa tgaatggtct ttggttaaca 120

agcaaaagcc catctggatg aggaagccag aagagatcac caaagaggag tacgctgcat 180

tttacaagag tcttaccaat g 201

<210> 201
<211> 201
<212> DNA
<213> Beta vulgaris

<400> 201
aagargarga gaagaaagat gaggarggta aggtagagga tgttgatgag gaaaaagaga 60

aagaagagaa gaagaaaaag aagattaarg aggtctccaa ygaatggtct ttggttaaca 120

agcaaaagcc catctggatg aggaagccag aagagatcac caaagaggag tacgctgcat 180

tttacaagag tcttaccaat g 201

<210> 202
<211> 201
<212> DNA
<213> Beta vulgaris

<400> 202
aacgaaaaca agagaaaact aacaagcatt tgcagatagc cagagacata magtcgattt 60

tttgttcaat aggagcttgt ggatggaaaa cagagagtcc acgagcgaac tcttcaaaac 120

ctaaaactcc attatgcttt gtgtcaaaca agtcaaagac cygcaasaag taaacaayat 180

tagcattkct gagaaaaata c 201

<210> 203
<211> 201
<212> DNA
<213> Beta vulgaris

<400> 203
aacgaaaaca agagaaaact aacaagcatt tgcagatagc cagagacata magtcgattt 60

tttgttcaat aggagcttgt ggatggaaaa cagagagtcc ccgagcgaac tcttcaaaac 120

ctaaaactcc attatgcttt gtgtcaaaca agtcaaagac cygcaasaag taaacaayat 180

tagcattkct gagaaaaata c 201

<210> 204
<211> 201
<212> DNA
<213> Beta vulgaris

<400> 204
aacgaaaaca agagaaaact aacaagcatt tgcagatagc cagagacata magtcgattt 60

tttgttcaat aggagcttgt ggatggaaaa cagagagtcc mcgagcgaac tcttcaaaac 120

ctaaaactcc attatgcttt gtgtcaaaca agtcaaagac cygcaasaag taaacaayat 180

tagcattkct gagaaaaata c 201

<210> 205
<211> 201
<212> DNA
<213> Beta vulgaris

<400> 205
tatcccacca ctctcgatcc ttaaacttca tcctcaaayy aataagcttc tccgcataag 60

gagaaggtga cataagctca gtagaacgct cagactcatt cgtaggctta atcacaaaat 120

gcttaatccc agtaacaaac tcagtcatat tatactgcat tgccaaccca tcaatctgat 180

cagacacygt ctcataaaac t 201

<210>  206
<211>  201
<212>  DNA
<213>  Beta vulgaris

<400>  206
tatcccacca ctctcgatcc ttaaacttca tcctcaaayy aataagcttc tccgcataag      60

gagaaggtga cataagctca gtagaacgct cagactcatt tgtaggctta atcacaaaat     120

gcttaatccc agtaacaaac tcagtcatat tatactgcat tgccaaccca tcaatctgat     180

cagacacygt ctcataaaac t                                               201


<210>  207
<211>  201
<212>  DNA
<213>  Beta vulgaris

<400>  207
tatcccacca ctctcgatcc ttaaacttca tcctcaaayy aataagcttc tccgcataag      60

gagaaggtga cataagctca gtagaacgct cagactcatt ygtaggctta atcacaaaat     120

gcttaatccc agtaacaaac tcagtcatat tatactgcat tgccaaccca tcaatctgat     180

cagacacygt ctcataaaac t                                               201


<210>  208
<211>  201
<212>  DNA
<213>  Beta vulgaris

<400>  208
cagaggaggt ggktgtttca aagttaaagc tccatttttc tagagaaatt gtcaaaaaaa      60

aaagaagcaa aaaaaggggc aarctttyac ctctatctct ctcttccact actattgttc     120

ttgtttctac acctctcctt ggtttttgct tcaaatctct tcaaggattg aaaagggttc     180

ttaaagatcc caawtttaag g                                               201


<210>  209
<211>  201
<212>  DNA
<213>  Beta vulgaris

<400>  209
cagaggaggt ggktgtttca aagttaaagc tccatttttc tagagaaatt gtcaaaaaaa      60

aaagaagcaa aaaaaggggc aarctttyac ctctatctct atcttccact actattgttc     120

ttgtttctac acctctcctt ggtttttgct tcaaatctct tcaaggattg aaaagggttc     180

ttaaagatcc caawtttaag g                                               201


<210>  210

<211>  201
<212>  DNA
<213>  Beta vulgaris

<400>  210
cagaggaggt ggktgtttca aagttaaagc tccatttttc tagagaaatt gtcaaaaaaa      60

aaagaagcaa aaaaaggggc aarctttyac ctctatctct mtcttccact actattgttc     120

ttgtttctac acctctcctt ggtttttgct tcaaatctct tcaaggattg aaaagggttc     180

ttaaagatcc caawtttaag g                                               201


<210>  211
<211>  201
<212>  DNA
<213>  Beta vulgaris

<400>  211
tatgcacaac gcatttagct gcataaagga ccgattggrt caatactaat attacaacat      60

gaaggctgaa gctgttaccg ttcaaaaggt ggcaacgctc gayttatagt tgcttgtagc     120

aactatatgc gacaccaact gagacagcag cacaracaat tgcaagggtt gcrtatgtat     180

cttcacgttc ccagctctcg a                                               201


<210>  212
<211>  201
<212>  DNA
<213>  Beta vulgaris

<400>  212
tatgcacaac gcatttagct gcataaagga ccgattggrt caatactaat attacaacat      60

gaaggctgaa gctgttaccg ttcaaaaggt ggcaacgctc aayttatagt tgcttgtagc     120

aactatatgc gacaccaact gagacagcag cacaracaat tgcaagggtt gcrtatgtat     180

cttcacgttc ccagctctcg a                                               201


<210>  213
<211>  201
<212>  DNA
<213>  Beta vulgaris

<400>  213
tatgcacaac gcatttagct gcataaagga ccgattggrt caatactaat attacaacat      60

gaaggctgaa gctgttaccg ttcaaaaggt ggcaacgctc rayttatagt tgcttgtagc     120

aactatatgc gacaccaact gagacagcag cacaracaat tgcaagggtt gcrtatgtat     180

cttcacgttc ccagctctcg a                                               201


<210>  214
<211>  201
<212>  DNA
<213>  Beta vulgaris

<400>  214
gttcgagagc tgggaacgtg aagatacata ygcaaccctt gcaattgtyt gtgctgctgt      60

ctcagttggt gtcgcatata gttgctacaa gcaactataa atygagcgtt gccacctttt     120

gaacggtaac agcttcagcc ttcatgttgt aatattagta ttgayccaat cggtccttta     180

tgcagctaaa tgcgttgtgc a                                               201


<210>  215
<211>  201
<212>  DNA
<213>  Beta vulgaris

<400>  215
gttcgagagc tgggaacgtg aagatacata ygcaaccctt gcaattgtyt gtgctgctgt      60

ctcagttggt gtcgcatata gttgctacaa gcaactataa gtygagcgtt gccacctttt     120

gaacggtaac agcttcagcc ttcatgttgt aatattagta ttgayccaat cggtccttta     180

tgcagctaaa tgcgttgtgc a                                               201


<210>  216
<211>  201
<212>  DNA
<213>  Beta vulgaris

<400>  216
gttcgagagc tgggaacgtg aagatacata ygcaaccctt gcaattgtyt gtgctgctgt      60

ctcagttggt gtcgcatata gttgctacaa gcaactataa rtygagcgtt gccacctttt     120

gaacggtaac agcttcagcc ttcatgttgt aatattagta ttgayccaat cggtccttta     180

tgcagctaaa tgcgttgtgc a                                               201


<210>  217
<211>  201
<212>  DNA
<213>  Beta vulgaris

<400>  217
acatgcagat tcccagcaat ggattctgtt tctgcttctt tytcttttaa ttttgcatgc      60

tcttttctca aattctctag ttcctctttc agagaatcca ccatgttttg gagggcactt     120

ccctcttcca acactttttg sagagattct ttcgcaccat csagctccaa tgtgacmgtt     180

ctcacagagt ctacatccaa t                                               201


<210>  218
<211>  201
<212>  DNA
<213>  Beta vulgaris

<400>  218
acatgcagat tcccagcaat ggattctgtt tctgcttctt tytcttttaa ttttgcatgc      60

tcttttctca aattctctag ttcctctttc agagaatcca acatgttttg gagggcactt 120

ccctcttcca acactttttg sagagattct ttcgcaccat csagctccaa tgtgacmgtt 180

ctcacagagt ctacatccaa t 201

<210> 219
<211> 201
<212> DNA
<213> Beta vulgaris

<400> 219
acatgcagat tcccagcaat ggattctgtt tctgcttctt tytcttttaa ttttgcatgc 60

tcttttctca aattctctag ttcctctttc agagaatcca mcatgttttg gagggcactt 120

ccctcttcca acactttttg sagagattct ttcgcaccat csagctccaa tgtgacmgtt 180

ctcacagagt ctacatccaa t 201

<210> 220
<211> 201
<212> DNA
<213> Beta vulgaris

<400> 220
tccctgagga tgcatgaagt tgcttgtaaa attttgaaat acttgaccaa cggtgcagtt 60

gtactcggag agtactgtta cgaccttcaa aagctataaa ctwgagtttc atctttattc 120

tattgacatg cttcacaaaa caacgtagtt atacaggatt tttatattat gacatgctrc 180

gctctttgtc acaagtagag c 201

<210> 221
<211> 201
<212> DNA
<213> Beta vulgaris

<400> 221
tccctgagga tgcatgaagt tgcttgtaaa attttgaaat acttgaccaa cggtgcagtt 60

gtactcggag agtactgtta cgaccttcaa aagctataaa ttwgagtttc atctttattc 120

tattgacatg cttcacaaaa caacgtagtt atacaggatt tttatattat gacatgctrc 180

gctctttgtc acaagtagag c 201

<210> 222
<211> 201
<212> DNA
<213> Beta vulgaris

<400> 222
tccctgagga tgcatgaagt tgcttgtaaa attttgaaat acttgaccaa cggtgcagtt 60

gtactcggag agtactgtta cgaccttcaa aagctataaa ytwgagtttc atctttattc 120

tattgacatg cttcacaaaa caacgtagtt atacaggatt tttatattat gacatgctrc     180

gctctttgtc acaagtagag c                                                201

<210>  223
<211>  517
<212>  PRT
<213>  Artificial Sequence

<220>
<223>  mutated EPSP

<400>  223

Met Ala Gln Ala Ser Thr Ile Asn Asn Gly Val Lys Ser Thr Gln Leu
1               5                   10                  15

Cys Pro Asn Leu Pro Lys Thr His Leu Ser Lys Ser Ser Lys Ser Val
            20                  25                  30

Lys Phe Gly Ser Asn Leu Arg Phe Ser Pro Lys Leu Lys Ser Phe Asn
        35                  40                  45

Asn Glu Arg Val Gly Gly Asn Ser Ser Val Val Phe Arg Val Arg Ala
    50                  55                  60

Ser Val Ala Ala Ala Ala Glu Lys Ser Ser Thr Val Pro Glu Ile Val
65                  70                  75                  80

Leu Gln Pro Ile Lys Glu Ile Ser Gly Thr Ile Gln Leu Pro Gly Ser
                85                  90                  95

Lys Ser Leu Ser Asn Arg Ile Leu Leu Leu Ala Ala Leu Ser Gln Gly
            100                 105                 110

Thr Thr Val Val Asp Asn Leu Leu Tyr Ser Asp Asp Ile Arg Tyr Met
        115                 120                 125

Leu Asp Ala Leu Arg Thr Leu Gly Leu Asn Val Glu Asp Asp Asn Ile
    130                 135                 140

Ala Lys Arg Ala Ile Val Glu Gly Cys Gly Gly Leu Phe Pro Val Gly
145                 150                 155                 160

Lys Asp Gly Lys Glu Ile Glu Leu Phe Leu Gly Asn Ala Gly Thr Ala
                165                 170                 175

Met Arg Ser Leu Thr Ala Ala Val Ala Val Ala Gly Gly Asn Ser Ser
            180                 185                 190

Tyr Val Leu Asp Gly Val Pro Arg Met Arg Glu Arg Pro Ile Gly Asp
        195                 200                 205

Leu Val Ala Gly Leu Lys Gln Leu Gly Ala Asp Val Asp Cys Tyr Leu
    210                 215                 220

Gly Thr Asn Cys Pro Pro Val Arg Val Asn Ala Lys Gly Gly Leu Pro
225                 230                 235                 240

Gly Gly Lys Val Lys Leu Ser Gly Ser Val Ser Ser Gln Tyr Leu Thr
                245                 250                 255

Ala Leu Leu Met Ala Thr Pro Leu Gly Leu Gly Asp Val Glu Val Glu
            260                 265                 270

Ile Ile Asp Lys Leu Ile Ser Val Pro Tyr Val Glu Met Thr Ile Lys
        275                 280                 285

Leu Met Glu Arg Phe Gly Val Ser Val Glu His Ser Ala Asp Trp Gly
    290                 295                 300

Arg Phe Leu Ile Arg Gly Gly Gln Lys Tyr Lys Ser Pro Gly Asn Ala
305                 310                 315                 320

Tyr Val Glu Gly Asp Ala Ser Ser Ala Ser Tyr Phe Ile Gly Gly Ala
                325                 330                 335

Ala Val Thr Gly Gly Thr Val Thr Val Glu Gly Cys Gly Thr Ser Ser
            340                 345                 350

Leu Gln Gly Asp Val Lys Phe Ala Glu Val Leu Glu Lys Met Gly Cys
        355                 360                 365

Lys Val Ser Trp Thr Glu Asn Ser Val Thr Val Thr Gly Pro Pro Arg
    370                 375                 380

Asp Ala Ser Gly Arg Lys His Leu Arg Ala Val Asp Val Asn Met Asn
385                 390                 395                 400

Lys Met Pro Asp Val Ala Met Thr Leu Ala Val Val Ala Leu Tyr Ala
                405                 410                 415

Asp Gly Pro Thr Thr Ile Arg Asp Val Ala Ser Trp Arg Val Lys Glu
            420                 425                 430

Thr Glu Arg Met Ile Ala Ile Cys Thr Glu Leu Arg Lys Leu Gly Ala
        435                 440                 445

Thr Val Glu Glu Gly Ser Asp Tyr Cys Val Ile Thr Pro Pro Glu Lys
    450                 455                 460

Leu Asn Val Thr Ala Ile Asp Thr Tyr Asp Asp His Arg Met Ala Met
465                 470                 475                 480

Ala Phe Ser Leu Ala Ala Cys Ala Asp Val Pro Val Thr Ile Lys Asp
                485                 490                 495

Pro Gly Cys Thr Arg Lys Thr Phe Pro Asp Tyr Phe Asp Val Leu Glu
            500                 505                 510

Arg Phe Ala Lys His
        515

<210>  224
<211>  7001
<212>  DNA
<213>  Artificial Sequence

<220>
<223>  genomic DNA of mutated EPSPS gene

<400>  224
aggaagtatt tgaatttgat atagatattg tgtctttgtg tgtgttgaat ttcaattccc      60

agttccctaa aaaaaattta caattgcaat ttcgagatta tgatgtaaat taaatttgag     120

agactagaaa gtatttggtc aacccaaaaa aaaaatatca atacttatat aaatcaaaaa     180

cataatagag aatccaattt tactaaaaat attagtaatt ttgattaaaa taatctatta     240

aaatgaactc taaccttcac ataatttcca catattatta atcaacaaaa taagcatcac     300

aaattattag aataggcgat ctaattttaa cataaaatta gacgaattca aattgaattt     360

ttctaacaag ctcattccat ttcacgcaac ccaaaattat cctagtcagt agtcatccat     420

tcttttctca ttcctttatt cttgattatc gaactacaac agataatttc aaaaaaaaac     480

taaattggta gtcttaactg attaaactac ttactaaatg gattaaagaa tgtcattact     540

gaatagatta aactgattac gaaatagatt aacttggtcc ctaaatagat taaattagtt     600

actatattaa aattaggcga tctcttacaa aaccaactga ataagcatag ctctgtatat     660

tacctagatt tcaactaaat caaaacccct tacagttcaa tctagagctg atcattttgg     720

ctcggcccgt cccatttttg ggccgggttt tagtcagatt tttttggccc gcggtcgggc     780

ccggcccgat ttttttggct ttgggcaagc caaaaacgac ttttcagttt attttttggc     840

ccgacccgtt tttacccgca aaagcccgct aatttaggtc cgcactttgg gcacaaaaat     900

ttagcccgaa cttaaacctg gcccgaccca tgatcacctc tagtttaatc caaactaaaa     960

aactacacaa gttagccaaa aattatgtct actttgtaca actttataaa atacacacag    1020

tagttgatat cttgatgatt aactcctttt gaagtttgac tacacaccaa ccccaaacac 1080

acccactttt tcccccctct tgtcaccaac cccccctcct ctttagccac caaagtttgg 1140

ttggtgagtc ctccataact gctaaattct ctcttttttc tctctcctaa aaaactaaaa 1200

cccaccaaaa tttcagacat caaaaaaatt acaagtgaag gaaacaataa tggctcaagc 1260

tagcaccata aacaatggtg tcaaaagcac ccaattatgc cccaatttac ccaaaaccca 1320

cttatccaaa tcttcaaaat ctgttaaatt tggatcaaat ttgagatttt ctccaaagtt 1380

gaaatctttt aacaatgaaa gagttggtgg gaattcatca gttgttttca gggttagggc 1440

ttcagttgca gcagcagctg aaaaatcatc aactgtacca gaaattgtgt tacaacccat 1500

caaagagatc tcgggtacca ttcaattgcc cggatccaag tcattatcta atcggattct 1560

acttcttgct gccctttctc aggtactttt caattgtttg atttcttttt ttcttagaac 1620

ttgtgaattt gtatacttta tccgtttcta aatacgtgca acatttgaat agtaacgagt 1680

atttatctac caacttattt aatattctct cacgaatgta tatgaaaaaa tatagtcatg 1740

cgtggtttta tttgattgat ctgcggactt ttataatatc aactttttat aatttagagg 1800

acaaagtagt gtattgggta gcgtgtaagg aggttgggaa actggaggaa ttttttaaca 1860

attcaagttt gatatttttc atagtgaaat gtttattagc atagaatcat gcttttagtt 1920

tttagtggag tgtgcattta ttctttaact tgttggatgg ctatgattaa gaattggatt 1980

ctggttattt gcttgagtat ttagaaatta attgtgggtg ttggtgataa tgtaacaaaa 2040

ttgttttgaa ggtgtgagaa tgtgatttta attatatgag gaaatgatgg gttatttatt 2100

gtaatgtggg aatttatgat aatatgttgg aggatgaaac aattgatgat tttgaagtgg 2160

ggtatgaagg ctttccccca tttttttacc tttcatgtgt tgtatgtagc agactgccaa 2220

agcaattctt ctgagcagct tggcatattt ccattgcaac tcataatctc aatccaagca 2280

caatcaatta cctgccacgc tagcacttta agtagcaaaa tcgcgcttga agaaaagata 2340

ataccacagg cttctatagt tctattcctt gtgatatagg acacaagtat catcctagat 2400

gacttcccta gccctgcaaa taattcctgg aaggaagtcc gttgagtatc cttgatcaag 2460

taaaattagc gctttctttt gggtaacaca aactatactc gaaccacttt atgctaaggg 2520

atcacattgc gtctcttgca gaacttttaa catttctaac atggcataag cacggaaaca 2580

ccctgtgaag ttgctagtga ccatcttttt tgataaatca agaatccctg ttcttatttg 2640

catacaaagg gttaagtctt gtctgagagg gattgacata tctcctgctg gatagacttt 2700

gtgttcatct ctaatgttat tctgagtttg cattgtatgt gttgtgtttt cgtttttctt 2760

cttgtttgtg tgtgtgtgtg tgtgtgtgtg tgtgtgttac ccaaacttgt acggaaattt 2820

ccatcctaac gagaattatg ctgcagggaa caacagtggt tgacaacttg ctatatagtg 2880

atgatatccg ttacatgctg gatgctttga gaactcttgg gttgaatgtg gaggatgata 2940

atatagccaa aagggcaatc gtggagggtt gcggtggttt gtttcctgtt ggaaaagatg 3000

ggaaagaaat tgaacttttt cttggaaatg caggaacagc aatgcgctca ttgacagctg 3060

cagttgctgt tgctggagga aattctaggt ttgtctacta aggccttcca tgtcatcgtg 3120

aggctgttaa tattctttta ttttgtgtgg agacttgttt cctcttgcta caattgcaaa 3180

gggggctggg attgattaac aatgcagaac tcaaacacta tctttggtat caccgtctgg 3240

caggtggcat ctgatcttgc aaaacagata tggagggttt tgccggaaag gggttggggg 3300

tgggacttgg gagtatagcc tcaatgttat agaagattgt gctatactac tgtaaaatta 3360

acttttggtt ggaaccagat atagttaata aagtattttt ttatctggtg aggggtgctt 3420

ctaggtcctg aaaagttcca ccatagaaaa aaattgaatt tcattgtgaa aagctaaggt 3480

gaattcatta aattcaatac atgttgttga ttgcttgaac tagaatactt cagatatcag 3540

ttaattgaat ttatttcttt tggagaacat gcatgaagag cttttaactc gcctctacta 3600

tcctgctctt aattattttt ataaatagat ttagcaattg ggaatatcct gtaatttgct 3660

ggttgctttt tcatatggta tatattgaat tggtgtggag gcgttttaca aagagtgaag 3720

gtaattgttg atgggtggat attgaatgga aatactttga tggcggatag catattcatc 3780

tgattggatg tctgaaattt gatgagctct agtaaatgtt cctccatgta tgcttgtgta 3840

ttgggaattt gcaatccagt agtagagaac acccctgata attgcactat tattcctgag 3900

ttgtgagagc tctcaagaag atagatgggg ttcttcaagt tttttaattg tctttaggta 3960

gtgtaaaggt tgcaatattt caaaggatct gtctgatcca acaagtctgc taaatccttc 4020

accaatctga aaggggagaa gatactgctc tctctgacct gcttgcatgc atataaagct 4080

tctaaaacaa ataggttaaa tgtaaaacaa gcgcagaagt tttcacttct cttgggaaat 4140

gtgtgccaag atctattgca tgacatgaat tcatatttat tgattatatg ctcaacatga 4200

atgggattaa gaatgaaata aagtagcatg agtaggagac aatccgcctg ctcttttaat 4260

aggcatatac aatcagtcat caacatcatt gctgctttag aatttgattt atgttgatct 4320

tggtttttgc agctatgtac ttgatggagt gccgagaatg agggagcgac ccattgggga 4380

tttggtagcg ggtctaaagc aacttggcgc cgatgttgac tgttatcttg gcacaaattg 4440

tcctcctgtt cgagtgaatg ctaaaggagg ccttcccggg ggcaaggtga attctcttgt 4500

tttgattaga gaaaaattat aagttagtgt tctatcatgc ttgacacgaa caagtttttt 4560

tatctgaaag agaaagaagt gtatgcctaa tgattaacag tcatattcaa aatgatataa 4620

aagagggtgg aaggttactt gctctatgat gatttatggt agtaccgtgt taccaatagc 4680

agaattcaag caatattctt tgaactggta ataacaagtt caagcaatca agcatagttt 4740

agattagatt ctatcaatct cttagcatag tttaaattcg cttccatcaa tctcttttgt 4800

gacgtaagaa gttctgttcc attatactta tgctttttgt gaaattttat ttgcaggtca 4860

agctctctgg atcagttagt agccaatatt tgactgcact gcttatggct actcctttgg 4920

gtcttggaga cgtggaggtt gaaatcattg ataaattgat ttctgtacca tatgtggaga 4980

tgacaataaa gctaatggaa aggtttggag tgtctgtaga gcatagtgct gactggggta 5040

ggttcttgat ccgaggtggt cagaagtaca agtaagtctc tcttttttac tacgtgtcct 5100

gaaaaatcta tgttttagta gactaagata ctataaaata catcagatct cctggaaatg 5160

cgtatgttga gggtgatgct tcaagtgcta gttacttcat aggaggggcc gcagtcactg 5220

gtgggactgt gactgttgag ggttgtggaa caagtagttt acaggtatga tttaaagcct 5280

tatttcacat ccttttactt ctctccttac tgcatctcag cttaattctg aagaactctt 5340

gttgctcaat ctctaggcat aagcttgttc tttcgacctt ctaaatttgc aaaatcattc 5400

tgttaatgta acaaacagaa gatgagatgg tgtttttgat cctgataaaa aaaattagga 5460

cattcatgat tggtttatgt tctatgacaa tttcgatgat ctcttcaatt gtaacttgag 5520

gacccttttc tgctagtaaa gattgtgtat aagctattct agactgttgt atacacctca 5580

tattccggta ttgtttatac ctaataaact tcttatgatt agtatcttac ttgtctcatg 5640

tgttttgcat agggtgatgt aaaatttgct gaagttcttg agaagatggg ttgcaaggta 5700

tcctggacag agaacagtgt cactgtcact ggaccaccca gggatgcatc tggaagaaaa 5760

cacttgcgcg ccgttgatgt caacatgaac aaaatgccag atgttgcaat gactcttgct 5820

gttgttgctc tttatgcaga tggacccacc accattagag acggtatgct taattccttt 5880

ctgtgaacat gacactcttc ttgtgccata ggtagattcc gtagttattg gtcacaatcc 5940

aaatttgcat tggtatttaa agcaagtctt aggtggtgag accgcttgaa acccataata 6000

taaaagaaag gtcaccttgg tctcaaagtt aattcttgtg agaccatctc atgcaagatt 6060

ttgccgggta tctaatcctt gtttgcactg aggaaaagga aaaaaattaa catgacctat 6120

tagtaagcca aaaaaattct aacaagagaa gtagccgaat aggtaataat ttttgtgata 6180

taatgatcag tggctagctg gagagtgaag gaaacagaac ggatgattgc gatttgcaca 6240

gagctcagaa aggttagcca ttttggatat acacattctt gaaggttttc aaccttgtag 6300

aagattgtac tattgataaa aaaaattgat gtttatccgg tgtacttcga aatttctttt 6360

cagctggggg caacagttga ggaaggatca gattactgtg tgatcactcc acctgagaaa 6420

ctaaatgtga cggccattga tacatacgat gatcaccgaa tggccatggc attctctctt 6480

gctgcctgcg ccgatgttcc tgttaccatc aaggacccgg gttgcactcg caagactttc 6540

ccagactact ttgatgtgtt ggaaaggttt gcaaagcatt aagtggtctc ctacatattc 6600

tataaagcat aagctgagat tttttgagag aattaggaga tgaaaaatgc tttctgcttg 6660

agttatcatc acattctttg tattatgatt gtaagattat tatagtatag agtttacaaa 6720

gtactactaa taattgttat gtatccgatt gatcagaaat aagttaattg gaaggctgga 6780

ctttgaaaat gtgaccaaga cactagtgtg accaagtcat tttgttaatg tgagttcaat 6840

gttattgatt caacatgtag agccaaatct caattctatc gtcacttcat atgaccaaaa 6900

atctaaagat gaaaaagtaa aaaagagcat gttggatcaa actctagctg tatctctgaa 6960

attcaatcac gcagttagat caaatgagga ttaaagggag t 7001

<210> 225
<211> 210
<212> DNA
<213> Cauliflower mosaic virus

<400> 225
tccactgacg taagggatga cgcacaatcc cactatcctt cgcaagaccc ttcctctata 60

taaggaagtt catttcattt ggagaggaca cgctgaaatc accagtctct ctctacaaat 120

ctatctctct ctattttctc cataataatg tgtgagtagt tcccagataa gggaattagg 180

gttcttatag ggtttcgctc acgtgttgag 210

<210> 226
<211> 5009
<212> DNA
<213> Beta vulgaris

<400> 226
caaatcttct ggcatcaatg gcggtgttgc cgttcatcaa tttaacatca atggaggtaa 60

gagtcatgtt ttttcaacaa tataaaactt atacttcctc tgttctgttt taaatgaaac 120

gtttgttttc tcacgcaacc caacccactt ttttaataat aaatattttt agttgtgtgc 180

acgtaaaaaa tataaaaaag ttataatttg atagtatctt gtttgagatt gtgattatta 240

agagagtcaa gtctcacaat attcgaaagt ctacgtaatc cacctcaaat tgacgaagaa 300

aacaagcagg aaaggattaa gtaagttcgt ggaaccacta gaattgattt tcaaatatag 360

ctctacctaa tatatggcct acttttaatt ttaaataagg agaaggtaat gtgattagaa 420

acaaattggt cttaaattat tcattaagct taataatgta taaacataat caagtgctat 480

cttcttttca gggccgtctt gaagattttg gggcccggtt ctattatgaa aattgggccc 540

ctaaatttat agaaaataaa gatggaaggt tagagttcta aagatagaaa gttgaaaatc 600

taaatataaa tcattgacaa atttattaag ggtgagaaac aagggtgttt tcttcaaata 660

tgaagcaaaa ttttcaaaat aatacttcct ccgtttctaa ataagtgcaa catttgcata 720

atgtttacta ttcacagttt aaactttaat tagctttggt gatttacatt ttaggaaaaa 780

acatagtcat gtgggatctt gttagattcg tctgaatgtg aattttttta atatcaactt 840

tttataattt ttacttattg ataattgaag atattaatgg ttaaaataat gcattggcaa 900

acgtgaaaac aagaagtgtt gcacttattt agaaacggag gaagtatatt ttgggccttt 960

ataattttgg agaccctgcg ctgttgggct ccttgcacac cttcatctac ccctctgctt 1020

cttttgatac aatttttcag cgacatgatt gtcgattgat gcatatatta ttgtatactc 1080

gatccatatt gtttaagatg aattgtttgt ctttgatggt ctccaatgca tattttgtat 1140

acttaggaat tctaattatg tactattagt agacattgag atgaatacat aattgccata 1200

atgaagtatg attattttag ttatatactt tctccattcc aaatatataa atgtaacact 1260

tgtgtacttt atgcgtacta atgcataaca acgtgcactc tcatgtgttt aattatatac 1320

tttttgagag aagtgttaca ttggggacca tgggactgtg tataatttga ccgcaaaatc 1380

gaagtgtcgc atttgattga aaatggagag agtagtatat agatggaaca cagcagagac 1440

tgctggtcat ctttggccaa caaacccaaa attgatatta atcccttatt caggtcattt 1500

catctttttg acacaaaatg gatgttgtag gcactgcgct atctgctgcc caatctctgt 1560

ttgcagccct gcaaagttct gagctcaaag agatcctctc gatctttggc tacaaatccc 1620

gacttgatga cctccaacgc actgtctcta ccatcaacgc tgtattccgt gatgctgaga 1680

ccaaacagga gctcactcat gaagcacagc attggctcga ggaactcaag gatgctgtct 1740

ttgaagcaga tgatctgttc gacgagtttg tcactcttgc cgagcagaag caacttgtag 1800

aggctggtgg cagtctttcc aaaaagatgc gccaattctt ttctgattcc aacccccttg 1860

gcattgctta taggatgtca cgagggttta agaagatcaa gaagaagttg gatgctatcg 1920

cttacaatca tcaatttagc tttaagattg atcttgagcc tatgaaagag agaaggctag 1980

agactggttc tgtcgtgaac gcaggtgata tcattggaag agaggacgac ttggagaaga 2040

ttgtaggttt gttgcttgat tctaacatcc aacgtgatgt gtctttcctt actattgtgg 2100

gaatgggagg gttgggtaaa actgctcttg cccaactcgt gtacaatgat ccaagggtca 2160

gaactgcttt tccattgaga tgttggaatt gtgtgtctga tcaagatcaa aagcaactag 2220

atgtgaaaga aattttgggt aagattctgg ctacagctac tggtaagaat catgagggtt 2280

caaccatgga tcaggtgcaa acccaactac gagaacaact atgtggcaag agatacttgc 2340

ttgttttgga tgatgtatgg aatgagaatc ctaatcaatt gcgtgatctg gtagaattct 2400

tcatgggagg tcgaagcaga aattggattg tggtaactac gcgttcgcac gagacagcga 2460

gaattataag agatggtcca ttgcacaagc tccaaggttt gtctgaggaa aactcttggc 2520

gtttatttgt caggtggacc ttcggatcag tgcaagcaaa attccctaat gactttatca 2580

tgattgcacg agatatagtt gacaaatgtg ctcgaaaccc tctggctata agagtggtag 2640

gaagtctttt gtgtggtcaa gacaagagta agtggctttc atttcatgag atcgatttag 2700

gcaacattag aaagagccat aatgatatca tgccaatact gaacctaagt taccatcatc 2760

ttgaacctcc aattaagaga tgctttagtt attgtgcagt gtttccaaag gatttcctta 2820

tagggaagca gacgctgata aacctctgga tggcacaagg ttatattgtt ccgttagaca 2880

aagatcaaag catagatgat gctagtgagg aatacatatc aattttgttg cggagatgtt 2940

ttttcgaaaa tgtcggagca gaaaaagatg gtgttattaa gatccatgat ctcatgcatg 3000

atattgctca aaatgtcatg gggaaggagc tttgtacgac taaaaacatt agtggcagct 3060

tggataaaag tgttcgccat ctatctcttg ccagaactag ttttgcaaga tactctttca 3120

atgcaactca tattcgctcc tatttctgtg ctggctactg gtgtcaggat gctgagataa 3180

accagttttc agttgaggca ttagtaccaa actgtttgta cctaagggca atggacctcg 3240

cttggtcgaa gataaaaagt ttaccagact cgattggtgg attgttgcat ttgaggtact 3300

tagatctttc gtataacgaa gatctggaag tacttccaaa ctcaattgct aaactatata 3360

atctacaaac cttacaattg aagggttgca agagattgga agggttacca aaacatttga 3420

gcaggctggt taagcttcaa actttggata tacatggttg caacaatgta acttatatgc 3480

ccaaaggcat gggtaagttg acttgccttc acactctcag taagtttata gtgggtggag 3540

aagggagttg ttcaagttgg aagcaatgtt ttgatgggtt ggaagatcta aaggctctca 3600

ataacctaaa gggtcatctg gaaatccaaa tcaggtggcc caaaaatact acagatgctg 3660

tcaaagaaga tgttacgagg gaaggattat acctgaatca taaggaacat ctcaatcaca 3720

ttgtggttga ttttagatgt gaggagggtg gtggaagaat ggatgatgag gaagcaagaa 3780

gattgatgga agagttgcgg ccacatcctt atcttgaaaa tttggctgtg aaagcatatt 3840

atggtgtgaa aatgcctggt tgggcaaccc ttctcccaaa tcttacagag ctttttcttt 3900

ctgattgtgg ggaactggag aaccttccat gcctgggaaa cttggatcat ctaaaagtcc 3960

tccgactttc gcatttggca aaattggagt acattgaaga agatagctca tcagctaatt 4020

tcaggtgtag gcctggacca gaaagtgcag gactatcatt atacttcccc tcccttgaac 4080

gccttgagtt gaagcgtttg tgtaagttaa aaggatggag gagaggggaa gggttaggag 4140

atgatcacca gccttttaat gaaagcagca gcaatacaca agtccaatta caattatgtc 4200

ttcctcaatt gaagtcattg agaatagaaa gatgcccatt gctgacattt atgccgctgt 4260

gtcccaagac agaaaaactg catttagttg tatttaatga acgactccgg atagtgcatg 4320

ctaagagaga tgagaatttc tatgctccat tacattcatc atcatctgat cctgaaaacc 4380

cgaggaacac tattcccatt cccatgttta gagaggtata cataaacaat gtggcgtggc 4440

taaattcgct gcctatggag gcttttaggt gtctcactca tatgacaata aaaaacgacg 4500

aggtagagag tttgggagaa gttggagagg tgtttcggag ctgctcatct tctttgcgat 4560

ccttgaatat cacaggttgc tccaacttaa gaagtgtttc tggagggctg gagcatctca 4620

ctgctttgga gatgttagaa atatacgaca cccataagct gagtctatca gaagacccag 4680

aaggtgttgt gccatggaaa tcccttcatc actccctcag ctacttgcaa ctgatgaatc 4740

tcccacagct ggtcaacctg cctgattcga tgcagttctt ggctgccctc cgaactcttt 4800

caatagtgca ttgcactaaa ctgcaatcag tgccagattg gatgcccaga ctcacttctc 4860

tcaggaagct tatggtttca ttctgttccg cacatctgga gagaagatgc caaaatccaa 4920

ctggggtgga ctggcctaac attcaacaca tcccctccat tgatgtcacc tctagccttc 4980

ctaagttttt agtgttgccg tatgaatag 5009

<210> 227
<211> 1163
<212> PRT
<213> Beta vulgaris

<400> 227

Met Asp Val Val Gly Thr Ala Leu Ser Ala Ala Gln Ser Leu Phe Ala
1               5                   10                  15

Ala Leu Gln Ser Ser Glu Leu Lys Glu Ile Leu Ser Ile Phe Gly Tyr
            20                  25                  30

Lys Ser Arg Leu Asp Asp Leu Gln Arg Thr Val Ser Thr Ile Asn Ala
        35                  40                  45

Val Phe Arg Asp Ala Glu Thr Lys Gln Glu Leu Thr His Glu Ala Gln
    50                  55                  60

His Trp Leu Glu Glu Leu Lys Asp Ala Val Phe Glu Ala Asp Asp Leu
65                  70                  75                  80

Phe Asp Glu Phe Val Thr Leu Ala Glu Gln Lys Gln Leu Val Glu Ala
                85                  90                  95

Gly Gly Ser Leu Ser Lys Lys Met Arg Gln Phe Phe Ser Asp Ser Asn
            100                 105                 110

Pro Leu Gly Ile Ala Tyr Arg Met Ser Arg Gly Val Lys Lys Ile Lys
        115                 120                 125

Lys Lys Leu Asp Ala Ile Ala Tyr Asn His Gln Phe Ser Phe Lys Ile
    130                 135                 140

Asp Leu Glu Pro Met Lys Glu Arg Arg Leu Glu Thr Gly Ser Val Val
145                 150                 155                 160

Asn Ala Gly Asp Ile Ile Gly Arg Glu Asp Asp Leu Glu Lys Ile Val
                165                 170                 175

Gly Leu Leu Leu Asp Ser Asn Ile Gln Arg Asp Val Ser Phe Leu Thr
            180                 185                 190

Ile Val Gly Met Gly Gly Leu Gly Lys Thr Ala Leu Ala Gln Leu Val
        195                 200                 205

Tyr Asn Asp Pro Arg Val Arg Thr Ala Phe Pro Leu Arg Cys Trp Asn
    210                 215                 220

Cys Val Ser Asp Gln Asp Gln Lys Gln Leu Asp Val Lys Glu Ile Leu
225                 230                 235                 240

Gly Lys Ile Leu Ala Thr Ala Thr Gly Lys Asn His Glu Gly Ser Thr
                245                 250                 255

Met Asp Gln Val Gln Thr Gln Leu Arg Glu Gln Leu Cys Gly Lys Arg
            260                 265                 270

Tyr Leu Leu Val Leu Asp Asp Val Trp Asn Glu Asn Pro Asn Gln Leu
        275                 280                 285

Arg Asp Leu Val Glu Phe Phe Met Gly Gly Arg Ser Arg Asn Trp Ile
    290                 295                 300

Val Val Thr Thr Arg Ser His Glu Thr Ala Arg Ile Ile Arg Asp Gly
305                 310                 315                 320

Pro Leu His Lys Leu Gln Gly Leu Ser Glu Glu Asn Ser Trp Arg Leu
                325                 330                 335

Phe Val Arg Trp Thr Phe Gly Ser Val Gln Ala Lys Phe Pro Asn Asp
            340                 345                 350

Phe Ile Met Ile Ala Arg Asp Ile Val Asp Lys Cys Ala Arg Asn Pro
        355                 360                 365

Leu Ala Ile Arg Val Val Gly Ser Leu Leu Cys Gly Gln Asp Lys Ser
    370                 375                 380

Lys Trp Leu Ser Phe His Glu Ile Asp Leu Gly Asn Ile Arg Lys Ser
385                 390                 395                 400

His Asn Asp Ile Met Pro Ile Leu Asn Leu Ser Tyr His His Leu Glu
                405                 410                 415

Pro Pro Ile Lys Arg Cys Phe Ser Tyr Cys Ala Val Phe Pro Lys Asp

            420                 425                 430

Phe Leu Ile Gly Lys Gln Thr Leu Ile Asn Leu Trp Met Ala Gln Gly
        435                 440                 445

Tyr Ile Val Pro Leu Asp Lys Asp Gln Ser Ile Asp Asp Ala Ser Glu
    450                 455                 460

Glu Tyr Ile Ser Ile Leu Leu Arg Arg Cys Phe Phe Glu Asn Val Gly
465                 470                 475                 480

Ala Glu Lys Asp Gly Val Ile Lys Ile His Asp Leu Met His Asp Ile
                485                 490                 495

Ala Gln Asn Val Met Gly Lys Glu Leu Cys Thr Thr Lys Asn Ile Ser
            500                 505                 510

Gly Ser Leu Asp Lys Ser Val Arg His Leu Ser Leu Ala Arg Thr Ser
        515                 520                 525

Phe Ala Arg Tyr Ser Phe Asn Ala Thr His Ile Arg Ser Tyr Phe Cys
    530                 535                 540

Ala Gly Tyr Trp Cys Gln Asp Ala Glu Ile Asn Gln Phe Ser Val Glu
545                 550                 555                 560

Ala Leu Val Pro Asn Cys Leu Tyr Leu Arg Ala Met Asp Leu Ala Trp
                565                 570                 575

Ser Lys Ile Lys Ser Leu Pro Asp Ser Ile Gly Gly Leu Leu His Leu
            580                 585                 590

Arg Tyr Leu Asp Leu Ser Tyr Asn Glu Asp Leu Glu Val Leu Pro Asn
        595                 600                 605

Ser Ile Ala Lys Leu Tyr Asn Leu Gln Thr Leu Gln Leu Lys Gly Cys
    610                 615                 620

Lys Arg Leu Glu Gly Leu Pro Lys His Leu Ser Arg Leu Val Lys Leu
625                 630                 635                 640

Gln Thr Leu Asp Ile His Gly Cys Asn Asn Val Thr Tyr Met Pro Lys
                645                 650                 655

Gly Met Gly Lys Leu Thr Cys Leu His Thr Leu Ser Lys Phe Ile Val
            660                 665                 670

Gly Gly Glu Gly Ser Cys Ser Ser Trp Lys Gln Cys Phe Asp Gly Leu
        675                 680                 685

Glu Asp Leu Lys Ala Leu Asn Asn Leu Lys Gly His Leu Glu Ile Gln
    690                 695                 700

Ile Arg Trp Pro Lys Asn Thr Thr Asp Ala Val Lys Glu Asp Val Thr
705                 710                 715                 720

Arg Glu Gly Leu Tyr Leu Asn His Lys Glu His Leu Asn His Ile Val
                725                 730                 735

Val Asp Phe Arg Cys Glu Glu Gly Gly Gly Arg Met Asp Asp Glu Glu
            740                 745                 750

Ala Arg Arg Leu Met Glu Glu Leu Arg Pro His Pro Tyr Leu Glu Asn
        755                 760                 765

Leu Ala Val Lys Ala Tyr Tyr Gly Val Lys Met Pro Gly Trp Ala Thr
    770                 775                 780

Leu Leu Pro Asn Leu Thr Glu Leu Phe Leu Ser Asp Cys Gly Glu Leu
785                 790                 795                 800

Glu Asn Leu Pro Cys Leu Gly Asn Leu Asp His Leu Lys Val Leu Arg
                805                 810                 815

Leu Ser His Leu Ala Lys Leu Glu Tyr Ile Glu Glu Asp Ser Ser Ser
            820                 825                 830

Ala Asn Phe Arg Cys Arg Pro Gly Pro Glu Ser Ala Gly Leu Ser Leu
        835                 840                 845

Tyr Phe Pro Ser Leu Glu Arg Leu Glu Leu Lys Arg Leu Cys Lys Leu
    850                 855                 860

Lys Gly Trp Arg Arg Gly Glu Gly Leu Gly Asp Asp His Gln Pro Phe
865                 870                 875                 880

Asn Glu Ser Ser Ser Asn Thr Gln Val Gln Leu Gln Leu Cys Leu Pro
                885                 890                 895

Gln Leu Lys Ser Leu Arg Ile Glu Arg Cys Pro Leu Leu Thr Phe Met
            900                 905                 910

Pro Leu Cys Pro Lys Thr Glu Lys Leu His Leu Val Val Phe Asn Glu
        915                 920                 925

Arg Leu Arg Ile Val His Ala Lys Arg Asp Glu Asn Phe Tyr Ala Pro
    930                 935                 940

Leu His Ser Ser Ser Ser Asp Pro Glu Asn Pro Arg Asn Thr Ile Pro
945                 950                 955                 960

Ile Pro Met Phe Arg Glu Val Tyr Ile Asn Asn Val Ala Trp Leu Asn
                965                 970                 975

Ser Leu Pro Met Glu Ala Phe Arg Cys Leu Thr His Met Thr Ile Lys
            980                 985                 990

Asn Asp Glu Val Glu Ser Leu Gly  Glu Val Gly Glu Val  Phe Arg Ser
        995                 1000                 1005

Cys Ser  Ser Ser Leu Arg Ser  Leu Asn Ile Thr Gly  Cys Ser Asn
    1010                 1015                 1020

Leu Arg  Ser Val Ser Gly Gly  Leu Glu His Leu Thr  Ala Leu Glu
    1025                 1030                 1035

Met Leu  Glu Ile Tyr Asp Thr  His Lys Leu Ser Leu  Ser Glu Asp
    1040                 1045                 1050

Pro Glu  Gly Val Val Pro Trp  Lys Ser Leu His His  Ser Leu Ser
    1055                 1060                 1065

Tyr Leu  Gln Leu Met Asn Leu  Pro Gln Leu Val Asn  Leu Pro Asp
    1070                 1075                 1080

Ser Met  Gln Phe Leu Ala Ala  Leu Arg Thr Leu Ser  Ile Val His
    1085                 1090                 1095

Cys Thr  Lys Leu Gln Ser Val  Pro Asp Trp Met Pro  Arg Leu Thr
    1100                 1105                 1110

Ser Leu  Arg Lys Leu Met Val  Ser Phe Cys Ser Ala  His Leu Glu
    1115                 1120                 1125

Arg Arg  Cys Gln Asn Pro Thr  Gly Val Asp Trp Pro  Asn Ile Gln
    1130                 1135                 1140

His Ile  Pro Ser Ile Asp Val  Thr Ser Ser Leu Pro  Lys Phe Leu
    1145                 1150                 1155

Val Leu  Pro Tyr Glu
    1160

<210>  228
<211>  1177
<212>  PRT
<213>  Beta vulgaris

<400>  228

Met Glu Arg Val Val Tyr Arg Trp Asn Thr Ala Glu Thr Ala Gly His
1               5                   10                  15

Leu Trp Pro Thr Asn Pro Lys Leu Ile Leu Ile Pro Tyr Ser Ala Leu
            20                  25                  30

Gln Ser Ser Glu Leu Lys Glu Ile Leu Ser Ile Phe Gly Tyr Lys Ser
        35                  40                  45

Arg Leu Asp Asp Leu Gln Arg Thr Val Ser Thr Ile Asn Ala Val Phe
    50                  55                  60

Arg Asp Ala Glu Thr Lys Gln Glu Leu Thr His Glu Ala Gln His Trp
65                  70                  75                  80

Leu Glu Glu Leu Lys Asp Ala Val Phe Glu Ala Asp Asp Leu Phe Asp
                85                  90                  95

Glu Phe Val Thr Leu Ala Glu Gln Lys Gln Leu Val Glu Ala Gly Gly
            100                 105                 110

Ser Leu Ser Lys Lys Met Arg Gln Phe Phe Ser Asp Ser Asn Pro Leu
        115                 120                 125

Gly Ile Ala Tyr Arg Met Ser Arg Gly Val Lys Lys Ile Lys Lys Lys
    130                 135                 140

Leu Asp Ala Ile Ala Tyr Asn His Gln Phe Ser Phe Lys Ile Asp Leu
145                 150                 155                 160

Glu Pro Met Lys Glu Arg Arg Leu Glu Thr Gly Ser Val Val Asn Ala
                165                 170                 175

Gly Asp Ile Ile Gly Arg Glu Asp Asp Leu Glu Lys Ile Val Gly Leu
            180                 185                 190

Leu Leu Asp Ser Asn Ile Gln Arg Asp Val Ser Phe Leu Thr Ile Val
        195                 200                 205

Gly Met Gly Gly Leu Gly Lys Thr Ala Leu Ala Gln Leu Val Tyr Asn
    210                 215                 220

Asp Pro Arg Val Arg Thr Ala Phe Pro Leu Arg Cys Trp Asn Cys Val
225                 230                 235                 240

Ser Asp Gln Asp Gln Lys Gln Leu Asp Val Lys Glu Ile Leu Gly Lys
                245                 250                 255

Ile Leu Ala Thr Ala Thr Gly Lys Asn His Glu Gly Ser Thr Met Asp
            260                 265                 270

Gln Val Gln Thr Gln Leu Arg Glu Gln Leu Cys Gly Lys Arg Tyr Leu
        275                 280                 285

Leu Val Leu Asp Asp Val Trp Asn Glu Asn Pro Asn Gln Leu Arg Asp
    290                 295                 300

Leu Val Glu Phe Phe Met Gly Gly Arg Ser Arg Asn Trp Ile Val Val
305                 310                 315                 320

Thr Thr Arg Ser His Glu Thr Ala Arg Ile Ile Arg Asp Gly Pro Leu
                325                 330                 335

His Lys Leu Gln Gly Leu Ser Glu Glu Asn Ser Trp Arg Leu Phe Val
            340                 345                 350

Arg Trp Thr Phe Gly Ser Val Gln Ala Lys Phe Pro Asn Asp Phe Ile
        355                 360                 365

Met Ile Ala Arg Asp Ile Val Asp Lys Cys Ala Arg Asn Pro Leu Ala
    370                 375                 380

Ile Arg Val Val Gly Ser Leu Leu Cys Gly Gln Asp Lys Ser Lys Trp
385                 390                 395                 400

Leu Ser Phe His Glu Ile Asp Leu Gly Asn Ile Arg Lys Ser His Asn
                405                 410                 415

Asp Ile Met Pro Ile Leu Asn Leu Ser Tyr His His Leu Glu Pro Pro
            420                 425                 430

Ile Lys Arg Cys Phe Ser Tyr Cys Ala Val Phe Pro Lys Asp Phe Leu
        435                 440                 445

Ile Gly Lys Gln Thr Leu Ile Asn Leu Trp Met Ala Gln Gly Tyr Ile
    450                 455                 460

Val Pro Leu Asp Lys Asp Gln Ser Ile Asp Asp Ala Ser Glu Glu Tyr

465                 470                 475                 480

Ile Ser Ile Leu Leu Arg Arg Cys Phe Phe Glu Asn Val Gly Ala Glu
                485                 490                 495

Lys Asp Gly Val Ile Lys Ile His Asp Leu Met His Asp Ile Ala Gln
            500                 505                 510

Asn Val Met Gly Lys Glu Leu Cys Thr Thr Lys Asn Ile Ser Gly Ser
        515                 520                 525

Leu Asp Lys Ser Val Arg His Leu Ser Leu Ala Arg Thr Ser Phe Ala
    530                 535                 540

Arg Tyr Ser Phe Asn Ala Thr His Ile Arg Ser Tyr Phe Cys Ala Gly
545                 550                 555                 560

Tyr Trp Cys Gln Asp Ala Glu Ile Asn Gln Phe Ser Val Glu Ala Leu
                565                 570                 575

Val Pro Asn Cys Leu Tyr Leu Arg Ala Met Asp Leu Ala Trp Ser Lys
            580                 585                 590

Ile Lys Ser Leu Pro Asp Ser Ile Gly Gly Leu Leu His Leu Arg Tyr
        595                 600                 605

Leu Asp Leu Ser Tyr Asn Glu Asp Leu Glu Val Leu Pro Asn Ser Ile
    610                 615                 620

Ala Lys Leu Tyr Asn Leu Gln Thr Leu Gln Leu Lys Gly Cys Lys Arg
625                 630                 635                 640

Leu Glu Gly Leu Pro Lys His Leu Ser Arg Leu Val Lys Leu Gln Thr
                645                 650                 655

Leu Asp Ile His Gly Cys Asn Asn Val Thr Tyr Met Pro Lys Gly Met
            660                 665                 670

Gly Lys Leu Thr Cys Leu His Thr Leu Ser Lys Phe Ile Val Gly Gly
        675                 680                 685

Glu Gly Ser Cys Ser Ser Trp Lys Gln Cys Phe Asp Gly Leu Glu Asp
    690                 695                 700

Leu Lys Ala Leu Asn Asn Leu Lys Gly His Leu Glu Ile Gln Ile Arg
705                 710                 715                 720

Trp Pro Lys Asn Thr Thr Asp Ala Val Lys Glu Asp Val Thr Arg Glu
                725                 730                 735

Gly Leu Tyr Leu Asn His Lys Glu His Leu Asn His Ile Val Val Asp
            740                 745                 750

Phe Arg Cys Glu Glu Gly Gly Gly Arg Met Asp Asp Glu Glu Ala Arg
        755                 760                 765

Arg Leu Met Glu Glu Leu Arg Pro His Pro Tyr Leu Glu Asn Leu Ala
    770                 775                 780

Val Lys Ala Tyr Tyr Gly Val Lys Met Pro Gly Trp Ala Thr Leu Leu
785                 790                 795                 800

Pro Asn Leu Thr Glu Leu Phe Leu Ser Asp Cys Gly Glu Leu Glu Asn
                805                 810                 815

Leu Pro Cys Leu Gly Asn Leu Asp His Leu Lys Val Leu Arg Leu Ser
            820                 825                 830

His Leu Ala Lys Leu Glu Tyr Ile Glu Glu Asp Ser Ser Ser Ala Asn
        835                 840                 845

Phe Arg Cys Arg Pro Gly Pro Glu Ser Ala Gly Leu Ser Leu Tyr Phe
    850                 855                 860

Pro Ser Leu Glu Arg Leu Glu Leu Lys Arg Leu Cys Lys Leu Lys Gly
865                 870                 875                 880

Trp Arg Arg Gly Glu Gly Leu Gly Asp Asp His Gln Pro Phe Asn Glu
                885                 890                 895

Ser Ser Ser Asn Thr Gln Val Gln Leu Gln Leu Cys Leu Pro Gln Leu
            900                 905                 910

Lys Ser Leu Arg Ile Glu Arg Cys Pro Leu Leu Thr Phe Met Pro Leu
        915                 920                 925

Cys Pro Lys Thr Glu Lys Leu His Leu Val Val Phe Asn Glu Arg Leu
    930                 935                 940

Arg Ile Val His Ala Lys Arg Asp Glu Asn Phe Tyr Ala Pro Leu His
945                 950                 955                 960

Ser Ser Ser Ser Asp Pro Glu Asn Pro Arg Asn Thr Ile Pro Ile Pro
                965                 970                 975

Met Phe Arg Glu Val Tyr Ile Asn Asn Val Ala Trp Leu Asn Ser Leu
            980                 985                 990

Pro Met Glu Ala Phe Arg Cys Leu Thr His Met Thr Ile Lys Asn Asp
        995                 1000                1005

Glu Val Glu Ser Leu Gly Glu Val Gly Glu Val Phe Arg Ser Cys
    1010                1015                1020

Ser Ser Ser Leu Arg Ser Leu Asn Ile Thr Gly Cys Ser Asn Leu
    1025                1030                1035

Arg Ser Val Ser Gly Gly Leu Glu His Leu Thr Ala Leu Glu Met
    1040                1045                1050

Leu Glu Ile Tyr Asp Thr His Lys Leu Ser Leu Ser Glu Asp Pro
    1055                1060                1065

Glu Gly Val Val Pro Trp Lys Ser Leu His His Ser Leu Ser Tyr
    1070                1075                1080

Leu Gln Leu Met Asn Leu Pro Gln Leu Val Asn Leu Pro Asp Ser
    1085                1090                1095

Met Gln Phe Leu Ala Ala Leu Arg Thr Leu Ser Ile Val His Cys
    1100                1105                1110

Thr Lys Leu Gln Ser Val Pro Asp Trp Met Pro Arg Leu Thr Ser
    1115                1120                1125

Leu Arg Lys Leu Met Val Ser Phe Cys Ser Ala His Leu Glu Arg
    1130                1135                1140

Arg Cys Gln Asn Pro Thr Gly Val Asp Trp Pro Asn Ile Gln His
    1145                1150                1155

Ile Pro Ser Ile Asp Val Thr Ser Ser Leu Pro Lys Phe Leu Val
    1160                1165                1170

Leu Pro Tyr Glu
    1175

## Description

### FIELD OF THE INVENTION

The present invention relates to a nucleic acid molecule which encodes a polypeptide, which nucleic acid molecule is able to confer a resistance to Cercospora - in particular, to the fungus *Cercospora beticola* in a plant, and, in particular, in a plant of the species *Beta vulgaris* in which the polypeptide is expressed - as well as to the polypeptide encoded by the nucleic acid molecule according to the invention. In particular, the nucleic acid molecule according to the invention is characterized in that the resistance effect to Cercospora that is conferred by the polypeptide is dominant. Furthermore, the invention relates to a Cercospora-resistant plant, plant cell, plant organ, plant tissue, plant part, or a seed or descendant of a plant, which comprises the nucleic acid molecule or portions thereof as an endogenous gene, as an edited gene, or as a transgene. Furthermore, the present invention also encompasses methods for increasing the resistance to Cercospora in a plant of the species *Beta vulgaris,* as well as methods for producing or identifying and possibly selecting a Cercospora-resistant plant. The present invention also encompasses methods for monitoring an infestation by the pathogen *Cercospora beticola,* as well as oligonucleotide probes and primers for hybridization with the nucleic acid molecule according to the invention.

### BACKGROUND OF THE INVENTION

Cercospora leaf spot is one of the most important, globally prevalent leaf diseases of different plants including the species *Beta vulgaris* and *Spinacia oleracea.* It is caused by the fungus *Cercospora beticola.* Plants infested by this disease typically form small, relatively round leaf spots (2-3 mm) that are light gray in the middle and are surrounded by a red-brown border. In a severe infestation, the leaf spots overlap, so that entire portions of the leaf blade dry out. Small black dots (pseudostromata) are visible within the fully formed spots, and a gray, felt-like covering (conidia bearers with conidia) forms under damp conditions - predominantly, on the leaf underside. Severely infested leaves first turn yellow, then turn brown and die. New leaf growth occurs in parallel, wherein the leaves become diseased again and die, however. At first, damage symptoms only on individual plants are visible; however, with spread of the disease, formation of persistent infestation nests often occurs. The further propagation over the entire field takes place via rain and wind. In case of chard plants the damaged leaves are not accepted by the consumers and even a low disease pressure can lead to severe crop shortfall.

The pathogen *Cercospora beticola* was first described in the second half of the 19th century, in Italy. Up to 40% crop losses may occur due to a severe infestation, which may be triggered by humid weather, early row closure, a high infection potential from previous years, or strong irrigation. These losses result from a reduced beet crop and reduced sugar content; see Holtschulte ((2000) "Cercospora beticola - worldwide distribution and incidence," pp. 5-16, in "Cercospora beticola Sacc. Biology, Agronomic Influence and Control Measures in Sugar Beet," vol. 2 (M.J.C. Asher, B. Holtschulte, M.R. Molard, F. Rosso, G. Steinrücken, R. Beckers, eds.). International Institute for Beet Research, Brussels, Belgium, 215 pp.). In order to fight back against the disease, intercropping or fungicides are often used. A chemical control of *Cercospora beticola* via fungicides incurs costs to the farmer and pollutes the environment. Moreover, the treatment of the edible chard leaves with chemicals reduces consumer acceptance. Repeated applications of fungicides additionally increase the selection pressure on fungicide-tolerant *Cercospora beticola* strains. This is contrary to a sustainable agricultural practice. It is worth mentioning that during the last few years stems of Cercospora beticola occurred which showed resistance against one or more fungicides; see for Trkulja, Nenad R., et al. "Molecular and experimental evidence of multi-resistance of Cercospora beticola field populations to MBC, DMI and QoI fungicides." European Journal of Plant Pathology 149.4 (2017): 895-910. The problem became such severe that the German Federal Office of Consumer Protection and Food Safety (BVL) approved the exemptional admission of copper-based fungicides for combating Cercospora. However, copper-based fungicides are generally regarded (depending on the dosage) as harmful for humans and environment. Copper is a heavy metal which may accumulate in the soil.

Indirect combat is done via the selection of cultivars with healthy leaves and cultivation of the beets with at least a 3-year crop rotation. Markedly better control of the infestation may be achieved with a combination of tolerant or resistant cultivars. Less susceptible Cercospora-tolerant beet cultivars have been offered on the market since 2000 (Steinrücken 1997, "Die Züchtung von Cercospora-resistenten Zuckerrüben." ["The breeding of Cercospora-resistant sugar beets."], Vorträge für Pflanzenzüchtung [Lectures on Plant Breeding], Volume 37, Lecture symposium, March 4-5, 1997, Kiel). These cultivars are furnished with a quantitative resistance to *Cercospora beticola.* The resistance of these cultivars is based upon several genes and is quantitatively passed down, wherein the exact number of the genes that are responsible for the resistance is not known; see Weiland and Koch (2004), Sugarbeet leaf spot disease (Cercospora beticola Sacc.), The Plant Journal, 5(3), 157-166. The complex quantitative heredity was confirmed via several Quantitative Trait Loci (QTL) analyses. This method allows the mapping of polygenic inherited resistances and is a reliable technique for identifying the number and position of genetic resistance factors on the genetic linkage map of a host plant. In this way, multiple causative QTL's could be determined on each chromosome of the sugar beet.

The mappings were performed with different Cercospora-resistance donors, wherein the observed QTL effects were, for the most part, small. The maximum declared phenotypical values were at 5%.

In continuative studies, lists of differentially expressed genes have been described. In a study by Weltmeier et al., ((2011) Transcript profiles in sugar beet genotypes uncover timing and strength of defense reactions to Cercospora beticola infection, Molecular plant-microbe interactions, 24(7), 758-772), a genome-side expression profile for various genotypes of sugar beet (i.e., *Cercospora-*resistant, -tolerant, -susceptible, etc.) was created with the aid of a microarray-based technology during the pathogen infection in order to analyze transcriptional changes in the expression profile in connection with leaf spot. Via these analyses, the authors were in a position to create a pathogen-induced transcription profile in various tested genotypes of sugar beet and to determine potential candidate genes. However, these genes have not yet been characterized in detail. The genetic and functional background of Cercospora resistance and the identity of the resistance genes have until now been entirely unclear.

However, with the quantitative heredity of QTL, not only is the desired resistance to *Cercospora beticola* introduced into the plant, but, rather, often unwanted features as well, such as, for example, reduced yield, due to the inheritance of additional genes that are linked with the positive feature of Cercospora resistance. This phenomenon is also known by the term, "linkage drag." Furthermore, the enormous breeding cost that is required in order to select for multiple resistance loci without thereby reducing the yield may have negative effects on the vitality of the plants; see Weiland and Koch, 2004.

Breeding companies have offered Cercospora-tolerant cultivars on the market for more than a decade. The resistance of these cultivars is inherited via multiple resistance genes with small effect. However, a disadvantage of these cultivars consists in the cultivar development being very laborious and complicated due to the complicated heredity, and in such cultivars having a markedly poorer yield performance relative to normal cultivars, in the absence of an infestation. Among other things, this may be linked to the epigenetic interaction of some resistance genes with genes that are responsible for sugar production, which leads to reduced fitness of the plants, in the absence of the pathogen. Furthermore, Cercospora shows the tendency to overcome the tolerance of long-established cultivars. Moreover, the so far available resistance scores of non-adapted, wild genetic resources is usually not reliable and not comparable among each other as the underlying studies took place at different environment conditions, under different infestation pressure and with different pathogenic stems of Cercospora. In this regard it should me mentioned that environmental parameters like moisture, temperature, wind etc. (which tend to be unstable) have significant influence on the progress of the Cercospora disease after infection. It is common that a specific genetic resource shows high level of tolerance / resistance in one study and tends to be completely susceptible in another study. Due to the above given factors it was so far not possible to identify a dominant resistance gene having a major effect towards Cercospora although there is a strong demand for such a gene which could be easily transferred into already existing cultivars and varieties to establish resistance towards Cercospora.

The use of new breeding techniques based upon gene editing, e.g., by means of TALE nucleases or CRISPR systems, and of transgenic approaches, is not applicable on the so far available genetic material due to the complicated heredity and the multitude of the genes which are involved in the resistance development, the majority of which have not yet been identified and characterized.

For sustainable breeding against Cercospora leaf spot that is to counteract the danger of Cercospora variants that overcome resistance, it is necessary to continuously identify new resistance genes and integrate these into the gene pools of cultivated plants such as sugar beets. In particular, the aim consisted in the provision of suitable resistance genes that, after expression in the plant, on their own already produce a very large, dominant resistance effect against *Cercospora beticola.* According to the invention, this aim is achieved via the embodiments characterized in the claims and in the specification.

### ABSTRACT OF THE INVENTION

The present invention relates to a nucleic acid molecule that is able to confer a resistance to Cercospora - in particular, to the fungus *Cercospora beticola* - in a plant, and, in particular, in *Beta vulgaris* subsp. *vulgaris.* The polypeptide which is encoded by the nucleic acid molecule is thereby produced in the plant. The nucleic acid molecule, after whose expression the polypeptide is produced, on its own, already produces in the plant a very large, dominant resistance effect against *Cercospora beticola.*

Furthermore, the invention relates to a Cercospora-resistant plant, plant cell, plant organ, plant tissue, plant part, a seed, seed stock, or descendant of a plant, which endogenously or transgenically comprises the nucleic acid molecule or portions thereof. According to a specific optional embodiment, those plants and their components that have been obtained via an essentially biological process are excluded.

Methods for increasing the resistance to Cercospora in a plant of the species *Beta vulgaris,* as well as methods for producing or identifying and possibly selecting a Cercospora-resistant plant, are likewise encompassed by the present invention. The present invention also encompasses methods for monitoring an infestation of the pathogen *Cercospora beticola,* as well as oligonucleotides as probes and primers for hybridization with the nucleic acid molecule according to the invention.

The present invention therefore relates to the embodiments that are listed in the following points and illustrated in the examples and figures.
[1] Nucleic acid molecule encoding a polypeptide that is able to confer resistance to Cercospora in a plant in which the polypeptide is expressed, characterized in that the nucleic acid molecule comprises a nucleotide sequence which is selected from
   (a) a nucleotide sequence encoding a polypeptide having an amino acid sequence according to SEQ ID No. 3;
   (b) a nucleotide sequence that comprises the DNA sequence according to SEQ ID No. 2;
   (c) a nucleotide sequence that comprises a DNA sequence selected from the group consisting of SEQ ID No. 1 or SEQ ID No. 53;
   (d) a nucleotide sequence that hybridizes to a nucleotide sequence which is complementary to the nucleotide sequence according to (a), (b), or (c), under stringent conditions;
   (e) a nucleotide sequence encoding a polypeptide which, via substitution, deletion, and/or addition of one or more amino acids of the amino acid sequence, differs from a polypeptide encoded by the nucleotide sequence according to (a), (b), or (c);
   (f) a nucleotide sequence encoding a polypeptide which has an amino acid sequence that is at least 70% identical to an amino acid sequence according to SEQ ID No. 3;
   (g) a nucleotide sequence that is at least 70% identical to a DNA sequence according to SEQ ID No. 1 or SEQ ID No. 2;
   wherein the resistance to Cercospora is preferably a resistance to *Cercospora beticola,* or wherein the plant is preferably a plant of the subspecies *Beta vulgaris* subsp. *vulgaris,* and is, particularly preferably, sugar beet.
[2] Nucleic acid molecule according to [1], characterized in that the resistance effect to Cercospora that is conferred by the polypeptide is dominant in the plant - preferably, wherein the polypeptide confers a resistance effect of at least one rating score, and, preferably, of more than one rating score, particularly preferably, of at least two rating scores, particularly preferably, of at least three rating scores, and, especially preferably, of at least four rating scores.
[3] Nucleic acid molecule according to [1] or [2], characterized in that the nucleic acid molecule originates from *Beta vulgaris* subsp. *maritima.*
[4] Polypeptide encoded by the nucleic acid molecule according to one of [1] through [3].
[5] Vector or expression cassette comprising the nucleic acid molecule according to one of [1] through [3], wherein the nucleic acid molecule is preferably heterologous to the vector or to the expression cassette.
[6] Cell which comprises the nucleic acid molecule according to one of [1] through [3], or the vector or the expression cassette according to [5], wherein the nucleic acid molecule or the expression cassette are preferably present as an endogene or transgene.
[7] Cercospora-resistant plant or a portion thereof, characterized in that the plant or its portion contains the nucleic acid molecule or the nucleotide sequence according to one of [1] through [3], or the vector or the expression cassette according to [5], wherein the plant which endogenously contains the nucleic acid molecule is a plant of the species *Beta vulgaris* - but not *Beta vulgaris* subsp. *maritima* - or of *Beta vulgaris* subsp. *vulgaris.* The nucleic acid molecule or the nucleotide sequence may be contained endogenously or transgenically. Seeds of a plant according to this paragraph may be obtained from a deposition with the NCIMB, Aberdeen, UK under access number NCIMB 43646. The plant according to this paragraph is obtainable from the deposited seeds.
[8] Plant according to [7], characterized in that the plant is a hybrid plant.
[9] Plant according to [7] or [8], characterized in that the nucleic acid molecule is present heterozygously or homozygously in the genome of the plant.
[10] A seed or descendant of the plant according to one of [7] through [9], wherein the seed or the descendant comprises the nucleic acid molecule or the nucleotide sequence according to one of [1] through [3], or the vector or the expression cassette according to [5]. The nucleic acid molecule may be present transgenically or non-transgenically or endogenously. Seeds according to this paragraph are obtainable from a deposition with the NCIMB, Aberdeen, UK under access number NCIMB 43646.
[11] Method for increasing the resistance to Cercospora in a plant, including the following steps:
   (i) integration of the nucleic acid molecule according to one of [1] through [3], or of the vector or of the expression cassette according to [5], by means of homology-directed repair or homologous recombination - preferably, supported by a site-directed nuclease - into the genome of at least one cell of a plant, and optional regeneration of a plant from the at least one plant cell; or
   (ii) increase in the expression of the nucleic acid molecule according to one of [1] through [3] in at least one cell of the plant - preferably, via modification of the native promoter, e.g., comprising a DNA sequence according to SEQ ID No. 7, or via linking of the nucleic acid molecule according to one of [1] through [3] with a heterologous promoter that has a higher level of activity in comparison to the native promoter, e.g., comprising a DNA sequence according to SEQ ID No. 7 - in particular, after Cercospora infection - and optional regeneration of a plant from the at least one plant cell; or
   (iii) increase in the activity and/or stability of the polypeptide according to [4] via modification of the nucleotide sequence of the nucleic acid molecule according to one of [1] through [3] in at least one cell of the plant, and optional regeneration of a plant from the at least one plant cell; or
   (iv) transformation of a plant cell with the nucleic acid molecule according to one of [1] through [3], or the vector or the expression cassette according to [5], and optional regeneration of a (transgenic) plant from the transformed plant cell;
   wherein the resistance to Cercospora is preferably a resistance to *Cercospora beticola,* or the plant is preferably a plant of the species *Beta vulgaris* - preferably, *Beta vulgaris* subsp. *vulgaris* - and, in particular, is sugar beet.
[12] Method for producing a Cercospora-resistant plant according to one of [7] through [9], including the following steps:
   (a) transformation of a plant cell with the nucleic acid molecule according to one of [1] through [3], or the vector or the expression cassette according to [5]; and
   (b) regeneration of the transgenic plant from the transformed plant cell; or
      (i) introduction of a site-directed nuclease and a repair matrix into a cell of a plant of the species *Beta vulgaris,* wherein the site-directed nuclease is able to generate at least one double-strand break of the DNA in the genome of the cell - preferably, upstream and/or downstream of a target region - and the repair matrix comprises the nucleic acid molecule according to one of [1] through [3];
      (ii) cultivation of the cell from (i) under conditions that allow a homology-directed repair or a homologous recombination, wherein the nucleic acid molecule is incorporated from the repair matrix into the genome of the plant; and
      (iii) regeneration of a plant from the cell modified in (ii).
[13] Method according to [12], characterized in that the target region comprises an allelic variant of the nucleic acid molecule according to one of [1] through [3], wherein the allelic variant encodes a polypeptide not conferring resistance or a slight resistance to Cercospora.
[14] Method according to [12] or [13], characterized in that the at least one double-strand break occurs at a position that is at most 10,000 base pairs upstream and/or downstream of the target region, or that is at most 10,000 base pairs distant from the allelic variant as defined in [13].
[15] Method according to [12] or [13], characterized in that the allelic variant of the nucleic acid molecule comprises a nucleotide sequence which is selected from
   (a) a nucleotide sequence that encodes a polypeptide having an amino acid sequence according to SEQ ID No. 6;
   (b) a nucleotide sequence that comprises the DNA sequence according to SEQ ID No. 5;
   (c) a nucleotide sequence that comprises a DNA sequence according to SEQ ID No. 4;
   (d) a nucleotide sequence that hybridizes to a nucleotide sequence which is complementary to the nucleotide sequence according to (a), (b), or (c), under stringent conditions;
   (e) a nucleotide sequence that encodes a polypeptide which, via substitution, deletion, and/or addition of one or more amino acids of the amino acid sequence, differs from a polypeptide that is encoded by the nucleotide sequence according to (a), (b), or (c); or
   (f) a nucleotide sequence that encodes a polypeptide which has an amino acid sequence that is at least 80% identical to an amino acid sequence according to SEQ ID No. 6.
[16] Plant, or a portion thereof, obtained or obtainable according to a method according to one of [12] through [15].
[17] Method for identifying, and optionally providing, a plant of the species *Beta vulgaris* that is resistant to Cercospora, characterized in that the method includes at least step (i) or (ii):
   (i) detection of the presence and/or expression of the nucleic acid molecule according to one of [1] through [3], or the presence of the polypeptide according to [4], in the plant or a portion of the plant; and/or
   (ii) detection of at least one marker locus in the nucleotide sequence of the nucleic acid molecule according to one of [1] through [3] or in a cosegregating region; and
   (iii) possible selection of the *Cercospora beticola-resistant* plant,
   wherein the detection in step (i) or step (ii) is based on the use of at least one molecular marker.
[18] Method for identification of a nucleic acid molecule which encodes a polypeptide that is able to confer a resistance to Cercospora in a plant of the species *Beta vulgaris* in which the polypeptide is expressed, characterized in that the method includes the following steps:
   (i) comparison of the amino acid sequence of the polypeptide according to [4] with amino acid sequences from a sequence database, or identification of allelic variants which encode the polypeptide according to [4] in genotypes of the species *Beta vulgaris;*
   (ii) identification of the amino acid sequence, or an allelic variant, encoding an amino acid sequence, wherein the amino acid sequence is at least 80% identical to the amino acid sequence of the polypeptide according to [4];
   (iii) introduction of a nucleic acid molecule, or the allelic variant, encoding the identified amino acid sequence into a plant of the species *Beta vulgaris,* and expression of the nucleic acid molecule in the plant; and
   (iv) detection of the resistance to Cercospora.
[19] Method for farming of plants of the species *Beta vulgaris,* including
   (i) the provision of plants according to one of [7] through [9], the planting of a pelleted seed of a sugar beet plant or of a plant of the genus *Beta* according to one of [26] - [39], the production of plants of the species *Beta vulgaris* with the aid of a method according to one of [12] through [15], or the identification and selection of plants of the genus Beta with the aid of a method according to [17], and
   (ii) cultivation of the plants from (i) or descendants thereof,
   wherein the method counteracts an infestation of the cultivated plants with Cercospora.
[20] Oligonucleotide of at least 15, 16, 17, 18, 19, or 20 - preferably, at least 21, 22, 23, 24, or 25, particularly preferably, at least 30, 35, 40, 45, or 50, and, especially preferably, at least 100, 200, 300, or 500 - nucleotides in length, which oligonucleotide hybridizes with a nucleotide sequence as defined in one of [1] through [3].
[21] A pair of oligonucleotides - preferably, oligonucleotides according to [20] or a kit containing these oligonucleotides - wherein the oligonucleotides are suitable for hybridization as forward primer and reverse primer to a region in the *Beta vulgaris* genome that, in *Beta vulgaris,* has a cosegregation with the Cercospora resistance conferred by the polypeptide according to [4], or with the nucleic acid molecule according to one of [1] through [3].
[22] Use of the nucleic acid molecule according to one of [1] through [3] in the production of Cercospora-resistant plants of the subspecies *Beta vulgaris* subsp. *vulgaris.*
[23] Method for the production of an organism which comprises a mutated version according to [1] and/or a mutated version of a promoter comprising a nucleic acid sequence selected from
   (a) SEQ ID NO: 7
   (b) a nucleotide sequence, which hybridizes under stringent conditions with a sequence which is complementary to the sequence according to (a)
   (c) a nucleotide sequence which is at least 70% identical to a sequence according to SEQ ID NO: 7
   wherein the method includes the following steps:
   (I) Provision of an organism or a cell comprising the nucleic acid molecule and/or the promoter
   (II) Increase of the mutation rate of the organism or the cell or mutagenesis of the organism or the cell
   (III) Phenotypic selection of an organism, which as a result of a mutation exhibits an altered resistance or altered resistance level towards *Cercospora beticola* or Genotypic selection of an organism or a cell which comprises a mutation in the nucleic acid molecule and / or the promoter wherein the mutation has been created via step (II) and optionally
   (IV) Regeneration of the organism from the cell obtained via step (III).
[24] Method according to [23], wherein the organism is a plant.
[25] Method according to [24] wherein the plant is a *Beta vulgaris,* preferably a *Beta vulgaris* subsp. *vulgaris,* more preferably a sugar beet or red beet.
[26] A pelleted seed of a sugar beet plant or a plant of the genus *Beta* including red beet comprising a nucleic acid molecule according to [1].
[27] The pelleted seed according to [26], wherein the beet body is suitable as raw material for industrial sugar production or for consumption as food.
[28] The pelleted seed according to [26] or [27], wherein the pelleted seed is a monogerm seed.
[29] The pelleted seed according to [26] to [28], wherein the sugar beet plant is harvestable before bolting.
[30] The pelleted seed according to [26] to [29], wherein the resistance to Cercospora is a resistance to Cercospora beticola.
[31] The pelleted seed according to [26] to [30], wherein the sugar beet plant is biannual.
[32] The pelleted seed according to [26] to [31] and [124], which has been technically treated, wherein the technical treatment is selected from the group consisting of:
   (a) polishing;
   (b) dressing;
   (c) incrustation; and
   (d) coloring.
[33] The pelleted seed according to [26] to [32], wherein the pellet comprises at least one chemical selected from the group selected of:
   (a) insecticide;
   (b) fungicide; and
   (c) fertilizer.
[34] The pelleted seed according to [26] to [33], wherein the seed has been subjected to priming or pre-germination before or during pelleting.
[35] The pelleted seed according to [26] to [34], wherein the sugar beet plant is a hybrid sugar beet plant.
[36] The pelleted seed according to [26] to [35] wherein the nucleotide sequence includes at least one mutation.
[37] The pelleted seed according to [36] wherein the at least one mutation is a mutation relative to SEQ ID No. 1 or SEQ ID No 2.
[38] The pelleted seed according to [36] or [37] wherein the nucleotide sequence including the at least one mutation encodes a polypeptide which has an amino acid sequence that is at least 99% identical to an amino acid sequence according to SEQ ID No. 3.
[39] The pelleted seed according to [38] wherein the nucleotide sequence including the at least one mutation encodes a polypeptide having an amino acid sequence according to SEQ ID No. 3.
[40] A packing containing the pelleted seed according to [26] to [39] or containing seed stock comprising the nucleic acid molecule according to [1] wherein the seed stock preferable is seed stock of a plant of the genus *Beta.*
[41] A mixture of a pelleting mass and a sugar beet plant seed wherein the sugar beet plant seed comprises a nucleic acid sequence which encodes a polypeptide that is able to confer resistance to Cercospora, wherein the nucleotide sequence is selected from the group consisting of
   (a) a nucleotide sequence encoding a polypeptide having an amino acid sequence according to SEQ ID No. 3;
   (b) a nucleotide sequence that comprises the DNA sequence according to SEQ ID No. 2;
   (c) a nucleotide sequence that comprises a DNA sequence selected from the group consisting of SEQ ID No. 1 or SEQ ID No. 53;
   (d) a nucleotide sequence that hybridizes to a nucleotide sequence which is complementary to the nucleotide sequence according to (a), (b), or (c), under stringent conditions;
   (e) a nucleotide sequence encoding a polypeptide which, via substitution, deletion, and/or addition of one or more amino acids of the amino acid sequence, differs from a polypeptide encoded by the nucleotide sequence according to (a), (b), or (c);
   (f) a nucleotide sequence encoding a polypeptide which has an amino acid sequence that is at least 70% identical to an amino acid sequence according to SEQ ID No. 3;
   (g) a nucleotide sequence that is at least 70% identical to a DNA sequence according to SEQ ID No. 1 or SEQ ID No. 2.
[42] A method for producing the pelleted sugar beet plant seed according to [26] to [39] comprising the following steps:
   a) providing a sugar beet plant seed comprising a nucleic acid sequence which encodes a polypeptide that is able to confer resistance to Cercospora,
      wherein the nucleotide sequence which is selected from the group consisting of
      (i) a nucleotide sequence that encodes a polypeptide which has an amino acid sequence that is at least 95% identical to an amino acid sequence according to SEQ ID No. 3; and
      (ii) a nucleotide sequence that is at least 95% identical to a DNA sequence according to SEQ ID No. 1 or SEQ ID No. 2
   b) embedding the sugar beet plant seed in a pelleting mass
   c) allow the pelleting mass to dry or dry the pelleting mass.
[43] The pelleted seed according to [36] to [39] wherein the nucleotide sequence including the at least one mutation is an artificial nucleotide sequence which does not occur naturally.
[44] The method according to [23] wherein during step (II) a mutagenizing chemical like EMS or mutagenizing radiation is applied.
[45] A variety or cultivar of the genus Beta comprising the nucleic acid molecule according to [1] or a pelleted seed the variety or cultivar.
[46] A leaf of a swiss chard plant comprising the Nucleic acid molecule according to [1].
[47] A bag - preferably a plastic bag - comprising one or more chard plant leaves according to [46].
[48] A method for identifying a plant, preferably a plant of the species *Beta vulgaris* or a plant of the subspecies *Beta vulgaris subsp. vulgaris,* that is resistant or tolerant to Cercospora, characterized in that the method includes the following steps:
   (i) detection of the presence and/or expression of the nucleic acid molecule according to [1] or the nucleotide sequence as defined in [1], or the presence of the polypeptide which is encoded by the nucleic acid molecule according to [1] or of the nucleotide sequence as defined in [1], in the plant or a portion of the plant; and/or
   (ii) detection of at least one marker locus in the nucleotide sequence of the nucleic acid molecule according [1] or in a cosegregating region,
   wherein the cosegregating region is a genomic region which cosegregates with the Cercospora resistance conferred by the polypeptide, or with the nucleic acid molecule or the nucleotide sequence and wherein the cosegregating region comprises and is flanked preferably by marker s4p1395s01 and s4p0421s01. More preferably the cosegregating region comprises and is flanked by a marker pair selected from the list consisting of: sxi0123s02 and s4p0238s01, s4p1396s01 and sxh1195s02, s4p1398s01 and s4p0234s01, s4p1462s01 and s4p0232s01, s4p1464s01 and s4p0323s01, s4p0044s01 and s4p0322s01, s4p0056s01 and s4p0320s01, s4p0058s01 and sxh0942s04, s4p0059s01 and sxh1834s05, s4p1564s01 and s4p0317s01, s4p1998s01 and s4p4308s01, s4p1343s01 and s4p4305d01, s4p1408s01 and sxh0678s01, s4p1565s01 and s4p4301s01, s4p1409s01 and s4p8772s01, s4p1411s01 and s4p4295s01. Most preferably the cosegregating region comprises and is flanked by a marker pair selected from the list consisting of: s4p1343s01 and s4p0421s01, s4p1408s01 and s4p0238s01, s4p1565s01 and sxh1195s02 s4p1409s01 and s4p0234s01, s4p1411s01 and s4p0232s01, s4p1414s01 and s4p0323s01, s4p1485s01 and s4p0322s01, s4p0257s01 and s4p0320s01, s4p0258s01 and sxh0942s04, s4p0260s01 and sxh1834s05, sxh0876s05 and s4p0317s01, s4p0262s01 and s4p4308s01, s4p0263s01 and s4p4305d01. For mapping purposes the following marker pairs are recommended: s4p4293s01 and s4p8772s01, preferably s4p4295s01 and s4p8772s01. The structural features of the markers are given in Table IB. The method may involve one or more of the following steps:
   - Provision of at least one plant, its tissue, a seed or at least one cell thereof
   - Extraction of DNA, preferably genomic DNA from the at least one plant, its tissue, the seed or from the at least one cell thereof and
   - Performing the detection on the extracted DNA
   - Selection of the Cercospora-resistant plant.
[49] The method according to anyone of [48] or [104] - [113] characterized in that the method includes a further step:
   - creating electronically transmittable and / or electronically storable data representing the detection of the presence of the nucleic acid molecule or the nucleotide sequence.
[50] The method according to [49] characterized in that the method includes a further step:
   - storing the data on a computer readable medium.
[51] The method according to one of [48] to [50] wherein the markers are molecular markers and / or diagnostic markers.
[52] A process of identifying a plant that displays resistance or tolerance to *Cercospora,* the process comprising detecting in the plant at least one polymorphism - preferably a single nucleotide polymorphism - by at least one marker, wherein the at least one marker is within a chromosomal interval flanked by the two markers s4p1395s01 and s4p0421s01 or within a chromosomal interval flanked by a pair of markers disclosed in [48] and wherein the plant comprises the nucleic acid molecule or the nucleotide sequence according to [1]. The markers may be diagnostic and / or molecular markers. The process may involve one or more of the following steps:
   - Provision of at least one plant, its tissue, a seed or at least one cell thereof
   - Extraction of DNA, preferably genomic DNA from the at least one plant, its tissue, the seed or from the at least one cell thereof
   - Performing the detection on the extracted DNA and
   - Selection of the Cercospora-resistant plant.
[53] The process according to [52] wherein
   (i) said at least one single nucleotide polymorphism is genetically linked to the nucleic acid molecule or nucleotide sequence according to [1] or has a recombination frequency of about 10%, preferably 5%, more preferably 1% or less to the nucleic acid molecule or nucleotide sequence according to [1], or
   (ii) said at least one single nucleotide polymorphism is located within 2562 kbp, 2300kbp, 2100kbp, 1900kbp, 1700kbp, 1500kbp, 1300kbp, 1100kbp, 900kbp, 700kbp, 500kbp, 300kbp, 100kbp, 50kbp, 25kbp, 10kbp, 5kbp or 1kbp or less of the nucleic acid molecule or the nucleotide sequence according to [1].
[54] The process or method according to anyone of [48], [52], [104] - [113] wherein the plant displays increased resistance or tolerance to *Cercospora,* compared to a plant lacking the nucleic acid molecule or nucleotide sequence of [1]
[55] The process according to anyone of [52] - [54] ,[57] - [70], [106] - [113] wherein the single nucleotide polymorphism is a nucleotide the presence of which is genetically and/or statistically linked to the presence of the nucleic acid molecule or the nucleotide sequence according to [1].
[56] The process according to [52] - [55] wherein the at least one marker is a marker given in Table 1b or the at least one marker is a marker pair as disclosed in [48].
[57] A process of identifying a plant that is resistant or tolerant to *Cercospora,* the process comprising detecting in the plant or in the DNA of the plant at least two single nucleotide polymorphisms by at least two markers, wherein at least one of said markers is on or within a first chromosomal interval flanked by a marker selected from the group consisting of:
   s4p1395s01, sxi0123s02, s4p1396s01, s4p1398s01, s4p1462s01, s4p1464s01, s4p0044s01, s4p0056s01, s4p0058s01, s4p0059s01, s4p1564s01, s4p1998s01, s4p1343s01, s4p1408s01, s4p1565s01, s4p1409s01, s4p1411s01, s4p1414s01, s4p1485s01, s4p0257s01, s4p0258s01, s4p0260s01, sxh0876s05, s4p0262s01, s4p0263s01, s4p0264s01, s4p2271s01, s4p4288s01, s4p4290d01, s4p4293s01
   and the nucleotide sequence according to [1], and at least one of said markers is on or within a second chromosomal interval flanked by the polynucleotide of claim 1 and a marker selected from the group consisting of:
   s4p0421s01, s4p0238s01, sxh1195s02, s4p0234s01, s4p0232s01, s4p0323s01, s4p0322s01, s4p0320s01, sxh0942s04, sxh1834s05, s4p0317s01, s4p4308s01, s4p4305d01.
[58] The process according to [57] wherein the at least two markers and the nucleotide sequence according to [1] flank the first and second chromosomal interval without being part of the first or second chromosomal interval.
[59] The process according to [57] or [58] wherein the at least two markers are each located within 2562kbp, 2300kbp, 2100kbp, 1900kbp, 1700kbp, 1500kbp, 1300kbp, 1100kbp, 900kbp, 700kbp, 500kbp, 300kbp, 100kbp, 50kbp, 25kbp, 10kbp, 5kbp or 1kbp or less of the nucleic acid molecule or the nucleotide sequence according to [1].
[60] The process according to [57] - [59] wherein at least one marker is located on a genomic region having an identity of at least 95%, at least 96%, at least 97%, at least 98%, at least 99% or 100% to SEQ ID No. 74 or SEQ ID No. 75, wherein the identity is preferably over the whole length of SEQ ID No. 74 or SEQ ID No. 75.
[61] The process according to [57] - [60] wherein one of the at least two markers is located on the genomic region having an identity of at least 95%, at least 96%, at least 97%, at least 98%, at least 99% or 100% to SEQ ID No. 74 and one of the at least two markers is located on the genomic region having an identity of at least 95%, at least 96%, at least 97%, at least 98%, at least 99% or 100% to SEQ ID No. 75, wherein the identity is preferably over the whole length of SEQ ID No. 74 or SEQ ID No. 75.
[62] The process according to [57] - [61] wherein at least one of the at least two markers is located within 2562kbp, 2300kbp, 2100kbp, 1900kbp, 1700kbp, 1500kbp, 1300kbp, 1100kbp, 900kbp, 700kbp, 500kbp, 300kbp, 100kbp, 50kbp, 25kbp, 10kbp, 5kbp or 1kbp or less of the nucleic acid molecule or the nucleotide sequence according to [1].
[63] The process according to [57] - [62] wherein each of the at least two markers is located within 2562kbp, 2300kbp, 2100kbp, 1900kbp, 1700kbp, 1500kbp, 1300kbp, 1100kbp, 900kbp, 700kbp, 500kbp, 300kbp, 100kbp, 50kbp, 25kbp, 10kbp, 5kbp or 1kbp or less of the nucleic acid molecule or the nucleotide sequence according to [1].
[64] The process according to [57] - [63] involving the detection of at least two single nucleotide polymorphisms in the first chromosomal interval and at least two single nucleotide polymorphisms in the second chromosomal interval wherein each single nucleotide polymorphism is detected by a different marker.
[65] The process according to [57] - [64] including the following steps
   - Provision of at least one plant, its tissue, a seed or at least one cell thereof
   - Extraction of DNA, preferably genomic DNA from the at least one plant, its tissue, a seed or from the at least one cell thereof and
   - Performing the detection step as defined in any of [57] - [64] on the extracted DNA.
[66] The process or method according to [48] - [65] wherein the detection step includes a polymerase chain reaction (PCR).
[67] The process or method according to [48] - [65] or [112] or [113] involving a polymerase chain reaction (PCR) wherein the PCR involves two allele-specific forward primers (or their use) and wherein the detection step involves fluorescence resonant energy transfer (FRET) wherein the presence, absence or kind of the fluorescence is determined by a sensor. The sensor signal may be turned into electronically transmittable and / or electronically storable data representing the detection of the presence of the nucleic acid molecule or the nucleotide sequence according to [1]. In addition, the electronically transmittable and / or electronically storable data may be stored on a computer readable medium. The kind of the fluorescence may be its color / wave length or the specific dye responsible for the fluorescence (like FAM or HEX).
[68] The process according to [67] furthermore involving one common reverse primer or involving the use of one common reverse primer. Examples for common primers are given in Tab. 1b.
[69] The process according to anyone of [67], [68] [112] and [113] wherein the determination of the fluorescence by the sensor is an end-point fluorescent read.
[70] The process according to anyone of [67] or [69], [112] and [113] wherein each of the two allele-specific forward primers comprise a unique tail sequence that undergoes chemical binding with a specific FRET cassette during the PCR.
[71] A method for identifying a plant, preferably a plant of the species *Beta vulgaris* or a plant of the subspecies *Beta vulgaris subsp. vulgaris,* that is resistant or tolerant to Cercospora, characterized in that the method includes the following steps:
   - Provision of at least one plant, its tissue, seed or at least one cell thereof
   - Extraction of DNA, preferably genomic DNA from the at least one plant, its tissue or from the at least one cell thereof and
   - Detecting the presence or absence of the nucleic acid molecule or the nucleotide sequence according to [1] in the extracted DNA, preferably by a marker as given in Tab. 1b or by a marker pair as disclosed in [48], by a pair of oligonucleotides according to [20], [21], [72] or [73] or [76], a set of three oligonucleotides according to [74] or [75] or a molecular marker or a set of molecular markers according to [84].
   - Optionally selecting the plant comprising the nucleic acid molecule or the nucleotide sequence according to [1] in the extracted DNA.
[72] A pair of oligonucleotides suitable for use as primer in a PCR which are able to hybridize to a genomic interval which comprises and is flanked by marker s4p1395s01 and s4p0421s01. Preferably the genomic interval comprises and is flanked by a marker pair selected from the list consisting of: sxi0123s02 and s4p0238s01, s4p1396s01 and sxh1195s02, s4p1398s01 and s4p0234s01, s4p1462s01 and s4p0232s01, s4p1464s01 and s4p0323s01, s4p0044s01 and s4p0322s01, s4p0056s01 and s4p0320s01, s4p0058s01 and sxh0942s04, s4p0059s01 and sxh1834s05, s4p1564s01 and s4p0317s01, s4p1998s01 and s4p4308s01, s4p1343s01 and s4p4305d01, s4p1408s01 and sxh0678s01, s4p1565s01 and s4p4301s01, s4p1409s01 and s4p8772s01, s4p1411s01 and s4p4295s01. Most preferably the genomic interval comprises and is flanked by a marker pair selected from the list consisting of: s4p1343s01 and s4p0421s01, s4p1408s01 and s4p0238s01, s4p1565s01 and sxh1195s02 s4p1409s01 and s4p0234s01, s4p1411s01 and s4p0232s01, s4p1414s01 and s4p0323s01, s4p1485s01 and s4p0322s01, s4p0257s01 and s4p0320s01, s4p0258s01 and sxh0942s04, s4p0260s01 and sxh1834s05, sxh0876s05 and s4p0317s01, s4p0262s01 and s4p4308s01, s4p0263s01 and s4p4305d01. The genomic interval may be the genomic interval of a plant of the species *Beta vulgaris* or subspecies *Beta vulgaris subsp. vulgaris.*
[73] A pair of oligonucleotides suitable for use as primer in a PCR which are able to hybridize to the nucleic acid sequence according to SEQ ID No. 74 or SEQ ID No. 75 or to a sequence that is at least 99% identical to the sequence according to SEQ ID No. 74 or SEQ ID No. 75 over the whole length of the sequence according to SEQ ID No. 74 or SEQ ID No. 75.
[74] A set of three oligonucleotides suitable for use as primer in a PCR involving two forward primers and a reverse primer wherein each primer has a different nucleotide sequence and wherein the reverse primer and only one of the two forward primers are able to hybridize to a genomic interval which comprises and is flanked by marker s4p1395s01 and s4p0421s01. Preferably the genomic interval comprises and is flanked by a marker pair selected from the list consisting of: sxi0123s02 and s4p0238s01, s4p1396s01 and sxh1195s02, s4p1398s01 and s4p0234s01, s4p1462s01 and s4p0232s01, s4p1464s01 and s4p0323s01, s4p0044s01 and s4p0322s01, s4p0056s01 and s4p0320s01, s4p0058s01 and sxh0942s04, s4p0059s01 and sxh1834s05, s4p1564s01 and s4p0317s01, s4p1998s01 and s4p4308s01, s4p1343s01 and s4p4305d01, s4p1408s01 and sxh0678s01, s4p1565s01 and s4p4301s01, s4p1409s01 and s4p8772s01, s4p1411s01 and s4p4295s01. Most preferably the genomic interval comprises and is flanked by a marker pair selected from the list consisting of: s4p1343s01 and s4p0421s01, s4p1408s01 and s4p0238s01, s4p1565s01 and sxh1195s02 s4p1409s01 and s4p0234s01, s4p1411s01 and s4p0232s01, s4p1414s01 and s4p0323s01, s4p1485s01 and s4p0322s01, s4p0257s01 and s4p0320s01, s4p0258s01 and sxh0942s04, s4p0260s01 and sxh1834s05, sxh0876s05 and s4p0317s01, s4p0262s01 and s4p4308s01, s4p0263s01 and s4p4305d01.
[75] A set of three oligonucleotides suitable for use as primer in a PCR involving two forward primers and a reverse primer wherein each primer has a different nucleotide sequence and wherein the reverse primer and only one of the two forward primers are able to hybridize to a sequence according to SEQ ID No. 74 or SEQ ID No. 75 or to a sequence that is at least 99% identical to the sequence according to SEQ ID No. 74 or SEQ ID No. 75 over the whole length of the sequence according to SEQ ID No. 74 or SEQ ID No. 75.
[76] The pair or set of oligonucleotides according to [72] - [75] wherein each oligonucleotide comprises or consists of 15 - 40 nucleotides, preferably 17 - 30 nucleotides, more preferably 19 - 25 nucleotides.
[77] A process for the quantification of the level of resistance or tolerance of a plant to *Cercospora* including the following steps:
   - Providing at least one first plant or at least one seed of the first plant comprising the nucleic acid molecule or the nucleotide sequence according to [1] and at least one second plant or at least one seed of the at least second plant lacking the nucleic acid molecule or the nucleotide sequence according to [1]
   - Cultivate the plants or allow the seeds to germinate and cultivate the germinated plants wherein a) the conditions of the cultivation allow the interaction with *Cercospora* or b) the plants are inoculated with *Cercospora*
   - Determine the infested surface of at least one leaf of each of the two plants.
   - Optionally compare the level of resistance or tolerance of the first plant with the level of resistance or tolerance of the second plant and/or determine a rating score for the first and or second plant selected from Table 1A.
[78] The process according to [77] wherein the cultivation step is replaced by the inoculation of a leaf of the first plant and a leaf of the second plant with or in a solution comprising *Cercospora.*
[79] A method for reducing the application of fungicidal agrochemicals including the following steps:
   I) Provide seeds or seed stock comprising the nucleic acid molecule or the nucleotide sequence according to [1].
   II) Plant or drill the seeds or the seed stock to allow the germination of the seeds or the seed stock.
   III) Cultivate the germinated plants derived by step II)
   IV) Harvest the plants cultivated during step III) or harvest plant parts like storage organs, taproots or harvest seeds of the plants cultivated during step III)
   wherein the application of fungicidal agrochemicals during step II) and or step III) is reduced or avoided.
[80] The method according to [79] wherein the fungicidal agrochemical is an agrochemical which is effective against *Cercospora* wherein these agrochemicals may include those which contain one or more of the following fungicides: epoxiconazole, kresoxim-methyl, thiophanate methyl, mancozeb and those fungicides effective against *Cercospora* mentioned elsewhere herein.
[81] The method according to [79] or [80] wherein the application of fungicidal agrochemicals during step II) and or step III) is reduced or avoided in comparison to the cultivation of plants lacking the nucleic acid molecule according to [1] under the same or comparable conditions as the plants in step III).
[82] Use of a marker or a molecular marker for the identification and / or selection of a plant comprising the nucleic acid molecule or nucleotide sequence according to [1].
[83] The use according to [82] wherein the marker or molecular marker is used in a process or method according to [48] - [71].
[84] A molecular marker or a set of molecular markers - preferably encompassing two or three molecular markers - suitable for use in a process or method according to [48] - [71].
[85] A method for the expression of the nucleic acid molecule or nucleotide sequence according to [1] comprising the following steps:
   i) Introduction of the nucleic acid molecule or nucleotide sequence according to [1] in one or more cells
   ii) Cultivating the one or more cells under conditions which allow the proliferation of the one or more cells and / or which allow the expression of the nucleic acid molecule or nucleotide sequence according to [1]
[86] The method according to [85] wherein the method is a method for *in vitro* expression.
[87] The method according to [85] or [86] wherein the introduction in step i) is by transformation, transfection, electroporation or infiltration.
[88] The method according to [85] - [87] wherein the introduction of the nucleic acid molecule or nucleotide sequence is the introduction of a vector, plasmid or transfer DNA (tDNA).
[89] The method according to [85] - [88] wherein the nucleic acid molecule or nucleotide sequence is part of an expression cassette and is operably linked to a promoter which is active, partly active or inducible in the one or more cells of i). The expression cassette may be the expression cassette according to [5].
[90] The method according to [89] wherein the promoter has at least 95% sequence identity to the 35S promoter derived from *Cauliflower mosaic virus* or is the 35S promoter derived from *Cauliflower mosaic virus.*
[91] The method according to [90] wherein the 35S promoter derived from *Cauliflower mosaic virus* comprises or consists of a nucleic acid sequence according to SEQ ID No. 225.
[92] The method according to [85] - [91] wherein the one or more cells are plant cells.
[93] The method according to [85] - [89] wherein the one or more cells are microorganisms preferably bacteria or fungal cells like yeast.
[94] The plant according to [7] - [9] characterized in that the plant furthermore comprises a nucleic acid molecule which encodes a polypeptide that is able to convey a resistance to the pathogen Beet Necrotic Yellow Vein Virus BNYVV in a plant in which the polypeptide is expressed, characterised in that the nucleic acid molecule comprises a nucleotide sequence selected from
   a) a nucleotide sequence that encodes a polypeptide having an amino acid sequence according to SEQ ID NO: 227 or SEQ ID NO: 228,
   b) a nucleotide sequence comprising the encoding sequence of the DNA sequence according to SEQ ID NO: 226,
   c) a nucleotide sequence that hybridises with the complementary sequence of the nucleotide sequence according to a) or b) under stringent conditions,
   d) a nucleotide sequence that encodes a polypeptide which is derived by substitution, deletion and/or addition of one or more amino acids of the amino acid sequence encoded by the nucleotide sequence according to a) or b) from a polypeptide encoded by the nucleotide sequence according to a) or b),
   e) a nucleotide sequence that encodes a polypeptide which has an amino acid sequence at least 90%, preferably at least 95%, most preferably 99% identical to an amino acid sequence encoded by the nucleotide sequence according to a) or b), or
   f) a nucleotide sequence that encodes at least one nucleotide-binding domain (NBS) corresponding to amino acid positions 168-227 of SEQ ID NO: 227 or corresponding to amino acid positions 182-241 of SEQ ID NO: 228, at least one leucine-rich domain (LRR) corresponding to amino acid positions 591-613 of SEQ ID NO: 227 or corresponding to amino acid positions 605-627 of SEQ ID NO: 228 and/or at least one internal repetitive domain (IR) corresponding to amino acid positions 1013-1072 of SEQ ID NO: 227 or corresponding to amino acid positions 1027-1086 of SEQ ID NO: 228.
[95] The plant according to [7] -[9] wherein the plant is obtainable from the seeds deposited with the NCIMB, Aberdeen, UK under access number NCIMB 43646.
[96] The seed or descendent according to [10] wherein the seed or descendent is obtainable from the deposition with the NCIMB, Aberdeen, UK under access number NCIMB 43646.
[97] The plant according to [7] - [9], [94] and [95] furthermore characterized in that the plant comprises SEQ ID NO. 182 or is detectable by marker s4p8772s01.
[98] A process for the breeding of a *Cercospora* resistant plant of the species *Beta vulgaris* including the following steps:
   i) Obtaining seeds from the deposition with the NCIMB, Aberdeen, UK under access number NCIMB 43646 or offspring of the deposited seeds.
   ii) Cultivate the seeds of step i) under conditions which allow the growth of plants
   iii) Cross the plants resulting from step ii) with a plant of the species *Beta vulgaris.* The method may optionally include a further step of
   iv) Determine the presence or absence of the nucleic acid molecule or the nucleotide sequence according to [1] in the offspring resulting from step iii) by a method or process described herein like for example a method or process according to [48] - [71].
[99] Use of electronically transmittable and / or electronically storable data derived from a process according to anyone of [49], [50], [69], [70] and [104] - [113] for determining the estimated breeding value of a plant in a population of plants.
[100] The use according to [99] wherein the estimated breeding value is at least partly dependent on *Cercospora* resistance and wherein the population of plants comprises 1000 or less plants.
[101] The plant according to one of [7] -[9] or a seed or descendant of the plant according to [10] wherein the nucleic acid molecule or the nucleotide sequence according to [1] is comprised or integrated as an introgression.
[102] A storage organ of the plant according to one of [7] -[9] or [101]. The storage organ may be a taproot, especially a beet body.
[103] A leaf or leaves of the plant according to one of [7] -[9] or [101], wherein the plant may be a swiss chard or a plant of the genus *Spinacia.*
[104] A method for identifying a plant that is resistant or tolerant to Cercospora, characterized in that the method includes the following steps:
   (i) detection of the presence or absence of a nucleotide sequence selected from the group consisting of
      (a) a nucleotide sequence that encodes a polypeptide having an amino acid sequence according to SEQ ID No. 3;
      (b) a nucleotide sequence that comprises the DNA sequence according to SEQ ID No. 2;
      (c) a nucleotide sequence that comprises a DNA sequence according to SEQ ID No. 1 or SEQ ID No. 53;
      (d) a nucleotide sequence that hybridizes with a complementary sequence of the nucleotide sequence according to (a), (b), or (c) in 4 x SSC (Saline-Sodium Citrate) at 65 °C, and subsequent repeated washing in 0.1 x SSC at 65 °C for approximately 1 hour in total;
      (e) a nucleotide sequence that encodes a polypeptide which has an amino acid sequence that is at least 90% identical to an amino acid sequence according to SEQ ID No. 3;
      (f) a nucleotide sequence that is at least 90% identical to a DNA sequence according to SEQ ID No. 1 or SEQ ID No. 2;
   (ii) detection of the presence or absence of the polypeptide which is encoded by the nucleotide sequence as defined in step (i) in the plant or a portion of the plant; and/or
   (iii) detection of at least one marker locus in the nucleotide sequence as defined in step (1) or in a cosegregating region,
   wherein the cosegregating region is a genomic region which cosegregates with the Cercospora resistance conferred by the polypeptide, or with the nucleic acid molecule or the nucleotide sequence and wherein the cosegregating region comprises and is flanked by marker s4p1395s01 and s4p0421s01.
[105] The method according to [104], characterized in that the method includes the further step of:
   (iv) selection of the Cercospora-resistant plant.
[106] The method according to [104] or [105] wherein the detection in step (i) or (iii) is based on at least one polymorphism or a single nucleotide polymorphism.
[107] The method according to [106] furthermore characterized in that
   a) said at least one polymorphism or single nucleotide polymorphism is genetically linked to the nucleotide sequence or has a recombination frequency of 10% or less to the nucleotide sequence,
   b) said at least one polymorphism or single nucleotide polymorphism is located within 2562kbp, 2300kbp, 2100kbp, 1900kbp, 1700kbp, 1500kbp, 1300kbp, 1100kbp, 900kbp, 700kbp, 500kbp, 300kbp, 100kbp, 50kbp, 25kbp, 10kbp, 5kbp or 1kbp or less of the nucleotide sequence,
   c) said at least one polymorphism or single nucleotide polymorphism is detectable in the seeds deposited with the NCIMB, Aberdeen, UK under access number NCIMB 43646 or
   d) said at least one polymorphism or single nucleotide polymorphism is part of the cosegregating region,
   wherein the nucleotide sequence is the nucleotide sequence defined in step (i) of [104] and wherein the cosegregating region is the cosegregating region defined in step (iii) of [104].
[108] The method according to anyone of [104]-[107] wherein step (i) and / or step (iii) furthermore includes the following steps which are concluded by the detection:
   a) Provision of at least one plant, its tissue, a seed or at least one cell thereof and
   b) Extraction of DNA, preferably genomic DNA from the at least one plant, its tissue, the seed or from the at least one cell thereof.
   This means for example:
   A method for identifying a plant that is resistant or tolerant to Cercospora, characterized in that the method includes the following steps:
   ia) Provision of at least one plant, its tissue, a seed or at least one cell thereof and
   ib) Extraction of DNA, preferably genomic DNA from the at least one plant, its tissue, the seed or from the at least one cell thereof and
   ic) detection of the presence or absence of a nucleotide sequence as defined in step (i) of [104]
   ii) detection of the presence or absence of the polypeptide which is encoded by the nucleotide sequence as defined in step (i) in the plant or a portion of the plant; and/or
   iiia) Provision of at least one plant, its tissue, a seed or at least one cell thereof and
   iiib) Extraction of DNA, preferably genomic DNA from the at least one plant, its tissue, the seed or from the at least one cell thereof and
   iiic) detection of at least one marker locus in the nucleotide sequence as defined in step (1) or in a cosegregating region,
   wherein the cosegregating region is a genomic region which cosegregates with the Cercospora resistance conferred by the polypeptide, or with the nucleic acid molecule or the nucleotide sequence and wherein the cosegregating region comprises and is flanked by marker s4p1395s01 and s4p0421s01.
[109] The method according to [104]-[108] involving the use of at least two oligonucleotides.
[110] The method according to [109] wherein the oligonucleotides are suitable for use as primer in a PCR and are able to hybridize to a genomic interval which comprises and is flanked by marker s4p1395s01 and s4p0421s01 or by a marker pair as disclosed in [48]. Preferably the oligonucleotides may hybridize during a PCR with a genomic template comprising the nucleotide sequence as defined in step (i) of [104] in such a way / in such a distance that the resulting amplificate is comprises up to 2000bp, preferably up to 1500bp, more preferably 1000bp, most preferably up to 500 or 200 or up to 100bp.
[111] The method according to anyone of the preceding claims wherein the plant is a plant of the genus *Beta, Spinacia, Glycine, Daucus* or *Pastinaca.*
[112] The method according to anyone of [104] - [111] involving a PCR in step (i) and / or (iii) wherein the PCR involves two allele-specific forward primers and wherein the detection step involves fluorescence resonant energy transfer and wherein the presence, absence or kind of the fluorescence is determined by a sensor wherein optionally a sensor signal is produced. The sensor signal may be turned into electronically transmittable and / or electronically storable data representing the detection of the presence of the nucleic acid molecule or the nucleotide sequence according to [1]. In addition, the electronically transmittable and / or electronically storable data may be stored on a computer readable medium. The determination of the fluorescence by the sensor may be an end-point fluorescent read.
[113] The method according to [112] involving furthermore one common reverse primer. Preferably the reverse primer and the allele-specific forward primers may hybridize during a PCR with a genomic template comprising the nucleotide sequence as defined in step (i) of [104] in such a way / in such a distance that the resulting amplificate is comprises up to 2000bp, preferably up to 1500bp, more preferably 1000bp, most preferably up to 500 or 200 or up to 100bp. The primer may be oligonucleotides for example the oligonucleotides mentioned in [109].
[114] A plant of the genus *Beta, Spinacia, Glycine, Daucus* or *Pastinaca* comprising a nucleic acid molecule encoding a polypeptide that is able to confer resistance to *Cercospora* in a plant in which the polypeptide is expressed, characterized in that the nucleic acid molecule comprises a nucleotide sequence which is selected from the group consisting of:
   (a) a nucleotide sequence that encodes a polypeptide having an amino acid sequence according to SEQ ID No. 3;
   (b) a nucleotide sequence that comprises the DNA sequence according to SEQ ID No. 2;
   (c) a nucleotide sequence that comprises a DNA sequence according to SEQ ID No. 1 or SEQ ID No. 53;
   (d) a nucleotide sequence that hybridizes with a complementary sequence of the nucleotide sequence according to (a), (b), or (c) in 4 x SSC at 65 °C, and subsequent repeated washing in 0.1 x SSC at 65 °C for approximately 1 hour in total;
   (e) a nucleotide sequence that encodes a polypeptide which has an amino acid sequence that is at least 90% identical to an amino acid sequence according to SEQ ID No. 3;
   (f) a nucleotide sequence that is at least 90% identical to a DNA sequence according to SEQ ID No. 1 or SEQ ID No. 2;
   wherein the plant of the genus *Beta* or *Spinacia* furthermore comprises an endogenous allele encoding an epsp synthase having at position 179 an amino acid different from proline. According to a specific embodiment this epsp synthase is a mutated epsp synthase and the mutation has been generated by a mutative reagent, radiation, gene editing, site-specific point mutation, zinc finger nucleases, TALENS or by *in vitro* culturing of the respective cells and subsequent selection by the use of a herbicide as glyphosate.
[115] The plant according to [114], further characterized in that the plant is a hybrid plant.
[116] The plant according to [114] or [115], further characterized in that the plant is a double haploid plant.
[117] The plant according to anyone of [114] to [116], further characterized in that the resistance against *Cercospora* is dominant.
[118] The plant according to anyone of [114] to [117], further characterized in that the nucleic acid molecule or nucleotide sequence is comprised as an introgression.
[119] The plant according to anyone of [114] to [118], further characterized in that the nucleic acid molecule or nucleotide sequence is homozygous.
[120] The plant according to anyone of [114] to [119], further characterized the plant has tolerance to glyphosate.
[121] The plant according to anyone of [114] to [120], further characterized in that the nucleic acid molecule or nucleotide sequence is comprised as a transgene.
[122] The plant according to anyone of [114] to [121], further characterized in that the nucleic acid molecule or nucleotide sequence is obtainable from the seeds deposited with the NCIMB, Aberdeen, UK under access number NCIMB 43646.
[123] The storage organ or a leaf of the plant according to anyone of [114] to [122].
[124] A pelleted seed of the plant according to anyone of [114] to [122], characterized in that the seed comprises the nucleic acid molecule and the endogenous allele.
[125] The plant according to anyone of [114] to [122], the storage organ or leaf according to claim 13 or the pelleted seed according to claim 14, wherein the epsp synthase having at position 179 an amino acid different from proline, comprises an amino acid sequence selected from
   i) the sequence of SEQ ID NO: 223
   ii) the sequence i) having at position 179 an amino acid different from serine and proline, or
   iii) a sequence having an identity of at least 90% to the sequence of i) or ii)
   over the entire length of the sequence.
[126] The method according to anyone of [104] - [113] wherein the cosegregating region is a cosegregating region as defined in [48].
[127] The method according to anyone of [109] - [113] wherein the at least two oligonucleotides are oligonucleotides as defined in [20], [21], [72] or [73].
[128] A mixture of a pelleting mass and a sugar beet plant seed characterized in that the sugar beet plant seed is a seed of the plant according to anyone of [114] to [122], characterized in that the seed comprises the nucleic acid molecule and the endogenous allele.

First, some of the terms used in this application are explained in detail in the following:
What is understood by "rating score" in the sense of the present invention is a qualitative assessment of the resistance to a *Cercospora* infestation that is represented using a scale from 1 to 9 (with 1 = strong resistance and 9 = no resistance).

**Table 1A: 9-level resistance rating for Cercospora**

| Rating score | Leaf phenotype | Whole plant phenotype |
|---|---|---|
| 1 | Healthy leaf | Healthy leaf, whole |
| 3 | Diseased leaf, spots on the outer leaves | Whole plant, beginning of disease, spots on the outer leaves |
| 5 | Diseased leaf, merging of the spots into dying areas | Whole plant, advanced disease, merging of the spots into dying areas |
| 7 | Diseased leaf, large part of the leaf brown and dead, only lower lamina is still alive | Whole diseased plant, large portions of the outer leaves are dying off |
| 9 | Diseased leaves, lamina and petiole are dead and dried | Whole diseased plant, outer leaves have died, inner leaves with severe damage, strong new leaf growth |

The genus *Cercospora* encompasses various species, e.g., the species *Cercospora arachidicola, Cercospora ariminiensis, Cercospora asparagi, Cercospora bertoreae, Cercospora beticola, Cercospora bizzozeriana, Cercospora canescens, Cercospora carotae, Cercospora chenopodii, Cercospora cistinearum, Cercospora cladosporioides, Cercospora diazu, Cercospora dulcamarae, Cercospora erysimi, Cercospora hayii, Cercospora kikuchii, Cercospora malvacearum, Cercospora malvicola, Cercospora medicaginis, Cercospora oryzaem, Cercospora personata, Cercospora plantaginis, Cercospora ricinella, Cercospora setariae, Cercospora unamunoi, Cercospora violae,* or *Cercospora zeae-maydis.*

In conjunction with the specification of a length of a nucleotide sequence, the term, "approximately," means a deviation by +/- 200 base pairs - preferably, by +/- 100 base pairs, and, particularly preferably, by +/- 50 base pairs.

A cosegregating region is a genomic region which is in most of the cases or always inherited together with the genomic region, locus, gene, polymorphism or single nucleotide polymorphism to which it is genetically coupled, genetically linked or adjacent or close by. For example the genetic linkage may be inherited in at least 95%, 96%, 97%, 98% or 99% to the next generation or the cosegregating region and the genomic region, locus, gene, polymorphism or single nucleotide polymorphism to which it is genetically coupled, genetically linked or adjacent or close have at distance of at most 2562 kbp, 2300kbp, 2100kbp, 1900kbp, 1700kbp, 1500kbp, 1300kbp, 1100kbp, 900kbp, 700kbp, 500kbp, 300kbp, 100kbp, 50kbp, 25kbp, 10kbp, 5kbp or 1kbp or less. The presence of the cosegregating region is indicative or diagnostic for the corresponding genomic region, locus, gene, polymorphism or single nucleotide polymorphism. Cosegregating regions may be detected / identified by the corresponding methods, markers and oligonucleotides included herein for example in the seeds deposited with the NCIMB, Aberdeen, UK under access number NCIMB 43646. In context of the present invention the cosegregating region may be especially a genomic region in a plant of the genus *Beta, Spinacia, Glycine, Daucus or Pastinaca.*

A "plant" comprises plants of different genera including but not limited to Beta, *Spinacia, Glycine, Daucus* and *Pastinaca.*

A "plant of the genus Beta" belongs to the amaranth family (Amaranthaceae). Numbering among these plants are plants of the species *Beta macrocarpa, Beta vulgaris, Beta lomatogona, Beta macrorhiza, Beta corolliflora, Beta trigyna,* and *Beta nana.* A plant of the species *Beta vulgaris* is, in particular, a plant of the subspecies *Beta vulgaris* subsp. *vulgaris.* For example, numbering among these are *Beta vulgaris* subsp. *vulgaris* var. *altissima* (sugar beet in a narrower sense), *Beta vulgaris* ssp. *vulgaris* var. *vulgaris* (chard), *Beta vulgaris* ssp. *vulgaris* var. *conditiva* (beetroot / red beet), *Beta vulgaris* ssp. *vulgaris* var. *crassa*/*alba* (fodder beet). It is noted that the nucleic acid according to the invention does not naturally occur in sugar beet, chard, beetroot, or fodder beet, but may be introduced into these via human action.

A "plant of the genus *Spinacia"* belongs to the amaranth family (Amaranthaceae). This genus especially encompasses *Spinacia oleracea* which is also known as spinach.

A "plant of the genus *Glycine"* belongs to the family of Fabaceae. This genus especially encompasses *Glycine max* which is also known as soybean.

A "plant of the genus *Daucus"* belongs to the family of Apiaceae. This genus especially encompasses *Daucus carota* and *Daucus carota* subsp. *sativus* which is also known as carrot.

A "plant of the genus *Pastinaca"* belongs to the family of Apiaceae. This genus especially encompasses *Pastinaca sativa* which is also known as parsnip.

A "functional fragment" of a nucleotide sequence means a segment of a nucleotide sequence which has a functionality identical or comparable to that of the complete nucleotide sequence from which the functional fragment originates. As such, the functional fragment may possess a nucleotide sequence which is identical or homologous to the total nucleotide sequence over a length of at least 50%, 55%, 60%, 65%, 70%, 75%, 80%, 85%, 90%, 92%, 94% 96%, 97%, 98%, or 99%. This also explicitly encompasses the range of 90 - 100%. Furthermore, a "functional fragment" of a nucleotide sequence may also mean a segment of a nucleotide sequence which modifies the functionality of the entire nucleotide sequence, e.g., in the course of post-transcriptional or transcriptional gene silencing. As such, the functional fragment of a nucleotide sequence may comprise at least 14, 15, 16, 17, 18, 19, 20, 21, 22, 23, 24, or 25 - preferably, at least 30, 35, 40, 45, 50, 60, 70, 80, 90, 100, 120, or 140, and, particularly preferably, at least 160, 180, 200, 250, 300, 350, 400, 450, 500, 600, 700, 800, 900, or 1,000 - successive nucleotides of the total nucleotide sequence. This also explicitly encompasses the range of 21 to 50 nucleotides.

A "functional part" of a protein means a segment of a protein, or a section of the amino acid sequence, that encodes the protein, wherein the segment may exert functionality identical or comparable to that of the entire protein in a plant cell. Over a length of at least 50%, 55%, 60%, 65%, 70%, 75%, 80%, 85%, 90%, 92%, 94% 96%, 97%, 98%, or 99%, a functional part of a protein has an amino acid sequence that is identical or, with consideration of conservative and semi-conservative amino acid exchanges, similar to the protein from which the functional part originates.

The term, "heterologous," means that the introduced polynucleotide originates from a cell or an organism with a different genetic background, of the same species or a different species, or is homologous to the prokaryotic or eukaryotic host cell, but is then located in a different genetic environment and thus differs from a corresponding polynucleotide that is possibly naturally present. A heterologous polynucleotide may be present in addition to a corresponding endogenous gene.

In the sense of the invention, what is understood by a "homolog" is a protein of the same phylogenetic origin; what is understood by an "analog" is a protein which exerts the same function, but has a different phylogenetic origin; what is understood by an "ortholog" is a protein from a different species that exerts the same function; and what is understood by a "paralog" is a protein that has appeared within a species due to duplication, wherein this copy either retains the same protein function, alters its expression template, but not the function, changes its protein function, or divides up the original gene function between both copies.

What is to be understood by "hybridizing" or "hybridization" is a process in which a single-stranded nucleic acid molecule binds to a nucleic acid strand that is complementary to the greatest possible extent, i.e., forms base pairs with this. Standard methods for hybridization are described in, for example, Sambrook et al., Molecular Cloning: A Laboratory Manual, 3rd ed., Cold Spring Harbor Laboratory Press, Cold Spring Harbor, NY, 2001. What is preferably understood by this is that at least 60% - more preferably, at least 65%, 70%, 75%, 80%, or 85%, and, particularly preferably, 90%, 91%, 92%, 93%, 94%, 95%, 96%, 97%, 98%, or 99% - of the bases of the nucleic acid molecule form a base pairing with the nucleic acid strand that is complementary to the greatest possible extent. The possibility of such an annealing depends upon the stringency of the hybridization conditions. The term, "stringency," relates to the hybridization conditions. High stringency is present when a base pairing is made more difficult; low stringency is present if a base pairing is made easier. For example, the stringency of the hybridization conditions depends upon the salt concentration or ionic strength and the temperature. In general, the stringency may be increased by increasing the temperature and/or decreasing the salt content. What are to be understood by "stringent hybridization conditions" are those conditions given which a hybridization predominantly occurs only between homologous nucleic acid molecules. The term, "hybridization conditions," thereby relates not only to the conditions prevailing in the actual addition of the nucleic acids, but also to the conditions prevailing in the following washing steps. For example, stringent hybridization conditions are conditions under which, predominantly, only those nucleic acid molecules hybridize that have at least 70% - preferably, at least 75%, at least 80%, at least 85%, at least 90%, or at least 95% - sequence identity. Stringent hybridization conditions are, for example: hybridization in 4 x SSC at 65 °C, and subsequent repeated washing in 0.1 x SSC at 65 °C for approximately 1 hour in total. A hybridization preferably occurs under stringent conditions.

Sequences showing a certain identity level to a starting sequence may show the given identity level over the whole length of the starting sequence. This is applicable towards nucleotide sequences as well as amino acid sequences.

In relation to a nucleic acid in the form of a double-stranded DNA, "complementary" nucleotide sequence means that the second DNA strand complementary to the first DNA strand has the nucleotides that correspond to the bases of the first strand, in accordance with the base pairing rules. A complementary sequence is, preferably, entirely complementary to the counter-sequence, and thus preferably has the same length.

What is understood by an "isolated nucleic acid molecule" is a nucleic acid molecule extracted from its natural or original environment. The term also encompasses a synthetically-produced nucleic acid molecule. What is understood by an "isolated polypeptide" is a polypeptide extracted from its natural or original environment. The term also encompasses a synthetically-produced polypeptide.

A "molecular marker" or "marker" is a nucleic acid that is polymorphic in a plant population and is used as a reference or orientation point. Depending on the technical context the term "marker" may be related to a specific genomic position which is detectable by a corresponding "molecular marker" wherein the "molecular marker" in most cases is sequentially compatible to the genomic position. A marker for the detection of a recombination event should be suitable for monitoring differences or polymorphisms within a plant population. Such a marker is thus able to detect and differentiate between various allelic states (alleles). The term, "molecular marker," also relates to nucleotide sequences which are complementary or at least largely complementary or homologous to genomic sequences - for example, nucleic acids which are used as probes or primers. These differences at the DNA level are to be found as markers and are, for example, polynucleotide sequence differences, e.g., SSR's (*simple sequence repeats*), RFLP's (*restriction fragment length polymorphisms*), FLP's (*fragment length polymorphisms*) or SNP's (*single nucleotide polymorphisms*). The markers may be derived from genomic or expressed nucleic acids, e.g., spliced RNA, cDNA, or EST's, and may also relate to nucleic acids that are used as probes or primer pairs and as such are suitable for amplifying a sequence fragment using PCR-based methods. Markers that describe genetic polymorphisms (between parts of a population) may be detected using well-established methods from the prior art (An Introduction to Genetic Analysis, 7th edition, Griffiths, Miller, Suzuki, et al., 2000). For example, among these are DNA sequencing, PCR-based, sequence-specific amplification, verification of RFLP's, verification of polynucleotide polymorphisms by means of allele-specific hybridization (ASH), detection of amplified variable sequences of the plant genome, detection of a 3 SR (*self-sustained sequence replication*), detection of SSR's, SNP's, RFLP's, or AFLP's (*amplified fragment length polymorphisms*). Furthermore, the methods for detection of EST's (*expressed sequence tags*) and SSR markers derived from EST sequences and RAPD (*randomly amplified polymorphic DNA*) are also known. Depending upon the context, the term, "marker," in the description may also mean a specific chromosome position in the genome of a species where a specific marker (SNP, for example) may be found.
Markers also include synthetic oligonucleotides that may be connected with one or more detection molecules, wherein the detection molecules may be used for a detection reaction or the generation of a signal within the scope of a verification method. Synthetic oligonucleotides also include labeled primers. Synthetic oligonucleotides and labeled primers are artificial compounds, do not occur in nature, and cannot be isolated from nature. The production of such compounds is explained further below.

A "single nucleotide polymorphism" or SNP is a genetic variation between two samples of DNA wherein at least one nucleotide between the two samples is different. In most cases the samples belong two the same species and the comparison or alignment of the two samples is performed on the basis of homologues genomic regions. SNPs may cause allelic variations but not all SNPs need to occur within a functional genomic element like a gene. SNPs can be used two differentiate between for example different genotypes / haplotypes or may be used to screen and select for the presence of absence of a functional genomic element like for example a specific gene or its allelic variant. Due to genetic linkage SNPs need not to be within the functional genomic element which is to be selected. Examples for SNPs in the sense of the invention are given in Tab. 1b, especially they are included in the common primer sequences. The detection or identification of SNPs may occur by a PCR involving two different forward primers and one common revers primer. The technical details for such a detection or identification may be derived from passages [67] - [70] above.

A "promoter" is a non-translated, regulatory DNA sequence, typically upstream of a coding region, which contains the binding point for the RNA polymerase and initiates the transcription of the DNA. A promoter additionally contains other elements that act as a regulator gene for gene expression (for example, cis-regulatory elements). A "core or minimal promoter" is a promoter that has the basic elements which are needed for transcription initiation (for example, TATA box and/or initiator).

A "pathogen" means an organism that, in interactions with a plant, leads to disease symptoms in one or more organs in the plant. For example, animal, fungal, bacterial, or viral organisms or oomycetes number among these pathogens.

What is to be understood by a "pathogenic infection" is the earliest point in time at which a pathogen interacts with a plant host tissue. In this sense, "infestation" means the occurrence of contact between pathogen and host. With an anchorage of a pathogen at a host, e.g., of a fungal spore on a leaf surface of a plant, mechanisms of pathogen detection and signal relaying begin in the plant host cell. In the case of *Cercospora beticola,* conidia are formed in humid, warm weather and transferred to neighboring plants by rain and wind. New infections most often show individual leaf spots first at the physiologically older outer leaves. These are most often quite clearly delimited from the healthy leaf tissue by a brown ring. The brown conidia carriers of the fungus in the middle part of the spots may be observed with the aid of a magnifying glass (rating score 3). The number of these brown spots increases rapidly, wherein the sporocarps initially overlap even smaller dead areas (rating score 5). In the further course of the disease, which now also spans to the inner leaves, dying-off of the outer leaves finally occurs for the first time (rating score 7), and, then, of practically all leaves (rating score 9). Course of disease and symptom severity are strongly dependent upon the site and on the annually fluctuating weather conditions.

Plant "organs" means, for example, leaves, shoot, stem, roots, hypocotyl, vegetative buds, meristems, embryos, anthers, ovula, seeds, or fruits. "Plant parts" include, but are not limited to, the shoot or the stalk, leaves, blossoms, inflorescence, roots, fruits, and seeds, as well as the pollen. The term, "plant parts," also means an association of multiple organs, e.g., a blossom or a seed, or a part of an organ, e.g., a cross-section through the plant shoot. Plant "tissues" are, for example, callus tissue, storage tissue, meristematic tissue, leaf tissue, shoot tissue, root tissue, plant tumor tissue, or reproductive tissue, as well as the cambium, parenchyma, vascular tissue, sclerenchyma, and epidermis. However, the tissue is not limited to this listing. For example, what are to be understood by plant "cells" are, for example, isolated cells having a cell wall or aggregates thereof, or protoplasts.

"Recombination frequency" means that between some genomic elements like single nucleotide polymorphisms, genes, loci, QTL or genetic regions a crossing over occurs during meiosis. A recombination frequency of 1% is equal to 1cM. It is possible to determine the recombination frequency by the generation of meiosis maps.

"Resistance gene according to the invention" is a nucleotide molecule or nucleic acid sequence as described under passage [1] above. The gene may be genetically linked to cosegregating regions.

"Variety" means a plant grouping within a single botanical taxon of the lowest known rank, which grouping, irrespective of whether the conditions for the grant of a breeder's right are fully met, can be - defined by the expression of the characteristics resulting from a given genotype or combination of genotypes,
- distinguished from any other plant grouping by the expression of at least one of the said characteristics and
- considered as a unit with regard to its suitability for being propagated unchanged.

In conjunction with the present invention, the term, "regulatory sequence," relates to a nucleotide sequence which influences the specificity and/or the expression strength, e.g., in that the regulatory sequence confers a defined tissue specificity. Such a regulatory sequence may be located upstream of the transcription initiation point of a minimal promoter, but also downstream thereof, e.g., in a transcribed, but not translated, leader sequence or within an intron.

The term, "resistance," is to be understood broadly and covers the range of the protection from a retardation up to a complete blocking of the development of the disease. One example of an important pathogen is *Cercospora beticola.* A resistant plant cell of the invention or resistant plant of the invention preferably achieves a resistance to *Cercospora beticola.* A resistance to a pathogen is to be equated to a resistance to the disease which this pathogen causes; for example, a resistance to *Cercospora beticola* is also a resistance to leaf spot disease. For example, an increase in the resistance can be measured via a reduced fungal biomass on the host plant; for this, the fungal DNA may be determined with the aid of quantitative PCR in comparison to the plant DNA in the infested plant tissue. An additional approach to the measurement of resistance is optical rating, wherein rating scores of 1 (not susceptible) to 9 (very susceptible) are awarded.

"Transgenic plant" relates to a plant into whose genome is integrated at least one polynucleotide. It may thereby be a heterologous polynucleotide. The polynucleotide is, preferably, stably integrated, which means that the integrated polynucleotide is stably preserved in the plant, is expressed, and also may be stably passed on to the descendants. The stable introduction of a polynucleotide into the genome of a plant also includes the integration into the genome of a plant of the preceding parental generation, wherein the polynucleotide may be stably passed on further. The term, "heterologous," means that the introduced polynucleotide originates from a cell or an organism with a different genetic background, of the same species or a different species, or is homologous to the prokaryotic or eukaryotic host cell, for example, but then is located in a different genetic environment and thus differs from a corresponding polynucleotide that is possibly naturally present. A heterologous polynucleotide may be present in addition to a corresponding endogenous gene.

"Raw material for industrial sugar production" means plant material which can be fed into a sugar production facility which is specialized in the extraction of sugar from sugar beets. Such raw material is typically the beet body (taproot) of the harvested sugar beet. To ensure the conformity with the extraction process the beet body needs to have sufficient mass, volume and a conical shape so that the raw material can be mechanically cut into shreds (beet strips). These beet strips maximize the surface area for sugar extraction and should have a low content of Sodium, Potassium and Nitrogen to allow an efficient extraction. After the extraction remaining beet pulp is pressed, dried and used as animal feed.

"Saccharose concentration" is expressed as percentage of the fresh weight of the root.

"Monogerm" means that a seed grows into exactly one plant whereas a polygerm or multigerm seed (also called "seed ball") grows into several plants.

"Bolting" is the production of a flowering stem (or stems) on a sugar beet in a natural attempt to produce seeds and reproduce. Bolting is triggered in sugar beet due to vernalization, i.e. a chilling stress which might occur e.g. during overwintering. However, commercially grown sugar beets are harvested before bolting as the bolting process and subsequent seed setting reduces the saccharose content in the beet body.

"Introgression" means that a nucleotide sequence has been transferred into the genome of a plant wherein this nucleotide sequence originates from a plant that does not belong to the same species or subspecies. This can for example mean that a nucleotide sequence deriving from a plant of the subspecies *Beta vulgaris maritima* has been transferred into a plant of the subspecies *Beta vulgaris vulgaris.*

Designs and embodiments of the present invention are described by way of example with reference to the pending sequences and figures.
- **Fig. 1:**: Protein sequence alignment between the resistant protein (protein which confers Cercospora resistance in a plant) and the sensitive protein (protein which does not confer Cercospora resistance in a plant). The polymorphisms are highlighted in gray.
- **Fig. 2:**: Genetic (left part) an physical (right part) map of the genomic region including the resistance gene showing the positions of some most important markers.
- **Fig. 3:**: Vector map of the vector pZFN-nptII including the LRR region.
- **Fig. 4:**: Statistical box-plot evaluation of the data generated eight days post infection during the transgenic verification of the resistance gene.
- **Fig. 5:**: Statistical box-plot evaluation of the data generated eleven days post infection during the transgenic verification of the resistance gene.
- **Fig. 6:**: Statistical box-plot evaluation of the data generated eight days post infection during the transgenic verification of the resistance gene.
- **Fig. 7:**: Statistical box-plot evaluation of the data generated fifteen days post infection during the transgenic verification of the resistance gene.

### DETAILED DESCRIPTION OF THE INVENTION

The present invention relates to a nucleic acid molecule that is able to confer a resistance to Cercospora in a plant - in particular, in *Beta vulgaris* subsp. *vulgaris* - in which the polypeptide which is encoded by the nucleic acid molecule is expressed. According to a preferred embodiment of the invention, the pathogen is the fungus *Cercospora beticola,* which is among the most important and destructive leaf pathogens of sugar beets, beetroot, and chard, among others, and may cause crop losses of over 40%. The fungus produces the secondary metabolite cercosporin, which reacts with oxygen in the presence of light and leads to the formation of reactive oxygen species (ROS). The ROS cause massive cell damage in the leaf tissue of the infested plant that visible in the form of necroses.

The present invention is based upon the genetic fine mapping, identification, isolation, and characterization of a gene or of a gene locus that originates from the donor *Beta vulgaris* subsp. *maritima,* whose presence in a plant - in particular, in *Beta vulgaris* subsp. *vulgaris* - correlates with or is causative of the resistance of the plant concerned to Cercospora leaf spot disease. Initial material was a *Beta vulgaris* subsp. *maritima* population which was developed from 37 *Beta vulgaris* subsp. *maritima* accessions from different sources. Seeds of a *Beta vulgaris* subsp. *vulgaris* plant (sugar beet cultivar 6626) comprising the resistance according to the invention have been deposited before the filing with the National Collection of Industrial Food and Marine Bacteria (NCIMB), Aberdeen, UK under access number NCIMB 43646. Especially a plant of the genus *Beta,* a plant of the species *Beta vulgaris* or a plant of the subspecies Beta vulgaris subsp. vulgaris or a sugar beet plant comprising the resistance gene according to the invention may be derived from the deposited seeds.

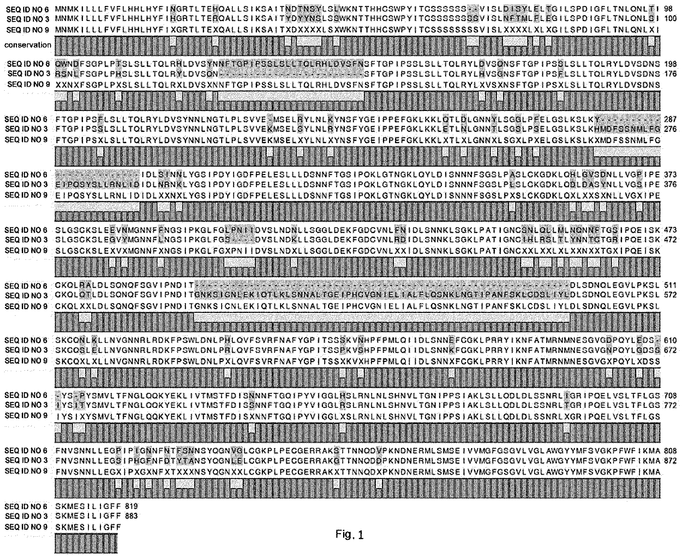

The nucleotide and amino acid encoding sequence of the nucleic acid molecule according to the invention is characterized by numerous polymorphisms, which differentiates the NPS-LRR gene identified according to the invention from the "sensitive" variant of the gene, i.e., the variant of the gene that does not confer resistance to Cercospora. Examples of polymorphisms are presented in Fig. 1.

The nucleic acid molecule according to the invention may be an isolated nucleic acid molecule. It is preferably DNA, and, particularly preferably, cDNA (coding DNA). The polypeptide which is encoded by the nucleic acid molecule according to the invention preferably confers a resistance to the pathogen *Cercospora beticola,* which causes the plant disease Cercospora leaf spot. Furthermore, the polypeptide which is encoded by the nucleic acid molecule according to the invention confers - in particular, in a plant of the genus Beta - a resistance to this pathogen. The plant is preferably a plant of the species *Beta vulgaris* - particularly preferably, a plant of the subspecies *Beta vulgaris* subsp. *vulgaris;* among these are, for example, the cultivars sugar beet, beetroot, fodder beet, chard, and Swiss chard.
In one embodiment of the present invention, the nucleic acid molecule according to the invention comprises a nucleotide sequence that encodes a polypeptide with an amino acid sequence according to SEQ ID No. 3 and/or the coding DNA sequence according to SEQ ID No. 2. Furthermore, the present invention provides a nucleotide sequence that comprises the DNA sequences according to SEQ ID No. 1 and SEQ ID No. 53.

The gene identified according to the invention is a resistance gene/protein of the type NBS-LRR, which is characterized by specific structural motifs. The general structure of such resistance proteins in plants has already been well-examined (Martin et al., Annual Review Plant Biology 54 (2003), 23-61). However, the principle of the structural embodiment - in particular, of what is known as the LRR domain, which applies as a potential detection domain for most unknown pathogenic effectors - is unpredictable, and the functional background of the resistance genes i.e., the genetic structure, is generally largely unknown. The identification of a Cercospora resistance-conferring gene or protein solely on the basis of the known structural motif is, consequently, impossible. Furthermore, the sequence region has turned out to be a highly repetitive region that contains, among other things, *tandem repeats* with very high sequence homology, which makes the development of diagnostic markers, as well as the assembly of sequence data, especially difficult.

With the aid of the setup of a population of over 4,000 dividing descendants and the development of special recombination screens, the target region was reduced, and thus ever further isolated, via analysis of informative recombinants (genotypical and phenotypical) in a series of resistance tests. This genetic mapping, as well as the creation of physical maps accompanied by WHG sequencing ("whole genome sequencing"), comparative BAC (Bac-by-Bac) sequencing, and bioinformatic analyses, led to the identification of three recombinant genotypes that confirmed the resistance gene (1 recombinant in the neighboring gene, on the one hand, and 2 recombinants in the neighboring gene, on the other). In light of particular requirements, the inventors placed the highly repetitive structure in the target region, which, among other things, contains *tandem repeats* with very high sequence homology, which made the marker development, and thus the identification of informative recombinants, enormously more difficult. The following steps were particularly decisive for the location of the genetic structure of the resistance gene:
- development of the markers s4p0264s01, s4p2271s01, sxh0678s01, s4p4293s01, s4p4295s01, s4p4301s01 (see Table 1B).
- Fine mapping coupled with intensive phenotyping. The phenotypes were verified with 90-180 descendants per plant in a greenhouse test, and with intensive statistical methods (for example, t-test, power analysis, etc.).
- BAC clone identification and sequencing from BAC pools of the resistant genotype.
- Sequence evaluation, as well as sequence and protein comparison between RR (i.e., resistant) and ss (i.e., sensitive) genotypes; an unambiguous assembly of the RR and ss sequence data was thereby not always possible, due to the sequence complexity.

**Table 1B: Marker in the target region relating to sensitive genotype, resistant genotype and consensus sequence.**

| **Marker** | **Sequences: sensitive / resistant / consensus** | **Position on genetic map [cM]** | **Position on physical map [bp]** |
|---|---|---|---|
| s4p1395s01 | SEQ ID No. 100 / SEQ ID No. 101 / SEQ ID No. 102 | 56,35582927 | 55312395 |
| sxi0123s02 | SEQ ID NO. 103 / SEQ ID NO. 104 / SEQ ID NO. 105 | 56,47317509 | 55357303 |
| s4p1396s01 | SEQ ID NO. 106/ SEQ ID NO. 107 / SEQ ID NO. 108 | 56,61163027 | 55409550 |
| s4p1398s01 | SEQ ID NO. 109 / SEQ ID NO. 110 / SEQ ID NO. 111 | 56,78339709 | 55473271 |
| s4p1462s01 | SEQ ID NO. 112 / SEQ ID NO. 113 / SEQ ID NO. 114 | 56,84738427 | 55496705 |
| s4p1464s01 | SEQ ID NO. 115 / SEQ ID NO. 116 / SEQ ID NO. 117 | 56,87553455 | 55506972 |
| s4p0044s01 | SEQ ID NO. 118 / SEQ ID NO. 119 / SEQ ID NO. 120 | 58,84432555 | 56160463 |
| s4p0056s01 | SEQ ID NO. 121 / SEQ ID NO. 122 / SEQ ID NO. 123 | 59,85754673 | 56456734 |
| s4p0058s01 | SEQ ID NO. 124 / SEQ ID NO. 125 / SEQ ID NO. 126 | 60,00588436 | 56498250 |
| s4p0059s01 | SEQ ID NO. 127 / SEQ ID NO. 128 / SEQ ID NO. 129 | 60,00633209 | 56498372 |
| s4p1564s01 | SEQ ID NO. 130 / SEQ ID NO. 131 / SEQ ID NO. 132 | 60,00815936 | 56498883 |
| s4p1998s01 | SEQ ID NO. 133 / SEQ ID NO. 134 / SEQ ID NO. 135 | 60,02385255 | 56503259 |
| s4p1343s01 | SEQ ID NO. 136 / SEQ ID NO. 137 / SEQ ID NO. 138 | 60,42647909 | 56613667 |
| s4p1408s01 | SEQ ID NO. 139 / SEQ ID NO. 140 / SEQ ID NO. 141 | 60,42651091 | 56613673 |
| s4p1565s01 | SEQ ID NO. 142 / SEQ ID NO. 143 / SEQ ID NO. 144 | 60,54596773 | 56645847 |
| s4p1409s01 | SEQ ID NO. 145 / SEQ ID NO. 146 / SEQ ID NO. 147 | 60,547695 | 56646306 |
| s4p1411s01 | SEQ ID NO. 148 / SEQ ID NO. 149 / SEQ ID NO. 150 | 60,86562555 | 56730683 |
| s4p1414s01 | SEQ ID NO. 151 / SEQ ID NO. 152 / SEQ ID NO. 153 | 60,99547782 | 56764641 |
| s4p1485s01 | SEQ ID NO. 154 / SEQ ID NO. 155 / SEQ ID NO. 156 | 61,54654291 | 56905647 |
| s4p0257s01 | SEQ ID NO. 157/ SEQ ID NO. 158/ SEQ ID NO. 159 | 62,42235182 | 57120242 |
| s4p0258s01 | SEQ ID NO. 160 / SEQ ID NO. 161 / SEQ ID NO. 162 | 62,42346318 | 57120506 |
| s4p0260s01 | SEQ ID NO. 163 / SEQ ID NO. 164 / SEQ ID NO. 165 | 62,59136591 | 57160401 |
| sxh0876s05 | SEQ ID NO. 166 / SEQ ID NO. 167 / SEQ ID NO. 168 | 62,730303 | 57193142 |
| s4p0262s01 | SEQ ID NO. 169 / SEQ ID NO. 170 / SEQ ID NO. 171 | 62,79537191 | 57208387 |
| s4p0263s01 | SEQ ID NO. 172 / SEQ ID NO. 173 / SEQ ID NO. 174 | 62,79557191 | 57208429 |
| s4p0264s01 | SEQ ID No. 54 / SEQ ID No. 55 / SEQ ID No. 10 | 62,79590373 | 57208510 |
| s4p2271s01 | SEQ ID No. 56 / SEQ ID No. 57 / SEQ ID No. 11 | 62,81185523 | 57212240 |
| s4p4288s01 | SEQ ID NO. 175 / SEQ ID NO. 176 / SEQ ID NO. 177 | 62,81950148 | 57214020 |
| s4p4290d01 | SEQ ID NO. 178 / SEQ ID NO. 179 / SEQ ID NO. 180 | 62,83393842 | 57217392 |
| s4p4293s01 | SEQ ID No. 58 / SEQ ID No. 59 / SEQ ID No. 12 | 62,84491806 | 57219956 |
| s4p4295s01 | SEQ ID No. 60 / SEQ ID No. 61 / SEQ ID No. 13 | 62,85399055 | 57222060 |
| s4p8772s01 | SEQ ID NO. 181 / SEQ ID NO. 182 / SEQ ID NO. 183 | | |
| s4p4301s01 | SEQ ID No. 62 / SEQ ID No. 63 / SEQ ID No. 14 | 62,94635089 | 57243521 |
| sxh0678s01 | SEQ ID No. 64 / SEQ ID No. 65 / SEQ ID No. 15 | 62,97474964 | 57250119 |
| s4p4305d01 | SEQ ID NO. 184 / SEQ ID NO. 185 / SEQ ID NO. 186 | 62,98035341 | 57251418 |
| s4p4308s01 | SEQ ID NO. 187 / SEQ ID NO. 188 / SEQ ID NO. 189 | 63,00151327 | 57256313 |
| s4p0317s01 | SEQ ID NO. 190 / SEQ ID NO. 191 / SEQ ID NO. 192 | 63,16585209 | 57294143 |
| sxh1834s05 | SEQ ID NO. 193 / SEQ ID NO. 194 / SEQ ID NO. 195 | 63,26778918 | 57317441 |
| sxh0942s04 | SEQ ID NO. 196 / SEQ ID NO. 197 / SEQ ID NO. 198 | 63,31312755 | 57327765 |
| s4p0320s01 | SEQ ID NO. 199 / SEQ ID NO. 200 / SEQ ID NO. 201 | 63,49891636 | 57369798 |
| s4p0322s01 | SEQ ID NO. 202 / SEQ ID NO. 203 / SEQ ID NO. 204 | 63,52934236 | 57376639 |
| s4p0323s01 | SEQ ID NO. 205 / SEQ ID NO. 206 / SEQ ID NO. 207 | 63,79248973 | 57435390 |
| s4p0232s01 | SEQ ID NO. 208 / SEQ ID NO. 209 / SEQ ID NO. 210 | 64,19409409 | 57523548 |
| s4p0234s01 | SEQ ID NO. 211 / SEQ ID NO. 212 / SEQ ID NO. 213 | 64,32903091 | 57552769 |
| sxh1195s02 | SEQ ID NO. 214 / SEQ ID NO. 215 / SEQ ID NO. 216 | 64,32903091 | 57552771 |
| s4p0238s01 | SEQ ID NO. 217/ SEQ ID NO. 218 / SEQ ID NO. 219 | 64,36171473 | 57559821 |
| s4p0421s01 | SEQ ID NO. 220/ SEQ ID NO. 221 / SEQ ID NO. 222 | 65,86730118 | 57873806 |

The compounds provided in Table 1B can be used as molecular markers according to the invention. All markers are suitable to detect genetic material comprising the resistance gene according to the invention (especially by the identification of genetically linked polymorphisms like single nucleotide polymorphisms) and to trace the inheritance of the genetic material from a source plant to the offspring of the source plant via marker assisted selection (MAS). In this regard the markers disclosed herein can be highly useful in transmitting the resistance gene according to the invention from one subspecies to a different subspecies. The markers disclosed herein can be furthermore used for genetic mapping and the tracing of recombination as a consequence meiosis. It seems that the resistance gene is located close to or in between the physical positions 57219956bp and 57243521bp. Markers disclosed herein which are located very close to this region are highly suitable for the detection of the resistance gene. Markers disclosed herein which are in larger distance to the resistance gene still show a strong genetic linkage to the resistance gene but can furthermore be used to detect rare recombination events and genetic exchange in the flanking areas of the resistance gene. This can be useful for example in backcrossing approaches and the transmittance of the resistance gene into a different genetic background. The markers disclosed herein and especially those which are in larger distance to the resistance gene are especially useful when the resistance gene is transmitted to the offspring as part of a cosegregating region. The resistance gene including cosegregating regions may also be transmitted from one subspecies to a different subspecies. The successful transmittance can be confirmed by the markers disclosed herein for example by the detection of the resistance gene or the cosegregating region. The markers disclosed herein can also be used to reduce the cosegregating regions in a backcrossing program involving several generations while keeping the resistance gene according to the invention in the offspring. Further markers for the detection of the resistance gene according to the invention can be deduced from the data disclosed herein. Especially those markers can be deduced which are located in a genomic interval that is flanked by markers s4p0421s01 and s4p1395s01 or which are flanked by those marker pairs which are given in passage [48] above. The deduction of further markers is facilitated by the genomic material comprised by the seeds which have been deposited with the NCIMB, Aberdeen, UK under access number NCIMB 43646. This genomic material allows for example the alignment of genetic material encoding the resistance according to the invention with genetic material lacking the resistance gene according to the invention. The genetic material lacking the resistance may be homologues or of the same physical position (compare Tab. 1b) as the genetic material encoding the resistance according to the invention. Those alignments may be used for the identification of polymorphisms like single nucleotide polymorphisms. On the basis of polymorphisms additional markers can be created for the detection of the identified polymorphisms, especially for those polymorphisms which are genetically linked to the resistance gene according to the invention. Alignments and detection of polymorphisms on the basis of alignment usually involve the use of computers and the storage of data on computer readable media. It is also suggested to convert the signal produced by molecular markers during the analysis of genetic material into electronically transmittable and / or electronically storable data which may be processed by the help of a computer. Such computer-based process may involve the determination or calculation of the estimated breeding value of a plant. The estimated breeding value may encompass the level of resistance or tolerance towards Cercospora. More information on the estimation of breeding values may be derived from US8321147B2.

Analyses yielded that the LRR gene has a moderate protein homology to the Cf-2 resistance protein from the tomato (UNIPROT|Q41397_SOLPIP. Cf-2.1) (sequence identity 322/830=38%). In fact, the identified Cercospora resistance-conferring protein is the best sugar beet protein homolog to the Cf-2 tomato resistance protein. The Cf-2 resistance protein from the tomato confers a resistance to *Cladosporium fulvum* - a type of black mold fungus (US 6,287,865 B1) - via interaction with the avirulence protein Avr2 from *C. fulvum.* This leads to the activation of the plant immune defense against the pathogen; see Dixon *et al.,* 1996 (Dixon, Mark S., et al., "The tomato Cf-2 disease resistance locus comprises two functional genes encoding leucine-rich repeat proteins." Cell 84.3 (1996): 451-459). Due to the sequence homology between the Cf-2 gene and the identified LRR gene, it is to be assumed - but without thereby being bound to one theory - that a similar defense mechanism forming the basis of Cercospora resistance also occurs in the case of the sugar beet. However, a different mechanism is not to be precluded, due to the moderate sequence homology.

Furthermore, substitutions, deletions, insertions, additions, and/or any other change may be introduced into the nucleotide sequence according to the invention that, alone or in combinations, do in fact change the nucleotide sequence, wherein the modified nucleotide sequence may, however, perform the same function as the initial sequence. The present case deals with the coding of an amino acid sequence which confers resistance to Cercospora leaf spot disease. In a further embodiment, the invention therefore includes a nucleotide sequence that encodes a polypeptide which represents a derivative of the polypeptide which is encoded by the nucleotide sequence according to the invention, or which includes the amino acid sequence according to the invention. A derived amino acid sequence which has at least one substitution, deletion, insertion, or addition of one or more amino acids, wherein the functionality of the encoded polypeptide/protein is preserved, represents a derivative of the polypeptide. Substitutions, deletions, insertions, additions, and/or any other change, either solely or in combinations, that do in fact change the nucleotide sequence, but perform the same function as the initial sequence, may thereby be introduced into the nucleotide sequence using conventional methods that are known in the prior art, e.g., via site-directed mutagenesis, PCR-mediated mutagenesis, transposon mutagenesis, genome editing, etc.

The substitution of one amino acid by a different amino acid having the same or equivalent or similar chemical/physical properties is referred to as a "conservative substitution" or "semi-conservative substitution." Examples of physical/chemical properties of an amino acid are, for example, hydrophobia or the charge. Which amino acid substitution represents a conservative or semi-conservative substitution is known to the person skilled in the art. Moreover, general expertise allows the person skilled in the art to recognize, identify, and detect which amino acid deletions and additions are harmless to the functionality of the resistance protein, and at which positions these are possible. The person skilled in the art is aware that, in the case of the present NBS-LRR protein for modifications of the amino acid sequence (substitutions, deletions, insertion, or additions of one or more amino acids), the functionality, in particular, of the conserved domains must be preserved, and that therefore only limited preceding modifications are possible in these domains.

The invention thus includes a functional fragment of the nucleotide sequence according to the invention. The term, "fragment," thereby includes genes with a nucleotide sequence sufficiently similar to the aforementioned nucleotide sequence. The term, "sufficiently similar," means that a first nucleotide sequence or amino acid sequence has a sufficient or minimum number of identical or equivalent nucleotides or amino acid groups relative to a second nucleotide sequence or a second amino acid sequence.

With regard to the amino acid sequence, after modification via an aforementioned method, this also has a common structural domain and/or possesses common functional activity. Nucleotide sequences or amino acid sequences that have an identity of at least approximately 70%, at least approximately 75%, at least approximately 80%, at least approximately 85%, at least approximately 90%, at least approximately 91%, at least approximately 92%, at least approximately 93%, at least approximately 94%, at least approximately 95%, at least approximately 96%, at least approximately 97%, at least approximately 98%, at least approximately 99%, or at least approximately 100% with the nucleotide sequence or amino acid sequence according to the invention are defined here as being sufficiently similar. This also explicitly encompasses the range of 90% to 100%. For the functional fragments, a sufficient similarity is established if the nucleotide sequence or amino acid sequence generally has the same property as the previously-named nucleotide sequence or amino acid sequence of the present invention. Those nucleotide sequences which encode a derivative or for a derived amino acid sequence are generated either directly or indirectly (for example, via amplification or replication steps) from an initial nucleotide sequence which corresponds to the nucleotide sequence according to the invention over the entire length, or at least in part.

Accordingly, the present invention includes a nucleotide sequence that is able to hybridize, under stringent conditions, with a nucleotide sequence complementary to a nucleotide sequence according to the invention or to the nucleotide sequence that encodes the amino acid sequence according to the invention.

In a further embodiment, the nucleic acid molecule according to the invention is characterized in that, after expression in a plant, it already, on its own, confers a dominant resistance effect against a pathogen - preferably, against *Cercospora beticola* - or that it encodes for a polypeptide that is able to confer a dominant resistance effect against Cercospora. In a preferred embodiment, the nucleic acid molecule or the polypeptide confers a resistance effect of at least one rating score-preferably, of at least two rating scores, and, particularly preferably, of three to four rating scores. Such a gene that already, on its own, confers such a strongly pronounced resistance to Cercospora in a plant, or that encodes a polypeptide that is able to confer such a pronounced resistance, is not known from the prior art. As was already described above, in previously available varieties on the market, the Cercospora resistance is transmitted via many resistance genes having little effect, and a disadvantage of such varieties is that their development is very slow and expensive due to the complicated transmission, and that such varieties have a markedly poorer crop yield relative to normal varieties, in the absence of an infestation. Among other things, this may be linked to the epigenetic interaction of some resistance genes with genes that are responsible for sugar production, which leads to reduced fitness of the plants, in the absence of the pathogen.

The inventors could thus for the first time provide a Cercospora resistance gene that may be used for markedly simplified breeding. Via the incorporation of this gene in elite lines, it is now possible to very quickly develop very high-yield varieties with a high Cercospora resistance. Accordingly, in the framework of the present invention there are provided for the first time a sugar beet plant, a chard plant, a red beet or beetroot plant, a fodder beet plant having the resistance according to the invention against *Cercospora beticola* and thus being encompassed by the present invention. As the listed plants are all cultivated plants, crops or plants which are suitable for the agricultural cultivation and which have the resistance according to the invention, are part of the invention. Especially such crops are part of the invention which comprise a subterrestrial storage organ usable as food, raw material or industrial source of sugar and which comprise the resistance according to the invention are a further aspect of the present invention. The storage organ can be for example the sugar containing beet body of the sugar beet, the consumable beet body of the red beet or the feedable beet body of the fodder beet. The subterrestrial storage organ can sum up to more than 50% and for the sugar beet even to more than 70% of the total mass of the full-grown plant. Furthermore, also seeds or seeding material of these plants are part of the invention. The seeds or the seeding material can be technically treated as described further below. Part of the invention are also plants of the genus *Spinacia, Glycine, Daucus* and *Pastinaca* comprising the resistance gene according to the invention. Especially plants of the species *Spinacia oleracea* and their varieties comprising the resistance gene according to the invention are included. The storage organ may be a taproot, especially a beet body.

The resistance gene according to the invention may be under control or may be operably linked to a promoter according to SEQ ID No. 7. The resistance gene may be also under control or may be operably linked to a nucleic acid molecule comprising a promoter according to SEQ ID No. 7. Sequences having 90%, 95% or 99% sequence identity over the whole length of the sequence to SEQ ID No. 7 are encompassed in this respect.

In this context, the invention also includes a nucleic acid that encodes the protein according to SEQ ID No. 3, wherein, in a specific embodiment, the nucleic acid according to SEQ ID No. 1 is excluded.

Furthermore, the present invention relates to a recombinant and/or heterologous DNA molecule that comprises the sequences of the nucleic acid molecule according to the invention. This DNA molecule, furthermore, preferably has a regulatory sequence. It may thereby be operatively linked with this regulatory sequence or be under the influence of this regulatory sequence. This regulatory sequence is preferably a promoter sequence and/or other sequences of transcription or translation control elements - for example, cis-elements. The regulatory sequence, which controls the expression of a gene that includes the nucleic acid molecule according to the invention, is preferably a sequence that is able to confer or modulate the expression, as a result of a pathogenic infection. This promoter is preferably able to control the expression of the DNA sequence specifically in leaves of the plant. The regulatory sequence may be heterologous to the expressing sequence. Such an approach has the advantage that the person skilled in the art may better adjust the expression rate of the expressing sequence, the tissue in which the expression occurs, and the point in time at which the expression occurs, in that he selects that regulatory sequence which is best suited to the respective use case. The heterologous DNA sequence preferably includes a nucleotide sequence which encodes a component of the plant pathogen defense (example: resistance genes (R-genes) or genes which encode enzymes involved in signal transfer, such as kinases or phosphatases, and for G-protein, or which encode a pathogenic effector (what are known as avirulence genes *(avr))).* The heterologous DNA sequence may be one of the DNA sequences according to the invention. The heterologous DNA sequence may also additionally encode further components of the plant pathogen defense. The heterologous DNA sequence may therefore be designed such that a polycistronic mRNA is created after its transcription.

The present invention furthermore also relates to a polypeptide which can be encoded by the nucleic acid molecule according to the invention and a functionally and/or immunologically active fragment thereof, as well as an antibody that specifically binds to the polypeptide or to its fragment. The polypeptide particularly preferably has an amino acid sequence according to SEQ ID No. 3. The recombinant production of proteins, polypeptides, and fragments is familiar to the person skilled in the art (Sambrook et al., Molecular Cloning: A Laboratory Manual, 3rd ed., Cold Spring Harbor Laboratory Press, Cold Spring Harbor, NY, 2001, or Wingfield, P. T., 2008, Production of Recombinant Proteins, Current Protocols in Protein Science, 52:5.0:5.0.1-5.0.4). Polyclonal or monoclonal antibodies to the protein according to the invention may be produced by the person skilled in the art according to known methods (E. Harlow et al., editor, Antibodies: A Laboratory Manual (1988)). The production of monoclonal antibodies, as well as of Fab and F(ab')₂ fragments that are also useful in protein detection methods, may be performed via various conventional methods (Goding, Monoclonal Antibodies: Principles and Practice, pp. 98-118, New York: Academic Press (1983)). The antibodies may then be used for the screening of expression cDNA libraries in order to identify identical, homologous, or heterologous genes by means of immunological screening (Sambrook et al., Molecular Cloning: A Laboratory Manual, Cold Spring Harbor Laboratory Press, Cold Spring Harbor, NY, 1989, or Ausubel et al., 1994, "Current Protocols in Molecular Biology." John Wiley & Sons), or may be used for western blot analyses. In particular, the present invention relates to antibodies that selectively detect a polypeptide encoded by the Cercospora resistance-conferring allele according to the invention, and essentially do not detect the polypeptide encoded by the correspondingly sensitive allele, i.e., that they detect less, by a factor of 2 - preferably, a factor of 5, and, more preferably, a factor or 10 or more - of the polypeptide encoded by the correspondingly sensitive allele than the polypeptide encoded by the Cercospora resistance-conferring allele according to the invention.

In a preferred embodiment, the antibody according to the invention is characterized in that it is a synthetic polypeptide which does not occur in nature.

Furthermore, the antibodies according to the invention may be linked with a fluorescent dye in order to be usable in an immunohistochemical method, for example, and evoke an antibody coloration. The fluorescent dye may be fluorochrome. The antibodies according to the invention may also be present linked with other signaling molecules. Among these are, for example, biotin, radioisotopes, reporter enzymes such as alkaline phosphatase, or oligonucleotides.

An additional subject matter of the invention is vectors or expression cassettes that include the nucleic acid molecule or the recombinant DNA molecule according to the invention - possibly under control of regulatory elements and, in particular, under control of functional regulatory elements in plants, as well as negative and/or positive selection markers. The vector backbone is thereby heterologous to the nucleic acid molecule according to the invention, which means that such a vector does not occur in nature and cannot be isolated from nature. The vector is a plasmid, a cosmid, a phage or an expression vector, a transformation vector, shuttle vector or cloning vector; it may be double-stranded or single-stranded, linear or circular; or it may transform a prokaryotic or eukaryotic organism either via integration into its genome or extrachromosomally. The nucleic acid molecule or DNA molecule according to the invention in an expression vector or expression cassette is, preferably, operatively linked with one or more regulatory sequences which allow the transcription and, optionally, the expression in a prokaryotic or eukaryotic cell; (Sambrook et al., Molecular Cloning: A Laboratory Manual, 3rd ed., Cold Spring Harbor Laboratory Press, Cold Spring Harbor, NY, 2001). These regulatory sequences are preferably promoters or terminators-in particular, a transcription initiation starting point, a ribosome binding location, an RNA-processing signal, a transcription termination location, and/or a polyadenylation signal. For example, the nucleic acid molecule is here under the control of a suitable promoter and/or a terminator. Suitable promotors may be constitutive promotors (example: 35S promoter from the "Cauliflower mosaic virus" (Odell et al., Nature 313 (1985), 810 - 812); those promoters which are pathogenically inducible are especially suitable (example: PR1 promoter from parsley (Rushton et al., EMBO J. 15 (1996), 5,690-5,700)). Particularly suitable pathogenically-inducible promoters are synthetic or chimeric promoters which do not occur in nature, are composed of multiple elements, and contain a minimal promoter, and have at least one *cis*-regulatory element upstream of the minimal promoter, which at least one *cis*-regulatory element serves as a binding location for special transcription factors. Chimeric promoters are designed according to the desired requirements and are induced or repressed via different factors. Examples of such promoters are found in WO 00/29592, WO 2007/147395, and WO 2013/091612. For example, a suitable terminator is the nos-terminator (Depicker et al., J. Mol. Appl. Genet. 1 (1982), 561-573). Suitable promoters and terminators may also be the native promoter and the native terminator, whose DNA sequences are reproduced in SEQ ID Nos. 7 and 8. The vectors or expression cassettes additionally contain for conventional indicator/reporter genes or resistance genes for the detection of the transfer of the desired vector or DNA molecule/nucleic acid molecule, and for selection of the individuals that contain these, since a direct detection via the expression of the gene is for the most part rather difficult. Since the nucleic acid molecule according to the invention here itself encodes for a polypeptide which confers resistance to Cercospora leaf spot disease, it is not essential for the expression in plant cells to provide an additional resistance gene; however, it is recommended, in order to allow a rapid selection.

Examples of indicator/reporter genes are, for example, the luciferase gene and the gene encoding green fluorescent protein (GFP). These, furthermore, also allow tests for the activity and/or regulation of a promoter of the gene. Examples of resistance genes - especially, for plant transformations - are the neomycin phosphotransferase gene, the hygromycin phosphotransferase gene, or the gene encoding phosphinothricin acetyltransferase. Additional positive selection markers may be enzymes which provide the transformed plant a selection advantage over the non-transformed plant - in particular, a nutrient advantage, e.g., the mannose-6-phosphate isomerase or the xylose isomerase. However, this does not preclude additional indicator/reporter genes or resistance genes known to the person skilled in the art. In a preferred embodiment, the vector is a plant vector. Furthermore, the expression cassette may be present as integrated into a plant genome.

In a further aspect, the present invention relates to cells that include the vectors, recombinant DNA molecules, and/or nucleic acid molecules according to the invention. A cell in the sense of the invention may be a prokaryotic (for example, bacterial) or eukaryotic cell (for example, a plant cell or a yeast cell). The cell is preferably an agrobacterium such as *Agrobacterium tumefaciens* or *Agrobacterium rhizogenes,* an *Escherichia coli* cell, or a plant cell; the plant cell is particularly preferably a cell of a plant of the genus *Beta,* the species *Beta vulgaris,* or the subspecies *Beta vulgaris* subsp. *vulgaris.* The cell may also be present as a culture. The invention also consequently covers a cell culture which contains such cells. The cell culture is preferably a pure culture or an isolate that contains no cells of another type.

Known to the person skilled in the art are both numerous methods, such as conjugation or electroporation, with which he may introduce the nucleic acid molecule according to the invention, the recombinant DNA molecule, and/or the vector or the expression cassette of the present invention into an agrobacterium, and methods such as diverse transformation methods (biolistic transformation, agrobacterium-mediated transformation) with which he may introduce the nucleic acid molecule according to the invention, the DNA molecule, and/or the vector of the present invention into a plant cell (Sambrook et al., Molecular Cloning: A Laboratory Manual, 3rd ed., Cold Spring Harbor Laboratory Press, Cold Spring Harbor, NY, 2001.

Furthermore, the present invention preferably relates to a Cercospora-resistant plant - preferably, a plant of the species *Beta vulgaris* subsp. *vulgaris* or a portion thereof - that contains the nucleic acid molecule according to the invention which confers the Cercospora resistance. The Cercospora-resistant plant may contain the nucleic acid molecule according to the invention as a transgene or as an endogene. Within the scope of the invention, for the first time, plants of the subspecies *Beta vulgaris* subsp. *vulgaris* were produced which contain the nucleic acid molecule according to the invention. The invention here also includes plants of the subspecies *Beta vulgaris* subsp. *vulgaris* which contain the nucleic acid molecule according to the invention as an endogene.

A portion may thereby be a cell, a tissue, an organ, or a combination of multiple cells, tissues, or organs. A combination of multiple organs is, for example, a blossom or a seed. A Cercospora-resistant plant of the present invention preferably shows a higher resistance to Cercospora - in particular, *Cercospora beticola* - than a corresponding plant that does not contain the nucleic acid molecule according to the invention (control plant). The control plant ideally has the identical genotype as the transgenic plant, and has been cultured under identical conditions, but does not contain the resistance-conferring nucleic acid molecule. The level of the resistance, e.g., to *Cercospora beticola,* may be qualitatively established in plants of the genus Beta by determining rating scores. A higher resistance manifests in an improvement in the resistance by at least one rating score, by at least two rating scores, and, preferably, by at least three or more rating scores.

A plant cell or plant or portion thereof of the present invention that contains the nucleic acid molecule according to the invention - in particular, a plant of the genus Beta - preferably shows a higher resistance to a pathogen - in particular, to *Cercospora beticola* - than a corresponding plant cell or plant or portion thereof that does not contain the nucleic acid molecule according to the invention, or contains a sensitive allelic variant of the nucleic acid molecule. The level of the resistance, e.g., to *Cercospora beticola,* may be qualitatively established in plants of the genus Beta by determining rating scores. A higher resistance manifests in an improvement in the resistance by at least one rating score, by at least two rating scores, and, preferably, by at least three or more rating scores.

In the case of a transgenic plant cell, or plant or portion thereof, this comprises the nucleic acid molecule or DNA molecule according to the invention as a transgene or the vector or the expression cassette of the present invention. Such a transgenic plant cell or plant or portion thereof is, for example, one that is transformed - preferably, stably - with the nucleic acid molecule, DNA molecule according to the invention, or with the vector or the expression cassette of the present invention. In a preferred embodiment, the nucleic acid molecule is operatively linked with one or more regulatory sequences which allow the transcription and, optionally, the expression in the plant cell. The total structure made up of the nucleic acid molecule according to the invention and the regulatory sequence(s) then represents the transgene. Such regulatory sequences are, for example, a promoter or a terminator. Numerous functional promoters and terminators that are applicable in plants are known to the person skilled in the art.

The invention also includes a vacuole of the cell according to the invention, and the content substances stored therein.

Furthermore, the invention also relates to the cell extract from a cell - preferably, from a plant cell, particularly preferably, from a cell of *Beta vulgaris,* and, especially preferably, from a cell of one of the following crops: sugar beet, chard, or beetroot. No plant can be regenerated from the cell extract.

Likewise encompassed by the invention is a plant genome containing the nucleic acid according to the invention.

The sugar concentration from the cell extract may thereby be increased relative to a cell that is not a cell according to the invention, but that belongs to the same species or crop. This applies, in particular, under the conditions when infested by *Cercospora.*

Also encompassed by the invention is the use of the cell extract for the production of sugar (saccharose) or for the production of juice - preferably, beetroot juice.

Likewise encompassed by the invention is the sugar - in particular, saccharose - contained in the cells according to the invention and their vacuoles.

An additional aspect of the invention is seed stock comprising seeds that contain the nucleic acid according to the invention. The nucleic acid according to the invention may be present transgenically or endogenously. The seed stock and the seeds may be technically treated. The invention thus also comprises technically-treated seed stock and technically-treated seeds. The various embodiments of technically-treated seed stock are explained in detail in the following whereby the term seed stock also includes seeds: Technically-treated seed stock may be present in polished form. The outermost layer of the seed is thereby removed, so that the seed assumes a more rounded form. This is helpful in sowing, where an optimally uniform shape leads to a uniform distribution of the seed stock grains. Technically-treated seed stock furthermore encompasses pelleted seed stock. The seed stock is thereby embedded in a pelleting mass that protects the seed stock contained therein and leads to a larger mass, such that the pelleted seed stock shows a greater resistance capability with regard to wind drift and is thus less susceptible to being blown away by the wind, and, at the same time, a more precise positioning during sowing is enabled. In a preferred embodiment of the invention, all pelleted seed stock grains of a batch or unit designated for sale have essentially the same shape and the same mass. Deviations of 5% in diameter and mass are possible. However, the deviations preferably do not exceed 1%. As one of the main components, the pelleting mass may contain for example a mineral compound such as clay, bentonite, kaolin, humus and/or peat, for example. It is possible to add an adhesive material like polyacylamide. Additional possible components are cited in US 4,067,141. Moreover, the pelleting mass may contain additional chemical agents that positively influence the cultivation in practice. These may here be substances that are counted among fertilizing agents. These include compounds rich of one or more of the following elements: Nitrogen, Phosphorus and Potassium (macronutrients). Therefore, the fertilizing ingredients may contain for example Nitrate nitrogen, Ammonium nitrogen, Magnesium Nitrate, Calcium Ammonium Nitrate, Mono Ammonium Phosphate, Mono Potassium Phosphate and Potassium Nitrate. Furthermore, pelleting mass may contain fungicides, insecticides, and/or antifeedants. The fungicides may be thiram and/or hymexazol and/or other fungicides. The insecticide may be a substance from the neonicotinoid group. The substance from the neonicotinoid group is preferably imidacloprid (ATC Code: QP53AX17) and/or clothianidin (CAS number 210880-92-5). Furthermore, the insecticide may also be cyfluthrin (CAS number 68359-37-5), beta-cyfluthrin or tefluthrin. It is worth mentioned that the compound included in the dressing or pelleting mass are taken up by the plant and show systemic effect thereby providing suitable protection of the whole plant. Plants resulting from pelleted seed including one or more pesticides therefore differ from naturally occurring plants and show better performance under biotic stress conditions. In this context the invention also encompasses a mixture of a pelleting mass and a seed according to the invention. Furthermore, the invention also encompasses a method for producing a pelleted seed according to the invention comprising the following steps:
a) providing a plant seed comprising the nucleic acid according to the invention,
b) embedding the plant seed in a pelleting mass, and
c) allow the pelleting mass to dry or drying the pelleting mass, wherein the plant seed may be optionally a primed or pregerminated plant seed or the plant seed may be allowed to be primed during step b). Optionally, the method can be concluded by additional step d)
d) Pack up the embedded plant seed as derived by step c) in a packing. Examples of suitable packings are given herein elsewhere. The plant seed may for example be a seed of the plant genus *Beta* as described herein including sugar beet or red beet.

The seed pellet may also comprise beneficial bacteria. Preferably the bacteria are in a vital status and can undergo proliferation. Symbiotic bacteria can have diverse positive effects on the germination of the seed including increased vigor, increased germination speed and better resistance towards stress. According to a preferred embodiment microorganisms of the species *Pseudomonas spp.* are incorporated in the seed pellets. Further strains of different bacteria may be added. A possible method for improving the viability of microorganisms comprises the steps of:
a) suspending one or more pre-cultures of microbial cells in polymeric substance solution,
b) immobilizing said microbial cells by dropping the solution of step a) into multivalent ion solution thereby obtaining polymeric particles,
c) cultivating the encapsulated microorganisms in said polymeric particles for at least 12 hours, preferably for at least 24 hours in liquid media until an increase of the cell density of at least 2 to 10 log is obtained,
d) collecting the polymeric particles, and e) drying the polymeric particles.
Pelleted seeds comprising the resistance gene according to the invention and comprising furthermore microorganisms which have been obtained by the method disclosed in this paragraph are part of the invention. Furthermore, the microorganisms may be encapsulated and / or may be embedded in a extracellular matrix comprising naphthazarin.

In this context technically-treated seed stock encompasses also the dressed seed stock. The invention may be applied with any form of dressed seed stock. Dry dressing, wet dressing, and suspension dressing are thus also encompassed. The dressing may thereby also contain at least one dye (coloring), such that the dressed seed stock may be quickly differentiated from undressed seed stock, and, furthermore, good visibility in the environment is ensured after sowing. The dressing may also contain those agrochemicals which are described in the context of the pilling mass. The invention includes thus such dressed seed stock whereby the dressing contains at least one anti-feedant such as an insecticide and / or at least one fungicide. Optionally, so called electronic dressing (dressing by application of electric energy) may be applied. Electronic dressing is not a dressing in the strict sense of the word but is very suitable to destroy plant pathogens which adhere to the seed or seed stock before planting the seed or seed stock. It is also beneficial that seeds or seed stock which have only been treated by use of electronic dressing (without using agrochemicals) can be fed to animals in case more seed or seed stock is available than needed to till a field.

An additional form of technically-treated seed stock is encrusted seed stock. What is known as coating is also spoken of in this context as well as of seed stock treated with a coating. The difference to pelleted seed stock is that the seed grains retain their original shape, wherein this method is especially economical. The method is described in EP 0 334 258 A1, for example. An additional form of technically-treated seed stock is sprouted or primed seed stock. Sprouted seed stock is pretreated via a pre-germination, whereas primed seed stock has been pretreated via a priming ("germination"). Pre-germinated and primed seed stock have the advantage of a shorter emergence time. The point in time of the emergence after sowing is, at the same time, more strongly synchronized. This enables better agrotechnical processing during cultivation and especially during the harvest, and, additionally, increases the yield quantity. In pre-germination, the seed stock is germinated until the radicle exits the seed stock shell, and the process is subsequently stopped. In the priming, the process is stopped before the radicle exits the seed stock shell. Compared to pre-germinated seed stock, seed stock that has been subjected to a priming is insensitive to the stress of a re-drying and, after such a re-drying, has a longer storage life in comparison to pre-germinated seed stock, for which a re-drying is generally not advised. In this context, technically pre-treated seed stock also includes primed and re-dried seed stock. The process of pre-germination is explained in US 4,905,411 A. Various embodiments of priming are explained in EP 0 686 340 A1. In addition to this, it is also possible to simultaneously pill and prime seed stock in one process. This method is described in EP 2 002 702 B1. Primed seed stock which is moreover pelleted, is encompassed by the present invention.

The technically-treated seed stock may additionally be furnished with one or more of the herbicide resistances explained above. This allows a further-improved agrotechnical cultivation, since the technically-treated seed stock may be deployed on a field that has previously been treated with weed killer, and that therefore is weed-free.

In addition to this, the invention also encompasses a mixture containing the seed stock according to the invention or the seeds according to the invention, and a dressing mass as defined above. The dressing mass is thereby preferably embodied as a pelleting mass, as defined above.

With storage of seed stock according to the invention, storage conditions are preferably to be chosen that do not negatively affect the stability or storage life of the seed stock. Fluctuations in humidity may, especially, have a disadvantageous effect here. Part of the invention is a method for the storage of the seed stock in a bag or container that is via simultaneously water-repellent and breathable. Such a bag or container may be designed as a carton or packing. Such a carton or packing may optionally possess an inner vapor barrier. If the carton or packing is designed as a duplex carton, its stability increases. A container, bag, carton or packing comprising the seed stock according to the invention, or technically-treated seed stock according to the invention, is likewise a part of the invention. It is likewise part of the invention to store seed stock according to the invention or technically-treated seed stock according to the invention in such a bag, container, box, packing or carton.

The present invention also encompasses varieties comprising the resistance gene according to the invention. Furthermore, plants, seeds and seedstock of such a variety are included. The seeds and seedstock of such a variety may be subject to a technical treating as described herein (e.g. pelleting). Suitable sugar beet varieties for the introduction of the resistance gene are for example BTS 7300 N, BTS 2045, BTS 3750, DAPHNA, KORTESSA KWS or SABATINA KWS. Sugar beet plants of the named varieties are also examples of hybrid sugar beet plants. Suitable red beet varieties for the introduction of the resistance gene are for example Jolie, Scarlett (PV-9503) or Diaz wherein Jolie and Diaz are also examples of hybrid red beet plants. Suitable Swiss Chard varieties for the introduction of the resistance gene are for example Fluence, Ion or Tesla / PV-9022. Suitable varieties of *Spinacia oleracea* (spinach) for the introduction of thre resistance gene are for example PV-9210, PV-1194 or La Paz / PV-1237. Hybrid plants take advantage from the heterosis effect.

In one embodiment, the plant according to the invention is a hybrid plant or a double haploid plant. Hybrid plants and double haploid plants do not occur in nature and cannot be isolated from nature. In a further embodiment of the plant according to the invention, the nucleic acid molecule according to the invention is present in heterozygous or homozygous form. In the case of a hybrid plant, the nucleic acid molecule may also be present in hemizygous form. The invention also encompasses hybrid seeds and double haploid seeds which contain a nucleic acid according to the invention or a polypeptide according to the invention.

A further embodiment of the present invention comprises a plant - preferably, of the species *Beta vulgaris* - that is characterized in that the resistance to Cercospora in this plant is further increased. For example, this may be realized by means of "gene stacking," i.e., the resistance is increased using this dose effect. For this, the plants according to the invention that contain the Cercospora resistance-conferring allele are over-transformed with this resistance allele in order to increase the amount of the transcription of the gene in the plant. An alternative approach includes the gene editing/site-directed mutagenesis or TILLING-mediated modification of the native promoter of the resistance-conferring allele, in order to increase its expression rate, or the modification of the resistance-conferring LRR gene allele itself, in order to increase its activity or stability. Such a method for increasing the activity by means of modification of a resistance gene is described in WO 2006/128444 A2, for example, and may be performed by means of the techniques known to the person skilled in the art. An additional approach may include the fusion of the nucleic acid molecule according to the invention with a heterologous promoter that exhibits a higher activity in comparison to the native promoter - in particular, after Cercospora infection.

An additional embodiment of the present invention relates to a sugar beet plant or a portion thereof or a pelleted seed of such a plant which is harvestable before bolting because no bolting of the sugar beet plant occurs during the first 10, 11, 12, 13, 14 or 15 months after germination and the development of a beet body is finished during this period. Suitable varieties to create a sugar beet plant according to this paragraph by introduction of the resistance according to the invention are for example DAPHNA, KORTESSA KWS or SABATINA KWS.

In one embodiment of the present invention the sugar beet plant or a portion thereof or a pelleted seed of such a plant has a genome allowing the development of a beet body having a mass summing up to at least 50%, 60%, 70%, 80% or even 90% of the total mass of the full-grown plant. Suitable varieties to create a sugar beet plant according to this paragraph by introduction of the resistance according to the invention are for example DAPHNA, KORTESSA KWS or SABATINA KWS. In another embodiment of the present invention the sugar beet plant or a portion thereof or a pelleted seed of such a plant has a genome allowing the development of a beet body having a minimum mass of 200g, 250g, 300g, 350g, 400g, 450g or 500g and a maximum mass of 1000g, 1100g, 1200g, 1300g, 1400g, 1500g, 1600g, 1700g, 1800g, 1900g or even 2000g via photosynthesis. Suitable varieties to create a sugar beet plant according to this paragraph by introduction of the resistance according to the invention are for example DAPHNA, KORTESSA KWS or SABATINA KWS.

An additional embodiment of the present invention is directed to a sugar beet plant or a portion thereof or a pelleted seed of such a plant wherein the genome of the sugar beet plant allows development of a beet body having a saccharose concentration of at least 10%, 11%, 12%, 13%, 14%, 15%, 16%, 17%, 18%, 19% or even 20%. Suitable varieties to create a sugar beet plant according to this paragraph by introduction of the resistance according to the invention are for example DAPHNA, KORTESSA KWS or SABATINA KWS.

In one embodiment of the present invention the sugar beet plant or a portion thereof or a pelleted seed of such a plant includes at least one, at least two, at least three, at least four, at least five, at least ten, at least twenty or even at least thirty mutation(s) relative to SEQ ID No. 1, 2 or 4.

The method for the production of an organism which comprises a mutated version of the nucleic acid molecule according to the above given embodiment [1] and/or a mutated version of a promoter comprising a nucleic acid sequence selected from the group consisting of (a) SEQ ID NO: 7, (b) a nucleotide sequence, which hybridizes under stringent conditions with a sequence which is complementary to the sequence according to (a), and (c) a nucleotide sequence which is at least 70% identical to a sequence according to SEQ ID NO: 7, wherein the method includes the following steps:
(I) Provision of an organism or a cell comprising the nucleic acid molecule and/or the promoter
(II) Increase of the mutation rate of the organism or the cell or mutagenesis of the organism or the cell
(III) Phenotypic selection of an organism, which as a result of a mutation exhibits an altered resistance or altered resistance level towards *Cercospora beticola* or Genotypic selection of an organism or a cell which comprises a mutation in the nucleic acid molecule and / or the promoter wherein the mutation has been created via step (II) and optionally
(IV) Regeneration of the organism from the cell obtained via step (III).

The organism can be a plant. Preferably the plant is a *Beta vulgaris.* However, it is also possible to use unicellular organisms as bacteria. The bacterium can be *E.coli.* If the organism is a plant then the method can be applied *in vivo* as well as *in vitro.* If the organism is a plant and the method is applied *in vitro,* a cell culture of the plant may be established and the increase of the mutation rate or the mutagenization may occur in the cell culture. The increase of the mutation rate encompasses for example the application of mutagenic agents like for example 5-bromouracil or ethylmethane sulfonate (EMS) or the application of physical mutagens like ionizing radiation or UV light. The mutagenization encompasses also the targeted mutagenesis. The targeted mutagenesis can be achieved by precise methods as gene editing (as explained further below). The regeneration of organism out of cells is explained in various standard references of the cell biology. The regeneration of plants is for example explained in the standard reference "Plant biotechnology: comprehensive biotechnology, second supplement" (Michael W. Fowler, Graham Warren, Murray Moo-Young - Pergamon Press - 1992). The regeneration of *Beta vulgaris* out of the cell culture is described in Lindsey & Gallois "Transformation of sugarbeet (Beta vulgaris) by Agrobacterium tumefaciens." Journal of experimental botany 41.5 (1990): 529-536.
These references also describe how plant cell cultures are established. As explained further above the mutated version of the nucleic acid molecule respectively the promoter characterizes themselves preferably due to the expression rate of the resistance imparting nucleic acid molecule which is increased by the mutation. Such an effect can also rely on the presence of several mutations. For example, it is possible to introduce two, three, four, five or more mutations in the promoter or the nucleic acid molecule.

By the introduction of mutations thus more resistance imparting protein can be built in the cell or the protein has a better effect. Thereby, the resistance in comparison to a control plant comprising the unaltered nucleic acid according to the invention can be increased for example by at least 1, 2, 3, 4, 5 or more percent. The increase can be measured as explained further below. Moreover, the resistance due to the mutation or mutations can be increased by at least one rating score. The determination of rating scores is explained elsewhere herein. Furthermore, the resistance protein can impart - as a result of the mutations - an altered effect and in some circumstances can exhibit effect against such pathogens which have adapted themselves to the initial resistance mechanism.

In this context the invention encompasses also such mutated variants of the nucleic acids according to the invention and mutated variants of the protein according to the invention. Preferably the invention encompasses such variants which do not occur in nature and cannot be isolated from nature to make sure that the pathogen had no opportunity to adapt itself to such variants. The above described method for the production of an organism which comprises a mutated version of the nucleic acid molecule may furthermore include a further step, in which those organisms or respectively plants are identified, which have a further increased resistance due to the mutation or mutations. If an increase of resistance has taken place may be determined by the herein explained rating scores or the measuring of the resistance level.

Besides the above described method for the production of organisms which comprise a mutated version of the nucleic acid molecule or of the promoter it is also possible to modify the according nucleic acids chemically in an isolated state to achieve the desired effects (as for example those which are described above). The advantage of this approach is that the compounds can be edited even more precisely. For this purpose, the following method is offered:
Production of a chemically modified nucleic acid molecule according to the above given embodiment [1] and / or a chemically modified promoter comprising a nucleotide sequence which is chosen from
(a) SEQ ID NO: 7;
(b) a nucleotide sequence which hybridizes under stringent conditions to a nucleotide sequence according to (a);
(c) a nucleotide sequence which is at least 70% identical to a sequence according to SEQ ID NO: 7;
wherein the method comprises the following steps:
(I) Provision of the nucleic acid molecule as stated above in isolated form
(II) chemical modification of the nucleic acid molecule or the promoter by one of the following steps:
   (IIa) Mutagenization
   (IIb) Gene editing
   (IIc) Restriction and ligation respectively insertion or deletion.

Furthermore, chemical modifications can be generated by such approaches as stated elsewhere herein in context of allelic variants. The gene editing given under step (II) above is equal to the term "Genome-Editing". Optionally the chemically modified nucleic acid molecule or the chemically modified promoter can be subsequently introduced into a cell or can be stably integrated. With the help of such a cell, the chemically modified nucleic acid molecule and the modified promoter can be propagated in context of the cell proliferation. They can be subsequently isolated in vast number and expression analyses may be performed. Expression analyses are especially suitable when the chemical modification concerns the promoter. It is possible to harvest the cells and to isolate the chemically modified resistance protein for chemical analyses. If the cell which comprises the chemically modified nucleic acid molecule or the modified promoter is a plant cell, a complete plant may be regenerated out of this cell. The approaches described within this passage can be performed subsequently to the above given method for the production of a modified form of the nucleic acid molecule and / or a modified promoter and the obtained variants are also a part of this invention. Moreover, a plant comprising the chemically modified nucleic acid molecule or the modified promoter are also part of the invention. Thus, the invention is also related to a plant obtained by this method. Furthermore, the invention relates also to the chemically modified nucleic acid molecules obtained by this method and to the encoded polypeptides. These compounds may be optimized versions of the original (not modified) compounds, wherein the resulting resistance level - as explained further above - may be increased by at least by 1, 2, 3, 4, 5, or more percent or may be increased by at least one rating score. In this regard the method for the production of a chemically modified nucleic acid molecule is also a method for the optimization of the nucleic acid molecule. The method for optimization may furthermore contain an additional step, in which those modified variants of the nucleic acid molecule are identified which lead in comparison to the unamended variants to an increased resistance in a plant.

In a further embodiment, the plant of the present invention additionally, transgenically or endogenously, comprises a second nucleic acid molecule at a different position in the genome, which encodes a polypeptide that is able to confer a resistance to Cercospora in the plant in which the polypeptide is expressed. For example, one or more of the resistance genes or resistance loci that are described in the prior art may - insofar as they are not already present in the initial genotype - be introduced into the present plant by means of crossing, transformation, homology-directed repair, or homologous recombination in the plant. Among these are, for example, the rhizomania resistance RZ1 (Lewellen, R. T., I. O. Skoyen, and A. W. Erichsen, "Breeding sugar beet for resistance to rhizomania: Evaluation of host-plant reactions and selection for and inheritance of resistance." 50th Winter Congress of the International Institute for Sugar Beet Research, Brussels (Belgium), Feb. 11-12, 1987. IIRB. Secretariat General, 1987), or the rhizomania resistance RZ3 (WO 2014/202044) wherein the embodiment concerning RZ3 is explained under passage [94] in more detail.

The present invention additionally relates to a method for increasing the resistance to Cercospora in a plant of the species *Beta vulgaris,* wherein the increase in the resistance takes place without the resistance-conferring gene according to the invention, in comparison to an isogenic plant. The increase in the resistance may take place via integration of the nucleic acid molecule according to the invention into the genome of at least one cell of a plant of the species *Beta vulgaris,* as well as possible regeneration of a plant from the plant cell. The integration may take place both by means of sexual crossing, e.g., with one of the aforementioned *Beta vulgaris* subsp. *maritima* and subsequent selection, or by means of homology-directed repair or homologous recombination. The two latter methods cited are preferably supported by site-directed nucleases which may be selected from, but are not limited to, the following: CRISPR nuclease, including Cas9, CasX, CasY, or Cpf1 nuclease, TALE nuclease, zinc finger nuclease, meganuclease, Argonaut nuclease, restriction endonuclease, including FokI or a variant thereof, recombinase, or two, site-specific, nicking endonucleases. The introduction of the resistance-conferring gene by means of CRISPR-mediated homologous recombination in *Beta vulgaris* subsp. *vulgaris* is shown in Example 1.

Moreover, the invention encompasses also a method of producing an agronomically sugar beet plant of the genus *Beta* that displays improved resistance to *Cercospora beticola,* the method comprising introgressing into said plant a chromosomal interval that confers the improved resistance to Cercospora beticola, wherein the chromosomal interval maps to a position between a sequence represented by a marker selected from the group consisting of
s4p4293s01 and s4p4295s01
and a sequence represented by a marker selected from the group consisting of
s4p4301s01 and sxh0678s01,
characterized in that the chromosomal interval comprises a nucleotide sequence encoding a polypeptide that is able to confer resistance to Cercospora beticola in a plant in which the polypeptide is expressed wherein the nucleotide sequence is selected from
(a) a nucleotide sequence encoding a polypeptide having an amino acid sequence according to SEQ ID No. 3;
(b) a nucleotide sequence that comprises the DNA sequence according to SEQ ID No. 2;
(c) a nucleotide sequence that comprises a DNA sequence selected from the group consisting of SEQ ID No. 1 or SEQ ID No. 53;
(d) a nucleotide sequence that hybridizes to a nucleotide sequence which is complementary to the nucleotide sequence according to (a), (b), or (c), under stringent conditions;
(e) a nucleotide sequence encoding a polypeptide which, via substitution, deletion, and/or addition of one or more amino acids of the amino acid sequence, differs from a polypeptide encoded by the nucleotide sequence according to (a), (b), or (c);
(f) a nucleotide sequence encoding a polypeptide which has an amino acid sequence that is at least 70% identical to an amino acid sequence according to SEQ ID No. 3;
(g) a nucleotide sequence that is at least 70% identical to a DNA sequence according to SEQ ID No. 1 or SEQ ID No. 2;

An alternative approach includes the increase in the expression of the nucleic acid molecule according to the invention in the plant. This may take place via modification of the native promoter, wherein the modification preferably takes place by means of gene editing or site-directed mutagenesis which is mediated via site-directed nucleases, and, optionally, repair models. Examples of such nucleases have already been cited above. The increase in the expression of the nucleic acid molecule according to the invention may likewise take place via fusion of the nucleic acid molecule with a heterologous promoter, which exhibits a higher activity in comparison to the native promoter - in particular, after Cercospora infection. The fusion may likewise take place via site-directed nuclease and repair models, but also by means of direct insertion after double-strand break.

As has already been mentioned above, a method for increasing the Cercospora resistance, may also result in the increase in the activity and/or stability of the polypeptide according to the invention, via modification of the nucleotide sequence of the nucleic acid molecule according to the invention. Such a method for increasing the activity by means of modification of a resistance gene is described in WO 2006/128444 A2, for example, and may be performed by means of the techniques known to the person skilled in the art. This approach is explained in detail further below.

Alternatively, a Cercospora-resistant genotype may be produced from a Cercospora-sensitive genotype by means of random or directed mutagenesis of the nucleic acid sequence of the sensitive gene, and thus the Cercospora resistance may be increased. Examples of polymorphisms which differentiate the sensitive allele from the resistant allele are presented in Fig. 1.

For example, the sensitive allele may be modified via gene mutation by means of TALE nucleases (TALEN's) or zinc finger nucleases (ZFN's), as well as CRISPR/Cas systems, which - among other things - are described by way of example in WO 2014/144155 A1 (Engineering plant genomes using CRISPR/Cas systems) and in Osakabe & Osakabe, Plant Cell Physiol., 56 (2015), 389-400. This may also be achieved via use of the method designated as TILLING (*Targeted Induced Local Lesions in Genomes*)*,* wherein it is described, e.g., in the German patent application DE 10 2013 101 617, how point mutations are caused in the sensitive gene, and plants are subsequently selected that exhibit a suitable, i.e., resistance-conferring, mutation, e.g., a barley resistant to yellow mosaic virus; see DE 10 2013 101 617 on pp. 4, 8, and 12, in paragraphs [0014], [0026], and [0038]. The TILLING method is also described in detail in the publication by Henikoff *et al.* (Henikoff et al., Plant Physiol. 135, 2004, 630-636).

These methods preferably lead to an improvement in the resistance by at least one rating score - particularly preferably, to an improvement in the resistance by at least two, three, or more rating scores. After mutagenesis of the plant cells and subsequent regeneration of plants from the mutagenized plant cells, or mutagenesis of plants, the plants may then be identified that exhibit one or more mutations, as depicted in Fig. 1, in an endogenous nucleic acid molecule. In this context the already mentioned plant according to the invention may be characterized by that the resistance is increased by at least one rating score, preferably by at least two or more rating scores. Alternatively, the resistance of the plants according to the invention may be increased for example by at least 1, 2, 3, 4, 5 or more percent in comparison to a control plant, which does not comprise the nucleic acid according to the invention. The increase can be measured by inoculation of respectively one healthy leaf with an isolate of the pathogen and the determination of the infested surface after 15 days. A reduce of 5% of the infested surface corresponds to an increase of the resistance of 5%. Further parameters for the conduction of the measuring can be derived from the below given embodiment "resistance rest".

An additional embodiment of the present invention is a method for producing a Cercospora-resistant plant, which may take place via transformation of a plant cell with the nucleic acid molecule according to the invention, the recombinant DNA molecule, or with the vector or the expression cassette, and regeneration of the transgenic plant from the transformed plant cell (see Example 2), as well as, as described above, by means of random or targeted mutagenesis of the nucleic acid sequence of the sensitive gene to generate a Cercospora-resistant genotype, or via crossing and selection, e.g., with one of the aforementioned *Beta vulgaris* subsp. *maritima.* Vectors or expression cassettes, as well as methods for transforming plants, have already been described above.

The method for production of a Cercospora-resistant plant alternatively includes, as described above, the introduction of a site-directed nuclease and a repair matrix into a cell of a plant of the species *Beta vulgaris,* wherein the site-directed nuclease is able to generate at least one double-strand break of the DNA in the genome of the cell - preferably, upstream and/or downstream of a target region - and the repair matrix comprises the nucleic acid molecule according to the invention. The method furthermore includes the cultivation of this cell under conditions that allow a homology-directed repair or a homologous recombination, wherein the nucleic acid molecule is incorporated from the repair matrix into the genome of the plant. Furthermore, the regeneration of a plant from the modified plant cell is encompassed (see Example 1).

In a preferred embodiment, the target region is an allelic variant of the nucleic acid molecule according to the invention, wherein the allelic variant encodes a polypeptide which does not confer resistance to Cercospora. In a further preferred embodiment, this allelic variant comprises a nucleotide sequence that encodes a polypeptide with an amino acid sequence according to SEQ ID No. 6 and/or comprises the encoded DNA sequence according to SEQ ID NO: 5 or the genomic DNA sequence according to SEQ ID No. 4.

As described in connection with the nucleic acid molecule according to the invention, substitutions, deletions, insertions, additions, and/or any other change may be introduced that, either alone or in combinations, do in fact change the nucleotide sequence, but perform the same function as the initial sequence - here, the nucleotide sequence of the allelic variant of the nucleic acid molecule according to the invention. Therefore, in a further embodiment, the invention includes a nucleotide sequence that encodes a polypeptide which represents a derivative of the polypeptide which is encoded by the allelic variant of the nucleic acid molecule according to the invention, or which comprises the amino acid sequence of the allelic variant of the nucleic acid molecule according to the invention. A derived amino acid sequence which has at least one substitution, deletion, insertion, or addition of one or more amino acids, wherein the functionality of the encoded polypeptide/protein is preserved, represents a derivative of the polypeptide. The nucleotide sequence, using conventional methods that are known in the prior art, e.g., via site-directed mutagenesis, PCR-mediated mutagenesis, transposon mutagenesis, genome editing, etc., substitutions, deletions, insertions, additions, and/or any other change, either solely or in combinations with the gene, may thereby be introduced, which do in fact change the nucleotide sequence, but perform the same function as the initial sequence.

With regard to the amino acid sequence, after modification via an aforementioned method, this also has a common structural domain and/or possesses common functional activity. Nucleotide sequences or amino acid sequences that at least approximately 80%, at least approximately 85%, at least approximately 90%, at least approximately 91%, at least approximately 92%, at least approximately 93%, at least approximately 94%, at least approximately 95%, at least approximately 96%, at least approximately 97%, at least approximately 98%, at least approximately 99%, or at least approximately 100% identical to the nucleotide sequence or amino acid sequence of the cited allelic variant of the nucleic acid molecule according to the invention are defined here as being sufficiently similar. Accordingly, the present invention includes a nucleotide sequence that is able to hybridize, under stringent conditions, with a nucleotide sequence that is complementary to a nucleotide sequence of the allelic variant of the nucleic acid molecule according to the invention or to the nucleotide sequence that encodes the corresponding amino acid sequence.

In a further preferred embodiment, the method according to the invention is characterized in that the double strand break occurs in an allelic variant of the nucleic acid molecule according to embodiment [1] or that the at least one double strand break occurs at a position which is at least 10,000 base pairs upstream or downstream of the allelic variant, wherein the allelic variant codes for a polypeptide which does not impart a resistance towards Cercospora.

For the person skilled in the art, it is obvious that numerous, different sensitive sequences may occur that derive from the nucleic acid molecule according to the invention, but do not confer resistance to Cercospora, such that the sequences listed above (SEQ ID Nos. 4, 5, and 6) should only be considered as an example of sequences, and the present invention is not limited to the aforementioned allelic variant of the nucleic acid molecule according to the invention. Such an allelic variant can comprise a nucleotide sequence, which is selected from:
(a) a nucleotide sequence encoding a polypeptide having an amino acid sequence according to SEQ ID No. 6;
(b) a nucleotide sequence that comprises the DNA sequence according to SEQ ID No. 5;
(c) a nucleotide sequence that comprises a DNA sequence according to SEQ ID No. 4;
(d) a nucleotide sequence that hybridizes with the complementary sequence according to (a), (b), or (c), under stringent conditions;
(e) a nucleotide sequence encoding a polypeptide which, via substitution, deletion, and/or addition of one or more amino acids of the amino acid sequence, differs from a polypeptide encoded by the nucleotide sequence according to (a), (b), or (c);
(f) a nucleotide sequence encoding a polypeptide which has an amino acid sequence that is at least 70%, 75%, 80%, 85%, 90%, 95%, 96%, 97%, 98% or 99% identical to an amino acid sequence according to SEQ ID No. 6;
(g) a nucleotide sequence that is at least 70%, 75%, 80%, 85%, 90%, 95%, 96%, 97%, 98% or 99% identical to a DNA sequence according to SEQ ID No. 4 or SEQ ID No. 5;

As described above, with quantitative heredity of QTL, not only is the desired resistance to often introduced into the plant, but, rather, also often unwanted features such as, for example, reduced yield, due to the inheritance of additional genes that are not linked with the positive feature of the resistance. This increasingly occurs if, as in the case of Cercospora resistance, the resistance is inherited in previously available cultivars via many resistance genes with small effect. Therefore, in a preferred embodiment, the introduction of the nucleic acid molecule according to the invention, which already shows, on its own, a dominant resistance effect, or of the vector or the expression cassette, is not linked with the introduction of unwanted features, wherein the yield is, preferably, not negatively affected. Furthermore, encompassed by the invention is the plant that is obtained via such a method.

Although the QTL analyses with that have previously been known from the prior art could detect actual QTL's, the underlying genomic regions that had shown a QTL effect also mediated the disadvantages described above, which is why "linkage drag" is also discussed in this context. At the same time, the QTL's and the effects connected therewith were not described uniformly in the respective prior art, and merely mediated a weak effect, such that the utilization of these results in the breeding of Cercospora-resistant plants was possible to only a limited extent, and was largely uncertain. Targeted breeding and controlled integration of the resistance gene into the gene pool of the sugar beet are now enabled by means of the identification of the resistance gene described herein. This ensures the breeding and generation of entirely new Cercospora-resistant cultivars that exhibit a high resistance to the pathogen, without negatively affecting the sugar yield.

The present invention likewise relates to methods / processes for the identification or detection, and optionally the selection and / or provision, of a plant of the species *Beta vulgaris* that is resistant to the pathogen Cercospora, characterized in that the method includes a step of the detection of the presence and/or of the expression of a nucleic acid molecule according to the invention or of the polypeptide according to the invention in the plant or a sample / portion thereof. The presence and/or the expression of a nucleic acid molecule according to the invention, or of the polypeptide according to the invention, may be tested by means of standard methods known to the person skilled in the art, e.g., by means of PCR, RT-PCR, or western blot. Preferably during the PCR the oligonucleotides or primers hybridize with a genomic template comprising the nucleotide sequence as defined in step (i) of [104] in such a way / in such a distance that the resulting amplificate is comprises up to 2000bp, preferably up to 1500bp, more preferably 1000bp, most preferably up to 500 or 200 or up to 100bp. The plant to be identified / detected and optionally selected and / or provided may be a plant comprising the resistance gene according to the invention and furthermore comprising a nucleotide sequence according to SEQ ID NO. 182 and / or the resistance allele according to marker s4p8772s01 or may be a plant obtainable from the seeds deposited with the NCIMB, Aberdeen, UK under access number NCIMB 43646. The methods and processes according to this paragraph may also be used to exclude or disregard plants which do not carry the resistance gene according to the invention. This is espacially useful for example in breeding programs where some but not all of the offspring plants include the resistance.

Furthermore, the identification method according to the invention also includes the detection of the nucleic acid molecule according to the invention by means of detection of at least one polymorphism between resistant and sensitive sequences, i.e., between the sequences of the nucleic acid molecule according to the invention and the sequences of the allelic variant of the nucleic acid molecule according to the invention that is described above, using molecular markers that detect one or more polymorphisms. As has already been described above, it is obvious to the person skilled in the art that numerous sensitive sequences exist, i.e., numerous sequences that encode the allelic variant of the nucleic acid molecule according to the invention. One of these is presented by way of example in the sequence comparison with the nucleotide sequence of the nucleic acid molecule according to the invention in Fig. 1. A preferred embodiment of the method according to the invention consequently includes the detection of at least one polymorphism that is presented in Fig. 1 using molecular markers which detect the polymorphisms - in particular, diagnostic polymorphisms. This detection preferably occurs using at least one molecular marker per polymorphism - in particular, per diagnostic polymorphism. It is known to the person skilled in the art which marker techniques are to be applied to detect a corresponding polymorphism, and how molecular markers for this are constructed (see Advances in Seed Science and Technology Vol. I, Vanangamudi et al., 2008). Furthermore, the present invention encompasses molecular markers which describe or detect a polymorphism according to Fig. 1, such as the use of a molecular marker for detection of a polymorphism according to Fig. 1. It is thereby also possible to use markers that do not differentiate between various polymorphisms, as long as the markers are able to detect such a polymorphism as it occurs in the nucleic acid molecule according to the invention, but is not contained the sensitive allelic variant.

Alternatively, or additionally, the identification method according to the invention includes a step of detecting at least one marker locus in the nucleotide sequence of the nucleic acid molecule according to the invention or in a cosegregating regions thereof. Preferably the cosegregating region is a genomic region in *Beta vulgaris* which cosegregates with the Cercospora resistance conferred by the polypeptide according to the present invention, or with the nucleic acid molecule according to the present invention, more preferably the cosegregating region comprises and is flanked by markers *sxh0678s01* and *s4p0264s01,* by markers *s4p4301s01* and *s4p2271s01,* by markers *s4p4301s01* and *s4p4293s01, or* by markers *s4p4301s01* and *s4p4295s01.* The detection may thereby take place via a method step in which at least one marker or at least one primer pair binds at the locus according to SEQ ID No. 74 or 75 - preferably, at the locus according to SEQ ID No. 76 or 77 - and, optionally as a result of this, a signal is generated, e.g., a fluorescence signal or a sequence amplificate. Thus, alternatively or additionally the cosegregating region may comprise a sequence according to SEQ ID NO 74 and/or 75, or SEQ ID NO: 76 and/or 77. Furthermore, the preceding identification methods also represent methods for selection of a plant which exhibits the resistance to Cercospora according to the invention. The method for selection includes a concluding step of selecting a resistant plant.

In this context, the present invention also includes the development or production of molecular markers that are suitable for detecting the aforementioned polymorphisms between the nucleic acid molecule according to the invention (resistant allele) and the sensitive allelic variant, wherein the markers are preferably suitable for detecting the polymorphisms presented in Fig. 1 or the construction of hybridization probes that specifically bind to the nucleotide sequence of the nucleic acid molecule according to the invention, or the production of a pair of nucleic acid molecules that is suitable for amplifying, in a PCR, a region that is specific to the nucleic acid molecule according to the invention, and thus for detecting these in a plant or plant cell.

The invention preferably includes a method for producing oligonucleotides of at least 15, 16, 17, 18, 19, or 20 - preferably, at least 21, 22, 23, 24, or 25, particularly preferably, at least 30, 35, 40, 45, or 50, and, especially preferably, at least 100, 200, 300, 500 or 1,000 - nucleotides in length that specifically hybridize with a nucleotide sequence of the nucleic acid molecule according to the invention or the nucleic acid molecule that is complementary thereto, or a pair of nucleic acid molecules - preferably, in the form of oligonucleotides - that is suitable for attachment as a forward and reverse primer to a region that is specific to the nucleic acid molecule according to the invention, and for amplifying this in a polymerase chain reaction (PCR), or that is suitable for hybridization as a forward and reverse primer to a region in the *Beta vulgaris* genome that, in *Beta vulgaris,* has a cosegregation with the Cercospora resistance conferred by the polypeptide according to the invention or with the nucleic acid molecule according to the invention. An example for suitable primers for the detection of a resistance-mediating nucleotide sequence according to the invention are given by SEQ ID NO 98 and SEQ ID NO 99. These two sequences build a primer pair which can by used in the PCR. The invention also includes a kit comprising oligonucleotides or molecular markers according to the invention.

The method for the production of oligonucleotides initially includes: the comparison of the nucleotide sequence of the nucleic acid molecule according to the invention with the nucleotide sequence of the corresponding nucleic acid molecule that does not confer resistance or of the sensitive allelic variant, which preferably has a nucleotide sequence according to SEQ ID No. 4 or 5; the identification of the sequence differences between the two nucleotide sequences; and the generation of nucleic acid molecules - here, meaning oligonucleotides - that specifically bind to the nucleic acid molecule according to the invention, but not to the nucleic acid molecule that does not mediate resistance.

Furthermore, the oligonucleotide according to the invention may be connected to a fluorescent dye in order to generate a fluorescence signal, e.g., under excitation via light of the corresponding wavelength. The fluorescent dye may be fluorochrome. The oligonucleotides according to the invention may be coupled with other compounds that are suitable for generating a signal. Such oligonucleotides do not occur in nature and also cannot be isolated from nature. The following is executed to produce such marked oligonucleotides: DNA may be marked bio-orthogonally. For this, DNA may be marked in vivo or in vitro with nucleoside analogs, which, for example, may subsequently be coupled with a fluorophore per Staudinger reaction. In addition to this, DNA may also be chemically provided with fluorophores. Oligonucleotides may be marked via a phosphoramidite synthesis with fluorophores that, for example, are used in QPCR, DNA sequencing, and in situ hybridization. Furthermore, DNA may be generated enzymatically in the course of a polymerase chain reaction with fluorescent nucleotides, or be marked with a ligase or a terminal deoxynucleotidyl transferase. DNA may also be detected indirectly via a biotinylation and fluorescent avidin. For couplings, fluorescein, fluorescent lanthanides, gold nanoparticles, carbon nanotubes, or quantum dots, among other things, are used as fluorophores. One of the most commonly used fluorescent substances is FAM (carboxyfluorescein). Consequently, oligonucleotides and, in particular, primers that possess a FAM marking are encompassed by the invention. FAM is preferably present as 6-FAM, wherein - depending upon the desired wavelength of the emission and excitation - other FAM variants, e.g., 5-FAM, may, however, also be used. Examples of additional fluorescence markers are AlexaFluor, ATTO, Dabcyl, HEX, Rox, TET, Texas Red, and Yakima Yellow. Depending upon the field of use, the oligonucleotides may be furnished with modifications of the bases or of the sugar phosphate spine. Among these are, among others, amino-dT, azide-dT, 2-aminopurine,5-Br-dC, 2'-deoxyinosine (INO), 3'-deoxy-A, C, G, 5-Met-dC, 5-OH-Met-dCN6-Met-dA, and others.

Furthermore, the present invention also relates to a marker chip ("DNA chip", "assay" or microarray) which contains at least one oligonucleotide according to the invention that is suitable for detection. The marker chip is suitable for application in one or more detection methods according to the invention.

The invention likewise includes a method for production of the protein according to the invention. The method includes the provision or cultivation of a cell culture which contains the SEQ ID No. 2, and the subsequent expression of the protein encoded by SEQ ID No. 2.

Furthermore, the present invention also relates to a Cercospora-resistant plant or a portion thereof which was identified, and, if applicable, selected, via a method as described in the preceding. In particular, the present invention relates to a population of plants comprising plants that are available according to one of the methods according to the invention as described in the preceding, and that preferably are resistant to *Cercospora* leaf spot disease or Cercospora infestation, and are characterized by the presence of a nucleic acid molecule according to the invention. The population preferably has at least 10 - preferably, at least 50, more preferably, at least 100, particularly preferably, at least 500, and, particularly in agricultural farming, preferably at least 1,000 - plants. The proportion of plants in the population that do not carry the nucleic acid molecule according to the invention and/or are susceptible to Cercospora leaf spot disease is preferably below 25% - preferably, below 20%, more preferably, below 15%, even more preferably, 10%, and, in particular, preferably below 5%, if present at all.

With the fine mapping described above, the position of the Cercospora resistance-conferring gene in the genome of *Beta vulgaris* subsp. *maritima* could be determined, and the gene itself and the surrounding sequence regions could be identified. This in turn represents the basis for the development of DNA hybridization probes or genetic markers in the target region, with the aid of which the Cercospora resistance-mediating gene could be detected, or could be differentiated from the gene that does not confer resistance.

DNA hybridization probes may be derived from the sequence of the Cercospora resistance-conferring gene and be used for the screening of genomic and/or cDNA banks of the desired organism. The probes may be used to amplify identified homologous genes via the known process of polymerase chain reaction (PCR), and to check whether the Cercospora resistance-conferring gene is present endogenously in an organism, or has been successfully introduced as heterologous genetic element.

The person skilled in the art may here resort to customary hybridization, cloning, and sequencing methods, which, for example, are listed in Sambrook et al., Molecular Cloning: A Laboratory Manual, 3rd ed., Cold Spring Harbor Laboratory Press, Cold Spring Harbor, NY, 2001. The person skilled in the art may also synthesize and use oligonucleotide primers to amplify sequences of the Cercospora resistance-conferring gene. In order to achieve a specific hybridization, such probes should be specific and have at least a length of 15 nucleotides - preferably, at least 20 nucleotides. A detailed guide to hybridization of nucleic acids may be found in Tijssen, Laboratory Techniques in Biochemistry and Molecular Biology - Hybridization with Nucleic Acid Probes, Part 1, Chapter 2, "Overview of principles of hybridization and the strategy of nucleic acid probe assays." Elsevier, New York (1993); and in Current Protocols in Molecular Biology, Chapter 2, Ausubel et al., eds., Greene Publishing and Wiley lnterscience, New York (1995).

Therefore, a nucleic acid molecule of at least 15, 16, 17, 18, 19, or 20 - preferably, at least 21, 22, 23, 24, or 25, particularly preferably, at least 30, 35, 40, 45, or 50, and, especially preferably, at least 100, 200, 300, 500, or 1,000 - nucleotides in length is the subject matter of the present invention, wherein this nucleic acid molecule specifically hybridizes with a previously-described nucleotide sequence according to the invention that comprises the Cercospora resistance-conferring gene. This also explicitly encompasses the range of 15 to 35 nucleotides.

The present invention thus also relates to markers as oligonucleotides - in particular, primer oligonucleotides. These comprise a nucleic acid molecule of at least 15 nucleotides in length that specifically hybridizes with a nucleotide sequence defined as in the preceding.

In particular, the present invention encompasses a pair of nucleic acid molecules - preferably, in the form of oligonucleotides or a kit containing this pair of oligonucleotides - that is suitable for hybridization as a forward and reverse primer to a region that is specific to the nucleic acid molecule according to the invention, and for amplifying this in a polymerase chain reaction (PCR), or that is suitable as a forward and reverse primer for hybridization to a region in the *Beta vulgaris* genome that, in *Beta vulgaris,* exhibits a cosegregation with the Cercospora resistance conferred by the polypeptide according to the invention, or with the nucleic acid molecule according to the invention.

The following advantages for the breeding and development of new resistant plant lines of the genus Beta may also be achieved via the present invention. Sequence information, as well as the identified polymorphisms which allow a differentiation between resistant and susceptible alleles of the disclosed gene, i.e., between the alleles that confer a Cercospora resistance and the alleles that are not capable of conferring this resistance, make possible the marker development directly in the gene, as described above, as well as in the regions situated upstream and downstream, which represents an important facilitation for the plant breeder - in particular, with regard to the development of optimized elite lines without "linkage drag." Moreover, knowledge about the sequential structure may be used for the identification of additional resistance genes - in particular, against Cercospora - which are homologous or orthologous, for example.

Therefore, the present invention also encompasses a method for the identification of additional nucleic acid molecules encoding polypeptides or additional proteins that are able to confer a resistance to Cercospora in a plant in which the polypeptide is expressed. The person skilled in the art may thereby use databases, employing suitable search profiles and computer programs for the screening for homologous sequences or for sequence comparisons. Moreover, by means of conventional molecular biology techniques, the person skilled in the art may himself derive additional DNA sequences encoding Cercospora resistance proteins, and use these within the scope of the present invention. For example, suitable hybridization probes may be derived from the sequence of the nucleic acid molecule according to the invention and be used for the screening of genomic and/or cDNA banks of the desired organism. The person skilled in the art may here resort to customary hybridization, cloning, and sequencing methods, which, for example, are listed in Sambrook et al., Molecular Cloning: A Laboratory Manual, 3rd ed., Cold Spring Harbor Laboratory Press, Cold Spring Harbor, NY, 2001. Using known sequences, the person skilled in the art may also synthesize and use oligonucleotide primers to amplify sequences of Cercospora resistance-conferring nucleic acid molecules.

In one embodiment, the present invention therefore encompasses a method for the identification of a nucleic acid molecule which encodes a polypeptide that is able to confer a resistance to Cercospora in a plant of the species *Beta vulgaris* in which the polypeptide is expressed. The method thereby includes the comparison of the amino acid sequence of the polypeptide according to the invention which, in *Beta vulgaris* subsp. *vulgaris,* confers a Cercospora resistance with amino acid sequences from a sequence database, or with sequences of allelic variants of the polypeptide according to the invention in genotypes of the species *Beta vulgaris.* Furthermore, the method according to the invention includes the identification of an amino acid sequence or of an allelic variant that is at least 80% identical to the amino acid sequence of the polypeptide according to the invention, as well as the introduction of a nucleic acid molecule encoding the identified amino acid sequence or allelic variant in a plant of the species *Beta vulgaris;* expression of the nucleic acid molecule in the plant; and, optionally, subsequent verification of the resistance to Cercospora.

As described in the preceding, additional Cercospora resistance-conferring proteins or their coding genes, i.e., homologs, analogs, and orthologs, that are at least 70% - preferably, at least 80%, particularly preferably, at least 90%, especially preferably, at least 95%, or even 98% - identical to the amino acid sequence of the polypeptide which is encoded by the nucleic acid molecule according to the invention may be identified via classical bioinformatic approaches (database searches and computer programs for screening for homologous sequences).

The term, homolog(s), thereby means that the genes concerned (from two different plant species) have essentially the same function and a common ancestor, and therefore typically show a significant identity in their nucleic acid or coded amino acid sequences. However, there are also many genes that are homologous to one another, without protein sequences resulting in a meaningful paired alignment. In contrast to this, the term, analog(s), describes genes or proteins that (likewise) have an identical or similar function, but are not created from the same structure, i.e., have no common ancestor. In this case, often, no significant identity can be established in their nucleic acid or encoded amino acid sequence, or, in the best case, in specific functional domains.

In the context of genome sequencing, homologs are, for annotation, more finely classified. The terms, orthology and paralogy, have been introduced for this. Orthologs are genes that are connected via a speciation event. Paralogs are genes that trace back to a duplication event.

A gene is, then, fundamentally a homolog or analog or ortholog in the sense of the present invention if it is able to confer Cercospora resistance in a plant. To check, methods, which have already been described in the preceding, known to the person skilled in the art are used, e.g., the amplification of the identified homolog or analog or ortholog by means of PCR, cloning in expression vectors, introduction into the target plant or plant cell, and checking the resistance.

As described above, the usage disclosed here of the resistant gene allele in cis- or transgenic approaches opens up the possibility of new resistant species of the genus Beta which, using the dose effect, exhibit a higher resistance, or in which a resistance break may be avoided and the resistance development optimized via the stacking of the disclosed gene with other resistance genes. Modifications of the gene by means of tilling or targeted engineering to optimize the codon selction for an increased expression or for the development of new or modified resistance alleles are also possible. According to a preferred embodiment the codon-optimized sequences or the modified resistance alleles are not occurring in nature but are artificial. An example of a modified genomic sequence is provided by SEQ ID No. 94 in which the codon at position 16 - 18 is modified but the encoded amino acid sequence is unchanged and corresponds to SEQ ID No. 3. An example of a modified cDNA sequence is provided by SEQ ID No. 95 in which the codon at position 55-57 is modified but the encoded amino acid sequence is unchanged and corresponds to SEQ ID No. 3. SEQ ID No. 94 and SEQ ID No. 95 are also examples for hybridizing sequences. An example of a modified resistance conferring allele is given by the amino acid sequence according to SEQ ID No. 96 in which the amino acid valine has been replaced with the amino acid leucine at position 209. The amino acid sequence according to SEQ ID No. 96 is encoded by the modified cDNA according to SEQ ID No. 97. These sequences do not occur in nature but are artificial. When replacing amino acids in for example the resistance-mediating Sequence according to SEQ ID No. 3 it is recommended to exchange amino acids within the following groups:
a) glycine, alanine, valine, leucine, isoleucine
b) serine, cysteine, selenocysteine, threonine, methionine
c) phenylalanine, tyrosine, tryptophan
d) histidine, lysine, arginine
e) aspartate, glutamate, asparagine, glutamine.

The present invention also relates to the use in a plant of the identified Cercospora resistance-conferring gene allele in a genetic or molecular stack with other genetic elements which may confer agronomically advantageous properties. The economic value of cultivated plants may thereby be markedly increased, in that, for example, the yield performance is increased in comparison to plants that possess the same genetics, but have not been furnished with the nucleic acid according to the invention. Furthermore, new crop areas for a plant may be opened up that were not previously accessible to the cultivation of this plant due to biotic factors such as strong pathogen pressure. In particular, the present invention relates to the use of the identified Cercospora resistance-conferring gene allele in methods for controlling an infestation with the pathogen *Cercospora beticola* in the agricultural or horticultural cultivation of plants of the genus Beta, e.g., encompassing the identification and selection of plants of the genus Beta with the aid of one of the methods described in the preceding and/or the cultivation of the plants so selected or descendants thereof. The present invention thus includes a method for the cultivation of plants of the species *Beta vulgaris,* including, in a first step, the provision of Cercospora-resistant plants of the species *Beta vulgaris* according to the invention, or the production of plants of the species *Beta vulgaris* with the aid of the production method according to the invention, or the identification and selection of plants of the species *Beta vulgaris* with the aid of the identification method according to the invention that has been described in the preceding; and including, in a second step, the cultivation of the plants from the first step, or the deployment of seed stock of the plants from the first step, or the raising of plants from the first step. The cultivation method thereby counteracts an infestation of the cultivated plants by Cercospora. The cultivation method may be part of a method for producing sugar. The method for the production of sugar includes the steps of the cultivation method, and additionally, as a penultimate step, the harvesting of the cultivated plants, and, as a last step, the extraction of sugar from the aforesaid plants. The methods for identification or detection, the marker and oligonucleotides as described herein may be for example used on the seeds deposited with the NCIMB, Aberdeen, UK under access number NCIMB 43646 or may be used on a plant resulting from the deposited seeds. Furthermore, they may be used a) on a plant which is the offspring of the deposited seeds or b) on a plant into which the resistance gene according to the invention has been transferred by one of the methods described herein (e.g. transformation etc.) or c) for the discrimination of a plant which comprises a resistance gene according to the invention and a plant lacking the resistance gene of the invention.

The cultivation method may also be part of a method for producing seed stock. The method for the production of seed stock includes the steps of the cultivation method, and additionally, as a penultimate step, the vernalization of the cultivated plants, and, as a last step, the extraction of seeds from the aforesaid plants.

The extracted seeds may optionally be pelleted, in order to obtain pelleted seed stock of the species *Beta vulgaris.* In this instance, it is a method for the production of pelleted seed stock.

Moreover, the method for the production of seed stock may be designed as a method for the production of Cercospora-resistant seed stock. The method for the production of Cercospora-resistant seed stock includes the steps of the method described above for the production of seed stock, and additionally, as a last step, the verification of the nucleic acid according to the invention according to a method described herein in at least one of the extracted seeds - preferably, in at least 0.1% or in at least 1% of the extracted seeds. The verification is particularly preferably implemented so that the seed remains germinable. This means that the extraction of the DNA required for verification from the seed does not neutralize the germinability of the seed. In such an instance, the verification of the nucleic acid according to the invention may have taken place in an especially large proportion of all extracted seeds. For example, the verification may take place in at least 2% - preferably, at least 3%, particularly preferably, at least 4% - of all extracted seeds.

The plants according to the invention, their cells, or seeds or seed stock according to the invention may possess additional, agronomically advantageous properties, or be furnished with such. One example is the tolerance or resistance to an herbicide such as glyphosate, glufosinate, or ALS inhibitors. The tolerance to glyphosate or an ALS-inhibitor herbicide is preferred. A specific embodiment of the glyphosate resistance is disclosed in US 7,335,816 B2. Such a glyphosate resistance is, for example, available from seed stock stored at the NCIMB, Aberdeen (Scotland, UK), under the access number, NCIMB 41158 or NCIMB 41159. Such seeds may be used in order to obtain a glyphosate-tolerant sugar beet plant. The glyphosate resistance may also be introduced into other species of the genus *Beta* via crossing.
In the context of glyphosate resistance the invention thus also encompasses plants, their cells, or seeds or seed stock, characterized in that these contain the nucleic acid according to the invention, and furthermore in that
a) a DNA fragment of the genomic DNA of the plant, portions, or seeds thereof may be amplified via polymerase chain reaction with a first primer that has the nucleotide sequence of SEQ ID No. 81, and a second primer that has the nucleotide sequence of SEQ ID No. 82, wherein the DNA fragment is at least 95% - preferably, 100% - identical to the nucleotide sequence of SEQ ID No. 83, and/or
b) a DNA fragment of the genomic DNA of the plant, portions, or seeds thereof may be amplified via polymerase chain reaction with a first primer that has the nucleotide sequence of SEQ ID No. 84, and a second primer that has the nucleotide sequence of SEQ ID No. 85, wherein the DNA fragment is at least 95% identical - preferably, 100% identical - to the nucleotide sequence of SEQ ID No. 86, and/or
c) a DNA fragment of the genomic DNA of the plant, portions, or seeds thereof may be amplified via polymerase chain reaction with a first primer that has the nucleotide sequence of SEQ ID No. 87, and a second primer that has the nucleotide sequence of SEQ ID No. 88, wherein the DNA fragment is at least 95% identical - preferably, 100% identical - to the nucleotide sequence of SEQ ID No. 89.

In certain embodiments, the wild type Beta vulgaris epsp synthase has an amino acid sequence as provided in NCBI reference sequence XP_010692222.1. This wild type epsp synthase may be mutated or modified (for example by one of the means for mutation or modification as disclosed herein) for conferring resistance to glyphosate. Such a mutation may create an endogenous allele encoding an epsp synthase having at position 179 an amino acid different from proline. Preferably the amino acid different from proline is serine. Plants comprising a mutated epsp synthase and the resistance gene according to the invention are encompassed herein. This includes plants comprising the resistance gene of the invention and furthermore a nucleic acid molecule encoding an epsp synthase having at position 179 an amino acid different from proline and comprising an amino acid sequence selected from
i) the sequence of SEQ ID NO: 223
ii) the sequence i) having at position 179 an amino acid different from serine
   and proline, or
iii) a sequence having an identity of at least 90%, more preferable 95%, most preferably 99% to the sequence of i) or ii)
   over the entire length of the sequence.
In this context the mutated EPSP synthase may be for example encoded by a nucleic acid sequence according to SEQ ID No. 224. Further technical details on the use of mutated EPSP synthase may be derived from WO2020064687 (A1). A plant comprising the resistance gene according to the invention and expressing a mutated EPSP synthase may furthermore comprise a resistance towards one or more ALS inhibitor herbicide as described herein. A plant comprising the resistance gene according to the invention and expressing a mutated EPSP synthase may be a non-transgenic plant. The mutated EPSP synthase in the context of this invention is an artificial compound which does not occur in nature. According to a specific embodiment the plant comprising the mutated EPSP synthase and the resistance gene according to the invention is not a product of an essentially biological process.

A specific embodiment of the ALS-inhibitor herbicide resistance is disclosed in the document, WO2012/049268 A1. For example, such an ALS-inhibitor herbicide resistance is available from a deposit of NCIMB, Aberdeen, UK, under the number NCIMB 41705. Furthermore, such an ALS-inhibitor resistance may be produced via tilling or site-directed mutagenesis, e.g., via gene editing, such as through the use of CRISPR/Cas, CRISPR/Cpf1, TALENS or zinc finger nucleases. The invention thus also encompasses plants, their cells, or seeds or seed stock, characterized in that these contain the nucleic acid according to the invention, and furthermore in that these exhibit a mutation in an endogenous acetolactate synthase gene, wherein the acetolactate synthase gene encodes an acetolactate synthase protein which, as a result of the mutation at position 569, has a different amino acid than tryptophan. As a result of the mutation, the amino acid at position 569 is preferably alanine, glycine, isoleucine, leucine, methionine, phenylalanine, proline, valine, or arginine. Position 569 is preferably defined via the position 569 of SEQ ID No. 90. Furthermore, the specific sequence of the mutated acetolactate synthase gene SEQ ID No. 91 is preferred. The mutated sequence of the acetolactate synthase gene, or the sequence according to SEQ ID No. 91, does not occur in nature and cannot be isolated from nature. Furthermore, the mutation may be present both heterozygously and homozygously in the plants, their cells or seeds, or the seed stock. We recommend the homozygous presence of the mutation, since this promotes a more stable or more intensive phenotypical occurrence of the resistance.

Numerous additional herbicides and their applicability are known to the person skilled in the art from the prior art. He may resort to the prior art in order to achieve knowledge of which genetic elements are to be used in what manner in order to implement a corresponding tolerance in plants.
Moreover, an herbicide tolerance has the synergistic effect that the occurrence of weeds is reduced via the use of herbicides. This is advantageous in combating Cercospora, because it is known that the conidia (asexual spores) or the pseudostroma (mycelium) of *Cercospora beticola* can survive for up to 2 years on plant material.

A further example of an agronomically advantageous property is an additional pathogen resistance, wherein pathogens may be insects, viruses, nematodes, bacteria, or fungi, for example. For example, a broad pathogen defense for a plant may be achieved via combination of different pathogen resistances/tolerances, since genetic elements may exhibit additive effects among one another. For example, numerous resistance genes for this are known to the person skilled in the art as genetic elements. For example, US20160152999A1 discloses an RZ resistance gene against the disease Rhizomania. This disease is caused by the agent, "Beet Necrotic Yellow Vein Virus." Several disease resistances contained in one plant have synergistic effects upon one another. If a plant is infested for the first time by a pathogen, its immune system is normally weakened, and the epidermis as an outer barrier is often damaged, such that the probability of further infections is increased. An additional example of an agronomically advantageous property is cold tolerance or frost tolerance. Plants which exhibit this property may already be sown earlier in the year, or may remain in the field longer, which may lead to increased yields, for example. Here, the person skilled in the art may also resort to the prior art to find suitable genetic elements. Additional examples of agronomically advantageous properties are water usage efficiency, nitrogen usage efficiency, and yield. Genetic elements which may be used to confer such properties might be found in the prior art.

Furthermore, numerous modifications for pathogen defense are known to the person skilled in the art. In addition to the families of the R-genes that are often described, the Avr/R approach, the Avr gene complementation (WO 2013/127379), the autoactivation of an R-gene (WO 2006/128444), or the HIGS (host-induced gene silencing) approach (e.g., WO2013/050024) may be advantageously used. In particular, the autoactivation of an R-gene might be important to the present invention. For this, a nucleic acid is to be created that encodes an autoactivated resistance protein for generation of a resistance to pathogens in plants. This nucleic acid then has only a limited portion of an NBS-LRR resistance gene, such as the *wb*-R-gene, which extends downstream from the 5' end of the coding region of the NBS-LRR resistance gene to the beginning of the coding for the NBS domain of the NBS-LRR resistance gene.

In this context, a method is also encompassed which contains the step of the removal of that region of the nucleic acid according to the invention which encodes the N-terminal region and which begins with the p-loop in the NBS domain, and extends up to the end of the N-terminal region.

The resistance proteins that are encoded for by such shortened nucleic acids are generally autoactivated, in that these resistance proteins trigger an immune reaction in the plant, even in the absence of the associated pathogen, and thus increase the base immunity of the plant. Furthermore, such a shortened nucleic acid according to the invention, and the polypeptide that is encoded by this, are encompassed.

Furthermore, the invention also includes the use of the Cercospora resistance-conferring gene allele, identified with a method described above, for combination with one of the preceding modifications, or with a genetic element described in the preceding which may convey in a plant one or more agronomically advantageous properties.

In addition to relating to the plant according to the invention, the present invention also relates to seeds or descendants, or to an organ, a plant part, a tissue, or a cell thereof in the production of products that are typically produced from sustainable raw materials, such as foodstuffs and animal feed - preferably, sugar or syrup (molasses), wherein the molasses is also used for industrial applications, e.g., in alcohol production or as a growing medium for the production of biotechnological products, in the production of materials or substances for the chemical industry, e.g., refined chemicals, pharmaceuticals or precursors thereof, diagnostics, cosmetics, bioethanol, or biogas. An example of the use of sugar beet as a biogenic raw material in biogas plants is described in the application DE 10 2012 022 178 A1; see, for example, paragraph 10.

The following examples explain the invention, but without limiting the subject matter of the invention. Unless indicated otherwise, standard molecular biology methods have been used; see, for example, Sambrook et al., Molecular Cloning: A Laboratory Manual, 3rd ed., Cold Spring Harbor Laboratory Press, Cold Spring Harbor, NY, 2001, Fritsch *et al.,* Cold Spring Harbor Laboratory Press: 1989; Mayer et al., Immunochemical Methods in Cell and Molecular Biology, eds., Academic Press, London, 1987, and Weir et al., Handbook of Experimental Immunology, Volumes I-IV, Blackwell, eds., 1986.

Some of the most important sequences according to the invention are explained in detail in the following:
- SEQ ID No. 1: genomic DNA sequence of the Cercospora resistance-conferring gene from *Beta vulgaris subsp. maritima.*
- SEQ ID No. 2: cDNA sequence of the Cercospora resistance-conferring gene as it does not occur in nature.
- SEQ ID No. 3: amino acid sequence of the Cercospora resistance-conferring protein as it is encoded by SEQ ID No. 1 or SEQ ID No. 2.
- SEQ ID No. 4: genomic DNA sequence of the sensitive Variant of the Cercospora resistance-conferring gene
- SEQ ID No. 5: cDNA of the sensitive Variant of the Cercospora resistance-conferring gene
- SEQ ID No. 6: Amino acid sequence of the sensitive Variant of the Cercospora resistance-conferring gene
- SEQ ID No. 7: native promoter of the Cercospora resistance-conferring gene from Beta vulgaris subsp. maritima.
- SEQ ID No. 8: native terminator of the Cercospora resistance-conferring gene from Beta vulgaris subsp. maritima.
- SEQ ID No. 53: sequence of the locus from Beta vulgaris subsp. maritima containing the Cercospora resistance-conferring gene according to SEQ ID No. 1.

### EXAMPLES

### Example 1: Introduction of the resistance-conferring gene by means of CRISPR-mediated homologous recombination in Beta vulgaris subsp. vulgaris

### Design and selection of the crRNA:

Suitable crRNA's for Cpfl-mediated induction of double-strand breaks have been designed with the aid of CRISPR RGEN Tools (Park J., Bae S., and Kim J.-S. Cas-Designer: A web-based tool for choice of CRISPR-Cas9 target sites. Bioinformatics 31, 4014-4016 (2015); Bae S., Park J., and Kim J.-S. Cas-OFFinder: A fast and versatile algorithm that searches for potential off-target sites of Cas9 RNA-guided endonucleases. Bioinformatics 30, 1473-1475 (2014)). For this, suitable protospacers were sought within the genomic DNA sequences having a length of 500-1,300 bp which flank the 5'- and 3'-end of the Cercospora resistance gene from *Beta vulgaris* subsp. *maritima.* In order to ensure the functionality of the endonuclease Cpfl from *Lachnospiraceae bacterium* ND2006 (Lb), protospacers having a length of 24 nt were selected whose genomic binding sequence at the 5'-end was flanked by an essential protospacer adjacent motif (PAM) having the sequence 5'-TTTV-3' (V=G or C or A). Suitable protospacers were selected according to the predetermined quality criteria of the tool and reconciled as to potential off-targets with a reference genome of *B. vulgaris* subsp. *vulgaris.* For the continuing tests, crRNA's, exclusively, were selected that, in addition to the actual target sequence, have at most 15 identical bases with a functional PAM. Since the first 18 nt of the protospacer are essential to the detection and cutting of the target sequence, an unwanted cutting within other genomic sequences could be precluded in this way (Tang, X., L. G. Lowder, T. Zhang, A. A. Malzahn, X. Zheng, D. F. Voytas, Z. Zhong, Y. Chen, Q. Ren, Q. Li, E. R. Kirkland, Y. Zhang, and Y. Qi (2017), "A CRISPR-Cpf1 system for efficient genome editing and transcriptional repression in plants." Nat Plants 3: 17018). In this way, four potential crRNA's at the 5'-flanking region (5'crRNA#1-4) and three crRNA's at the 3'-flanking region (3'crRNA#1-3) of the resistance gene could be identified (see Table A).

**Table A: Selected target sequences within the 5'- and 3'-flanking DNA sequences of the resistance gene in B. vulgaris. The PAM is underlined.**

| Name of the crRNA | Genomic target sequence with 5'-flanking PAM (underlined) | Binding at the +/strand |
|---|---|---|
| 5'crRNA#1 | TTTATTTCGATTTCGATTCTTGGATTAT (SEQ ID No. 16) | - |
| 5'crRNA#2 | TTTC AACCCAGTATCCTTATCCGTCACT (SEQ ID No. 17) | - |
| 5'crRNA#3 | TTTATTTAAACATGATACGTATCATATT (SEQ ID No. 18) | + |
| 5'crRNA#4 | TTTAAACATGATACGTATCATATTGAGT (SEQ ID No. 19) | + |
| 3' crRNA# 1 | TTTGTGGGTGGGTGGTTTTCACGTGTGT (SEQ ID No. 20) | - |
| 3'crRNA#2 | TTTCCCCTCCCTTTGCCGCTGCGAAGTT | - |
| | (SEQ ID No. 21) | |
| 3' crRNA#3 | TTTCTTCTTCTTGCTTCCACCATAACAC (SEQ ID No. 22) | - |

Cloning of the genetic elements: For the cloning of the *cpf1*-expression cassette and the crRNA-expression cassette, first, a detection sequence of the restriction enzyme of BbsI that prevents cloning was removed from the target vector pZFNnptII via introduction of a point mutation (T to G). The mutagenesis was performed with a mutagenesis kit according to the specification of the manufacturer, using two mutagenesis primers (see Table B).

**Table B: Mutagenesis primer used to introduce a point mutation (T to G, underlined) to remove the BbsI detection sequence.**

| Name | Sequence 5'→3' |
|---|---|
| Mutagenesis primer 1 | TCAGTGCAGCCGTCGTCTGAAAACGACA (SEQ ID No. 23) |
| Mutagenesis primer 2 | TGTCGTTTTCAGACGACGGCTGCACTGA (SEQ ID No. 24) |

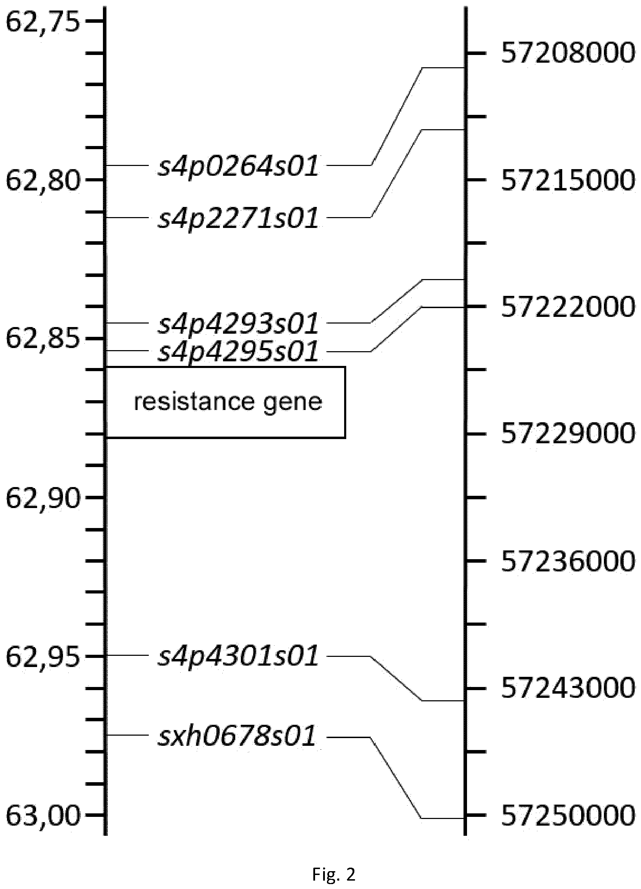

For the expression of the *Lbcpf1* gene in *B. vulgaris,* a DNA sequence codon-optimized for *A. thaliana,* with 5'-flanking PcUbi promoter sequence from *Petroselinum crispum* (SEQ ID No. 79) and a 3'-flanking 3A terminator sequence from *Pea sp.* as a DNA fragment, was synthetically produced. The restriction interface (HindIII) that is relevant to cloning within the *Lbcpfl-*coding sequence (CDS) [SEQ ID No. 78] were removed via the introduction of a silent mutation (base exchange, without modifying the amino acid sequence), in order to avoid an unintended cutting within the coding region. The codon optimization was performed with the aid of the GeneArt algorithm from Invitrogene/ThermoScientific. In order to enable the transport of the Cpfl in the cell nucleus, the coding sequence of the nucleus location signal (NLS) of the SV40 was integrated into the *cpf1* CDS at the 5'-end, and the NLS of the nucleoplasmin was integrated at the 3'-end. For the ligation in the binary target vector pZFNnptII (Fig. 2), the expression cassette was flanked by two HindIII restriction interfaces and subsequently ligated to pZFNnptII_LbCpf1. The successful insertion of the PcUbi::Cpf1::TPea expression cassette was verified by means of sequencing, wherein the binding regions of the primers used for the sequencing were situated both in the flanking vector regions and within the expression cassette (see Table C).

**Table C: Primer used for the sequencing of the PcUbi::Cpf1::TPea expression cassette integrated into pZFNnptII**

| Name | Sequence 5'→3' |
|---|---|
| pSeq_CRBM_F1 | SEQ ID No. 25 |
| pSeq_CRBM_R1 | SEQ ID No. 26 |
| pSeq_CRBM_F2 | SEQ ID No. 27 |
| pSeq_CRBM_R2 | SEQ ID No. 28 |
| pSeq_CRBM_F3 | SEQ ID No. 29 |
| pSeq_CRBM_R3 | SEQ ID No. 30 |
| pSeq_CRBM_F4 | SEQ ID No. 31 |
| pSeq_CRBM_R4 | (SEQ ID No. 32) |

After transcription into the plant cell, the crRNA's should be cut out via two flanking ribozymes. For this, the precursor crRNA was flanked by the coding sequences of a hammerhead ribozyme and an HDV ribozyme (Tang, X., L. G. Lowder, T. Zhang, A. A. Malzahn, X. Zheng, D. F. Voytas, Z. Zhong, Y. Chen, Q. Ren, Q. Li, E. R. Kirkland, Y. Zhang, and Y. Qi (2017), "A CRISPR-Cpf1 system for efficient genome editing and transcriptional repression in plants." Nat Plants 3: 17018).

For a perfect ligation of the individual protospacers at the coding sequence of the crRNA repeat, two *BbsI* detection sequences were integrated between crRNA repeat and HDV ribozyme, wherein the overhangs that were used for the cloning were adapted accordingly. In order to ensure an identical expression strength of the *cpf1* and the crRNA's, the crRNA ribozyme cassette was bounded, at the 5'-end, by the PcUbi promoter sequence and, at the 3'-end, by [a/the] 3A terminator sequence. For the later ligation in the target vector pZFNnptII_Cpfl, the crRNA expression cassette was flanked by two *PstI* interfaces and ordered as a synthetic DNA fragment. The protospacers were synthesized as complementary oligonucleotides and annealed according to a standard protocol. The 24-bp-long DNA fragment that was generated in this way was flanked by the 4-nt overhangs that are relevant to the ligation (see Table D).

**Table D: Sequence of oligonucleotides that were used for the generation of short 24-bp protospacers. The 4-nt overhangs that are used for the ligation are the respective four first nucleotides of each listed sequence.**

| Name of the crRNA | Sequence 5'→3' |
|---|---|
| 5'crRNA#1 | SEQ ID No. 33 |
| | SEQ ID No. 34 |
| 5'crRNA#2 | SEQ ID No. 35 |
| | SEQ ID No. 36 |
| 5'crRNA#3 | SEQ ID No. 37 |
| | SEQ ID No. 38 |
| 5'crRNA#4 | SEQ ID No. 39 |
| | SEQ ID No. 40 |
| 3'crRNA#1 | SEQ ID No. 41 |
| | SEQ ID No. 42 |
| 3'crRNA#2 | SEQ ID No. 43 |
| | SEQ ID No. 44 |
| 3'crRNA#3 | SEQ ID No. 45 |
| | SEQ ID No. 46 |

The efficiency of the four crRNA's was tested by means of agrobacteria-mediated gene transfer in leaves of *B. vulgaris.* The pZFNtDTnptII plasmid was co-transformed in order to check the transformation efficiency. The transformation of the leaf explant took place via vacuum infiltration according to a standard protocol. The fluorescence of the tDT was checked after six days by means of fluorescence microscopy, and leaf explants with heterogeneous fluorescence were discarded. Ten days after infiltration took place, the leaf explants were quick-frozen in liquid nitrogen, pestled, and the genomic DNA was isolated by means of the CTAB method (Clarke, Joseph D., "Cetyltrimethyl ammonium bromide (CTAB) DNA miniprep for plant DNA isolation." Cold Spring Harbor Protocols 2009.3 (2009): pdb-prot5177). The efficiency of the individual crRNA's was determined by an external service provider, using the frequency of the inserted editions (e.g., insertions, deletions, or base exchange) in comparison to unedited sequences in the genomic DNA, by means of NGS.

As a synthetic DNA construct, the most efficient crRNA's - 5'crRNA#3 and 3'crRNA#1 - with the previously described ribozymes, promoter, and terminator sequences, were ordered as reverse-oriented expression cassettes. The entire DNA construct was flanked by two PstI restriction interfaces for cloning in the target vector pZFNnptII_LbCpf1. After insertion of the crRNA's has taken place, the *LbCpf1* and crRNA expression cassettes were ligated from the vector pZFNnptII_LbCpf1_crRNA into the pUbitDTnptII vector via HindIII.

As a repair template which should be integrated into the genome of *B. vulgaris* via homologous recombination, the resistance gene expression cassette was flanked, at the 5'-end, by the 5'crRNA#3 and, at the 3'-end, by the 3'crRNA#1 binding sequence. This enabled the excision of the resistance gene expression cassette from the plasmid via Cpfl. The entire DNA template was synthesized as an 87,326-bp-long synthetic DNA fragment (SEQ ID No. 80) and used directly in the vector backbone for the transformation. The resistance gene plasmid and the pUbitDTnptII_LbCpf1_crRNA plasmid were introduced into *B. vulgaris* callus cultures with the aid of a gene cannon.

The transformation efficiency was determined using the transient tDT fluorescence, one day after the transformation, by means of fluorescence microscopy. The callus cultures were cultivated on shoot induction medium without selection pressure (without Kanamycin), and the regenerated shoots were subsequently checked for the site-directed integration of the resistance-conferring resistance gene cassette. For this, the genomic DNA was isolated by means of CTAB. The integration of the resistance-conferring gene was amplified by means of PCR using the primers pCRBM_F1 according to SEQ ID No. 47 and pCRBM_R1 according to SEQ ID No. 48 (see Table E), and the PCR products were subsequently sequenced with both primers. Shoots, in which the successful insertion of the expression cassette could be verified in this manner, were identified in the following analyses of the integration site of the resistance gene. In order to verify the insertion within the desired target sequence in the genome, the flanking regions of the resistance gene expression cassette were amplified by means of PCR. The binding of a primer here took place within the resistance gene DNA sequence; the binding of the second primer took place outside of the 5'- or 3'-flanking homologous region of the inserted expression cassette (see Table E). The amplified DNA sequences were sequenced using the same primers, and the integration at the desired location was confirmed in this way. In order to preclude the binding of the primers pCRBM_F1 (SEQ ID No. 47), pCRBM_R1 (SEQ ID No. 48), pCRBM_R2 (SEQ ID No. 50) and pCRBM_F3 (SEQ ID No. 51) in sequence-similar regions of the genome, all primer sequences were compared beforehand with the *B. vulgaris* genome. For the primer pCRBM_F3 (SEQ ID No. 51), it was not possible to select the nucleotide sequence such that a binding to the wild-type sequence could be precluded. Therefore, the 3'-flanking region was amplified in all shoots that tested positive for the resistance gene, and the site-specific insertion was verified exclusively via the subsequent sequencing. The generated PCR product thereby differs by 18 bp from the wild-type sequence. In order to enable the complete sequencing of the amplified sequences, the PCR products were additionally sequenced via a third primer with a binding location within the amplified sequence (pCRBM_S2, pCRBM_S3; see Table E). In order to preclude the nonspecific binding of the primers pCRBMF_F1 (SEQ ID No. 47), pCRBM_R1 (SEQ ID No. 48) and pCRBM_R2 (SEQ ID No. 50) within the wild-type genome, the nucleotide sequences were compared with an internal reference genome of *B. vulgaris.* The primers were additionally tested by means of PCR for the binding in genomic sequences of *B. vulgaris* wild-type plants.

In order to preclude the integration of the resistance gene in other regions of the genome, a targeted amplification of the target location was performed (Targeted Locus Amplification, TLA).

**Table E: Primer used to verify the insertion of the resistance gene expression cassette at the desired integration site.**

| Name | Sequence 5'→3' | Size of the PCR product | Binding |
|---|---|---|---|
| pCRBM_F1 | SEQ ID No. 47 | 450 bp | within the resistance gene expression cassette |
| pCRBM_R1 | SEQ ID No. 48 | | within the resistance gene expression cassette |
| pCRBM_F2 | SEQ ID No. 49 | 1,140 bp | up-strand of the 5'-flanking homologous region |
| pCRBM_R2 | SEQ ID No. 50 | | within the resistance gene promoter sequence |
| pCRBM_S2 | SEQ ID No. 66 | | |
| pCRBM_F3 | SEQ ID No. 51 | 1,280 | within the resistance gene terminator sequence |
| pCRBM_R3 | SEQ ID No. 52 | | down-strand of the 3'-flanking homologous region |
| pCRBM_S3 | SEQ ID No. 67 | | |

In addition to the verification and the successful insertion of the resistance gene expression cassette into the genome of *B. vulgaris,* the unwanted integration of plasmid DNA was also checked. For this, genomic DNA, in which the verification had already yielded a successful insertion of the resistance gene at the desired target site, was checked for the presence of plasmid DNA by means of PCR. Sequence regions within the *cpfl,* the crRNA ribozyme cassette, and the *tDT* were thereby amplified using the primers listed in Table F, and subsequently sequenced.

**Table F: Primers used to verify stably-integrated, plasmid-specific sequences in the genome of the regenerated B. vulgaris shoots**

| **Name** | **Sequence 5'→3'** | **Size of the PCR product** | **Binding** |
|---|---|---|---|
| pSeq_LbCpf1_F4 | SEQ ID No. 68 | 214 | Cpfl |
| pSeq_LbCpf1_R3 | SEQ ID No. 69 | | |
| pSeq_Ribozyme_F | SEQ ID No. 70 | 172 | crRNA ribozyme cassette |
| pSeq_Ribozyme_R | SEQ ID No. 71 | | |
| pSeq_tDT_F | SEQ ID No. 72 | 400 | *tDT* |
| pSeq_tDT_R | SEQ ID No. 73 | | |

### Example 2: Introduction of the resistance-conferring gene as a transgene by means of gene transformation in Beta vulgaris subsp. vulgaris

The transgenic approach to the production of *Cercospora-*resistant plants served not only for the alternative validation of the LRR gene as the resistance-conferring gene, but also as a means of producing transgenic resistance events that confers a novel *Cercospora* resistance or improve already existing *Cercospora* resistances.

The binary vector pZFN-nptII-LRR was generated by means of the following standard cloning procedures: Within the T-DNA of this vector, the cDNA of the resistance gene according to SEQ ID NO 2 was cloned together with its native promoter sequence. The T-DNA furthermore included the neomycin phosphotransferase II (*nptII*) gene, which confers resistance to a bandwidth of aminoglycoside antibiotics such as kanamycin or paromomycin. These antibiotic resistances were used for the selection of the transgenic plant cells and tissues. The *NOS* promoter and the *pAG7* terminator flanked the *nptII* gene. The backbone of the binary vector furthermore contained the *colE1* and the *pVS1* origin for the plasmid replication in *Escherichia coli* or *Agrobacterium tumefaciens.* The *aadA* gene confers streptomycin / spectinomycin resistance for bacteria selection. The pZFN-nptII-LRR plasmid was transformed in agrobacterium strain AGL-1 by means of standard procedure.

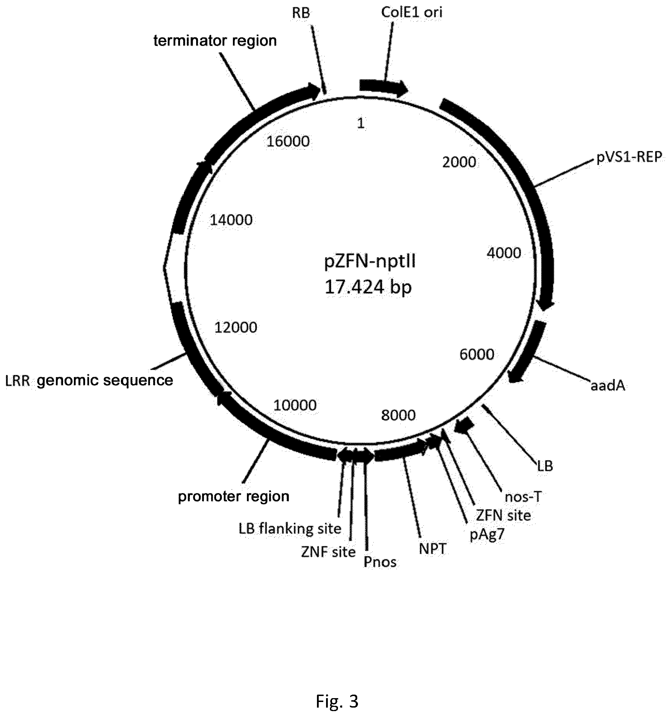

The transformation of the sugar beets took place according to Lindsey & Gallois (1990), "Transformation of sugarbeet (Beta vulgaris) by Agrobacterium tumefaciens." Journal of experimental botany 41.5, 529-536.). For this, "micropropagated shoots" of genotype 04E05B1DH5, which did not carry the resistance gene according to the invention, were used as starting material. Shoots were multiplied in the corresponding medium according to Lindsey & Gallois (1990). In order to induce as many meristems as possible, the "shoots" were transferred into a different medium (see Lindsey & Gallois (1990)) and incubated in darkness for several weeks at approximately 30 °C. Agrobacterium strain AGL-1 with vector pZFN-nptII-LRR (Fig. 3) was cultured in an additional medium (see Lindsey & Gallois (1990)), additionally provided with corresponding antibiotics for selection. Sections of meristematic tissue based upon the shoot to be treated were incubated with agrobacterium for several hours in an additional medium (see Lindsey & Gallois (1990)). Plant explants and agrobacteria were co-cultivated in darkness for at least 2 days in medium (see Lindsey & Gallois (1990)), and inoculated explants were subsequently incubated in darkness for approximately 2 weeks in an additional medium (see Lindsey & Gallois (1990)). The explants were thereupon further propagated in an additional medium (see Lindsey & Gallois (1990)) and sub-cultivated, in order to enable the selection of the transgenic tissue. In order to conclude the selection phase and to reduce the extent of chimera formation, green "shoots" were transferred to medium H, and all were propagated for 2 weeks. Leaf material was then extracted from the green, growing "shoots" and examined by means of PCr for the presence of the transgene. Suitable "shoots" were rooted in medium I and subsequently transferred to a greenhouse for production of T1 seed stock. Furthermore, leaf material derived from these "shoots" was used to analyse the expression of the transformed resistance gene.

### Analysis of the expression level

RNA was isolated from the leafes of the *in vitro* "shoots" and used within an qRT-PCR. The qRT-PCR was performed according to Weltmeier et al. 2011 (s. background of invention). Measured values were normalized against the reference gene PLT3_075_F09 (s. Weltmeier *et al.* 2011). The expression was determined by the use of the following primer sequences:

| **Sequence** | **Size [No. nucleotides]** | **Tₘ [C°]** | **Size of amplification product [No. nucleotides]** |
|---|---|---|---|
| SEQ ID No. 92 | 21 | 59,8 | 170 |
| SEQ ID No. 93 | 21 | 58,9 | 170 |

### Resistance test in sugar beet after inoculation with Cercospora beticola under greenhouse conditions:

A pure *Cercospora beticola* culture with a known high virulence was propagated on vegetable juice agar in Petri dishes (9 cm diameter) at 20 °C under near-ultraviolet (NUV) light. After 14 days, the surface of the agar on which the mold was grown was flooded with 10 ml of sterile water per Petri dish, and the conidia and mycelium fragments were carefully scraped off with the aid of a subject carrier. An inoculum density of 20,000 conidia/mycelium fragments per ml, plus 0.1% TWEEN 20, was used to inoculate the plants. At the point in time of the inoculation, the plants had been cultivated for 8 to 9 weeks under greenhouse conditions. The top side and underside of the leaves were treated with the inoculum. The plants were subsequently incubated for 5 to 7 days at 25 °C, 18h/6h light/dark, and approximately 100% humidity. The first Cercospora symptoms on the sugar beet leaves occurred after 12 to 14 hours. An assessment of the symptoms of the individual plants was performed regularly, with the assistance of the assessment of the rating scores shown in Tab. 1A. The results are shown below.

**Table G: Results of transgenic verification of the function of the resistance gene according to the invention in transformed plants; LSD = least significant difference; dpi = days post infection**

| **Test group** | **Number of Individuals** | **Average rating score** | | | | **Function** | **Expression level of the resistance gene according to the invention** |
|---|---|---|---|---|---|---|---|
| | | **8dpi** | **11dpi** | **13dpi** | **15dpi** | | |
| 1 | 57 | 1,60 | 3,82 | 5,24 | 6,46 | negative control | 0,0 |
| 2 | 38 | 1,28 | 3,07 | 4,42 | 6,04 | transgenic validation | 4,6 |
| 3 | 60 | 1,26 | 2,83 | 4,38 | 6,20 | transgenic validation | 12,5 |
| 4 | 45 | 1,50 | 3,40 | 4,62 | 6,18 | no validated expression *in vitro* | 0,0 |
| 5 | 60 | 1,44 | 3,62 | 5,29 | 6,38 | transgenic validation | 2,6 |
| 6 | 57 | 1,68 | 3,69 | 5,30 | 6,52 | transgenic validation | 3,3 |
| 7 | 66 | 1,58 | 3,84 | 5,43 | 6,57 | transgenic validation | 2,9 |
| 8 | 60 | 1,31 | 2,81 | 4,19 | 5,48 | transgenic validation | 11,3 |
| 9 | 72 | 1,25 | 3,07 | 4,61 | 5,97 | transgenic validation | 26,8 |
| 10 | 60 | 1,44 | 3,26 | 4,77 | 6,00 | transgenic validation | 10,7 |
| 11 | 57 | 1,60 | 3,83 | 5,48 | 6,64 | transgenic validation | 4,4 |
| 12 | 72 | 1,34 | 2,62 | 3,75 | 5,19 | resistant source plant | not determined |
| **mean value overall** | 58,66 | 1,44 | 3,32 | 4,8 | 6,13 | | |
| **LSD value** | - | 0,17 | 0,47 | 0,48 | 0,4 | | |

### Results of the transgenic validation of the resistance gene according to the invention (s. Table G)

Test group 1 represents a negative control. The genotype is the same as for test groups 2 to 11 but no transformation has taken place. Therefore, no expression could be detected. Test group 4 has been transformed but no expression could be detected. Test groups 2, 3 and 5 to 11 represent transformants which carry the resistance gene according to the invention only due to the transformation. Test group 12 represents a breeding line comprising the resistance gene according to the invention in a non-transgenic version. The rating scores of all lines has been established after inoculating the plant material with Cercospora beticola as described above. Test group 12 shows the highest resistance which is indicated by a final value of 5,19.

The transgenic lines showed a rating score according to the following table:

**Table H: Rating scores of the transgenic lines of table G**

| | **8dpi** | **11dpi** | **13dpi** | **15dpi** |
|---|---|---|---|---|
| **mean value transgenic validation** | 1,42 | 3,33 | 4,87 | 6,2 |
| **mean value transgenic validation for lines having an expression level >10** | 1,315 | 2,99 | 4,48 | 5,91 |

Table H shows that the rating scores for transgenic validation groups only. First the mean value for all transgenic test groups (excluding group 4) are shown. Below the rating scores only for those transgenic lines which showed an expression level of at least 10 (groups 3, 8, 9, 10; s. Table G) are given. Here, the final rating score is 5,91. That is a significantly higher resistance than the negative conrol of group 1 which has a rating score of only 6,46 (least significant difference = 0,4; s. Table G). The best transgenic test group (group 8) shows an even better resistance due to a rating score is 5,48 (s. Table G).

It is worth mentioning that the expression level of transgenic insertions may be influenced by the integration locus. As the expression level was measured *in vitro* the actual expression level under infection conditions could be higher - especially under when the resistance gene is under control of a pathogen inducible promoter.

### Statistical evaluation of the results of the transgenic validation

**Table I: statistic clustering**

| **test group** | **cluster 8dpi** | **cluster 11dpi** | **cluster 13dpi** | **cluster 15dpi** |
|---|---|---|---|---|
| 1 | ab | a | a | ab |
| 2 | e | de | bc | cd |
| 3 | e | ef | c | bcd |
| 4 | bc | bcd | bc | bcd |
| 5 | cd | abc | a | abc |
| 6 | a | ab | a | ab |
| 7 | ab | a | a | a |
| 8 | de | ef | c | e |
| 9 | e | de | bc | d |
| 10 | cd | cd | b | d |
| 11 | ab | a | a | a |
| 12 | de | f | d | e |

Table I shows a statistical evaluation of the rating scores contained in Table G. Each letter symbolizes the allocation to a statistical group. For example, it is evident that after the final evaluation (15dpi) test group 8 (transgenic verification) is in the same cluster as test group 12 (resistant source) but in a different cluster than test group 1 (negative control). According to this test group 8 is significantly different from test group 1 but not significantly different than test group 12.

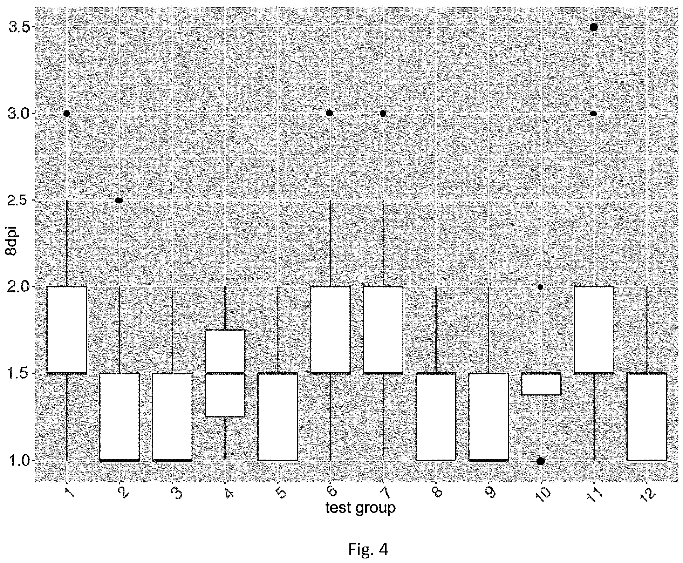

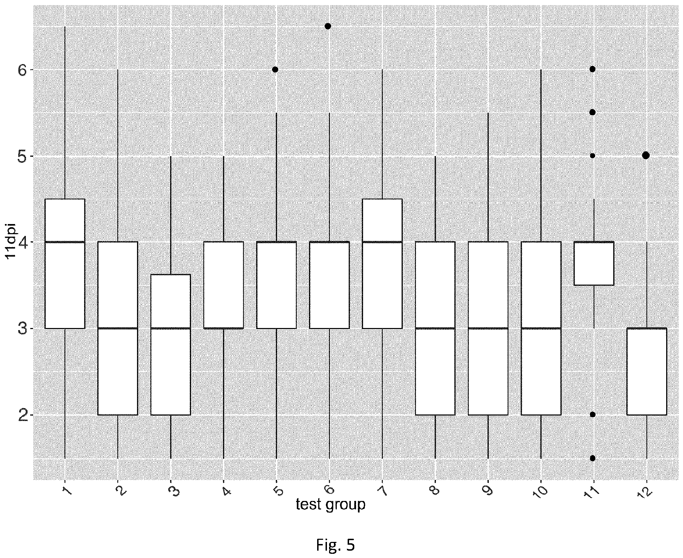

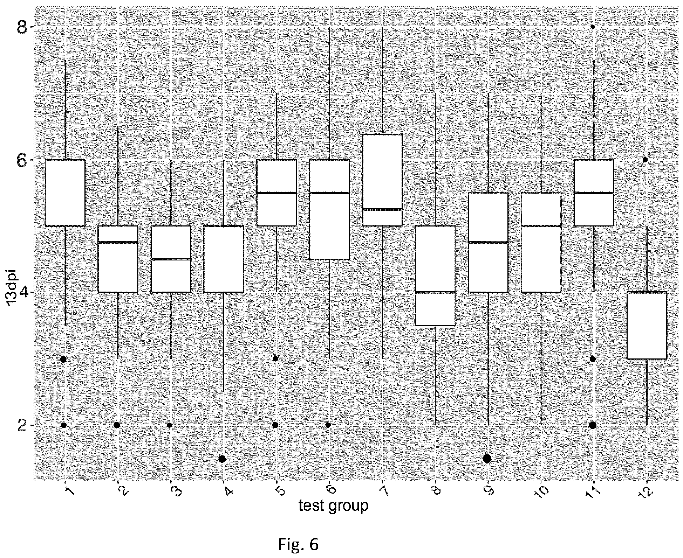

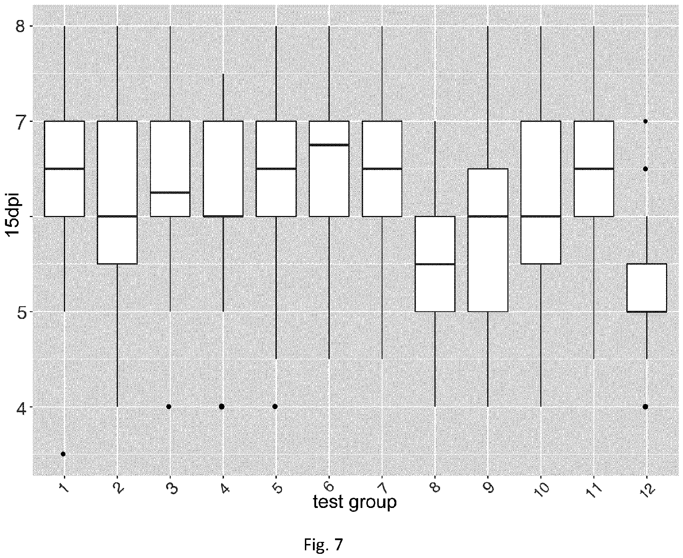

In addition, a box-plot analysis has been performed. The illustration of the the box-plots is available from Fig. 4 - 7.

### Example 3: Production of a resistant sugar beet plant according to the invention on the basis of genetic material accessed from Beta vulgaris supsp. maritima

The process described hereafter was based on pooling wild beet material to generate a Cercospora resistance genepool. The *Beta vulgaris* subsp. *maritima* accessions used as starting material for the breeding program are listed in the following table.

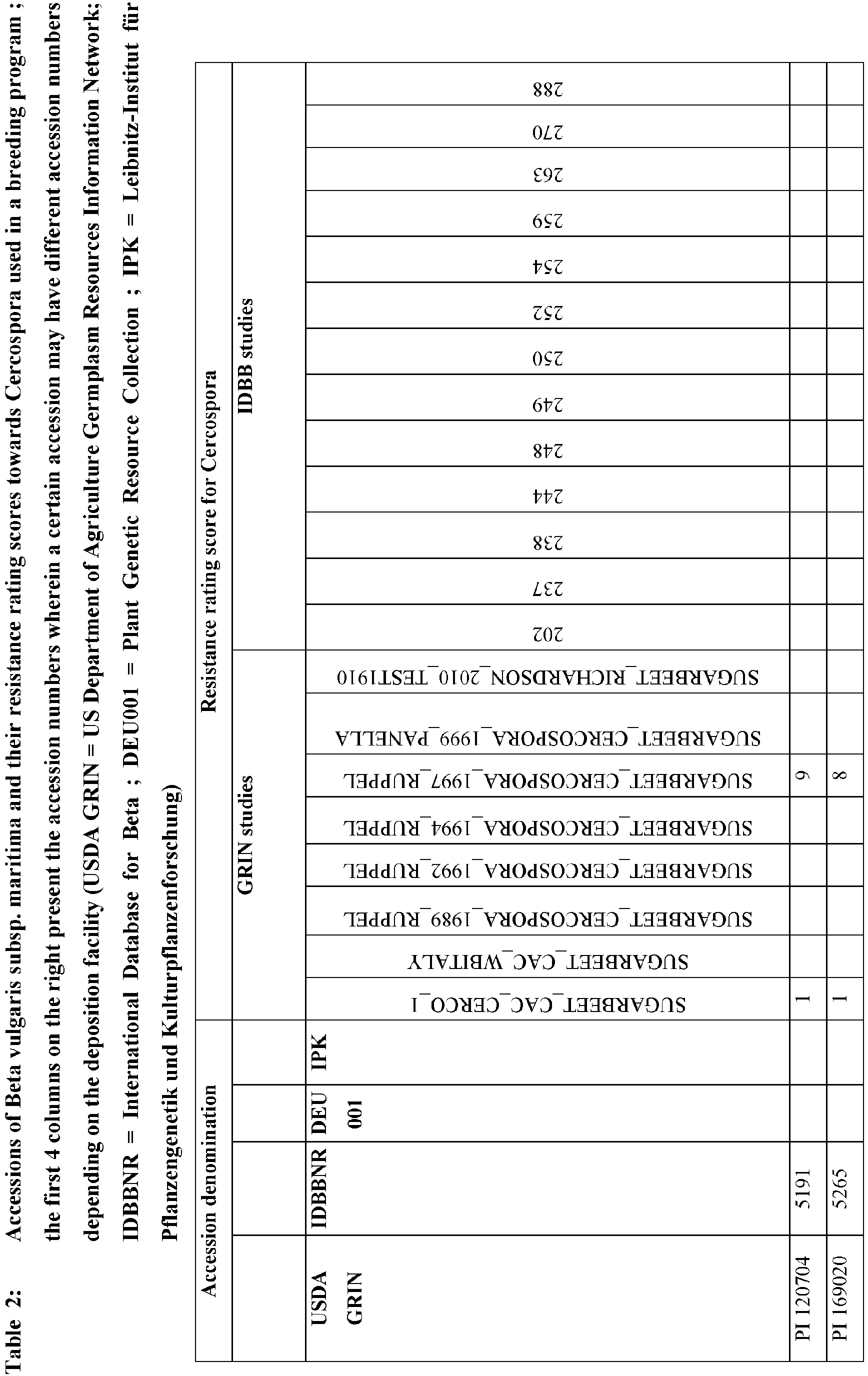

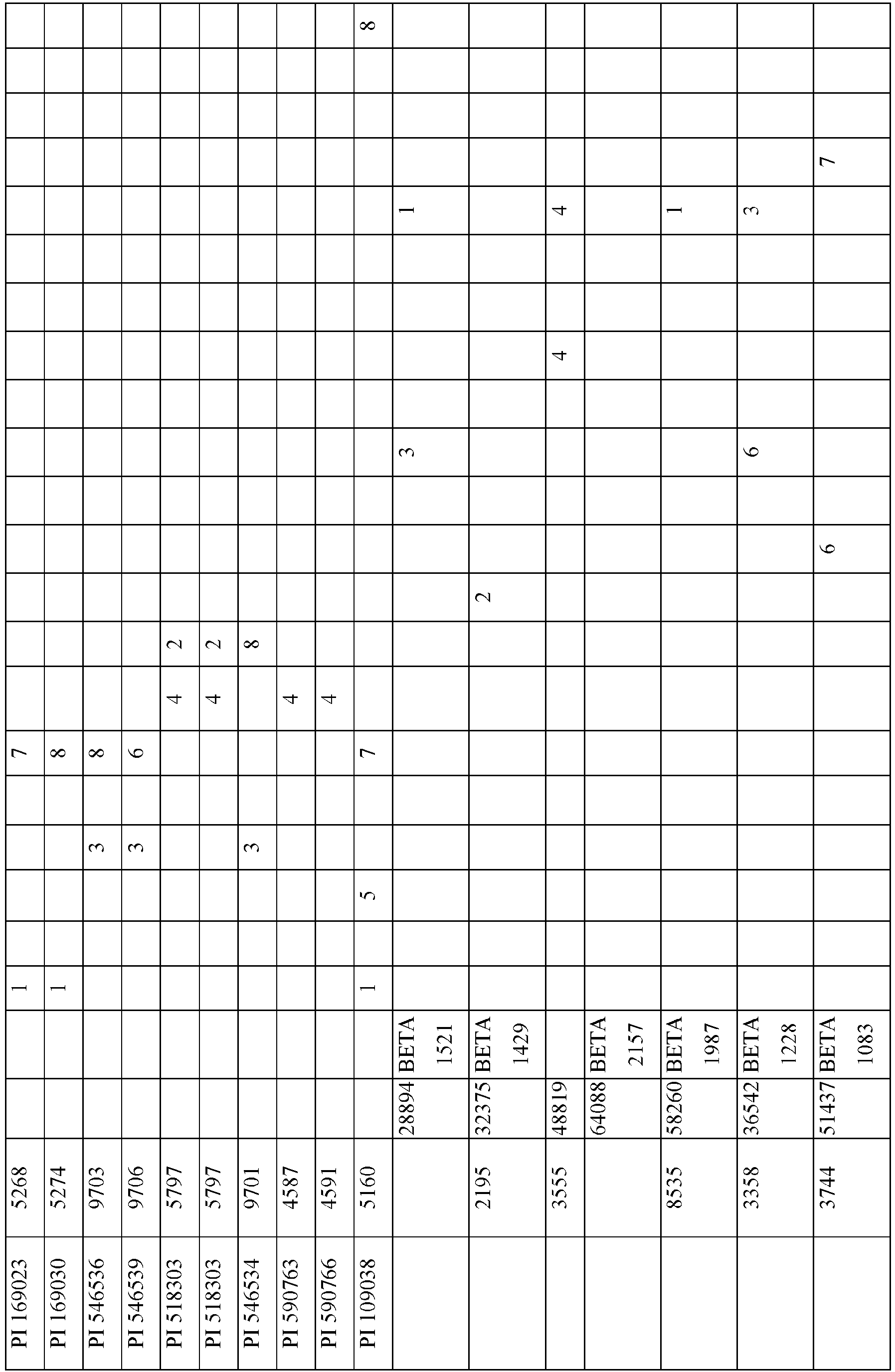

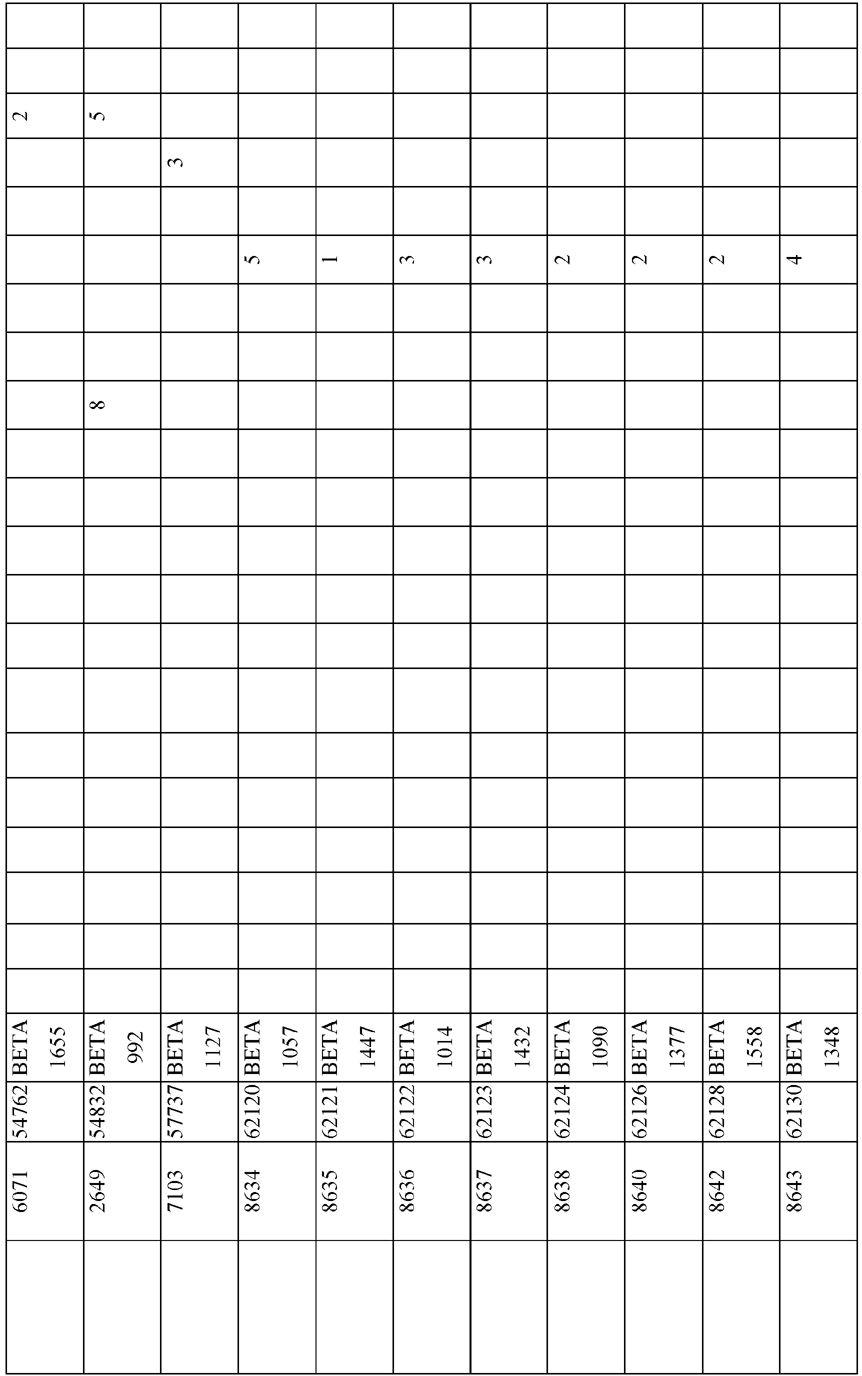

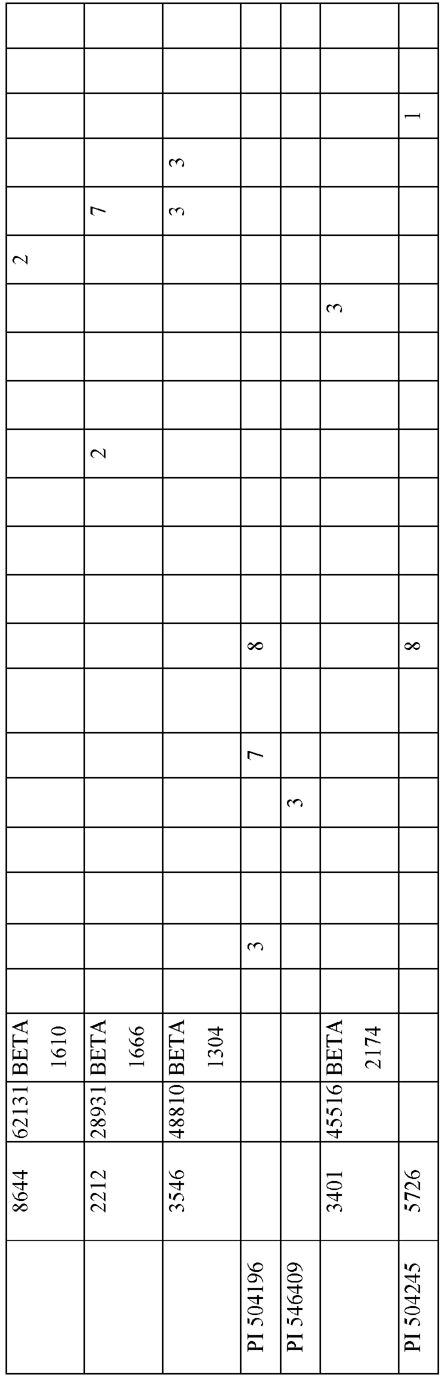

As it is apparent from Tab. 2 the accessed genetic material had shown previously a nonuniform resistance level against Cercospora and the degree of resistance varied throughout the different studies. For example, the accession "PI 120704" showed a score of 1 in one study and a score of 9 in another study. As this publicly available data seemed to be unreliable, seed material from the accessions has been planted and the resulting plants were screened phenotypically for Cercospora resistance. About 150 partial resistant plants have been selected. However, as the seen resistance in each plant could have been the result of a plenty of genes all having a small contribution the chances to identify a single gene suitable for establishing a resistance or increasing the resistance level in a measurable manner was limited. It was decided to cross the about 150 resistant plants among each other using an open pollination scenario. This approach also allowed for the generation of recombinations within the genetic material. Crossing and selection have been repeated for several generations to improve the resistance level. The best descendants have been cloned and prepared for a genetic mapping approach. The mapping of the herein described resistance was coupled with intensive phenotyping. With the aid of the setup of a population of over 4,000 dividing descendants and the development of special recombination screens, the target region was reduced, and thus ever further isolated, via analysis of informative recombinants (genotypical and phenotypical) in a series of resistance tests. This genetic mapping, as well as the creation of physical maps accompanied by WHG sequencing ("whole genome sequencing"), comparative BAC (Bac-by-Bac) sequencing, and bioinformatic analyses, led to the identification of three recombinant genotypes that confirmed the resistance gene (1 recombinant in the neighboring gene, on the one hand, and 2 recombinants in the neighboring gene, on the other). In light of particular requirements, the inventors placed the highly repetitive structure in the target region, which, among other things, contains *tandem repeats* with very high sequence homology, which made the marker development, and thus the identification of informative recombinants, enormously more difficult. The following steps were particularly decisive for the location of the genetic structure of the resistance gene:
- development of the markers s4p0264s01, s4p2271s01, sxh0678s01, s4p4293s01, s4p4295s01, s4p4301s01 (see Table 1B).
- Fine mapping coupled with intensive phenotyping. The phenotypes were verified with 90-180 descendants per plant in a greenhouse test, and with intensive statistical methods (for example, t-test, power analysis, etc.).
- BAC clone identification and sequencing from BAC pools of the resistant genotype.
- Sequence evaluation, as well as sequence and protein comparison between RR (i.e., resistant) and ss (i.e., sensitive) genotypes; an unambiguous assembly of the RR and ss sequence data was thereby not always possible, due to the sequence complexity.

In the framework of the breeding program, the *Beta vulgaris* subsp. *maritima* derived resistance was crossed with an elite sugar beet line. Several back crossings via marker assisted selection allowed to transfer the resistance gene in established sugar beet germplasm. Surprisingly, no undesired effects towards sugar yield etc. could be observed. Subsequently, a proof of concept for the resistance gene within sugar beet has been established via transformation and the generation of sugar beets which were transgenic for the resistance gene (s. above). After this successful proof of concept the generated sugar beet germplasm comprising the resistance gene could be used for the generation of a Cercospora resistant sugar beet variety.

### Example 4: Screening the starting accessions for the identified resistance gene

After the resistance gene has been identified the genetic source material (accessions according to Tab. 2) was screened by the help of markers to identify the accession which carried the resistance gene. The number of the analyzed plants per accession was dependent on the availability of seeds and is given in the table below.

**Tab. 3 Number of plants per accession analyzed for the presence of the identified resistance gene**

| **Accession denomination** | | | | **Plants [No.]** |
|---|---|---|---|---|
| **USDA GRIN** | **IDBBNR** | **DEU001** | **IPK** | |
| PI 120704 | 5191 | | | 40 |
| PI 169020 | 5265 | | | 40 |
| PI 169023 | 5268 | | | 18 |
| PI 169030 | 5274 | | | 18 |
| PI 546536 | 9703 | | | 50 |
| PI 546539 | 9706 | | | 34 |
| PI 518303 | 5797 | | | 2 |
| PI 518303 | 5797 | | | 16 |
| PI 546534 | 9701 | | | 40 |
| PI 590763 | 4587 | | | 12 |
| PI 590766 | 4591 | | | 28 |
| PI 109038 | 5160 | | | 23 |
| | | 28894 | BETA 1521 | 40 |
| | 2195 | 32375 | BETA 1429 | 40 |
| | 3555 | 48819 | | 14 |
| | | 64088 | BETA 2157 | 12 |
| | 8535 | 58260 | BETA 1987 | 40 |
| | 3358 | 36542 | BETA 1228 | 40 |
| | 3744 | 51437 | BETA 1083 | 31 |
| | 6071 | 54762 | BETA 1655 | 40 |
| | 2649 | 54832 | BETA 992 | 12 |
| | 7103 | 57737 | BETA 1127 | 40 |
| | 8634 | 62120 | BETA 1057 | 18 |
| | 8635 | 62121 | BETA 1447 | 40 |
| | 8636 | 62122 | BETA 1014 | 40 |
| | 8637 | 62123 | BETA 1432 | 37 |
| | 8638 | 62124 | BETA 1090 | 11 |
| | 8640 | 62126 | BETA 1377 | 40 |
| | 8642 | 62128 | BETA 1558 | 40 |
| | 8643 | 62130 | BETA 1348 | 40 |
| | 8644 | 62131 | BETA 1610 | 40 |
| | 2212 | 28931 | BETA 1666 | 36 |
| | 3546 | 48810 | BETA 1304 | 40 |
| PI 504196 | | | | 37 |
| PI 546409 | | | | |
| | 3401 | 45516 | BETA 2174 | 40 |
| PI 504245 | 5726 | | | 40 |

Each of the given plants of each accession has been screened by the use of 572 SNP markers which were located 5' as well as 3' to the resistance gene. Due to the large amount of markers a haplotype pattern could be derived. However, none of the accessions used as starting material showed the haplotype of the line CRBM which carried the identified resistance gene. Most similarities have been found to the accession 48819 (DEU001 denomination) / 3555 (IDBBNR) (s. Tables 2 and 3). The following table shows an extract of the entire marker analysis including positions 5' and 3' of the resistance gene.

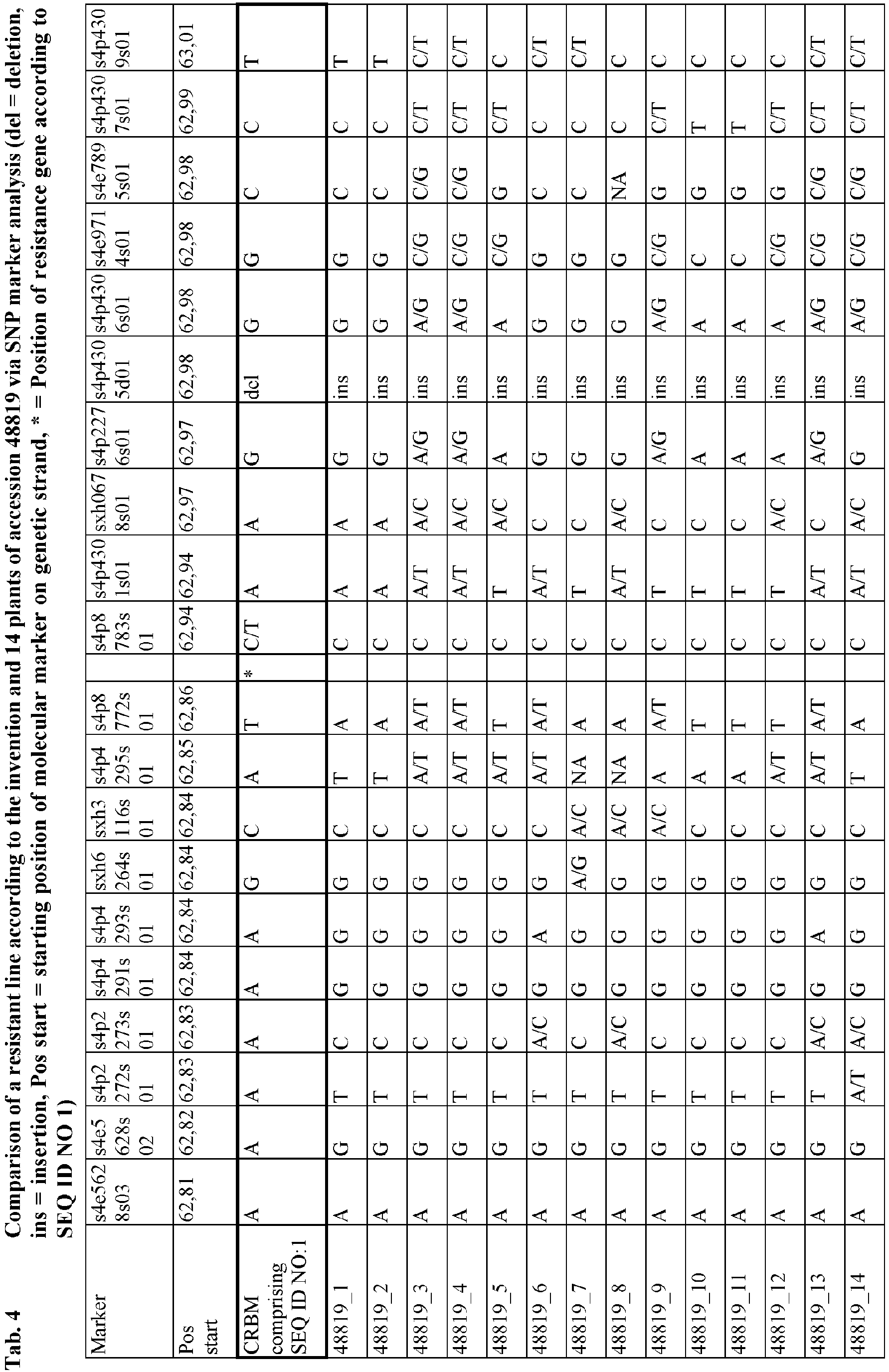

The results of the marker analysis (as exemplified by the data given in Tab. 4) show that the resistance gene according to the invention could not be traced back to one of the accessions according to Tab. 2. Even plants of the accession 48819 which shared the strongest marker overlap with the resistant line according to the invention had significant differences. Noticeable, was the detection of a deletion within the resistant line whereas accession 48819 showed a deletion at the same position. This could be an indication that a significant genetic restructuring at this locus took place during the generation of the resistance gene according to the invention. This assumption would also explain why it was not possible to trace back the resistance gene back to the starting material of the breeding program.

### Example 5: Creation of Cercospora resistant seed stock

The generated sugar beet germplasm comprising the resistance gene (outcome of Example 3) could be used for the generation of a Cercospora resistant sugar beet variety. For this purpose the gene was transmitted via crossing into a DH parent line which was crossed with a DH parent line originating from the other hybrid breeding pool. The result was a hybrid variety comprising the resistance towards Cercospora according to the present invention. The seeds of the variety were separated from each other (singularized), cleaned and polished. Afterwards, the seeds have been subjected to priming and pelleting as described in EP2002702A1. The resulting seed stock has been filed in a packaging made of cardboard which comprised an interlayer as vapor barrier. The resulting seed stock was suitable for sowing, growing, harvesting and subsequent industrial sugar production.

## Claims

1. A method for identifying a plant that is resistant or tolerant to *Cercospora,* **characterized in that** the method includes the following steps:
(i) detection of the presence or absence of a nucleotide sequence selected from the group consisting of:
(a) a nucleotide sequence that encodes a polypeptide having an amino acid sequence according to SEQ ID No. 3;
(b) a nucleotide sequence that comprises the DNA sequence according to SEQ ID No. 2;
(c) a nucleotide sequence that comprises a DNA sequence according to SEQ ID No. 1 or SEQ ID No. 53;
(d) a nucleotide sequence that hybridizes with a complementary sequence of the nucleotide sequence according to (a), (b), or (c) in 4 x SSC at 65 °C, and subsequent repeated washing in 0.1 x SSC at 65 °C for approximately 1 hour in total;
(e) a nucleotide sequence that encodes a polypeptide which has an amino acid sequence that is at least 90% identical to an amino acid sequence according to SEQ ID No. 3;
(f) a nucleotide sequence that is at least 90% identical to a DNA sequence according to SEQ ID No. 1 or SEQ ID No. 2;
(ii) detection of the presence or absence of the polypeptide which is encoded by the nucleotide sequence as defined in step (i) in the plant or a portion of the plant; and/or
(iii) detection of at least one marker locus in the nucleotide sequence as defined in step (1) or in a cosegregating region,
wherein the cosegregating region is a genomic region which cosegregates with the *Cercospora* resistance conferred by the polypeptide, or with the nucleotide sequence and wherein the cosegregating region comprises and is flanked by marker s4p1395s01 and s4p0421s01.

2. The method according to claim 1, **characterized in that** the method includes a further step of:
(iv) selection of the Cercospora-resistant plant.

3. The method according to claim 1 or 2 wherein the detection in step (i) or (iii) is based on at least one polymorphism or single nucleotide polymorphism.

4. The method according to claim 3 furthermore **characterized in that**
a) said at least one polymorphism or single nucleotide polymorphism is genetically linked to the nucleotide sequence or has a recombination frequency of 10% or less to the nucleotide sequence,
b) said at least one polymorphism or single nucleotide polymorphism is located within 2562kbp, 2300kbp, 2100kbp, 1900kbp, 1700kbp, 1500kbp, 1300kbp, 1100kbp, 900kbp, 700kbp, 500kbp, 300kbp, 100kbp, 50kbp, 25kbp, 10kbp, 5kbp or 1kbp or less of the nucleotide sequence,
c) said at least one polymorphism or single nucleotide polymorphism is detectable in the seeds deposited with the NCIMB, Aberdeen, UK under access number NCIMB 43646 or
d) said at least one polymorphism or single nucleotide polymorphism is part of the cosegregating region
wherein the nucleotide sequence is the nucleotide sequence defined in step (i) of claim 1 and wherein the cosegregating region is the cosegregating region defined in step (iii) of claim 1.

5. The method according to anyone of the preceding claims wherein step (i) and / or step (iii) furthermore includes the following steps which are concluded by the detection:
a) Provision of a plant, its tissue, a seed, or a cell thereof and
b) Extraction of DNA, preferably genomic DNA from the plant, its tissue, the seed or from the cell thereof.

6. The method according to anyone of the preceding claims involving the use of at least two oligonucleotides.

7. The method according to claim 6 wherein the oligonucleotides are suitable for use as primer in a PCR and are able to hybridize to a genomic interval which comprises and is flanked by marker s4p1395s01 and s4p0421s01.

8. The method according to anyone of the preceding claims wherein the plant is a plant of the genus *Beta* or a plant of the genus *Spinacia.*

9. The method according to anyone of the preceding claims involving a PCR in step (i) and / or (iii) wherein the PCR involves two allele-specific forward primers and wherein the detection step involves fluorescence resonant energy transfer and wherein the presence, absence or kind of the fluorescence is determined by a sensor.

10. The method according to claim 9 involving one common reverse primer.

11. A plant of the genus *Beta* or *Spinacia* comprising a nucleic acid molecule encoding a polypeptide that is able to confer resistance to *Cercospora* in a plant in which the polypeptide is expressed, **characterized in that** the nucleic acid molecule comprises a nucleotide sequence which is selected from the group consisting of:
(a) a nucleotide sequence that encodes a polypeptide having an amino acid sequence according to SEQ ID No. 3;
(b) a nucleotide sequence that comprises the DNA sequence according to SEQ ID No. 2;
(c) a nucleotide sequence that comprises a DNA sequence according to SEQ ID No. 1 or SEQ ID No. 53;
(d) a nucleotide sequence that hybridizes with a complementary sequence of the nucleotide sequence according to (a), (b), or (c) in 4 x SSC at 65 °C, and subsequent repeated washing in 0.1 x SSC at 65 °C for approximately 1 hour in total;
(e) a nucleotide sequence that encodes a polypeptide which has an amino acid sequence that is at least 90% identical to an amino acid sequence according to SEQ ID No. 3;
(f) a nucleotide sequence that is at least 90% identical to a DNA sequence according to SEQ ID No. 1 or SEQ ID No. 2;
wherein the plant of the genus *Beta* or *Spinacia* furthermore comprises an endogenous allele encoding an epsp synthase having at position 179 an amino acid different from proline.

12. The plant according to claim 11, further **characterized by** a feature or a combination of features selected from the list consisting of: the plant is a hybrid and / or a double haploid plant, the resistance against *Cercospora* is dominant, the nucleic acid molecule or nucleotide sequence is comprised as an introgression or is comprised homozygous and / or the plant has tolerance to glyphosate.

13. A storage organ or a leaf of the plant according to claim 11 or claim 12.

14. A pelleted seed of the plant according to claim 11 or claim 12, **characterized in that** the seed comprises the nucleic acid molecule and the endogenous allele.

15. The plant according to claim 11 or 12, the storage organ or leaf according to claim 13 or the pelleted seed according to claim 14, wherein the epsp synthase having at position 179 an amino acid different from proline, comprises an amino acid sequence selected from
i) the sequence of SEQ ID NO: 223
ii) the sequence i) having at position 179 an amino acid different from serine and proline, or
iii) a sequence having an identity of at least 90% to the sequence of i) or ii) over the entire length of the sequence.
